# EUROPEAN PATENT SPECIFICATION

(11) **EP 0 509 769 B1**
(45) Date of publication and mention of the grant of the patent: **11.09.1996**
(21) Application number: 92303358.3
(22) Date of filing: 15.04.1992
(51) Int. Cl.: C07K 5/08, C07K 5/06, A61K 38/55

(54) **Heterocyclic amides having HLE inhibiting activity**
HLE-inhibierende heterozyklische Amide
Amides hétérocycliques ayant une activité inhibante de HLE

(30) Priority: 18.04.1991 GB 9108358; 18.04.1991 GB 9108357; 12.03.1992 GB 9205392
(43) Date of publication of application: 21.10.1992
(73) Proprietor: ZENECA LIMITED, London W1Y 6LN (GB)
(72) Inventor: Bernstein, Peter Robert, ICI Americas Inc, Wilmington DE (US); Shaw, Andrew, ICI Americas Inc, Wilmington DE (US); Thomas, Royston Martin, ICI Americas Inc, Wilmington DE (US); Wolanin, Donald John, ICI Americas Inc, Wilmington DE (US); Warner, Peter, Macclesfield, Cheshire SK10 4TG (GB)
(74) Representative: Smith, Stephen Collyer

(56) References cited:
- EP-A- 0 189 305
- US-A- 4 474 778
- BIOCHEMISTRY. vol. 25, no. 13, 1 July 1986, EASTON, PA US pages 3760 - 3767 B IMPERIALI AND R H ABELES 'inhibition of serine proteases by peptidyl fluoromethyl ketones'

## Description

The present invention relates to certain heterocyclic amides, in particular, certain 1-pyridylacetamide compounds, which are inhibitors of human leukocyte elastase (HLE), also known as human neutrophil elastase (HNE), making them useful whenever such inhibition is desired, such as for research tools in pharmacological, diagnostic and related studies and in the treatment of diseases in mammals in which HLE is implicated. For example, HLE has been implicated in the pathogenesis of acute respiratory distress syndrome (ARDS), rheumatoid arthritis, atherosclerosis, pulmonary emphysema, and other inflammatory disorders, including airway inflammatory diseases characterized by increased and abnormal airway secretion such as chronic bronchitis and cystic fibrosis. Also, HLE has been implicated in certain vascular diseases and related conditions (and their therapy) in which neutrophil participation is involved or implicated, for example, in hemorrhage associated with acute non-lymphocytic leukemia, as well as in reperfusion injury associated with, for example, myocardial ischaemia and related conditions associated with coronary artery disease such as angina and infarction, cerebrovascular ischaemia such as transient ischaemic attack and stroke, peripheral occlusive vascular disease such as intermittent claudication and critical limb ischaemia, venous insufficiency such as venous hypertension, varicose veins and venous ulceration, as well as impaired reperfusion states such as those associated with reconstructive vascular surgery, thrombolysis and angioplasty. The invention also includes intermediates useful in the synthesis of these heterocyclic amides, processes for preparing the heterocyclic amides, pharmaceutical compositions containing such heterocyclic amides and methods for their use.

In U.S. Patent 4,910,190, of 20 March 1990, assigned to ICI Americas Inc., there is disclosed a series of peptidoyl trifluoromethane derivatives which are HLE inhibitors. Disclosed herein is a series of substituted 2-(2-oxo-1,2-dihydro-1-pyridyl)-N-[3,3,3-trifluoro-1-(lower alkyl)-2-oxopropyl]acetamide derivatives, which unexpectedly possess inhibitory properties against HLE, which provides the basis for the present invention.

According to the invention there is provided a Compound of the invention which is a compound of formula I (formula set out, together with other formulae referred to by Roman numerals, following the Examples) wherein:
R⁰ is (1-5C)alkyl;
R is hydrogen, formyl or trifluoroacetyl; or
R is an acyl group of formula A.X.CO- in which A.X-, taken together, is amino, RbRcN.O-, RaOCONH-, R¹SO₂NH-, RaOCO-, RbRcNCO- or RaCO-; or
R is an acyl group of formula A.X.CJ- in which
J is oxygen or sulfur;
X is a direct bond, imino, oxy or thio; and
A is (1-6C)alkyl, (3-6C)cycloalkyl, (3-6C)cycloalkyl-(1-3C)alkyl, aryl, aryl(1-3C)alkyl, heteroaryl or heteroaryl(1-3C)alkyl wherein an aryl or heteroaryl moiety may bear one to three halogeno, methyl or trifluoromethyl groups and further wherein the group A may bear one or more substituents selected from a group consisting of hydroxy, lower alkoxy, lower acyloxy, COORa, CONRbRc, COO(CH₂)₂NReRf, cyano, SO₂R¹, CONRdSO₂R¹, NReRf, NRgCOR², NRgCOOR², NRhCQNRiRj, NRkSO₂R³, SO₂NRlRm, SO₂NRnCOR⁴ and P(O)(ORa)₂ in which
Q is oxygen or sulfur;
Ra-Rn are independently hydrogen, benzyl or lower alkyl; or, independently, a group NRbRc, NReRf, NRiRj or NRlRm is a cyclic radical selected from a group consisting of 1-pyrrolidinyl, piperidino, morpholino and 1-piperazinyl which may bear a lower alkyl substituent at the 4-position; or, independently, a group NReRf is a cyclic radical selected from a group consisting of 2-pyrrolidinon-1-yl, succinimido, oxazolidin-2-on-3-yl, 2-benzoxazolinon-3-yl, phthalimido and cis-hexahydrophthalimido; and
R¹-R⁴ are independently trifluoromethyl, (1-6C)alkyl, (3-6C)cycloalkyl, aryl or heteroaryl in which the aryl or heteroaryl may bear one or more substituents selected from a group consisting of lower alkyl, hydroxy, lower alkoxy, halogeno and trifluoromethyl; or
A is tetrahydropyran-4-yl, 1-methylpiperid-4-yl, or 5-methyl-1,3-dioxacyclohex-5-ylmethyl;
Each of R⁵ and R⁶ is, independently, hydrogen or lower alkyl; or
One of R⁵ and R⁶ is hydrogen or methyl and the other of R⁵ and R⁶ is a radical of formula B.Y- in which
B is aryl or heteroaryl, which aryl or heteroaryl independently may bear one or more of the substituents defined for A or an aryl or heteroaryl moiety thereof;
Y is a direct bond, methylene, ethylene or trans-vinylene; and
provided that no aliphatic carbon is bonded to more than one nitrogen or oxygen, except as part of a cyclic ketal or where the nitrogen bears a carbonyl group; or,
for a compound of formula I which is acidic or basic, a pharmaceutically acceptable salt thereof.

In this specification, the following definitions are used, unless otherwise described: Halogeno is fluoro, chloro, bromo or iodo. Alkyl, alkoxy, etc. denote both straight and branched groups; but reference to an individual radical such "propyl" embraces only the straight chain ("normal") radical, a branched chain isomer such as "isopropyl" being specifically referred to. Lower alkyl and lower alkoxy refer to radicals containing one to about four carbon atoms. Lower acyloxy refers to a radical containing one to about five carbon atoms. Aryl denotes a phenyl radical or an ortho-fused bicyclic carbocyclic radical having about nine to ten ring atoms in which at least one ring is aromatic. Heteroaryl encompasses a radical attached via a ring carbon of a monocyclic aromatic ring containing five or six ring atoms consisting of carbon and one to four heteroatoms selected from the group consisting of oxygen, sulfur and nitrogen, as well as a radical of an ortho-fused bicyclic heterocycle of about eight to ten ring atoms derived therefrom, particularly a benz-derivative or one derived by fusing a propenylene, trimethylene or tetramethylene diradical thereto, as well as a stable N-oxide thereof.

It will be appreciated that, owing to the asymmetrically substituted carbon atom at the chiral center indicated by "*" in formula I, a compound of formula I may exist in, and be isolated in, optically active and racemic forms. If a compound of formula I contains an additional chiral element, such compound of formula I may exist in, and be isolated in, the form of a diastereomeric mixture or as a single diastereomer. It is to be understood that the present invention encompasses a compound of formula I as a mixture of diastereomers, as well as in the form of an individual diastereomer, and that the present invention encompasses a compound of formula I as a mixture of enantiomers, as well as in the form of an individual enantiomer. When R⁰ is isopropyl, a compound of formula I may be viewed as an alanyl trifluoromethane derivative. In general, a compound of formula I having the (S)-configuration at the chiral center indicated by "*", which corresponds to the L-alanyl configuration, is preferred as more potent than the corresponding (R)-isomer. Accordingly, it may be preferred to use the compound of formula I in a form which is characterized as containing, for example, at least 95%, 98% or 99% enantiomeric excess (ee) of the (S)-form. However, owing to the interconvertability of the (S)-isomer and the (R)-isomer by the facile epimerization of the chiral center indicated by "*" in formula I, it may be preferred to utilize a compound of formula I as a mixture of the (S)- and (R)-isomers at the center indicated by "*" in formula I.

As will be appreciated by those skilled in the art, a trifluoromethyl ketone of formula I can exist as a solvate, particularly a hydrate; and such a solvate of a compound of formula I is encompassed by the present invention.

A compound of formula I may exhibit polymorphism. The compound may form solvates in addition to a ketone solvate mentioned above. A compound may exist in more than one tautomeric form. It is to be understood, therefore, that the present invention encompasses any racemic or optically-active form, any polymorphic form, any tautomer or any solvate, or any mixture thereof, which form possesses inhibitory properties against HLE, it being well known in the art how to prepare optically active forms (for example, by resolution of the racemic form or by synthesis from optically-active starting materials) and how to determine the inhibitory properties against HLE by the standard tests described hereinafter.

It is preferred that the radicals R⁰, R, R⁵ and R⁶ not contain nor introduce an additional element of chirality into the molecule beyond the chiral center indicated by "*" in formula I.

Particular values are listed below for radicals, substituents and ranges for illustration only and they do not exclude other defined values or other values within defined ranges for the radicals and substituents.

A particular value for R⁰ is ethyl or isopropyl.

A particular value of (1-6C)alkyl is methyl, ethyl, propyl, isopropyl, butyl, isobutyl, t-butyl, pentyl, 3-methylbutyl, 1-ethylpropyl, hexyl or 4-methylpentyl. A particular value of (3-6C)cycloalkyl is cyclopropyl, cyclopentyl or cyclohexyl. A particular value for the (1-3C)alkyl portion of (3-6C)cycloalkyl-(1-3C)alkyl, aryl(1-3C)alkyl or heteroaryl(1-3C)alkyl is methylene, ethylene or trimethylene. A particular value for aryl is phenyl, indenyl or naphthyl. A particular value for heteroaryl is furyl, imidazolyl, tetrazolyl, pyridyl (or its N-oxide), thienyl, pyrimidinyl (or its N-oxide), indolyl or quinolinyl (or its N-oxide). A particular value for lower alkyl is methyl, ethyl, propyl, isopropyl, butyl, isobutyl or t-butyl. A particular value for lower acyloxy is acetoxy. A particular value for lower alkoxy is methoxy, ethoxy, propoxy, isoproxy or t-butoxy. A particular value for halogeno is bromo, chloro or fluoro.

A particular value for COORa is carboxy or methoxycarbonyl. A particular value for CONRbRc is carbamoyl or N,N-dimethylcarbamoyl. A particular value of CONRdSO₂R¹ is N-phenylsulfonylcarbamoyl. A particular value for A.X-, taken together, is tris(hydroxymethyl)methylamino, tris(acetoxymethyl)methylamino or 2,2-bis(hydroxymethyl)propoxy.

A more particular value for R⁰ is isopropyl. A more particular value for J is oxygen. A more particular value for X is a direct bond, imino or oxy. A more particular value for A is methyl, ethyl, phenyl, benzyl, phenethyl, pyridyl, thienyl, 5-tetrazolyl, thiazolyl, pyridylmethyl, thenyl, 5-tetrazolylmethyl, 2-(pyridyl)ethyl, 2-(thienyl)ethyl or 2-(thiazolyl)ethyl wherein the phenyl or heteroaryl group may bear one or two halogeno or methyl groups and further wherein the group A may bear a substituent selected from hydroxy, methoxy, t-butoxy, acetoxy, pivaloyloxy, carboxy, methoxycarbonyl, ethoxycarbonyl, carbamoyl, dimethylcarbamoyl, 2-(dimethylamino)ethoxycarbonyl, cyano, methylsulfonyl, phenylsulfonyl, N-methylsulfonylcarbamoyl, N-phenylsulfonylcarbamoyl, amino, dimethylamino, oxazolidin-2-on-3-yl, acetylamino, trifluoroacetylamino, ureido, methylsulfonyl, sulfamoyl, dimethylphosphoryl or diethylphosphoryl.

A particular value for R is, for example, hydrogen, trifluoroacetyl, hydroxyoxalyl, methoxycarbonyl, ethoxycarbonyl, isopropoxycarbonyl, 4-fluorophenoxycarbonyl, 4-bromophenoxycarbonyl, 4-methoxyphenoxycarbonyl, benzyloxycarbonyl, 4-fluorobenzyloxycarbonyl, 4-pyridylmethoxycarbonyl, 3-methylpyrid-4-ylmethoxycarbonyl, 2,6-dimethylpyrid-4-ylmethoxycarbonyl, 2-pyridylmethoxycarbonyl, 6-methylpyrid-2-ylmethoxycarbonyl, 2-dimethylaminoethoxycarbonyl, acetyl, carbamoylmethylaminocarbonyl or 4-(N-phenylsulfonylcarbamoyl)phenylacetyl; and a more particular value for R is, for example hydrogen, trifluoroacetyl, methoxycarbonyl, 4-bromophenoxycarbonyl, benzyloxycarbonyl, or 4-fluorobenzyloxycarbonyl.

A particular group of compounds of formula I is one in which R⁰ and R have any of the values defined above, R⁵ is benzyl, the phenyl ring of which may bear a 3-fluoro, 4-fluoro, 4-trifluoromethyl, 4-methoxycarbonyl, 3-acetoxy, 3-hydroxy, 3-pivaloyloxy, 4-hydroxy, 4-pivaloyloxy, 3-trifluoroacetylamino or 3-amino substituent, and R⁶ is hydrogen.

Another particular group of compounds of formula I is one in which R⁰ and R have any of the values defined above, R⁵ is hydrogen, and R⁶ is 2-furyl, 2-thienyl, 3-pyridyl or phenyl in which the phenyl may bear one or two halogeno, trifluoromethyl, methyl, hydroxy, methoxy, tert-butoxy, methoxycarbonyl or carboxy substituents; and, more particularly, R⁶ is phenyl, 4-fluorophenyl or 2-thienyl.

Specific compounds of formula I are described in the accompanying Examples. Of these, compounds of particular interest, along with their pharmaceutically acceptable salts, include those described in Examples 35, 49, 157, 159 and 178, based upon their activity in an in vivo test.

Pharmaceutically acceptable salts of an acidic compound of formula I include alkalai metal salts (especially lithium, sodium and potassium), alkaline earth metal salts (especially calcium and magnesium), aluminum salts and ammonium salts, as well as salts made from appropriate organic bases such as triethylamine, morpholine, piperidine and triethanol amine. Pharmaceutically acceptable salts of a basic compound of formula I include acid-addition salts such as those made with a strong acid, for example hydrochloric, sulfuric or phosphoric acid, which acid provides a pharmaceutically acceptable anion.

A compound of formula I may be made by processes which include processes known in the chemical art for the production of structurally analogous heterocyclic and peptidic compounds. Such processes and intermediates for the manufacture of a compound of formula I as defined above are provided as further features of the invention and are illustrated by the following procedures in which the meanings of generic radicals are as defined above:
(A) Oxidizing a corresponding alcohol of formula II. A convenient method is the use of excess dimethyl sulfoxide and a water soluble carbodimide, with dichloroacetic acid as a catalyst, in a inert solvent such as toluene at about room temperature, for example as described in Example 1. Other methods which may be useful include the use of alkaline aqueous potassium permanganate solution; the use of oxalyl chloride, dimethyl sulfoxide and a tertiary amine; the use of acetic anhydride and dimethyl sulfoxide; the use of chromium trioxide pyridine complex in methylene chloride; and the use of a hypervalent iodine reagent, such as a periodinane, for example 1,1,1-triacetoxy-2,1-benzoxidol-3(3H)-one with trifluoroacetic acid in dichloromethane.
(B) For a compound of formula I which bears a hydroxy substituent on an aryl or heteroaryl group, cleaving the alkyl ether or acyloxy ester of a corresponding compound of formula I which bears a lower alkoxy or lower acyloxy substituent on an aryl or heteroaryl group. Convenient methods include, for example, the cleavage of a methoxy group using boron tribromide and the cleavage of a t-butoxy group using trifluoroacetic acid as described in Examples 20 and 13, respectively, for an alkyl ether; and the acidic or alkaline hydrolysis of an acyloxy group.
(C) For a compound of formula I wherein R is not hydrogen, acylation of a corresponding amine of formula I wherein R is hydrogen. Convenient methods include those described below for acylation of an amine of formula IX, for example, when J is oxygen, the use of an activated carboxylic acid derivative, such as an acid halide, the use of a carboxylic acid and a coupling reagent, the use of an isocyanate for a compound wherein X is imino, and the use of a diactivated carbonic acid derivative, for example, carbonyldiimidazole, phosgene, diphosgene (trichloromethyl chloroformate) or triphosgene (bis(trichloromethyl) carbonate) with an alcohol of formula A.OH, a thiol of formula A.SH or an amine of formula A.NH₂ and a base, such as triethylamine or, when J is sulfur, the use of an activated thiocarboxylic acid derivative, such as a thioyl chloride or a lower alkyl ester of a dithioic acid, the use of a thioic acid and a coupling reagent, the use of an isothiocyanate for a compound wherein X is imino, and the use of a diactivated thiocarbonic acid derivative, for example, dimethyl trithiocarbonate, with an alcohol of formula A.OH, a thiol of formula A.SH or an amine of formula A.NH₂.
(D) For a compound of formula I which bears a group of formula COORa in which Ra is hydrogen (a carboxy group), decomposing the ester group of a corresponding ester made with a conveniently removed acid protecting group, for example a corresponding compound of formula I in which Ra is not hydrogen. The decomposition may be carried out using any one of the variety of procedures well known in organic chemistry, for example basic hydrolysis using lithium or sodium hydroxide, or by hydrogenolysis of a benzyl ester.
(E) For a compound of formula I which contains an amino N-H residue, removal of the nitrogen protecting group of a corresponding compound bearing a conventional nitrogen protecting group by using a conventional method, for example, removal of a benzyloxycarbonyl group by hydrogenolysis, removal of a benzyloxycarbonyl or tert-butoxycarbonyl group by treatment with a strong acid, for example with trifluoromethanesulfonic acid in an inert solvent such as dichloromethane, or basic hydrolysis of a trifluoroacetyl group.
(F) For a compound of formula I bearing a moiety of formula COORa, CONRbRc, COO(CH₂)₂NReRf or CONRdSO₂R¹, acylation of a corresponding compound of formula HORa, HNRbRc, HO(CH2)₂NReRf or HNRdSO₂R¹ with a corresponding acid of formula I bearing a moiety of formula COORa in which Ra is hydrogen, or an activated derivative thereof.
(G) For a compound of formula I bearing a lower acyloxy group or a group of formula NRgCOR², NRgCOOR², NRhCQNRiRj or NRkSO₂R³, acylation or sulfonation of a corresponding compound of formula I bearing a hydroxy group or an amino group of formula NHRg, NHRh or NHRk (i.e. an amino group of formula NReRf is which Re is hydrogen and Rf is Rg, Rh or Rk) with an activated derivative of a corresponding acid of formula HOCOR², HOCOOR², HOCQNRiRj (including an isocyanate or isothiocyanate) or HOSO₂R³, respectively, using a conventional method.
(H) For a compound of formula I which bears a heteroaryl N-oxide group, oxidation of a corresponding compound of formula I which bears a heteroaryl group using a conventional oxidant, such as for example dioxirane in acetone.
(I) For a compound of formula I which bears a primary amino group, reduction of a corresponding compound bearing a nitro group using a conventional reducing method, such as for example, hydrogenation over a palladium catalyst, or reduction with tin(II) chloride.

Whereafter, for any of the above procedures, when a pharmaceutically acceptable salt of an acidic or basic compound of formula I is required, it may be obtained by reacting the acidic or basic form of such a compound of formula I with a base or acid affording a physiologically acceptable counterion or by any other conventional procedure.

If not commercially available, the necessary starting materials for the above procedures may be made by procedures which are selected from standard techniques of heterocyclic chemistry and peptide chemistry, techniques which are analogous to the synthesis of known, structurally similar compounds, and techniques which are analogous to the above described procedures or the procedures described in the Examples. For uniformity and clarity, compounds herein are represented as the 2-pyridone, rather than the 2-hydroxypyridine, tautomers.

As will be clear to one skilled in the art, a variety of sequences is available for preparation of the starting materials. According to one of the available routes, a key intermediate pyrid-2-one-3-carboxylic acid of formula III may be prepared as shown in Scheme I (set out, together with other Schemes, following Examples) and as described in the Examples. In the Schemes, CBZ represents a benzyloxycarbonyl group.

In general, in a formal sense, a ketone of formula R⁵.CH₂.CO.R⁶ may be formylated, then cyclized with cyanoacetamide to afford a pyrid-2-one-3-carbonitrile of formula IV. Methods of preparation of a nitrile of formula IV and related pyridones are described in Example 1, part a (Cyclization Method A), Example 2, part a (Cyclization Method B) and Example 3, part a (Cyclization Method C). Where more than one product is possible from the cyclization reaction, the product selectivity may be controlled by the cyclization (and formylation) method chosen. For example, cyclization of phenylacetone by Cyclization Method A affords 6-methyl-5-phenylpyrid-2-one-3-carbonitrile (Example 60, part a); but cyclization of phenylacetone by Cyclization Method C affords 6-benzylpyrid-2-one-3-carbonitrile (Example 11, part a). Hydrolysis of the cyano group of a compound of formula IV, for example by heating with 48% hydrobromic acid in acetic acid (Hydrolysis Method A, Example 1, part b) or with sodium hydroxide solution in a pressure vessel (Hydrolysis Method B, Example 2, part b), affords a corresponding carboxy derivative of formula III. For a compound in which R⁶ is B.Y-and Y is ethylene or trans-vinylene, it may be preferred to proceed via an alternative route to an acid of formula III, for example as described in Examples 3 and 12. Thus, cyclization of a ketone of formula R⁵.CH₂.CO.CH₃ affords a 6-methyl pyridone derivative of formula IVa. Bis-metallation, followed by alkylation with a reagent of, for example, formula B.CH₂.Br affords a corresponding nitrile of formula IV in which Y is ethylene, for example as described in Example 3. Alternatively, for example as described in Example 12, bis-metallation of a 6-methyl pyridone of formula IVa, followed by condensation with an aldehyde of formula B.CHO, affords a pyrid-2-one-3-carbonitrile of formula IVb which may be converted by acid hydrolysis and dehydration into a corresponding pyride-2-one-3-carboxylic acid of formula III in which Y is trans-vinylene.

An acid of formula III may be converted into a corresponding isocyanate of formula VI by a conventional method, for example by using diphenylphosphoryl azide in an inert solvent, as described in Example 1, part c. Conveniently, the isocyanate is not isolated, but is converted into a benzyl urethane of formula VII as also is shown in Scheme I. It will be clear to one skilled in the art that, in general, treatment of an isocyanate of formula VI with a selected alcohol or amine of formula A.X.H in which X is oxy or imino will provide a corresponding product of formula VIIa in which X is oxy or imino, and that the product of formula VIIa may be carried forward to an alcohol of formula II using one of the routes outlined below. (An isocyanate of formula VI may undergo intramolecular cyclization to the oxygen at the pyridone 2-position, thereby forming a corresponding cyclic carbamate, which carbamate similarly may afford a corresponding compound of formula VII or VIIa.)

Elaboration of a substituted amino pyridone of formula VII (or VIIa) into a corresponding intermediate alcohol of formula II may be carried out as outlined in Scheme II. Alkylation of a compound of formula VII with an iodoacetamide derivative, for example as described in Example 1, part d, for a compound in which R⁰ is isopropyl, affords a 1-substituted pyridone of formula VIII, wherein Rp represents an alcohol protecting group, conveniently t-butyldimethylsilyl. (The corresponding 2-alkoxypyridine resulting from 0-alkylation is also obtained. When R⁶ is subject to hindered rotation, for example when R⁵ is methyl and R⁶ is phenyl, as in Example 9, or, for example, when R⁵ is hydrogen and R⁶ is 2-chlorophenyl as in Example 21, the ratio of N-alkylated product to 0-alkylated product is increased.) If an alcohol of formula II wherein R is benzyloxycarbonyl is required, it may be obtained directly from a compound of formula VIII by removal of the protecting group Rp, such as by the desilylation reaction described in Example 1, part e. When an alcohol of formula II with a different value of R is desired, the benzyloxycarbonyl group of a compound of formula VIII may be removed by a conventional method, for example by hydrogenolysis as described in Example 14, part a, to afford a corresponding 3-amino pyridone of formula IX. A 3-amino pyridone of formula IX may then be acylated by using a conventional method to afford a corresponding pyridone of formula X. Conventional acylation methods include the use of an acyl halide (for example as described in Example 14, part b, Acylation Method A), the use of a carboxylic acid and a coupling reagent (for example as described in Example 15, part a, Acylation Method B), the use of an isocyanate for a compound wherein X is imino (for example as described in Example 16, part a, Acylation Method C) and the use of triphosgene (bis(trichloromethyl) carbonate) with an alcohol of formula A.OH or an amine of formula A.NH₂ and a base, such as for example triethylamine (for example as described in Example 22, part e, Acylation Method D). Finally, removal of the alcohol protecting group Rp of a compound of formula X affords a corresponding alcohol of formula II. Instead of the deprotection method described in Example 1, part e, it may be preferred to use the alternative buffered deprotection as described in Example 19, part b.

A different route which obviates the need for an alcohol deprotection step is also shown in Scheme 11. Thus, a pyridone of formula VII (or VIIa) may be alkylated, for example with ethyl or t-butyl iodoacetate, to afford a corresponding ester of formula XI, wherein Rq is a conveniently removable acid protecting group, for example ethyl or t-butyl. Removal of the acid protecting group of an ester of formula XI by a conventional method, for example by base catalyzed hydrolysis or by acid catalyzed elimination as described in Example 3, part f, affords a corresponding acid of formula XII. An acid of formula XII may be coupled with 3-amino-1,1,1-trifluoro-4-methyl-2-pentanol, for example as described in Example 3, part g, to afford a corresponding alcohol of formula II.

An alternative route for the preparation of an intermediate acid of formula XII, beginning with a ketone of formula R⁵.CH₂.CO.R⁶ and involving a novel pyridone synthesis, which may be a preferred route, is described in Example 49, parts d.-i., for the conversion of acetophenone into 3-benzyloxycarbonylamino-2-oxo-6-phenyl-1,2-dihydro-1-pyridylacetic acid. The coupling to provide the corresponding alcohol of formula II, 2-(3-benzyloxycarbonylamino-2-oxo-6-phenyl-1,2-dihydro-l-pyridyl)-N-(3,3,3-trifluoro-2-hydroxy-1-isopropylpropyl)acetamide, is also described in Example 49.j.

Synthesis routes involving a cross coupling reaction to introduce a substituent R⁵ into intermediate compounds are outlined in Scheme III. These routes may be preferred when R⁵ has the value B.Y- and Y is methylene, ethylene or trans-vinylene. Thus, a pyridone of formula VII in which R⁵ is hydrogen may be converted into a corresponding 5-iodo pyridone of formula XXI by treatment with an iodinating agent, for example N-iodosuccinimide. An appropriate halide, for example a bromide of formula B.CH₂.Br, may be converted into a corresponding organozinc reagent, for example B.CH₂.Zn.Br, by treatment with zinc dust in tetrahydrofuran, and cross-coupled with an iodide of formula XXI using a palladium catalyst, such as dichloro[1,1'-bis(diphenylphosphino)ferrocene]palladium(II) to afford a corresponding compound of formula VII in which R⁵ is B.Y- and Y is methylene. A similar cross coupling utilizing a bromide of formula B.Y.Br in which Y is trans-vinylene may be useful to convert an iodide of formula XXI into a corresponding compound of formula VII in which R⁵ is B.Y- and Y is trans-vinylene. At a convenient point in a synthesis, a compound in which R⁵ is B.Y- and Y is trans-vinylene may be hydrogenated to afford a corresponding compound in which R⁵ is B.Y- and Y is ethylene.

Alternatively, an iodide of formula XXI may be alkylated to afford a corresponding iodide of formula XXII or XXIII which may be further cross coupled as described above to provide a corresponding compound of formula VIII or XI.

Alternative synthesis routes in which a 3-nitro pyridone serves as a precursor to a 3-amino pyridone are outlined in Scheme IV. They may be particularly useful when the 3-nitro derivative is readily available, such as when R⁵ and R⁶ are hydrogen. Alternatively, beginning with a ketone of formula R⁵.CH₂.CO.R⁶, the corresponding 3-nitropyridone may be prepared as described in Example 185, parts a. and b., beginning with 3-methoxycarbonylacetophenone. Direct reduction of the nitro group, followed by acylation of the amine obtained, provides a pyridone of formula VIIb, which may be converted into a corresponding intermediate of formula II using a route similar to one outlined in Scheme II for a compound of formula VII. Using a different order of steps, the 3-nitro pyridone may be alkylated first to provide an ester of formula XXIV. The ester of formula XXIV may be converted into the corresponding acid of formula XXV. The acid of formula XXV also may be obtained by allylation of the starting 3-nitro pyridone, followed by oxidative cleavage of the 1-allyl group using potassium permanganate. By coupling with the appropriate amino alcohol, an acid of formula XXV may be converted into a nitro alcohol of formula XXVI. A nitro alcohol of formula XXVI may be reduced to afford a corresponding 3-amino pyridone of formula XXVII. Acylation of a 3-amino pyridone of formula XXVII affords a corresponding intermediate alcohol of formula II. In addition, a nitro alcohol of formula XXVI may be oxidized to a corresponding nitro ketone of formula XXVIII. Reduction of the nitro group of a nitro ketone of formula XXVIII affords an intermediate amine of formula V. An analogous route from a nitro compound of formula XXIV involves first reducing the nitro group to afford a corresponding amino compound of formula XXIX. Acylation of a compound of formula XXIX affords a compound of formula XIb, which may be further converted into a corresponding compound of formula II using a similar method to that described in Scheme II for a compound of formula XI, that is, conversion into a corresponding acid of formula XIIb, followed by coupling with a requisite amino alcohol.

The trifluoromethyl amino alcohols required for the synthesis routes described above may be prepared by known routes. For example, 3-amino-1,1,1-trifluoro-4-methyl-2-pentanol (as its hydrochloride salt) conveniently may be obtained as described in U.S. Patent 4,910,190 in Example 4 (as a single diastereomer) or Example 6 (as a single enantiomer of a single diastereomer). If it is desired to carry out a chiral synthesis of a compound of formula I, using the single enantiomer in a substantially enantiomerically pure form and using methods and conditions which avoid epimerization at the center indicated by "*" in formula I provide such a synthesis.

It may be desired optionally to use a protecting group during all or portions of the above described processes; the protecting group then may be removed when the final compound or a required starting material is to be formed. As will be clear to one skilled in the art, the order of steps in the sequences leading to the starting materials and products of the invention may be altered if appropriate considerations relative to coupling methods, racemization, deprotection methods, etc. are followed.

The utility of a compound of the invention or a pharmaceutically acceptable salt thereof (hereinafter, collectively referred to as a "Compound") may be demonstrated by standard tests and clinical studies, including those described below.

### Inhibition Measurements:

The potency of a Compound to act as an inhibitor of human leukocyte elastase (HLE) on the low molecular weight peptide substrate methoxy-succinyl-alanyl-alanyl-prolyl-valine-p-nitroanilide is determined as described in U.S. Patent 4,910,190. The potency of an inhibitor is evaluated by obtaining a kinetic determination of the dissociation constant, Kᵢ, of the complex formed from the interaction of the inhibitor with HLE. If a Compound is found to be a "slow-binding" inhibitor of HLE, special methods of analysis to accurately determine Kᵢ values for the inhibition of HLE are carried out as described in U.S. Patent 4,910,190. Although some of the Compounds of the invention wherein R⁵ and R⁶ are both hydrogen exhibited Kᵢ values in the micromolar range, in general, the Kᵢ values for Compounds of the invention which were tested are generally on the order of 10⁻⁷ M or much less. For example a Kᵢ of 39 nM was measured for the Compound of the invention described in Example 167.

### Acute Lung Injury Model:

Animal models of emphysema include intratracheal (i.t.) administration of an elastolytic protease to cause a slowly progressive, destructive lesion of the lung. These lesions are normally evaluated a few weeks to a few months after the initial insult. However, these proteases also induce a lesion that is evident in the first few hours. The early lesion is first hemorrhagic, progresses to an inflammatory lesion by the end of the first 24 hours and resolves in the first week post insult. To take advantage of this early lesion, the following model was used.

Hamsters are first lightly anesthetized with Brevital. Phosphate buffered saline (PBS) pH 7.4, either alone or containing human leukocyte elastase (HLE), is then administered directly into the trachea. Twenty-four hours later the animals are killed and the lungs removed and carefully trimmed of extraneous tissue. Following determination of wet lung weight, the lungs are lavaged with PBS and total lavagable red and white cells recovered are determined. The values for wet lung weights, total lavagable red cells and total lavagable white cells are elevated in a dose-dependent manner following administration of HLE. Compounds that are effective elastase inhibitors can prevent or diminish the severity of the enzyme-induced lesion resulting in lower wet lung weight and reduced values for total lavagable cells, both red and white, relative to administration of HLE alone. Compounds can be evaluated by administering them intratracheally as solutions or suspensions in PBS, either with or at various times prior to the HLE challenge (400 µg), or by dosing them intravenously or orally as solutions at various times prior to the HLE challenge (100 µg) to determine their utility in preventing an HLE lesion. A solution of a Compound is conveniently prepared using 10% polyethylene glycol 400/PBS or 10% polyethylene glycol 400/water. For a Compound which is acidic or basic, base (e.g. sodium hydroxide solution) or acid (e.g. hydrochloric acid) may be added as indicated to achieve solution. Compounds of this invention produced statistically significant reductions in wet lung weight and total lavagable cells relative to HLE alone.

### Acute Hemorrhagic Assay:

This assay relies on monitoring only the amount of hemorrhage in the lung following intratracheal administration of human neutrophil elastase (HNE). Hemorrhage is quantified by disrupting erythrocytes recovered in lung lavage fluid and comparing that to dilutions of whole hamster blood. The screening protocol, similar to that described in Fletcher et al., American Review of Respiratory Disease (1990), 141, 672-677, is as follows. Compounds demonstrated to be HNE inhibitors in vitro are conveniently prepared for dosing as described above for the Acute Lung Injury Model. The compounds are then dosed by mouth to male Syrian hamsters at a fixed time, such as 30 or 90 min, prior to intratracheal administration of 50 µg/animal of HNE in 300 µL phosphate buffered saline (PBS) pH 7.4. Four hours after enzyme administration, the animals are killed with an overdose of pentobarbital sodium, the thorax opened and the lungs and trachea removed. The excised lungs are lavaged with three changes of 2 mL normal saline via a tracheal cannula. The recovered lavages are pooled, the volumes (about 5 mL) are recorded and the lavages stored at 4 °C until assayed. For calculation of the amount of blood in each sample, the thawed lavages and a sample of whole hamster blood are sonicated to disrupt erythrocytes and appropriately diluted into individual wells of a 96-well microtiter plate. The optical densities (OD) of the disrupted lavages and blood samples are determined at 405 nm. The (µL blood equivalents) / (mL lavage) are determined by comparing the OD of the test samples with the OD of the standard curve prepared from whole hamster blood. The total µL equivalents of blood recovered is determined by multiplying recovered lavage volume by the (µL blood equivalents) / (mL lavage) for each sample. Results are reported as % inhibition of hemorrhage with respect to PBS treated controls when the test compound is given at a specified dose and time prior to administration of HNE. For example, the Compound of the invention described as Example 167 provided statistically significant inhibition of hemorrhage when administered at a dose of 5 mg/kg 30 min prior to administration of HNE.

No overt toxicity was observed when Compounds of the invention were administered in the above in vivo tests.

It will be appreciated that the implications of a Compound's activity in the Acute Lung Injury Model or Acute Hemorrhagic Assay are not limited to emphysema, but, rather, that the test provides evidence of general in vivo inhibition of HLE.

Compounds of the present invention which were tested exhibited activity in at least one of the tests described above under Inhibition Measurement, Acute Lung Injury Model and Acute Hemorrhagic Assay. It should be noted that there was not always a direct correlation between the activities of the compounds measured as Kᵢ values in the Inhibition Measurement test and the reduced values for total lavagable cells and wet lung weights relative to the administration of HLE alone obtained in the Acute Lung Injury Model test or inhibition of hemorrhage in the Acute Hemorragic Assay.

According to a further feature of the invention, there is provided a pharmaceutical composition comprising a pharmaceutically effective amount of a Compound and a pharmaceutically acceptable diluent or carrier. As noted above, another feature of the invention is a method of using a Compound of the invention in the treatment of a disease or condition in a mammal, especially a human, in which HLE is implicated.

A Compound of the present invention may be administered to a warm-blooded animal, particularly a human, in need thereof for treatment of a disease in which HLE is implicated, in the form of a conventional pharmaceutical composition, for example as generally disclosed in U.S. Patent 4,910,190. The preferred mode of administration may be via a powdered or liquid aerosol. In a powdered aerosol, a Compound of the invention may be administered in the same manner as cromolyn sodium via a 'Spinhaler' (a trademark) turbo-inhaler device obtained from Fisons Corp. of Bedford, Massachusets at a rate of about 0.1 to 50 mg per capsule, 1 to 8 capsules being administered daily for an average human. Each capsule to be used in the turbo-inhaler contains the required amount of a Compound of the invention with the remainder of the 20 mg capsule being a pharmaceutically acceptable carrier such as lactose. In a liquid aerosol, a Compound of the invention may be administered using a nebulizer such as, for example, a 'Retec' (trademark) nebulizer, in which the solution is nebulized with compressed air. The aerosol may be administered, for example, at the rate of one to about eight times per day as follows: A nebulizer is filled with a solution of a Compound, for example 3.5 mL of solution containing 10 mg/mL; the solution in the nebulizer is nebulized with compressed air; and the patient breathes normally (tidal volume) for eight minutes with the nebulizer in his mouth.

Alternatively, the mode of adminstration may be oral or parenteral, including subcutaneous deposit by means of an osmotic pump. A compound of the invention may be conventionally formulated in an oral or parenteral dosage form by compounding about 10 to 250 mg per unit of dosage with conventional vehicle, excipient, binder, preservative, stabilizer, flavor or the like as called for by accepted pharmaceutical practice, e.g. as described in U.S. Patent 3,755,340. For parenteral administration, a 1 to 10 mL intravenous, intramuscular or subcutaneous injection would be given containing about 0.02 mg to 10 mg/kg of body weight of a compound of the invention 3 or 4 times daily. The injection would contain a compound of the invention in an aqueous isotonic sterile solution or suspension optionally with a preservative such as phenol or a solubilizing agent such as ethylenediaminetetraacetic acid (EDTA).

For parenteral administration or use in an aerosol, an 10 mg/mL aqueous formulation of an acidic Compound may be prepared, for example by dissolving the Compound (10 mg), dibasic sodium phosphate heptahydrate, USP (11.97 mg), monobasic sodium phosphate, USP (0.74 mg), sodium chloride, USP (4.50 mg) and sufficient 1 N sodium hydroxide solution or 0.05 M monobasic sodium phosphate solution to achieve pH 7.0-7.5 in sufficient water for injection, USP to afford 1.0 mL (1.01 g), followed by aseptic filtration, and sterile storage using standard procedures.

In general, a Compound of the invention will be administered to humans at a daily dose in the range of, for example, 5 to 100 mg of the Compound by aerosol or 50 to 1000 mg intravenously, or a combination of the two. However, it readily will be understood that it may be necessary to vary the dose of the Compound adminstered in accordance with well known medical practice to take account of the nature and severity of the disease under treatment, concurrent therapy, and the age, weight and sex of the patient receiving treatment. It similarly will be understood that generally equivalent amounts of a pharmaceutically acceptable salt of the Compound also may be used. Protocols for the administration of the HLE inhibitor and evaluation of the patients are described in the European Patent Applications with Publication Numbers 458535, 458536, 458537, and 463811 for the treatment or prevention of cystic fibrosis, ARDS, bronchitis, and hemorrhage associated with acute non-lymphocytic leukemia or its therapy, respectively; and a Compound of the invention may be used similarly for the treatment of those diseases and conditions either alone or in combination with another therapeutic agent customarily indicated for the treatment of the particular condition. For therapeutic or prophylactic treatment of a vascular disease or related condition in a mammal in which neutrophils are involved or implicated, a Compound of the invention may conveniently be administered by a parenteral route, either alone or simultaneously or sequentially with other therapeutically active agents customarily administered for the condition.

The invention will now be illustrated by the following non-limiting examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous sodium sulfate; evaporation of solvent was carried out using a rotary evaporator under reduced pressure (600-4000 pascals; 4.5-30 mm Hg) with a bath temperature of up to 60 °C;
(iii) chromatography means 'flash chromatography' (method of Still) carried out on Merck Kieselgel (Art 9385 from E. Merck, Darmstadt, Germany); thin layer chomatography (TLC) was carried out on 0.25 mm silica gel GHLF plates (Art 21521 from Analtech, Newark, DE, USA);
(iv) in general, the course of reactions was followed by TLC and reaction times are given for illustration only;
(v) melting points are uncorrected and (dec) indicates decomposition; the melting points given are those obtained for the materials prepared as described; polymorphism may result in isolation of materials with different melting points in some preparations;
(vi) final products had satisfactory nuclear magnetic resonance (NMR) spectra; and, where examined, were substantially pure by HPLC;
(vii) yields are given for illustration only and are not necessarily those which may be obtained by diligent process development; preparations were repeated if more material was required;
(viii) when given, NMR data is in the form of delta values for major diagnostic protons, given in parts per million (ppm) relative to tetramethylsilane (TMS) as an internal standard, determined at 250 MHz using DMSO-d₆ as solvent; conventional abbreviations for signal shape are used; for AB spectra the directly observed shifts are reported;
(ix) chemical symbols have their usual meanings; SI units and symbols are used;
(x) reduced pressures are given as absolute pressures in pascals (Pa); elevated pressures are given as gauge pressures in bars;
(xi) solvent ratios are given in volume:volume (v/v) terms;
(xii) mass spectra (MS) were run with an electron energy of 70 electron volts in the chemical ionizaton mode using a direct exposure probe; where indicated ionization was effected by electron impact (EI) or fast atom bombardment (FAB); generally, only peaks which indicate the parent mass are reported; and
(xiii) when high pressure liquid chromatography (HPLC) data is reported, t_{R} (retention time) is given in min, FR (flow rate) is given in ml/min, Col A is a Zorbax (trademark) ODS analytical column (4.6 mm x 25 cm) and Col B is a Phenomenex (trademark) Zorbax (trademark) C-8 analytical column (4.6 mm x 35 cm); solvent system A is water:acetonitrile:tetrahydrofuran:trifluoroacetic acid (55:35:15:0.1).

### EXAMPLE 1

### 2-(3-Benzyloxycarbonylamino-2-oxo-6-phenyl-1,2-dihydro-1-pyridyl)-N-(3,3,3-trifluoro-1-isopropyl-2-oxopropyl)acetamide.

A solution of 2-(3-benzyloxycarbonylamino-2-oxo-6-phenyl-1,2-dihydro-1-pyridyl)-N-(3,3,3-trifluoro-2-hydroxy-1-isopropylpropyl)acetamide (0.78 g) in dry dimethyl sulfoxide (4 mL) and toluene (4 mL) was treated with 1-(3-dimethylaminopropyl)-3-ethyl-carbodiimide hydrochloride (2.81 g) and dichloroacetic acid (0.48 mL). After overnight stirring, the reaction mixture was diluted with ethyl acetate (100 mL); washed successively with 10% hydrochloric acid (twice), saturated aqueous sodium bicarbonate (twice) and brine; dried; and evaporated to give an off-white solid (0.75 g). Flash chromatography, eluting with dichloromethane:ethyl acetate (95:5), and drying overnight in a vacuum oven gave the title compound as an off-white solid (0.53 g); TLC: R_{f}=0.29, dichloromethane:ethyl acetate (20:1); HPLC: t_{R}=7.19, FR=3.0, column A, solvent system A; NMR: 0.83 (d,3, J=6.8), 0.89 (d,3, J=6.7), 2.10-2.21 (m,1), 4.46 (d,1, J=16), 4.54 (d,1, J=16), 4.63 (t,1, J=6), 5.19 (s,2), 6.23 (d,1, J=7.6), 7.33-7.49 (m,10), 7.92 (d,1, J=7.6), 8.55 (s,1), 8.74 (d,1, J=7.0); MS: m/z=530(M+1).

| Analysis for C₂₇H₂₆F₃N₃O₅: | | | |
|---|---|---|---|
| Calculated: | C, 61.24; | H, 4.95; | N, 7.94 |
| Found: | C, 61.17; | H, 5.06; | N, 7.91 |

The intermediate 2-(3-benzyloxycarbonylamino-2-oxo-6-phenyl-1,2-dihydro-1-pyridyl)-N-(3,3,3-trifluoro-2-hydroxy-1-isopropylpropyl)acetamide may be prepared as follows:

### a. 6-Phenylpyrid-2-one-3-carbonitrile.

A solution of acetophenone (30.6 g) and N,N-dimethylformamide dimethyl acetal (94% by weight; 100 g) in acetonitrile was heated under reflux overnight. The reaction mixture was cooled, evaporated and dried overnight under vacuum to give a yellow semi-solid (36.68 g). To a solution of this material in dimethylformamide (400 mL) were added cyanoacetamide (19.4 g) and sodium methoxide (27.18 g). The red-orange solution was heated for 5 h at 100 °C, cooled, diluted with water (1200 mL), and acidified to about pH 5 with 10% hydrochloric acid. The yellow precipitate was filtered and dried overnight under vacuum at 40 °C to yield 6-phenylpyrid-2-one-3-carbonitrile(35.28 g); TLC: R_{f}=0.21, dichloromethane:methanol (50:1); 300 MHz NMR: 6.77 (broad d,1, J=6), 7.55 (m,3), 7.80 (m,2), 8.20 (dd,1, J=0.8, J=7); MS: m/z=197(M+1).

| Analysis for C₁₂H₈N₂O: | | | |
|---|---|---|---|
| Calculated: | C, 73.46; | H, 4.11; | N, 14.28 |
| Found: | C, 73.09; | H, 4.11; | N, 14.24 |

The method of 2-pyridone preparation described above in Example 1.a. is denoted herein as Cyclization Method A.

### b. 6-Phenylpyrid-2-one-3-carboxylic acid.

A suspension of 6-phenylpyrid-2-one-3-carbonitrile (8.54 g) in a mixture of glacial acetic acid (100 mL) and 48% aqueous hydrobromic acid (50 mL) was heated under reflux overnight, cooled, diluted with water (50 mL) and brought to about pH 5 with 10% NaOH. The precipitate was filtered, washed succesively with 10% hydrochloric acid and water, and dried overnight under vacuum to afford 6-phenylpyrid-2-one-3-carboxylic acid (8.49 g); 300 MHz NMR: 7.02 (d,1, J=7.6), 7.58 (m,3), 7.85 (m,2), 8.42 (d,1, J=7.6); MS: m/z=216(M+1).

The method of hydrolysis of the 3-cyano group to a 3-carboxy group described above in Example 1.b. is denoted herein as Hydrolysis Method A.

### c. 3-Benzyloxycarbonylamino-6-phenylpyrid-2-one.

To 6-phenylpyrid-2-one-3-carboxylic acid (10 g), suspended in dry dioxane (260 mL), triethylamine (7.8 mL) was added dropwise rapidly with stirring followed by diphenylphosphoryl azide (11.1 mL). The suspension was heated under reflux for 4 h using a preheated 120 °C oil bath. Benzyl alcohol (9.58 mL) was then added and the mixture stirred under reflux overnight. The suspension was cooled and evaporated. The resulting semisolid was suspended in water (600 mL) and filtered. The solid filter cake was washed with 10% hydrochloric acid (twice), saturated aqueous sodium bicarbonate and water. Trituration with chloroform turned the solid into an oil which was solidified with ether prior to suctioning off the solvents. Recrystallization from chloroform (600 mL) and methanol (450 mL) yielded a yellow solid (2.42 g), which was triturated with ether and dried under vacuum at 40 °C. The mother liquor was evaporated, and the residue was recrystallized from chloroform. The total amount of 3-benzyloxycarbonylamino-6-phenylpyrid-2-one isolated was 4.75 g; TLC: R_{f}=0.76, chloroform:methanol (20:1), R_{f}=0.32, dichloromethane:ethyl acetate (10:1); 300 MHz NMR: 5.19 (s,2), 6.61 (d,1, J=7.6), 7.33-7.49 (m,8), 7.70 (m,2), 7.93 (d,1, J=7.6), 8.47 (s,1).

### d. 2-(3-Benzyloxycarbonylamino-2-oxo-6-phenyl-1,2-dihydro-1-pyridyl)-N-(2-tert-butyldimethylsilyloxy-3,3,3-trifluoro-1-isopropylpropyl)-acetamide.

3-Benzyloxycarbonylamino-6-phenylpyrid-2-one (1.7 g) was added to a suspension of NaH (0.14 g) in dry dimethylformamide (50 mL). After 15 min stirring, the turbid, orange solution was treated with N-(2-tert-butyldimethylsilyloxy-3,3,3-trifluoro-1-isopropylpropyl)-2-iodoacetamide (2.65 g) and the mixture was stirred overnight. The mixture was diluted with 10% hydrochloric acid (125 mL) and extracted with ethyl acetate (2 times 150 mL). The combined extracts were washed with 10% hydrochloric acid and water (twice), dried and evaporated to an orange-brown glass. Purification by flash chromatography (gradient elution, 1%-4% ethyl acetate in dichloromethane) and overnight drying under vacuum yielded the N-alkylated product 2-(3-benzyloxycarbonylamino-2-oxo-6-phenyl-1,2-dihydro-1-pyridyl)-N-(2-tert-butyldimethylsilyloxy-3,3,3-trifluoro-1-isopropylpropyl)acetamide (0.97 g, 28%); TLC: R_{f}=0.35, dichloromethane:ethyl acetate (97:3); 300 MHz NMR: 0.08 (s,3), 0.10 (s, 3), 0.81 (d,3, J=6.6), 0.86 (s,9), 0.93 (d,3, J=6.6), 1.67-1.78 (m,1), 3.81 (t,1, J=10), 4.22-4.40 (m,2), 4.64 (broad d,1, J=15), 5.19 (s,2), 6.22 (d,1, J=7.6), 7.33-7.48 (m,10), 7.63 (d,1, J=9.9), 7.92 (d,1, J=7.6), 8.53 (s,1). Also obtained was the O-alkylated product 2-(3-benzyloxycarbonylamino-6-phenylpyrid-2-yloxy)-N-(2-tert-butyldimethylsilyloxy-3,3,3-trifluoro-1-isopropylpropyl)acetamide (2.11 g, 62%); TLC: R_{f}=0.61, dichloromethane:ethyl acetate (97:3), 300 MHz NMR: 0.01 (s,3), 0.07 (s,3), 0.75 (s,9), 0.77 (d,3, J=6.6), 0.89 (d,3, J=6.6), 1.67-1.81 (m,1), 3.84 (t,1, J=10), 4.26 (m,1), 4.81 (d,1, J=15), 5.14 (d,1, j=15), 5.20 (s,2), 7.35-7.48 (m,9), 7.62 (d,1, J=8), 7.99 (dd,2, J=1.6, J=8.4), 8.12 (d,1, J=8), 9.31 (s,1); MS: m/z=646(M+1). (A mixed fraction (0.25 g, 7%) was also obtained).

### e. 2-(3-Benzyloxycarbonylamino-2-oxo-6-phenyl-1,2-dihydro-1-pyridyl)-N-(3,3,3-trifluoro-2-hydroxy-1-isopropylpropyl)acetamide.

A solution 2-(3-benzyloxycarbonylamino-2-oxo-6-phenyl-1,2-dihydro-1-pyridyl)-N-(2-tert-butyldimethylsilyloxy-3,3,3-trifluoro-1-isopropylpropyl)acetamide (0.96 g) in dry tetrahydrofuran (8 mL) was treated with tetrabutylammonium fluoride (1M in tetrahydrofuran; 1.62 mL) and the mixture was stirred for 4.5 h. The reaction mixture was diluted with ethyl acetate (75 mL), washed with water (twice) and brine, dried and evaporated to yield a yellow foam. Purification by flash chromatography, eluting with ethyl acetate:chloroform (first 5:95, then 10:90), and overnight drying under vacuum gave 2-(3-benzyloxycarbonylamino-2-oxo-6-phenyl-1,2-dihydro-1-pyridyl)-N-(3,3,3-trifluoro-2-hydroxy-l-isopropylpropyl)acetamide as a white solid (0.793 g); TLC: R_{f}=0.19, chloroform:ethyl acetate (20:1); 300 MHz NMR: 0.81 (d,3, J=6.7), 0.88 (d,3, J=6.7), 1.67-1.84 (m,1), 3.82 (t,1, J=8.8), 4.0-4.17 (m,1), 4.34 (d,1, j=15), 4.50 (m,1), 5.18 (s,2), 6.20 (d,1, J=7.7), 6.49 (d,1, J=7), 7.31-7.47 (m,10), 7.86 (d,1, J=9.7), 7.91 (d,1 J=7.7), 8.53 (s,1); MS: m/z=532(M+1).

The iodide used in step d. was prepared as follows:

### f. 2-Chloro-N-(3,3,3-trifluoro-2-hydroxy-1-isopropylpropyl)acetamide.

To a solution of 3-amino-1,1,1-trifluoro-4-methyl-2-pentanol hydrochloride (20 g) in distilled tetrahydrofuran (480 mL) under nitrogen was added 4-methylmorpholine (21.8 mL) resulting in a white precipitate. A solution of chloroacetyl chloride (7.7 mL) in distilled tetrahydrofuran (40 mL) was added dropwise over 1 hour, and the mixture was stirred overnight. The mixture was diluted with ethyl acetate and filtered to remove undissolved solids. The filtrate was washed with 10% hydrochloric acid, water, saturated aqueous sodium bicarbonate and brine. The solid that had been filtered was dissolved in water, the aqueous phase was extracted with ethyl acetate (twice), and the extracts were washed as the first extract had been. The organic phases were combined, dried, and evaporated to give 2-chloro-N-(3,3,3-trifluoro-2-hydroxy-1-isopropyl-propyl)acetamide as an oil (23.8 g); TLC: R_{f}=0.70, dichloromethane:methanol (95:5); MS: m/z=248(M+1 for ³⁵Cl).

### g. N-(2-tert-Butyldimethylsilyloxy-3,3,3-trifluoro-1-isopropylpropyl) -2-chloroacetamide.

2-Chloro-N-(3,3,3-trifluoro-2-hydroxy-1-isopropylpropyl)acetamide prepared as above (23.79 g) and used directly was dissolved in dichloromethane (96 mL), and 2,6-lutidine (22.5 mL) was added. tert-Butyldimethylsilyl triflate (33 mL) was added rapidly dropwise. The reaction exothermed vigorously, producing white smoke. Cooling is advised. The mixture was stirred overnight; diluted with ethyl acetate (500 mL); and washed with 10% hydrochloric acid (twice), saturated aqueous sodium bicarbonate, and brine. The ethyl acetate solution was adsorbed onto silica gel (120 mL) by evaporation, and chromatographed, eluting with hexane:ethyl acetate (gradient, 100:0, 93:7, 85:15 and 80:20), to afford N-(2-tert-butyldimethylsilyloxy-3,3,3-trifluoro-1-isopropylpropyl)-2-chloroacetamide as a white solid (20.49 g); TLC: R_{f}=0.19, hexane:ethyl acetate (9:1);
MS: m/z=362(M+1, ³⁵Cl).

### h. N-(2-tert-Butyldimethylsilyloxy-3,3,3-trifluoro-1-isopropylpropyl)-2-iodoacetamide.

N-(2-tert-Butyldimethylsilyloxy-3,3,3-trifluoro-1-isopropylpropyl)-2-chloroacetamide (15.56 g) was added to a solution of NaI (19.3 g) in acetone (130 mL). The mixture was stirred overnight and the yellow reaction mixture was diluted with water (180 mL). The resulting precipitate was filtered; washed with water and saturated aqueous sodium thiosulfate; and dried under vacuum at 40 °C overnight. After spectral data indicated the presence of starting material, the product was subjected to another iteration of the above reaction conditions. The subsequent work-up was identical except that no sodium thiosulfate wash was performed. Purification by chromatography, eluting with hexane:ethyl acetate (gradient, 80:20 and 50:50), and drying under vacuum afforded N-(2-tert-butyldimethylsilyloxy-3,3,3-trifluoro-1-isopropylpropyl)-2-iodoacetamide (17.91 g); TLC: R_{f}=0.30, hexane:ethyl acetate (9:1); MS: m/z=454(M+1).

### EXAMPLE 2

### 2-[3-Benzyloxycarbonylamino-6-(4-methoxyphenyl)-2-oxo-1,2-dihydro-1-pyridyl]-N-(3,3,3-trifluoro-l-isopropyl-2-oxopropyl)acetamide.

Using a similar procedure to that described in Example 1, using dichloromethane:ethyl acetate:ethanol (100:3:0.5) for elution in the chromatography, 2-[3-benzyloxycarbonylamino-6-(4-methoxyphenyl)-2-oxo-1,2-dihydro-1-pyridyl]-N-(3,3,3-trifluoro-2-hydroxy-1-isopropylpropyl)acetamide was oxidized to afford the title compound; TLC: R_{f}=0.16, dichloromethane:ethyl acetate:ethanol (92:3:5); HPLC: t_{R}=6.82, FR=2, column A, acetonitrile:water (1:1); MS: m/z=560(M+1).

| Analysis for C₂₈H₂₈F₃N₃O₆: | | | |
|---|---|---|---|
| Calculated: | C, 60.13; | H, 5.04; | N, 7.51 |
| Found: | C, 59.85; | H, 5.19; | N, 7.13 |

The intermediate 2-[3-benzyloxycarbonylamino-6-(4-methoxyphenyl)-2-oxo-1,2-dihydro-1-pyridyl]-N-(3,3,3-trifluoro-2-hydroxyl-1-isopropylpropyl)acetamide may be prepared as follows:

### a. 6-(4-Methoxyphenyl)pyrid-2-one-3-carbonitrile.

To a stirred mixture of dry tetrahydrofuran (60 mL), dry ether (60 mL), and sodium methoxide (8.95 g) a solution of ethyl formate (11.55 g), 4′-methoxyacetophenone (10.06 g), and dry tetrahydrofuran (90 mL) was added dropwise over 35 min. After addition was complete, the addition funnel was replaced with a reflux condenser and the mixture was warmed to 40 °C (bath) for 3 h. The condenser was then replaced with a distillation head the mixture was heated to 90 °C (bath); and most of the solvents were distilled from the mixture. The remaining solvents were evaporated and the cooled residue was dissolved in water (240 mL) and acetic acid was added to adjust the solution to pH 9. Cyanoacetamide (10.06 g) was added to the solution and the mixture was heated at 90 °C for 18 h. After the mixture was cooled, the solvent was decanted from the gummy residue. The residue was sequentially triturated with aqueous 10% hydrochloric acid and chloroform to give a solid which was filtered, washed with ether, and dried under vacuum to give 6-(4-methoxyphenyl)pyrid-2-one-3-carbonitrile (2.97 g); TLC: R_{f}=0.50, methanol:dichloromethane (4:96); MS: m/z=227(M+1).

The method of 2-pyridone preparation described above in Example 2.a. is denoted herein as Cyclization Method B.

### b. 6-(4-Methoxyphenyl)pyrid-2-one-3-carboxylic acid.

A suspension of 6-(4-methoxyphenyl)pyrid-2-one-3-carbonitrile (2.95 g) in 50% NaOH (w/w; 13 mL) was heated at 140 °C overnight in a sealed pressure vessel. The reaction mixture was cooled to room temperature and allowed to stand for 24 h. The reaction mixture was diluted with water (150 mL) and acidified to pH 1 with concentrated hydrochloric acid. The solids were collected by filtration, washed with water (three times), and dried in a vacuum oven at 40 °C overnight. The dried 6-(4-methoxyphenyl)pyrid-2-one-3-carboxylic acid (3.22 g) required no further purification; MS: m/z=246(M+1).

The method of hydrolysis of the 3-cyano group to a 3-carboxy group described above in Example 2.b. is denoted herein as Hydrolysis Method B.

### c. 3-Benzyloxycarbonylamino-6-(4-methoxyphenyl)pyrid-2-one.

Using a similar procedure to that described in Example 1.c., 6-(4-methoxyphenyl)pyrid-2-one-3-carboxylic acid was treated with diphenylphosphoryl azide, followed by benzyl alcohol. The crude solid was suspended in dichloromethane and washed with 10% hydrochloric acid. Filtration of the solids afforded a crude product which was further purified by trituration with chloroform to afford slightly impure urethane. Washing the initial dichloromethane solution with saturated sodium bicarbonate afforded an additional precipitation of pure urethane. Evaporation of the remaining dichloromethane solution, followed by trituration of the residue with chloroform, afforded an additional portion of pure urethane, which was combined with the other pure portion. The urethane was washed with hot water and dried under vacuum to afford 3-benzyloxycarbonylamino-6-(4-methoxyphenyl)pyrid-2-one; TLC: R_{f}=0.56, dichloromethane:ethyl acetate:ethanol (95:3:2); MS: m/z=351(M+1).

### d. 2-[3-Benzyloxycarbonylamino-6-(4-methoxypheny1)-2-oxo-1,2-dihydro-l-pyridyl]-N-(2-tert-butyldimethylsilyloxy-3,3,3-trifluoro-1-isopropylpropyl)acetamide.

Using a similar procedure to that described in Example 1.d., but using two successive purifications by flash chromatography, eluting with ethyl acetate:dichloromethane (initially 1.5:100, then 2.5:100 in the second chromatography), 3-benzyloxycarbonylamino-6-(4-methoxyphenyl)pyrid-2-one was converted into 2-[3-benzyloxycarbonylamino-6-(4-methoxyphenyl)-2-oxo-1,2-dihydro-1-pyridyl]-N-(2-tert-butyldimethylsilyloxy-3,3,3-trifluoro-1-isopropylpropyl)acetamide; TLC: R_{f}=0.26, ethyl acetate:dichloromethane (3:97); MS: m/z=676(M+1).

### e. 2-[3-Benzyloxycarbonylamino-6-(4-methoxypheny1)-2-oxo-1,2-dihydro-1-pyridyl]-N-(3,3,3-trifluoro-2-hydroxy-1-isopropylpropyl)-acetamide.

Using a similar procedure to that described in Example 1.e., but omitting the chromatography, 2-[3-benzyloxycarbonylamino-6-(4-methoxyphenyl)-2-oxo-1,2-dihydro-1-pyridyl]-N-(2-tert-butyldimethylsilyloxy-3,3,3-trifluoro-1-isopropylpropyl)acetamide was converted into 2-[3-benzyloxycarbonylamino-6-(4-methoxyphenyl)-2-oxo-1,2-dihydro-1-pyridyl]-N-(3,3,3-trifluoro-2-hydroxy-1-isopropylpropyl)acetamide; TLC: R_{f}=0.52, ethyl acetate:dichloromethane (3:97); MS: m/z=562(M+1).

### EXAMPLE 3

### 2-(3-Benzyloxycarbonylamino-2-oxo-6-phenethyl-1,2-dihydro-1-pyridyl)-N-(3,3,3-trifluoro-1-isopropyl-2-oxopropyl)acetamide.

Using a similar procedure to that described in Example 1, using ethyl acetate:dichloromethane (5:95) as the chromatography solvent, 2-(3-benzyloxycarbonylamino-2-oxo-6-phenethyl-1,2-dihydro-1-pyridyl)-N-(3,3,3-trifluoro-2-hydroxy-1-isopropylpropyl)acetamide was oxidized to afford the title product; HPLC: t_{R}=6.16, FR=2.0, column A, water:acetonitrile (2:3); MS: m/z=558(M+1).

| Analysis for C₂₉H₃₀F₃N₃O₅: | | | |
|---|---|---|---|
| Calculated: | C, 62.57; | H, 5.42; | N, 7.54 |
| Found: | C, 62.31; | H, 5.47; | N, 7.42 |

The starting material alcohol used for the above preparation was prepared as follows:

### a. 6-Methylpyrid-2-one-3-carbonitrile.

To a mixture of sodium methoxide (46.5 g) in dry ether (950 mL), stirred with a mechanical stirrer and cooled with an ice bath, was added a mixture of acetone (distilled from K₂CO₃ under nitrogen, 46.5 g) and ethyl formate (distilled from P₂O₅ under nitrogen, 59.6 g) dropwise over 1 hour. When addition was complete, the cooling bath was removed and the reaction mixture was warmed to room temperature over 1 hour. The reflux condenser was replaced with a condenser set for distillation, and volatile materials were distilled by heating with an oil bath which was not allowed to rise above 60 °C. To the solid residue were added a solution of cyanoacetamide (67.0 g) in water (400 mL) and piperidine acetate (prepared by adding piperidine to a solution of 8 mL of glacial acetic acid in 20 mL of water until the solution was basic to litmus). The flask was fitted with a reflux condenser, and the mixture was heated for 2 h under reflux. The mixture was cooled to room temperature and acidified to pH 5 with glacial acetic acid. After standing overnight at room temperature, the mixture was cooled in an ice bath for about about 45 min; and the yellow solid product was filtered, washed with ice water (four times) and dried in a vacuum oven at 80 °C overnight. Crystallization from 50% (v/v) ethanol yielded 6-methylpyrid-2-one-3-carbonitrile as a yellow solid (52.6 g); TLC: R_{f}=0.29, chloroform:methanol (95:5); NMR: 2.27 (s,3), 6.20 (d,1, J=7.4), 8.01 (d,1, J=7.4); MS: m/z=135(M+1).

The method of 2-pyridone preparation described above in Example 3.a. is denoted herein as Cyclization Method C.

### b. 6-Phenethylpyrid-2-one-3-carbonitrile.

Using a similar procedure to that described below in Example 12.a., but using benzyl bromide in place of benzaldehyde, 6-methylpyrid-2-one-3-carbonitrile was alkylated to provide 6-phenethylpyrid-2-one-3-carbonitrile, which was washed with isopropanol and ether and dried under vacuum; TLC: R_{f}=0.49, methanol:chloroform (3:97); MS: m/z=225(M+1).

### c. 6-Phenethylpyrid-2-one-3-carboxylic acid.

Using Hydrolysis Method A, 6-phenethylpyrid-2-one-3-carbonitrile was hydrolyzed to 6-phenethylpyrid-2-one-3-carboxylic acid, which was filtered, washed with water and dried under vacuum; MS: m/z=244(M+1).

### d. 3-Benzyloxycarbonylamino-6-phenethylpyrid-2-one.

Using a similar method to that described in Example 1.c., isolating the product by filtration, washing with ethyl acetate, and drying under vacuum, 6-phenethylpyrid-2-one-3-carboxylic acid was converted into 3-benzyloxycarbonylamino-6-phenethylpyrid-2-one; TLC: R_{f}=0.48, methanol:chloroform (4:96); MS: m/z=349(M+1).

### e. tert-Butyl (3-Benzyloxycarbonylamino-2-oxo-6-phenethyl-1,2-dihydro-1-pyridyl)acetate.

Using a similar procedure to that of Example 1.d., above, 3-benzyloxycarbonylamino-6-phenethylpyrid-2-one (1.64 g) was suspended in dry dimethylformamide (20 mL) and to this suspension was added NaH (0.22 g of a 60% mineral oil dispersion). The mixture was stirred for 1.5 h, at which point all solids were in solution. tert-Butyl bromoacetate (0.92 g) was added, and the mixture was stirred overnight. The mixture was diluted with water (100 mL) and extracted with ethyl acetate (four times). The extracts were washed with brine, dried and evaporated. The residue was purified by chromatography, eluting with methanol:dichloromethane (0:100 then 3:97), to give tert-butyl (3-benzyloxycarbonylamino-2-oxo-6-phenethyl-1,2-dihydro-1-pyridyl)acetate (0.578 g); TLC: R_{f}=0.17, dichloromethane; MS: m/z=463(M+1).

NOTE: Also isolated from the chromatography was the corresponding O-alkylated product (0.512 g); TLC: R_{f}=0.61, dichloromethane.

### f. (3-Benzyloxycarbonylamino-2-oxo-6-phenethyl-1,2-dihydro-1-pyridyl)acetic acid.

To a solution of tert-butyl (3-benzyloxycarbonylamino-2-oxo-6-phenethyl-1,2-dihydro-1-pyridyl)acetate (0.554 g) in dichloromethane (distilled from CaH) was added trifluoroacetic acid (1.50 ml); and the mixture was stirred overnight, evaporated, and dried under vacuum to afford (3-benzyloxycarbonylamino-2-oxo-6-phenethyl-1,2-dihydro-1-pyridyl)acetic acid; MS: m/z=407(M+1).

### g. 2-(3-Benzyloxycarbonylamino-2-oxo-6-phenethyl-1,2-dihydro-1-pyridyl)-N-(3,3,3-trifluoro-2-hydroxy-1-isopropylpropyl)acetamide.

(3-Benzyloxycarbonylamino-2-oxo-6-phenethyl-1,2-dihydro-1-pyridyl)acetic acid (0.50 g) was dissolved in dry tetrahydrofuran (15 mL) along with 3-amino-1,1,1-trifluoro-4-methyl-2-pentanol hydrochloride (0.25 g), 1-(3-dimethylaminopropyl)-3-ethylcarbodiimide hydrochloride (0.23 g), 4-methylmorpholine (0.27 g) and 1-hydroxybenzotriazole hydrate (0.47 g). The mixture was stirred for 2 days. The reaction mixture was evaporated and the residue partitioned between ethyl acetate (50 mL) and 10% hydrochloric acid (25 mL). The layers were separated and the organics were washed with 10% hydrochloric acid, saturated sodium bicarbonate (twice) and brine; dried and evaporated to a foam. Chromatography, eluting with methanol:dichloromethane (4:96), gave 2-(3-benzyloxycarbonylamino-2-oxo-6-phenethyl-1,2-dihydro-1-pyridyl)-N-(3,3,3-trifluoro-2-hydroxy-1-isopropyl)acetamide (0.51 g); TLC: R_{f}=0.48, methanol:dichloromethane (4:96); MS: m/z=560(M+1).

### EXAMPLES 4-12

Using similar procedures to that described in Example 1, the following compounds of formula I wherein R⁰ is isopropyl, R is benzyloxycarbonyl and R⁵ and R⁶ have the indicated values were prepared by oxidation of the corresponding alcohols of formula II:

Example 4: R⁵=hydrogen, R⁶=3-pyridyl: Chromatography solvent: methanol:dichloromethane (1.5:98.5); TLC: R_{f}=0.24, methanol:dichloromethane (3:97); HPLC: t_{R}=6.80, FR=2.0, column A, water:acetonitrile (1:1); MS: m/z=531(M+1).

| Analysis for C₂₆H₂₅F₃N₄O₅·0.25 H₂O: | | | |
|---|---|---|---|
| Calculated: | C, 58.37; | H, 4.80; | N, 10.47 |
| Found: | C, 58.36; | H, 4.89, | N, 10.27 |

An alternative synthesis for this compound is described in Example 170.

Example 5: R⁵=hydrogen, R⁶=4-chlorophenyl: Chromatography solvent: chloroform:ethyl acetate (20:1); TLC: Rf=0.29, chloroform:ethyl acetate (20:1); MS: m/z=564(M+1 for ³⁵Cl).

| Analysis for C₂₇H₂₅ClF₃N₃O₅: | | | |
|---|---|---|---|
| Calculated: | C, 57.50; | H, 4.47; | N, 7.45 |
| Found: | C, 57.45; | H, 4.66; | N, 7.47 |

### Example 6: R⁵=hydrogen, R⁶=3-tert-butoxyphenyl:

Chromatography solvent: ethanol:ethyl acetate:dichloromethane (0.25:2.5:97.25); TLC: Rf=0.59, methanol:ethyl acetate:dichloromethane (2:3:95); HPLC: t_{R}=5.54, FR=2.0, column A; water:acetonitrile (2:3); MS: m/z=602(M+1).

| Analysis for C₃₁H₃₄F₃N₃O₆·0.5 H₂O: | | | |
|---|---|---|---|
| Calculated: | C, 60.98; | H, 5.78; | N, 6.88 |
| Found: | C, 60.96; | H, 5.69; | N, 6.66 |

Example 7: R⁵=hydrogen, R⁶=4-methylphenyl: Chromatography solvent (chromatographed twice): ethanol:ethyl acetate:dichloromethane (0.5:5:94.5 - first time) then (0.25:2.5:97.25 - second time); TLC: R_{f}=0.21, ethanol:ethyl acetate:dichloromethane (0.5:5:94.5); HPLC: t_{R}=8.27, FR=1, column A, water:acetonitrile (2:3); MS: m/z=544(M+1).

| Analysis for C₂₈H₂₈F₃N₃O₅: | | | |
|---|---|---|---|
| Calculated: | C, 61.87; | H, 5.19; | N, 7.73 |
| Found: | C, 61.62; | H, 5.21; | N, 7.58 |

Example 8: R⁵=hydrogen, R⁶=3-chlorophenyl: Chromatography solvent: dichloromethane:ethyl acetate (gradient, 50:1 then 50:2); HPLC: t_{R}=9.36, FR=2.0, column A, water:acetonitrile (1:1); MS: m/z=564(M+1 for ³⁵Cl).

| Analysis for C₂₇H₂₅ClF₃N₃O₅·0.2 H₂O: | | | |
|---|---|---|---|
| Calculated: | C, 57.13; | H, 4.52; | N, 7.40 |
| Found: | C, 57.07; | H, 4.55; | N, 7.30 |

Example 9: R⁵=methyl, R⁶=phenyl: Chromatography solvent: dichloromethane:ethyl acetate:ethanol (95:5:1); TLC: R_{f}=0.57, dichlomethane:ethyl actate:ethanol; HPLC: t_{R}=5.84, FR=3.0, column A, water:acetonitrile (1:1); MS: m/z=544(M+1).

| Analysis for C₂₈H₂₈F₃N₃O₅·0.35 H₂O: | | | |
|---|---|---|---|
| Calculated: | 61.16; | H, 5.26; | N, 7.73 |
| Found: | 61.13; | H, 5.31; | N, 7.53 |

Example 10: R⁵=hydrogen, R⁶=3,5-dimethoxyphenyl: Chromatography solvent: ethanol:ethyl acetate:dichloromethane (gradient, 0:0:1, 0:2:98, 1:2:98); TLC: R_{f}=0.32, ethanol:ethyl acetate:dichloromethane (1:2:97); HPLC: t_{R}=6.44, FR=3.0, column A, water:acetonitrile (1:1); MS: m/z=590(M+1).

| Analysis for C₂₉H₃₀F₃N₃O₇: | | | |
|---|---|---|---|
| Calculated: | C, 59.07; | H, 5.13; | N, 7.13 |
| Found: | C, 58.80; | H, 5.16; | N, 6.96 |

Example 11: R⁵=hydrogen, R⁶=benzyl: Chromatography solvent: chloroform:ethyl acetate (gradient, 30:1 to 20:1); TLC: R_{f}=0.31, chloroform:methanol (50:1); MS: m/z=544(M+1).

| Analysis for C₂₈H₂₈F₃N₃O₅·0.4 H₂O: | | | |
|---|---|---|---|
| Calculated: | C, 61.06; | H, 5.27; | N, 7.63 |
| Found: | C, 61.08; | H, 5.26; | N, 7.54 |

Example 12: R⁵=hydrogen, R⁶=trans-styryl: Chromatography solvent: ethyl acetate:dichloromethane (5:95); TLC: R_{f}=0.46, ethyl acetate:dichloromethane (1:9); HPLC: t_{R}=6.48, FR=2, column A, water:- acetonitrile (2:3); MS: m/z=556(M+1).
Analysis for C₂₉H₂₈F₃N₃O₅:
Calculated: C, 62.70; H, 5.08; N, 7.56
Found: C, 62.64; H, 5.18; N, 7.55

The corresponding alcohols of formula II for Examples 4-12 were prepared as follows:

### EXAMPLES 4.a.-11.a.

Pyrid-2-one-3-carbonitriles bearing the substituents R⁵ at the 5-position and R⁶ at the 6-position were prepared from the corresponding ketones of formula R⁵.CH₂.CO.R⁶ using similar procedures to those of the indicated Cyclization Methods described above:

Example 4.a.: R⁵=hydrogen, R⁶=3-pyridyl: Cyclization Method A; MS: m/z=198(M+1).

Example 5.a.: R⁵=hydrogen, R⁶=4-chlorophenyl: Cyclization Method A; TLC: R_{f}=0.17, chloroform:methanol (50:1); MS: m/z=231(M+1 for ³⁵Cl).

Example 6.a.: R⁵=hydrogen, R⁶=3-tert-butoxyphenyl: Cyclization Method A; chromatographed, eluting with ethyl acetate:dichloromethane (10:90) followed by trituration with ethyl acetate and precipitation of additional material by adding hexane to the ethyl acetate supernatant; MS: m/z=269(M+1).

The 3'-tert-butoxyacetophenone used for the cyclization was prepared as follows:

A mixture of 3'-hydroxyacetophenone (22.64 g), isobutylene (300 mL) and concentrated H₂SO₄ (1 mL) in dry dichloromethane (310 mL) was stirred in a sealed pressure vessel at room temperature for 3 days. After the reaction vessel was vented, the organic phase was washed (10% NaOH, water and brine), dried and evaporated to an oil (25.95 g) which was purified by chromatography, eluting with dichloromethane:ethyl acetate, to give 3'-tert-butoxyacetophenone (22.62 g); TLC: R_{f}=0.55, ethyl acetate:dichloromethane (3:97); MS: m/z=193(M+1).

Example 7.a.: R⁵=hydrogen, R⁶=4-methylphenyl: Cyclization Method B; after the reaction mixture was filtered at about pH 8, the collected solid was first stirred with 10% hydrochloric acid then triturated with chloroform and ether before air drying; TLC: R_{f}=0.43, chloroform:methanol (20:1); MS: m/z=211(M+1).

Example 8.a.: R⁵=hydrogen, R⁶=3-chlorophenyl: Cyclization Method A; triturated with ether; TLC: R_{f}=0.22, dichloromethane:methanol (95:5); MS: m/z=231(M+1).

Example 9.a.: R⁵=methyl, R⁶=phenyl: Cyclization Method A; 300 MHz NMR: 1.96 (s,3), 7.46-7.54 (m,5), 8.15 (s,1), 12.53 (broad s, 1); MS: m/z=211(M+1).

Example 10.a.: R⁵=hydrogen, R⁶=3,5-dimethoxyphenyl: Cyclization Method B; TLC: R_{f}=0.44, methanol:dichloromethane (2:98); MS: m/z=257(M+1).

Example 11.a. : R⁵=hydrogen, R⁶=benzyl: Cyclization Method C; recrystallized from hot ethanol, washed with ether; TLC: R_{f}=0.44, chloroform:methanol (20:1); 300 MHz NMR: 3.90 (s,2), 6.16 (d,1, J=7), 7.32 (m,5), 8.03 (d,1, J=7), 12.8 (broad, 1); MS: m/z=211(M+1).

| Analysis for C₁₃H₁₀N₂O: | | | |
|---|---|---|---|
| Calculated: | C, 74.27; | H, 4.79, | N, 13.32 |
| Found: | C, 74.20; | H, 5.01, | N, 13.31 |

Example 12.a.: R⁵=hydrogen, R⁶=trans-styryl: A solution of dry diisopropylamine (6.6 mL, 4.77 g) in dry tetrahydrofuran (200 mL) under nitrogen in a 500 mL round-bottomed flask was cooled with a -78 °C bath. A solution of n-butyl lithium (2.14 M in hexanes; 19.0 mL) was added, and the resultant solution was stirred at -78 °C for 20 min. 6-Methylpyrid-2-one-3-carbonitrile (2.50 g) was added as a solid. After 5 min of stirring at -78 °C, the mixture was allowed to warm to 0 °C. After the mixture was stirred for 2.5 h at 0 °C, a solution of freshly distilled benzaldehyde (1.90 mL; 1.98 g) in dry tetrahydrofuran (4 mL) was added via syringe; and the orange reaction mixture was stirred at °0 C for 2 h before warming to room temperature and stirring overnight. The mixture was evaporated, and the residue dissolved in water (100 mL). The aqueous phase was washed with ether and petroleum ether. Acidification with 10% hydrochloric acid to about pH 3 gave 6-(2-hydroxy-2-phenylethyl)pyrid-2-one-3-carbonitrile as a yellow solid (3.17 g) which was collected by filtration, washed with isopropanol and ether, dried under vacuum, and used in the next step without purification; MS: m/z=241(M+1).

### EXAMPLES 4.b.-11.b.

Pyrid-2-one-3-carboxylic acids bearing the substituents R⁵ at the 5-position and R⁶ at the 6-position were prepared from the corresponding pyrid-2-one-3-carbonitriles using similar procedures to those of the Hydrolysis Methods described above:

Example 4.b.: R⁵=hydrogen, R⁶=3-pyridyl: Hydrolysis Method A; MS: m/z=217(M+1).

Example 5.b.: R⁵=hydrogen, R⁶=4-chlorophenyl: Hydrolysis Method A; TLC: R_{f}=0.33, chloroform:methanol:acetic acid (50:1:trace); MS: m/z=250(M+1 for ³⁵Cl).

Example 6.b.: R⁵=hydrogen, R⁶=3-tert-butoxyphenyl: Hydrolysis Method B; MS: m/z=288(M+1).

Example 7.b.: R⁵=hydrogen, R⁶=4-methylphenyl: Hydrolysis Method A; TLC: R_{f}=0.41, chloroform:methanol:acetic acid (20:1:0.1); MS: m/z=230(M+1).

Example 8.b.: R⁵=hydrogen, R⁶=3-chlorophenyl: Hydrolysis Method A; TLC: R_{f}=0.99, chloroform:methanol:acetic acid (85:10:5); MS: m/z=250(M+1 for ³⁵Cl).

Example 9.b.: R⁵=methyl, R⁶=phenyl: Hydrolysis Method A; TLC: R_{f}=0.34, chloroform:methanol (20:1); MS: m/z=230(M+1).

Example 10.b.: R⁵=hydrogen, R6=3,5-dimethoxyphenyl: Hydrolysis Method B; MS: m/z=276(M+1).

Example 11.b.: R⁵=hydrogen, R⁶=benzyl: Hydrolysis Method A; TLC: R_{f}=0.42, dichloromethane:methanol:acetic acid (50:1:trace); MS: m/z=230(M+1).

### EXAMPLE 12.b.

6-(2-Hydroxy-2-phenylethyl)pyrid-2-one-3-carbonitrile prepared according to the procedure of Example 12.a. (3.12 g) was suspended in glacial acetic acid (27 mL) and 48% hydrobromic acid (13 mL). The mixture was heated at reflux overnight, cooled and evaporated. The residue was suspended in water and made basic with 50% NaOH. Undissolved solids were removed by filtration. The filtrate was brought to about pH 3 with concentrated HCl. The precipitate which formed was collected by filtration, washed with water and dried under vacuum overnight to afford trans-6-styrylpyrid-2-one-3-carboxylic acid (1.58 g) which was used in the next step without further purification; MS: m/z=242(M+1).

### EXAMPLES 4.c.-12.c.

Using similar procedures to that described above in Example 1.c., except omitting the trituration, 3-benzyloxycarbonylaminopyrid-2-ones bearing the substituents R⁵ at the 5-position and R⁶ at the 6-position were prepared from the corresponding pyrid-2-one-3-carboxylic acids:

Example 4.c.: R⁵=hydrogen, R⁶=3-pyridyl: TLC: R_{f}=0.70, chloroform:methanol (9:1); MS: m/z=322(M+1).

Example 5.c.: R⁵=hydrogen, R⁶=4-chlorophenyl: Purified by chromatography, eluting with chloroform:methanol (50:1), or by crystallization from chloroform; TLC: R_{f}=0.72, chloroform:methanol (20:1); MS: m/z=355(M+1 for ³⁵Cl).

Example 6.c.: R⁵=hydrogen, R⁶=3-tert-butoxyphenyl: Purified by chromatography, eluting with ethyl acetate:dichloromethane (3:97); TLC: R_{f}=0.67, methanol:chloroform (4:96); MS: m/z=393(M+1).

Example 7.c.: R⁵=hydrogen, R⁶=4-methylphenyl: Purification by trituration with chloroform; TLC: R_{f}=0.14, chloroform:ethyl acetate (50:1); MS: m/z=335(M+1).

Example 8.c.: R⁵=hydrogen, R⁶=3-chlorophenyl: Purification by recrystallization from hot ethanol and chloroform; TLC: R_{f}=0.57, dichloromethane:methanol (95:5); MS: m/z=355(M+1).

Example 9.c.: R⁵=methyl, R⁶=phenyl: Purified by chromatography, eluting with chloroform:methanol (50:1); TLC: R_{f}=0.35, chloroform:methanol (50:1); MS: m/z=335(M+1).

Example 10.c.: R⁵=hydrogen, R⁶=3,5-dimethoxyphenyl: Purified by chromatography, eluting with ethanol:ethyl acetate:dichloromethane (1:2:97), followed by trituration with ether; TLC: R_{f}=0.48, ethanol:ethyl acetate:dichloromethane (2:3:95); MS: m/z=381(M+1).

Example 11.c.: R⁵=hydrogen, R⁶=benzyl: Purified by recrystallization from ethanol; TLC: R_{f}=0.75, chloroform:methanol (20:1), R_{f}=0.10, hexane:ethyl acetate (3:1); MS: m/z=335(M+1).

Example 12.c.: R⁵=hydrogen, R⁶=trans-styryl: filtered and dried under vacuum; MS: m/z=347(M+1).

### EXAMPLES 4.d.-12.d.

Using similar procedures to that described above in Example 1.d., 2-(3-benzyloxycarbonylamino-2-oxo-1,2-dihydro-1-pyridyl)-N-(2-tert-butyldimethylsilyloxy-3,3,3-trifluoro-1-isopropylpropyl)-acetamides bearing the substituents R⁵ at the 5-position of the pyridyl ring and R⁶ at the 6-position of the pyridyl ring were prepared from the corresponding 3-benzyloxycarbonylaminopyrid-2-ones and N-(2-tert-butyldimethylsilyloxy-3,3,3-trifluoro-1-isopropylpropyl)-2-iodoacetamide:

Example 4.d.: R⁵=hydrogen, R⁶=3-pyridyl: Chromatography solvent: methanol:chloroform (0.5:99.5); TLC: R_{f}=0.37, methanol:dichloromethane (2:98); MS: m/z=647(M+1).

Example 5.d.: R⁵=hydrogen, R⁶=4-chlorophenyl: Chromatography solvent: dichloromethane:ethyl acetate (100:1 then 50:1); TLC: R_{f}=0.23, dichloromethane:ethyl acetate (50:1); MS: m/z=680(M+1 for ³⁵Cl).

Example 6.d.: R⁵=hydrogen, R⁶=3-tert-butoxyphenyl: Purified by three successive chromatographies, eluting with (1) chloroform, (2) ethanol:ethyl acetate:dichloromethane (0.5:0.5:99), and (3) ethanol:ethyl acetate:dichloromethane (0.25:0.5:99.25), which resulted in a product which still contained a small amount of the 0-alkylated isomer and was used for Example 6.e.; TLC: R_{f}=0.31, methanol:ethyl acetate:dichloromethane (0.5:1:98.5); MS: m/z=718(M+1).

Example 7.d.: R⁵=hydrogen, R⁶=4-methylphenyl: Chromatography solvent: ethanol:ethyl acetate:dichloromethane (0.25:2:97.75); TLC: R_{f}=0.25, ethyl acetate:dichloromethane (3:97); MS: m/z=660(M+1).

Example 8.d.: R⁵=hydrogen, R⁶=3-chlorophenyl: Purified by three successive chromatographies, eluting with (1) chloroform:ethyl acetate:methanol (75:1:0.5), (2) chloroform:ethyl acetate:acetic acid (50:1:0.15), and (3) dichloromethane:ethyl acetate (70:1-then 60:1); TLC (of crude reaction mixture): R_{f}=0.40, chloroform:ethyl acetate (50:1); MS: m/z=681(M+1 for ³⁵Cl).

Example 9.d: R⁵=methyl, R⁶=phenyl: Chromatography solvent: chloroform:ethyl acetate:methanol (50:1:0 then 75:1:0.5); TLC: R_{f}=0.31, chloroform:ethyl acetate (50:1); MS: m/z=660(M+1).

Example 10.d.: R⁵=hydrogen, R⁶=3,5-dimethoxyphenyl: Chromatography solvent: dichloromethane:ethyl acetate:ethanol (98:2:0.5) which afforded a mixture of N- and 0-alkylated products which was carried through to the next step before separation; TLC: R_{f}=0.36 (N-alkylated) and 0.43 (0-alkylated), dichloromethane:ethyl acetate:ethanol (97:2:1); MS: m/z=706(M+1).

Example 11.d.: R⁵=hydrogen; R⁶=benzyl: Chromatography solvent: chloroform:ethyl acetate (20:1); TLC: R_{f}=0.53, hexane:ethyl acetate (3:1); MS: m/z=660 (M+1).

Example 12.d.: R⁵=hydrogen, R⁶=trans-styryl: Chromatography solvent: methanol:ethyl acetate:dichloromethane (successively: 0:1:99, 0:5:95, 0:15:85, 5:0:95); TLC: R_{f}=0.51, ethyl acetate:dichloromethane (5:95).

### EXAMPLES 4.e.-12.e.

Using similar procedures to that described above in Example 1.e., 2-(3-benzyloxycarbonylamino-2-oxo-1,2-dihydro-1-pyridyl)-N-(3,3,3-trifluoro-2-hydroxy-1-isopropylpropyl)acetamides bearing the substituents R⁵ at the 5-position of the pyridyl ring and R⁶ at the 6-position of the pyridyl ring were prepared from the corresponding 2-(3-benzyloxyaminocarbonylamino-2-oxo-1,2-dihydro-1-pyridyl)-N-(2-tert-butyldimethylsilyloxy-3,3,3-trifluoro-1-isopropylpropyl)-acetamides:

Example 4.e.: R⁵=hydrogen, R⁶=3-pyridyl: Isolated and dried without chromatography; TLC: R_{f}=0.15, methanol:dichloromethane (2:98); MS: m/z=533(M+1).

Example 5.e.: R⁵=hydrogen, R⁶=4-chlorophenyl: Chromatography solvent: chloroform:ethyl acetate (20:1 then 10:1); TLC: R_{f}=0.17, chloroform:ethyl acetate (20:1); MS: m/z=566(M+1 for ³⁵Cl).

Example 6.e.: R⁵=hydrogen; R⁶=3-tertbutoxyphenyl: Chromatography solvent: methanol:ethyl acetate:dichloromethane (0.5:4:95.5); TLC: R_{f}=0.18, methanol:ethyl acetate:dichloromethane (1:4:95); MS: m/z=604(M+1).

Example 7.e.: R⁵=hydrogen, R⁶=4-methylphenyl: Chromatography solvent: ethanol:ethyl acetate:dichloromethane (1:3:96); TLC: R_{f}=0.19, ethanol:ethyl acetate:dichloromethane (1:4:95).

Example 8.e.: R⁵=hydrogen, R⁶=3-chlorophenyl: Purified by washing with water and brine and drying under vacuum; TLC (of crude reaction mixture): R_{f}=0.15, chloroform:methanol (50:1); MS: m/z=566(M+1 for ³⁵Cl).

Example 9.e.: R⁵=methyl, R⁶=phenyl: Isolated directly, not further purified; TLC: R_{f}=0.32, dichloromethane:ethyl acetate (9:1); MS: m/z=546(M+1).

Example 10.e.: R⁵=hydrogen, R⁶=3,5-dimethoxyphenyl: Chromatography solvent: ethanol:ethyl acetate:dichloromethane (0.5:2:97.5); TLC: R_{f}=0.22, ethanol:ethyl acetate:dichloromethane (1:2:97); MS: m/z=592(M+1).

Example 11.e.: R⁵=hydrogen, R⁶=benzyl: Chromatography solvent: chloroform:methanol (20:1); TLC: R_{f}=0.26, chloroform:methanol (50:1); MS: m/z=546(M+1).

Example 12.e.: R⁵=hydrogen, R⁶=trans-styryl: Chromatography solvent: ethyl acetate:dichloromethane (1:9); TLC: R_{f}=0.33, ethyl acetate:dichloromethane (1:9); MS: m/z=558(M+1).

### EXAMPLE 13

### 2-[3-Benzyloxycarbonylamino-6-(3-hydroxyphenyl)-2-oxo-1,2-dihydro-1-pyridyl]-N-(3,3,3-trifluoro-1-isopropyl-2-oxopropyl)acetamide.

To a solution of 2-[3-benzyloxycarbonylamino-6-(3-tert-butoxyphenyl)-2-oxo-1,2-dihydro-1-pyridyl]-N-(3,3,3-trifluoro-1-isopropyl-2-oxopropyl)acetamide (78 mg) in dry dichloromethane (3 mL) was added trifluoroacetic acid (57 mg). After 5 h the reaction was incomplete, and additional trifluoroacetic acid (57 mg) was added. After 18 h the reaction mixture was diluted with dichloromethane (50 mL), washed with water and brine, dried, and evaporated to a crude oil which was purified by chromatography, eluting with ethanol:ethyl acetate:dichloromethane (0.5:5:94.5), to give the title compound as a white solid (60 mg); TLC: R_{f}=0.15, ethanol:ethyl acetate:dichloromethane (0.5:5:94.5); HPLC: t_{R}=6.04, FR=2.0, column A, water:acetonitrile (55:45); MS: m/z=546(M+1).

| Analysis for C₂₇H₂₆F₃N₃O₆: | | | |
|---|---|---|---|
| Calculated: | C, 59.44; | H, 4.80; | N, 7.70 |
| Found: | C, 59.58; | H, 4.91; | N, 7.41 |

### EXAMPLE 14

### 2-(2-Oxo-6-phenyl-3-phenylacetylamino-1,2-dihydro-1-pyridyl)-N-(3,3,3-trifluoro-1-isopropyl-2-oxopropyl)acetamide.

Using a similar procedure to that described in Example 1, using methanol:dichloromethane (1:99) as the chromatography solvent, 2-(2-oxo-6-phenyl-3-phenylacetylamino-1,2-dihydro-1-pyridyl)-N-(3,3,3-trifluoro-2-hydroxy-1-isopropylpropyl)acetamide was oxidized to afford the title product; HPLC: t_{R}=6.92, FR=2.0, column A, water:acetonitrile (55.45); MS: m/z=514(M+1).

| Analysis for C₂₇H₂₆F₃N₃O₄: | | | |
|---|---|---|---|
| Calculated: | C, 63.15; | H, 5.10; | N, 8.18 |
| Found: | C, 63.03; | H, 4.98; | N, 8.02 |

The starting material alcohol used for the above preparation was prepared as follows:

### a. 2-(3-Amino-2-oxo-6-phenyl-1,2-dihydro-1-pyridyl)-N-(2-tert-butyldimethylsilyloxy-3,3,3-trifluoro-1-isopropylpropyl)acetamide.

To a solution of 2-(3-benzyloxycarbonylamino-2-oxo-6-phenyl-1,2-dihydro-1-pyridyl)-N-(2-tert-butyldimethylsilyloxy-3,3,3-trifluoro-1-isopropylpropyl)acetamide (2.41 g) in dry tetrahydrofuran (8 mL) was added 10% (w/w) palladium-on-carbon (0.50 g) in dry tetrahydrofuran (8 mL) under nitrogen. This mixture was stirred under hydrogen at atmospheric pressure for 5 h. The catalyst was removed by filtration through a plug of diatomaceous earth and the plug was washed with ethanol. The filtrate was evaporated and dried under vacuum to give 2-(3-amino-2-oxo-6-phenyl-1,2-dihydro-1-pyridyl)-N-(2-tert-butyldimethylsilyloxy-3,3,3-trifluoro-1-isopropylpropyl)-acetamide (1.78 g); TLC: R_{f}=0.06, ethyl acetate:dichloromethane (6:94); MS: m/z=512(M+1).

Alternatively, the 3-amino-6-phenylpyridone may be prepared from the 3-benzyloxycarbonyl-6-(2-chlorophenyl)pyridone as follows: To a solution of 2-[3-benzyloxycarbonylamino-6-(2-chlorophenyl)-2-oxo-1,2-dihydro-1-pyridyl]-N-(2-tert-butyldimethylsilyloxy-3,3,3-trifluoro-1-isopropylpropyl)acetamide (Example 21.d.) (0.35 g) in dry tetrahydrofuran (3 mL) was added 10% (w/w) palladium-on-carbon (91 mg). This mixture was stirred under hydrogen at atmospheric pressure overnight. The catalyst was removed by filtration through a plug of diatomaceous earth and the plug was washed with tetrahydrofuran. The filtrate was evaporated and dried under vacuum to give a white powder (0.28 g). To a solution of this powder in absolute ethanol (3 mL) was added sodium methoxide (30 mg), and the mixture was stirred until dissolution of all solid had occurred. To the solution was added 10% (w/w) palladium-on-carbon (90 mg), and the mixture was stirred under hydrogen at atmospheric pressure overnight. The catalyst was removed by filtration through a plug of diatomaceous earth and the plug was washed with ethanol. The filtrate was evaporated and the residue was partitioned between chloroform and water. The organic layer was washed with brine, dried and evaporated to give 2-(3-amino-2-oxo-6-phenyl-1,2-dihydro-1-pyridyl)-N-(2-tert-butyldimethylsilyloxy-3,3,3-trifluoro-1-isopropyl-propyl)acetamide as a white solid (0.23 g).

### b. N-(2-tert-Butyldimethylsilyloxy-3,3,3-trifluoro-1-isopropylpropyl)-2-(2-oxo-6-phenyl-3-phenylacetylamino-1,2-dihydro-1-pyridyl)acetamide.

A 25 mL flask was charged with 2-(3-amino-2-oxo-6-phenyl-1,2-dihydro-1-pyridyl)-N-(2-tert-butyldimethylsilyloxy-3,3,3-trifluoro-1-isopropylpropyl)acetamide (0.301 g) dissolved in freshly distilled tetrahydrofuran (6 mL) under nitrogen. Triethylamine (0.09 mL) was added, followed by phenylacetyl chloride (0.08 mL). The cloudy, yellowish solution was stirred overnight; diluted with ethyl acetate (50 mL); washed with 10 % hydrochloric acid (three times), saturated aqueous sodium bicarbonate (three times) and brine; dried; and evaporated to give an oil which solidified under vacuum. The soft solid was purified by chromatography, eluting with ethyl acetate:dichloromethane (3:97), to afford N-(2-tert-butyldimethylsilyloxy-3,3,3-trifluoro-1-isopropylpropyl)-2-(2-oxo-6-phenyl-3-phenylacetylamino-1,2-dihydro-1-pyridyl)acetamide (0.272 g); TLC: R_{f}=0.54, methanol:dichloromethane (1:99); MS: m/z=630(M+1).

The above described acylation using an acid chloride is denoted herein as Acylation Method A.

### c. 2-(2-Oxo-6-phenyl-3-phenylacetylamino-1,2-dihydro-1-pyridyl)-N-(3,3,3-trifluoro-2-hydroxy-1-iso-propylpropyl)acetamide.

Using a similar procedure to that described in Example 1.e., diluting the reaction mixture with ethyl acetate, washing it with water, and evaporating it, N-(2-tert-butyldimethylsilyloxy-3,3,3-trifluoro-1-isopropylpropyl)-2-(2-oxo-6-phenyl-3-phenylacetylamino-1,2-dihydro-1-pyridyl)acetamide was converted into 2-(2-oxo-6-phenyl-3-phenylacetylamino-1,2-dihydro-1-pyridyl)-N-(3,3,3-trifluoro-2-hydroxy-1-isopropylpropyl)acetamide; TLC: R_{f}=0.23, methanol:dichloromethane (1:99); MS: m/z=516(M+1).

### EXAMPLE 15

### 2-[3-(4-Methoxyphenyl)acetylamino-2-oxo-6-phenyl-1,2-dihydro-1-pyridyl]-N-(3,3,3-trifluoro-1-iso-propyl-2-oxopropyl)acetamide.

Using a similar procedure to that described in Example 1, preadsorbing the crude product onto silica gel before chromatography, eluting with methanol:ethyl acetate:dichloromethane (0.5:5:94.5), 2-[3-(4-methoxyphenyl)acetylamino-2-oxo-6-phenyl-1,2-dihydro-1-pyridyl]-N-(3,3,3-trifluoro-2-hydroxy-1-isopropylpropyl)acetamide was oxidized to afford the title product; HPLC: t_{R}=6.18, FR=2.0, column A, water:acetonitrile (55:45); MS: m/z=544(M+1).

| Analysis for C₂₈H₂₈F₃N₃O₅·0.5 H₂O: | | | |
|---|---|---|---|
| Calculated: | C, 60.86; | H, 5.29; | N, 7.60 |
| Found: | C, 60.85; | H, 5.36; | N, 7.43 |

The starting material alcohol for the above preparation was prepared as follows:

### a. N-(2-tert-Butyldimethylsilyloxy-3,3,3-trifluoro-1-isopropylpropyl)-2-[3-(4-methoxyphenyl)acetylamino-2-oxo-6-phenyl-1,2-dihydro-1-pyridyl]acetamide.

To 2-(3-amino-2-oxo-6-phenyl-1,2-dihydro-1-pyridyl)-N-(2-tert-butyldimethylsilyloxy-3,3,3-trifluoro-1-isopropylpropyl)-acetamide (0.295 g) suspended in redistilled tetrahydrofuran (5 mL) was added 4-methoxyphenylacetic acid (0.97 g), followed by 1-hydroxybenzotriazole hydrate (0.156 g) and 1-(3-dimethylaminopropyl)-3-ethylcarbodiimide hydrochloride (0.123 g). The reaction was shown by TLC to be incomplete after overnight stirring; therefore, triethylamine (0.08 mL) and 4-dimethylaminopyridine (catalytic amount) were added to the mixture. After stirring 1 h, additional acid (0. 098 g), 1-(3-dimethylaminopropylpropyl)-3-ethylcarbodiimide hydrochloride (0.123 g), triethylamine (0.08 mL), and 1-hydroxybenzotriazole (0.156 g) were added. Additional tetrahydrofuran (2 mL) was added to facilitate stirring. After stirring overnight TLC indicated little change in the reaction mixture which was diluted with ethyl acetate (50 mL); washed with water (twice), saturated aqueous sodium bicarbonate (three times) and brine; dried; and evaporated. Purification by chromatography, eluting with ethanol:ethyl acetate:dichloromethane (0.5:5:94.5) gave N-(2-tert-butyldimethylsilyloxy-3,3,3-trifluoro-1-isopropylpropyl)-2-[3-(4-methoxyphenyl)acetylamino-2-oxo-6-phenyl-1,2-dihydro-1-pyridyl]-acetamide (0.235 g); TLC (of crude reaction mixture): R_{f}=0.88, methanol:dichloromethane (4:96); MS: m/z=660(M+1).

The above described acylation using 1-(3-dimethylaminopropyl)-3-ethylcarbodiimide for the coupling of an acid with a 3-aminopyridyl derivative is denoted herein as Acylation Method B.

### b. 2-[3-(4-Methoxyphenyl)acetylamino-2-oxo-6-phenyl-1,2-dihydro-1-pyridyl]-N-(3,3,3-trifluoro-2-hydroxy-1-isopropylpropyl)acetamide.

Using a similar procedure to that described in Example 1.e., diluting the reaction mixture with ethyl acetate, washing it with water (3 times) and brine, drying it, and evaporating it, N-(2-tert-butyldimethylsilyloxy-3,3,3-trifluoro-1-isopropylpropyl)-2-[3-(4-methoxyphenyl)acetylamino-2-oxo-6-phenyl-1,2-dihydro-1-pyridyl]-acetamide was converted into 2-[3-(4-methoxyphenyl)acetylamino-2-oxo-6-phenyl-1,2-dihydro-1-pyridyl]-N-(3,3,3-trifluoro-2-hydroxy-1-isopropylpropyl)acetamide; TLC: R_{f}=0.18, ethyl acetate:dichloromethane (1:9); MS: m/z=546(M+1).

### EXAMPLES 16-19

Using similar procedures to that described in Example 1, the following compounds of formula I wherein R⁰ is isopropyl, R is the indicated acyl group, R⁵ is hydrogen and R⁶ is phenyl were prepared by oxidation of the corresponding alcohols of formula II:

Example 16: R=benzylaminocarbonyl: Chromatography solvent: acetic acid:ethyl acetate:dichloromethane (0.5:10:89.5); TLC: R_{f}=0.23, acetic acid:ethyl acetate:dichloromethane (0.5:10:89.5); HPLC: t_{R}=8.35, FR=1.0, column A, water:acetonitrile (1:1); MS: m/z=529(M+1).

| Analysis for C₂₇H₂₇F₃N₄O₄·1.0 CH₃COOH: | | | |
|---|---|---|---|
| Calculated: | C, 59.18; | H, 5.31; | N, 9.52 |
| Found: | C, 59.18; | H, 5.30; | N, 9.62 |

Example 17: R=4-methoxybenzoyl: Chromatography solvent: ethyl acetate:dichloromethane (5:95 then 10:90), the crude product was preadsorbed onto diatomaceous earth and placed atop the column before elution; HPLC: t_{R}=4.84, FR=2.0, column A, water:acetonitrile (1:1); MS: m/z=530(m+1).

| Analysis for C₂₇H₂₆F₃N₃O₅: | | | |
|---|---|---|---|
| Calculated: | C, 61.24; | H, 4.95; | N, 7.94 |
| Found: | C, 60.93; | H, 5.01; | N, 7.79 |

Example 18: R=(3,4-dimethoxyphenyl)acetyl: Chromatography solvent: ethyl acetate:dichloromethane (2:98); TLC: R_{f}=0.34, ethyl acetate:dichloromethane (3:97); HPLC: t_{R}=6.89, FR=2.0, column A, water:acetonitrile (55:45); MS: m/z=574(M+1).

| Analysis for C₂₉H₃₀F₃N₃O₆: | | | |
|---|---|---|---|
| Calculated: | C, 60.73, | H, 5.27, | N, 7.33 |
| Found: | C, 60.57; | H, 5.52, | N, 7.17 |

Example 19: R=phenoxycarbonyl: Chromatography solvent: ethyl acetate:dichloromethane (5:95); HPLC: t_{R}=8.26, FR=2.0, column A, water:acetonitrile (55:45); MS: m/z=516(M+1).

| Analysis for C₂₆H₂₄F₃N₃O₅·0.3 H₂O: | | | |
|---|---|---|---|
| Calculated: | C, 59.95; | H, 4.76; | N, 8.06 |
| Found: | C, 59.93; | H, 4.86; | N, 7.80 |

The corresponding alcohols of formula II for Examples 16-19 were prepared as follows:

### EXAMPLES 16.a.-19.a.

2-(3-Acylamino-2-oxo-6-phenyl-1,2-dihyro-1-pyridyl)-N-(2-tert-butyldimethylsilyloxy-3,3,3-trifluoro-1-isopropylpropyl)-acetamides having the indicated acyl group R were prepared from 2-(3-amino-2-oxo-6-phenyl-1,2-dihydro-1-pyridyl)-N-(2-tert-butyl-dimethylsilyloxy-3,3,3-trifluoro-1-isopropylpropyl)acetamide as follows:

Example 16.a.: R=benzylaminocarbonyl: To a solution of 2-(3-amino-2-oxo-6-phenyl-1,2-dihydro-1-pyridyl)-N-(2-tert-butyl-dimethylsilyloxy-3,3,3-trifluoro-1-isopropylpropyl)acetamide (300 mg) in dry tetrahydrofuran (10 mL) was added benzyl isocyanate (86 mg). After 20 h triethyl amine (65 mg) and benzyl isocyanate (43 mg) were added. After another 18 h the reaction mixture was diluted with ethyl acetate and 10% hydrochloric acid. The organic portion was separated, washed with 10% hydrochloric acid and brine, and evaporated to give a crude oil. The reaction was found to be incomplete. To a solution of the recovered oil in dry tetrahydrofuran (6 mL) was added triethyl amine (80 mg) and benzyl isocyanate (86 mg). The solution was heated under reflux for 6 h and stirred 18 h. The reaction mixture was diluted with ethyl acetate; washed with saturated aqueous sodium bicarbonate, 10% hydrochloric acid and brine; dried; evaporated; and dried under vacuum to give a crude solid. Purification by chromatography, eluting with ethanol:ethyl acetate:dichloromethane (0.25:3:96.75), gave N-(2-tert-butyldimethylsilyloxy-3,3,3-trifluoro-1-isopropylpropyl)-2-[2-oxo-6-phenyl-3-(3-benzylureido)-1,2-dihydro-1-pyridyl]acetamide (295 mg); TLC: R_{f}=0.20, ethanol:ethyl acetate:dichloromethane (0.5:4:95.5); MS: m/z=645(M+1).

The above described acylation using an isocyanate is denoted herein as Acylation Method C.

Example 17.a.: R=4-methoxybenzoyl: Acylation Method A using 4-methoxybenzoyl chloride; chromatography solvent: acetone:dichloromethane (2:98); TLC (of crude product): R_{f}=0.43, methanol:dichloromethane (1:99); MS: m/z=646(m+1).

Example 18.a.: R=(3,4-dimethoxyphenyl)acetyl: Acylation Method A using (3,4-dimethoxyphenyl)acetyl chloride; chromatography solvent: ethanol:ethyl acetate:dichloromethane (0.5:5:95); TLC: R_{f}=0.40, ethanol:ethyl acetate:dichloromethane (0.5:5:95); MS: m/z=690(M+1).

Example 19.a.: R=phenoxycarbonyl: Acylation Method A using phenyl chloroformate; chromatography solvent: ethyl acetate:dichloromethane (3:97); TLC (of crude product): R_{f}=0.96, methanol:dichloromethane (3:97); MS: m/z=632(M+1).

### EXAMPLES 16.b.-19.b.

2-(3-Acylamino-2-oxo-6-phenyl-1,2-dihydro-1-pyridyl)-N-(3,3,3-trifluoro-2-hydroxy-isopropylpropyl)acetamides of formula II having the indicated acyl group R, R⁰ as isopropyl, R⁵ as hydrogen and R⁶ as phenyl were prepared by cleavage of the corresponding silyl ethers described above. The cleavage was carried out using a similar procedure to that described in Example 1.e. for Examples 16.b.-18.b.

Example 16.b.: R=benzylaminocarbonyl: Chromatography solvent: ethanol:ethyl acetate:dichloromethane (1:3:96 then 2:4:94); TLC: R_{f}=0.10, ethanol:ethyl acetate:dichloromethane (2:4:94); MS: m/z=531(M+1).

Example 17.b.: R=4-methoxybenzoyl: Chromatography solvent: methanol:dichloromethane (2:98), the crude product was preadsorbed onto diatomaceous earth and placed atop the column before elution; TLC (of crude product): R_{f}=0.45, methanol:dichloromethane (3:97); MS: m/z=532(m+1).

Example 18.b.: R=(3,4-dimethoxyphenyl)acetyl: TLC: R_{f}=0.41; methanol:dichloromethane (1:20); MS: m/z=576(M+1).

Example 19.b.: R=phenoxycarbonyl: Tetra-n-butylammonium fluoride (1.0M in tetrahydrofuran, 0.34 mL) and glacial acetic acid (0.02 mL) were added to dry tetrahydrofuran (1 mL). A solution of N-(2-tert-butyldimethylsilyloxy-3,3,3-trifluoro-1-isopropylpropyl)-2-(2-oxo-3-phenoxycarbonylamino-6-phenyl-1,2,-dihydro-1-pyridyl)-acetamide in dry tetrahydrofuran (2 mL) was added to the reaction vessel, and the addition syringe was washed out with an additional 2 mL tetrahydrofuran. The reaction mixture was stirred for 3 h; diluted with ethyl acetate (50 mL); washed with water and brine; dried and evaporated to an oil which was purified by flash chromatography, eluting with dichloromethane:ethyl acetate (90:10), to afford 2-(2-oxo-3-phenoxycarbonylamino-6-phenyl-1,2-dihydro-1-pyridyl)-N-(3,3,3-trifluoro-2-hydroxy-1-isopropylpropyl)acetamide as a white solid (0.110 g); TLC: R_{f}=0.30, ethyl acetate:dichloromethane (1:9); MS: m/z=518(M+1).

### EXAMPLE 20

### 2-[3-(4-Hydroxyphenyl)acetylamino-2-oxo-6-phenyl-1,2-dihydro-1-pyridyl]-N-(3,3,3-trifluoro-1-isopropyl-2-oxopropyl)acetamide.

2-[3-(4-Methoxyphenyl)acetylamino-2-oxo-6-phenyl-1,2-dihydro-l-pyridyl]-N-(3,3,3-trifluoro-1-isopropyl-2-oxopropyl-acetamide (65 mg) was suspended in dry dichloromethane (1.5 mL) and cooled in an ice-water bath. To this suspension was added BBr₃ (1M in dichloromethane, 0.4 mL). The mixture was stirred for 5.5 h in the cold bath and at room temperature for 19 h. Additional BBr₃ (0.14 mL) was added after the reaction was found to be incomplete. After 5 h the mixture was diluted with water and extracted with dichloromethane. The combined extracts were washed with water and brine, dried, and evaporated to yield a crude oil which was purified by chromatography, eluting with ethanol:dichloromethane (gradient 1:99 to 2:98), to give the title compound (50 mg); TLC: R_{f}=0.38, ethanol:dichloromethane (3:97); HPLC: t_{R}=5.45, FR=2.0, column A, water:acetonitrile (3:2); MS: m/z=530(M+1).

| Analysis for C₂₇H₂₆F₃N₃O₅·0.1 H₂O: | | | |
|---|---|---|---|
| Calculated: | C, 61.04; | H, 4.97; | N, 7.91 |
| Found: | C, 61.03; | H, 5.16; | N, 7.66 |

### EXAMPLE 21

### 2-[3-Benzyloxycarbonylamino-6-(2-chlorophenyl)-2-oxo-1,2-dihydro-1-pyridyl]-N-(3,3,3-trifluoro-1-isopropyl-2-oxopropyl)acetamide.

Using a similar procedure to that described in Example 1, using chloroform:ethyl acetate (50:1) for elution in the chromatography, 2-[3-benzyloxycarbonylamino-6-(2-chlorophenyl)-2-oxo-1,2-dihydro-1-pyridyl]-N-(3,3,3-trifluoro-2-hydroxy-1-isopropylpropyl)acetamide was oxidized to afford the title compound; TLC: R_{f}=0.16, chloroform:ethyl acetate (50:1); HPLC: t_{R}=7.08 and 8.22, FR=2, column A, acetonitrile:water (1:1); MS: m/z=560(M+1, ³⁵Cl).

| Analysis for C₂₇H₂₅ClF₃N₃O₅: | | | |
|---|---|---|---|
| Calculated: | C, 56.60; | H, 4.58; | N, 7.33 |
| Found: | C, 56.70; | H, 4.73; | N, 6.95 |

The intermediate 2-[3-benzyloxycarbonyl-amino-6-(2-chlorophenyl)-2-oxo-1,2-dihydro-1-pyridyl]-N-(3,3,3-trifluoro-2-hydroxy-1-isopropylpropyl)acetamide may be prepared as follows:

### a. 6-(2-Chlorophenyl)pyrid-2-one-3-carbonitrile.

Using a similar method to that described in Example 1.a., except heating the solution under reflux using an oil bath at 140 °C following the addition of cyanoacetamide and sodium methoxide, and trituration of the initial precipitate with ether, 6-(2-chloro-phenyl)pyrid-2-one-3-carbonitrile was obtained; TLC: R_{f}=0.39, chloroform:methanol (20:1); MS: m/z=231(M+1, ³⁵Cl).

### b. 6-(2-Chlorophenyl)pyrid-2-one-3-carboxylic acid.

Using Hydrolysis Method A, 6-(2-chlorophenyl)pyrid-2-one-3-carbonitrile was converted into 6-(2-chlorophenyl)pyrid-2-one-3-carboxylic acid; TLC: R_{f}=0.37; MS: m/z=250(M+1, ³⁵Cl).

### c. 3-Benzyloxycarbonylamino-6-(2-chlorophenyl)pyrid-2-one.

Using a similar procedure to that described in Example 1.c., 6-(2-chlorophenyl)pyrid-2-one-3-carboxylic acid was converted into 3-benzyloxycarbonylamino-6-(2-chlorophenyl)pyrid-2-one; TLC: R_{f}=0.24, chloroform:ethyl acetate (50:1); MS: m/z=355(M+1, ³⁵Cl).

### d. 2-[3-Benzyloxycarbonylamino-6-(2-chlorophenyl)-2-oxo-1,2-dihydro-1-pyridyl]-N-(2-tert-butyldimethylsilyloxy-3,3,3-trifluoro-1-isopropylpropyl)acetamide.

Using a similar procedure to that described in Example 1.d., but using dichloromethane:hexanes (1:1, then 2:1) for flash chromatography, 3-benzyloxycarbonylamino-6-(2-chlorophenyl)pyrid-2-one was converted into 2-[3-benzyloxycarbonylamino-6-(2-chlorophenyl)-2-oxo-1,2-dihydro-1-pyridyl]-N-(2-tert-butyldimethylsilyloxy-3,3,3-trifluoro-1-isopropylpropyl)acetamide; TLC: R_{f}=0.34, chloroform:ethyl acetate (50:1); MS: m/z=680(M+1, ³⁵Cl).

### e. 2-[3-Benzyloxycarbonylamino-6-(2-chlorophenyl)-2-oxo-1,2-dihydro-1-pyridyl)-N-(3,3,3-trifluoro-2-hydroxy-1-isopropylpropyl)acetamide.

Using a similar procedure to that described in Example 1.e., 2-[3-benzyloxycarbonylamino-6-(2-chlorophenyI)-2-oxo-1,2-dihydro-1-pyridyl]-N-(2-tert-butyldimethylsilyloxy-3,3,3-trifluoro-1-isopropylpropyl)acetamide was converted into 2-[3-benzyloxycarbonylamino-6-(2-chlorophenyl)-2-oxo-1,2-dihydro-1-pyridyl]-N-(3,3,3-trifluoro-2-hydroxy-1-isopropylpropyl)acetamide; TLC: R_{f}=0.42 and 0.45, dichloromethane:methanol (95:5); MS: m/z=566(M+1, ³⁵Cl).

### EXAMPLE 22

### 2-[2-Oxo-6-phenyl-3-(4-pyridylmethoxycarbonylamino)-1,2-dihydro-1-pyridyl]-N-(3,3,3-trifluoro-1-isopropyl-2-oxopropyl)acetamide.

Using a similar method to that described in Example 1, using chloroform:methanol (gradient, 40:1, 30:1, 20:1) for elution in the chromatography, 2-[2-oxo-6-phenyl-3-(4-pyridylmethoxycarbonylamino)-1,2-dihydro-1-pyridyl]-N-(3,3,3-trifluoro-2-hydroxy-1-isopropylpropyl)acetamide was oxidized to afford the title compound; TLC: R_{f}=0.29, chloroform:methanol (20:1); MS: m/z=531(m+1).

| Analysis for C₂₆H₂₅F₃N₄O₅: | | | |
|---|---|---|---|
| Calculated: | C, 58.86; | H, 4.75; | N, 10.56 |
| Found: | C, 58.88; | H, 5.08; | N, 10.11 |

The intermediate 2-[2-oxo-6-phenyl-3-(4-pyridylmethoxycarbonylamino)-1,2-dihydro-1-pyridyl]-N-(3,3,3-trifluoro-2-hydroxy-1-isopropylpropyl)acetamide may be prepared as follows:

### a. 6-(2-Chlorophenyl)pyrid-2-one-3-carbonitrile.

To a 5 liter, 3-necked flask equipped with a mechanical stirrer, thermometer, reflux condenser capped with a nitrogen inlet, and a heating mantle were added o-chloroacetophenone (305.8 g, 1.98 mol), N,N-dimethylformamide dimethyl acetal (707.0 g, 5.93 mol), and acetonitrile (dried over molecular sieves, 3.0 L) at room temperature, giving an orange solution. The mixture was heated gradually to reflux (83 °C) over 1.5 hours, then maintained at reflux for 18 hours. After the dark red solution was cooled to room temperature, the acetonitrile was evaporated, leaving a heavy red oil. The oil was redissolved in toluene (1 L). The toluene was evaporated to afford the crude enamine which was further dried under high vacuum overnight to afford 2'-chloro-3-dimethylaminopropenophenone (422 g, quantitative) which was used without further purification.

To a 12-liter, 3-necked flask equipped with a mechanical stirrer, a Claisen adapter holding a thermometer and a reflux condenser capped with a nitrogen inlet, and a heating mantle were added the crude enamine (422 g, 1.98 mol) and N,N-dimethylformamide (dried over molecular sieves, 4.0 L), giving a reddish-brown solution. Cyanoacetamide (189.2 g, 2.25 mol) was added as a dry solid and washed down with N,N-dimethylformamide (500 mL). Lastly, sodium methoxide (235.1 g, 4.35 mol) was added as a dry solid and washed down with N,N-dimethylformamide (500 mL). The mixture was heated gradually over 4 hours to 130 °C, then maintained at 135-140 °C for 16 hours. The effluent line from the nitrogen bubbler was trapped through a solution of 3N HCl (2 L). The cooled reaction mixture was evaporated under pump vacuum (bath temperature 50 °C) until approximately 4 liters of N,N-dimethylformamide was removed. The residue was poured into ice/water (6 L) with vigorous stirring. The pH was adjusted to pH 5 by portionwise addition of concentrated hydrochloric acid (300 mL). This produced a suspension of reddish-orange solid. The solid was collected by suction filtration, washed with cold water (2 x 1.5 L) followed by ether (3 x 500 mL), leaving a pinkish-tan powder which was dried in the vacuum oven at 60 °C to afford 6-(2-chlorophenyl)pyrid-2-one-3-carbonitrile (205.5 g, 44.3%); mp 242-245 °C (dec).

### b. 6-(2-Chlorophenyl)pyrid-2-one-3-carboxylic acid.

To a 5 liter, 3-necked flask equipped with a mechanical stirrer, thermometer, reflux condenser capped with a nitrogen inlet, and a heating mantle were added 6-(2-chlorophenyl)pyrid-2-one-3-carbonitrile (205.5 g, 0.891 mol), 48% hydrobromic acid (1500 mL), and glacial acetic acid (1500 mL) at ambient temperature. The golden-brown suspension was heated gradually over a four hour period to gentle reflux (117 °C). During this time, all solids dissolved giving a dark brown solution. The reaction mixture was maintained at reflux for 20 hours, then cooled to room temperature and evaporated until approximately 2 liters of distillate were collected. The remaining suspension was poured into ice/water (5 L) with vigorous stirring, precipitating a tan solid. The solid was collected by suction filtration, washed with cold water (2 x 1.5 L) and dried in the vacuum oven at 60 °C to afford 6-(2-chlorophenyl)pyrid-2-one-3-carboxylic acid (189.7 g, 85.3%); mp 234 °C (dec).

### c. 3-Benzyloxycarbonylamino-6-(2-chlorophenyl)pyrid-2-one.

To a 5 liter, 3-necked flask equipped with a mechanical stirrer, a Claisen adaptor holding a thermometer and a reflux condenser capped with a nitrogen inlet, and a heating mantle were added 6-(2-chlorophenyl)pyrid-2-one-3-carboxylic acid (189.7 g, 0.760 mol) and dioxane (dried over molecular sieves, 3.0 L) at ambient temperature, giving a tan suspension. Triethylamine (92.3 g, 0.910 mol) was added in one portion and caused the solids to dissolve. Diphenylphosphoryl azide (232.0 g, 0.843 mol) was added in one portion and washed down with dioxane (100 mL). The reaction mixture was heated gradually over one hour to reflux (103 °C). At approximately 70 °C, nitrogen evolution began as the intermediate acylazide began to decompose. The reaction mixture was heated at reflux for two hours (until nitrogen evolution ceased) and then was cooled to 90 °C. Benzyl alcohol (dried over molecular sieves, 169.0 g, 1.563 mol) was added in one portion. The reaction mixture was heated at reflux for 45 hours. After this time, the reaction was checked by TLC using two different solvent systems: A. Dichloromethane:methanol:acetic acid (95:5:trace), R_{f}(starting acid)=0.55, R_{f}(benzyl alcohol)=0.80, R_{f}(product)=0.80; and B. Dichloromethane:ethyl acetate (9:1), R_{f}(starting acid)=0-0.15, R_{f}(benzyl alcohol)=0.6, R_{f}(product)=0.4. The reaction appeared complete by TLC; so the mixture was cooled to ambient temperature and stirred overnight. During this time, a crop of tan crystals precipitated. The material was collected by suction filtration, washed with dioxane (200 mL) and ether (2 x 200 mL), then dried to afford crude product (148.0 g). The filtrate was evaporated and the residue was redissolved in methylene chloride (4 L), and washed with aqueous sodium bicarbonate solution (2 x 800 mL) and brine (1 L), dried (MgSO₄), and evaporated leaving a brown semisolid which was triturated with ether and filtered to afford a second crop of crude product (72.5 g). Both fractions of material were shown by NMR to be contaminated with benzyl alcohol and triethylamine hydrochloride. The combined crude material (220.5 g) was stirred for five hours in a mixture of methylene chloride (2.5 L) and 1N hydrochloric acid (1 L), then filtered. The solid was washed with water (3 x 500 mL) and ether (2 x 300 mL) to afford pure product (158.0 g). The organic phases were separated from the filtrates and evaporated. The residual solid was pressure filtered through a silica gel plug (eluent: dichloromethane:ethyl acetae, 3:1) to afford a second fraction of pure product (26.5 g). Thus was obtained 3-benzyloxycarbonylamino-6-(2-chlorophenyl)pyrid-2-one (184.5 g, 68.4%); mp 213-215 °C.

### d. 2-[3-Benzyloxycarbonylamino-6-(2-chlorophenyl)-2-oxo-1,2-dihydro-1-pyridyl]-N-(2-tert-butyldimethylsilyloxy-3,3,3-trifluoro-1-isopropylpropyl)acetamide.

To a 5 liter, 3-necked flask equipped with a mechanical stirrer, thermometer, and a nitrogen inlet were added 3-benzyloxycarbonylamino-6-(2-chlorophenyl)pyrid-2-one (158.0 g, 0.445 mol) and N,N-dimethyformamide (dried over molecular sieves, 3.0 L) giving a tan suspension. Sodium hydride (60% mineral oil dispersion, 19.6 g, 0.490 mol) was added in one portion at ambient temperature. The reaction mixture was stirred at room temperature for 1.5 h, gradually becoming a clear amber solution; then it was was cooled with an ice/water bath to 5 °C. N-(2-tert-butyldimethylsilyloxy-3,3,3-trifluoro-1-isopropylpropyl)-2-iodoacetamide (222.5 g, 0.490 mol) was added in one portion as a dry solid and washed down with N,N-dimethylformamide (200 mL). The reaction flask was packed in ice and the mixture stirred over the weekend (50 h), gradually warming to room temperature. For analysis of the reaction mixture an aliquot was partitioned between 1N hydrochloric acid and ethyl acetate for TLC: dichloromethane:ethyl acetate (95:5), R_{f}(starting pyridone)=0.15, R_{f}(N-alkyated pyridone)=0.70, R_{f}(0-alkylated pyridone)=0.75. The reaction mixture was treated with water (100 mL) and acetic acid (25 mL) to neutralize any excess sodium hydride; then it was evaporated under pump vacuum at a bath temperature of 35 °C to remove most of the N,N-dimethylformamide. The residue was diluted with water (4 L) yielding a gummy precipitate. The aqueous mixture was extracted with ethyl acetate (1 L, followed by 4 x 500 mL). The combined extracts were washed successively with 1N hydrochloric acid (2 x 500 mL), saturated sodium bicarbonate solution (2 x 500 mL), and brine (500 mL); dried (MgSO₄), and evaporated to yield a thick amber syrup which was then pumped down under high vacuum. The crude syrup was redissolved in a mixture of hexane (1000 mL) and ether (200 mL) and seeded with authentic product. Crystallization ensued, and the mixture was stirred periodically with a spatula to complete the crystallization. The mixture was allowed to stand overnight at room temperature before collecting the crystals. The crystals were suction filtered, washed with ether/hexane (1:9) (3 x 200 mL) then dried in the vacuum oven at 50 °C to yield the N-alkylated product 2-[3-benzyloxycarbonylamino-6-(2-chlorophenyl)-2-oxo-1,2-dihydro-1-pyridyl]-N-(2-tert-butyldimethylsilyloxy-3,3,3-trifluoro-1-isopropylpropyl)acetamide as off-white crystals (193.5 g, 63.9%); mp 139-141 °C.

The filtrates were evaporated to an oil (138 g). This material was purified by column chromatography using gradient elution with ethyl acetate (0%-10%) in methylene chloride to afford a small second fraction of pure N-alkylated product (15.2 g, 5.0%). Also isolated was a fraction of the 0-alkylated isomeric product (77.4 g, 25.5%) as a heavy yellow syrup.

### e. 2-(3-Amino-2-oxo-6-phenyl-1,2-dihydro-1-pyridyl)-N-(2-tert-butyldimethylsilyloxy-3,3,3-trifluoro-1-isopropylpropyl)acetamide.

2-[3-Benzyloxycarbonylamino-6-(2-chlorophenyl)-2-oxo-1,2-dihydro-1-pyridyl]-N-(2-tert-butyldimethylsilyloxy-3,3,3-trifiuoro-1-isopropylpropyl)acetamide (100.0 g, 0.147 mol), 10% (w/w) palladium on carbon catalyst (10.0 g), and absolute ethanol (1200 mL), were charged under a nitrogen blanket into a 2-L stainless steel hydrogenation bottle. The mixture was shaken under a hydrogen atmosphere (3.4 bar) for 6 hours. Hydrogen uptake stopped after approximately two hours. The reaction mixture was checked by TLC, eluting with dichloromethane:methanol (97:3) which showed all of the starting material (R_{f}=0.85) to be consumed, essentially a single spot for the intermediate 3-amino-6-(2-chlorophenyl) product (R_{f}=0.55), and a trace spot for the final 3-amino-6-phenyl reduction product (R_{f}=0.48).

Sodium methoxide (8.10 g, 0.150 mol) was added to the reaction mixture. The hydrogenation bottle was placed back on the shaker, and the hydrogenation continued overnight (18 hours). TLC analysis at this time indicated a single spot (R_{f}=0.50). The reaction mixture was suction filtered through a pad of diatomaceous earth to remove the catalyst. The catalyst was washed thoroughly with ethanol (4 x 200 mL). The filtrate was evaporated to dryness, leaving a quantitative yield of the crude product.

After a total of 208.7 g of starting material was hydrogenolyzed in this manner, the combined crude product (160 g) was redissolved in methylene chloride:ethyl acetate (1:1) (5 L), washed with water (2 x 800 mL) and brine (800 mL), dried (MgSO₄), and evaporated to afford a glossy, off-white solid which was triturated with hexane:ether (4:1, 500 mL) to provide 2-(3-amino-2-oxo-6-phenyl-1,2-dihydro-1-pyridyl)-N-(2-tert-butyldimethylsilyloxy-3,3,3-trifluoro-1-isopropyl-propyl)acetamide as a white glossy solid (144.8 g, 92.2%); mp 146-148 °C.

### f. N-(2-tert-Butyldimethylsilyloxy-3,3,3-trifluoro-1-isopropylpropyl)-2-[2-oxo-6-phenyl-3-(4-pyridylmethoxycarbonylamino)-1,2-dihydro-1-pyridyl]acetamide.

To a solution of 2-(3-amino-2-oxo-6-phenyl-1,2-dihydro-1-pyridyl)-N-(2-tert-butyldimethylsilyloxy-3,3,3-trifluoro-1-isopropylpropyl)acetamide (1.00 g) in dry methylene chloride (17 mL) was added dry triethylamine (1.91 mL). The resultant solution was cooled to 0 °C and was treated with a solution of triphosgene (288 mg) in methylene chloride (3 mL) over 5 min. The addition syringe was washed with 2 mL fresh methylene chloride which was then added to the reaction mixture. The reaction mixture was stirred at 0 °C for 45 min, at which time 4-pyridylcarbinol (688 mg) was added as a solid. Stirring was continued 1 h at 0 °C, then overnight at room temperature. The mixture was diluted with methylene chloride, washed (saturated sodium bicarbonate solution), dried and evaporated to a brown foam. Purification by flash chromatography, eluting with methylene chloride:methanol (50:1) gave N-(2-tert-butyldimethylsilyloxy-3,3,3-trifluoro-1-isopropylpropyl)-2-[2-oxo-6-phenyl-3-(4-pyridylmethoxycarbonylamino)-1,2-dihydro-1-pyridyl]acetamide as a yellow-brown solid (1.18 g); TLC: R_{f}=0.36, eluted twice with dichloromethane:methanol (50:1); MS: m/z=647(M+1).

The above described acylation, using triphosgene, triethylamine and an alcohol of formula A.OH or an amine of formula A.NH₂, is denoted herein as Acylation Method D.

### g. 2-[2-Oxo-6-phenyl-3-(4-pyridylmethoxycarbonylamino)-1,2-dihydro-1-pyridyl]-N-(3,3,3-trifluoro-2-hydroxy-1-isopropylpropyl)acetamide.

Using a similar procedure to that described in Example 1.e., purifying the crude product by trituration with ethyl acetate, N-(2-tert-butyldimethylsilyloxy-3,3,3-trifluoro-1-isopropylpropyl)-2-[2-oxo-6-phenyl-3-(4-pyridylmethoxycarbonylamino)-1,2-dihydro-1-pyridyl]acetamide was converted into 2-[2-oxo-6-phenyl-3-(4-pyridylmethoxycarbonylamino)-1,2-dihydro-1-pyridyl]-N-(3,3,3-trifluoro-2-hydroxy-1-isopropylpropyl)acetamide; TLC: R_{f}=0.23, chloroform:methanol (20:1); ms: m/z=533(M+1).

### EXAMPLES 23-34

Using similar procedures to that described in Example 1, the following compounds of formula I wherein R⁰ is isopropyl, R is the indicated acyl group, R⁵ is hydrogen and R⁶ is phenyl were prepared by oxidation of the corresponding alcohols of formula II.

The corresponding alcohols of formula II for Examples 23-34 were prepared as follows:

### Examples 23.a.-34.a.

2-(3-acylamino-2-oxo-6-phenyl-1,2-dihydro-1-pyridyl)-N-(2-tert-butyldimethylsilyloxy-3,3,3-trifluoro-1-isopropylpropyl)acetamides having the indicated acyl group R were prepared from 2-(3-amino-2-oxo-6-phenyl-1,2-dihydro-1-pyridyl)-N-(2-tert-butyldimethylsilyloxy-3,3,3-trifluoro-1-isopropylpropyl)acetamide using triphosgene, triethylamine and an alcohol of formula A.OH or an amine of formula A.NH₂ by Acylation Method D, as described in Example 22.f., except as otherwise noted. Certain of the requisite pyridylcarbinols of formula A.OH which were not commerically available were prepared by the method of Katz et al. (R. B. Katz, J. Mistry, and M. B. Mitchell, Synthetic Communications (1989) 19, 317) with the following modified work up: The crude reation mixture was evaporated and the residue partitioned between ethyl acetate and water. The pH was adjusted to 7 by addition of saturated aqueous sodium bicarbonate. The phases were separated and the aqueous further extracted with ethyl acetate. The total organic phase was dried (MgSO₄), evaporated and purified as described for each individual alcohol.

### Examples 23.b.-34.b.

2-(3-acylamino-2-oxo-6-phenyl-1,2-dihydro-1-pyridyl)-N-(3,3,3-trifluoro-2-hydroxy-1-isopropylpropyl)acetamides of formula II having the corresponding acyl group R, R⁰ as isopropyl, R⁵ as hydrogen and R⁶ as phenyl were prepared by cleavage of the corresponding silyl ethers described above using a similar procedure to that described in Example 1.e., except as noted.

Example 23: R=3-pyridylmethoxycarbonyl: Chromatography solvent: dichloromethane:ethyl acetate (first column, gradient 97:3, 95:5; second column, gradient 100:0, 50:50, 30:70), then trituration with petroleum ether; TLC: R_{f}=0.33, dichloromethane:methanol (95:5); HPLC: t_{R}=6.15, FR=0.5, column A, water:acetonitrile (1:1); MS: m/z=531(M+1).

| Analysis for C₂₆H₂₅F₃N₄O₅: | | | |
|---|---|---|---|
| Calculated: | C, 58.87; | H, 4.75; | N, 10.56 |
| Found: | C, 58.51; | H, 4.98; | N, 10.58 |

Example 23.a.: Chromatography solvent: dichloromethane:methanol (gradient, 100:0 to 97:3); TLC: R_{f}=0.28, dichloromethane:methanol (95:5); MS: m/z=647(M+1).

Example 23.b.: Not chromatographed, but recrystallized from boiling methanol; TLC: R_{f}=0.60, eluted twice, first dichloromethane:methanol (95:5), then dichloromethane:ethyl acetate (6:4); MS: m/z=533(M+1).

Example 24: R=2-pyridylmethoxycarbonyl: Chromatography solvent: chloroform:methanol (40:1); TLC: R_{f}=0.30; chloroform:methanol (20:1); HPLC: t_{R}=6.73, FR=1, column A, water:acetonitrile (1:1); MS: m/z=531(M+1).

| Analysis for C₂₆H₂₅F₃N₄O₅·0.1 H₂O: | | | |
|---|---|---|---|
| Calculated: | C, 58.67; | H, 4.77; | N, 10.53 |
| Found: | C, 58.42; | H, 4.78; | N, 10.51 |

Example 24.a.: Chromatography solvent: dichloromethane:methanol (50:1); TLC: R_{f}=0.28, chloroform:methanol (50:1); MS: m/z=647(M+1).

Example 24.b.: Not chromatographed, but triturated with ethyl acetate; TLC: R_{f}=0.24, chloroform:methanol (20:1); MS: m/z=533(M+1).

Example 25: R=4-methoxypyrid-2-ylmethoxycarbonyl: Chromatography solvent: dichloromethane:methanol (gradient, 95.5:0.5 to 97.0:3.0); TLC: R_{f}=0.35, dichloromethane:methanol (95:5); NMR: 0.77-0.91 (m,6), 2.1-2.3 (m,1), 3.88 (s,3), 4.07-4.66 (m,3), 5.18 (s,2), 6.20-6.25 (m,1), 6.93 (d,1), 7.17 (d,1), 7.34-7.50 (m,5), 7.93 (d,1, J=8.7), 8.36 (d,1, J=5.7), 8.76 (d,1, J=6.9), 8.84 (d,1); MS: m/z=561(M+1).

| Analysis for C₂₇H₂₇F₃N₄O₆·1.0 H₂O: | | | |
|---|---|---|---|
| Calculated: | C, 56.05; | H, 5.05; | N, 9.68 |
| Found: | C, 56.03; | H, 4.90; | N, 9.58 |

Example 25.a.: Chromatography solvent: dichloromethane:methanol (gradient, 99.5:0.5 to 97:3); TLC: R_{f}=0.30, dichloromethane:methanol (98:2); MS: m/z=677(M+1).

4-Methoxypyrid-2-ylcarbinol was prepared by the method of Katz, et al. and purified by chromatography, eluting with dichloromethane:methanol (95:5); TLC: R_{f}=0.45, dichloromethane:methanol (9:1); 300 MHz NMR: 3.82 (s,3), 4.50 (d,2), 5.40 (t,1), 6.81 (dd,1), 7.00 (d,1), 8.28 (d,1); MS: m/z=140(M+1).

Example 25.b.: Isolated and used without further purification; TLC: R_{f}=0.25, dichloromethane:methanol (95:5); MS: m/z=563(M+1).

Example 26: R=2,6-dimethylpyrid-4-ylmethoxycarbonyl: Omitting chromatography, but triturating with dichloromethane to afford a white solid; TLC: R_{f}=0.40, dichloromethane:methanol (95:5); 300 MHz NMR: 0.77-0.90 (m,6), 2.07-2.33 (m,1), 2.41 (s,6), 4.07-4.67 (m,3), 5.14 (s,2), 6.19-6.22 (m,1), 6.90 (s,2), 7.37-7.53 (m,5), 7.70 (d,1, J=10.1), 7.89-7.93 (dd,1), 8.73-8.76 (m,1); MS: m/z=559(M+1).

| Analysis for C₂₈H₂₉F₃N₄O₅·1.2 H₂O: | | | |
|---|---|---|---|
| Calculated: | C, 57.96; | H, 5.45; | N, 9.65 |
| Found: | C, 57.97; | H, 5.24; | N. 9.60 |

Example 26.a.: Chromatography solvent: dichloromethane:methanol (gradient, 99.5:0.5 to 97:3); TLC: R_{f}=0.35, dichloromethane:methanol (98:2); MS: m/z=675(M+1).

2,6-Dimethylpyrid-4-ylcarbinol was prepared by the method of Katz et al. and purified by trituration with diethyl ether, TLC: R_{f}=0.20; dichloromethane:methanol (96:4); 300 MHz NMR: 2.38 (s,6), 4.44 (d,2), 5.30 (t,1), 6.94 (s,1); MS: m/z=138(M+1).

Example 26.b.: Chromatography solvent: dichloromethane:methanol (gradient, 99.5:0.5 to 97.5:2.5); TLC: R_{f}=0.40, dichloromethane:methanol (95:5); 300 MHz NMR: 0.80-0.90 (dd,6), 1.67-1.90 (m,1), 2.41 (s,6), 3.83 (t,1), 1.09 (q,1), 4.30-4.63 (m,2), 5.14 (s,2), 6.20 (d,1, J=7.6), 6.49 (d,1, J=6.7), 7.10 (s,2), 7.39-7.43 (m,5), 7.84-7.92 (m,2), 8.76 (s,1); MS: m/z=561(M+1).

Example 27: R=4-cyanopyrid-2-ylmethoxycarbonyl: Omitting the chromatography, but triturating with dichloromethane:methanol (95:5) to afford a white solid; TLC: R_{f}=0.50, dichloromethane:methanol (95:5); 300 MHz NMR: 0.82-0.95 (m,6), 2.13-2.40 (m,1), 4.09-4.67 (m,3), 5.33 (s,2), 6.23-6.28 (m,1), 7.38-7.51 (m,5), 7.75 (d,1, J=10.6), 7.86 (d,1), 7.99 (d,1, J=7.6), 8.2 (d,1), 8.86 (d,1), 9.07 (s,1); MS: m/z=556(M+1).

| Analysis for C₂₇H₂₄F₃N₅O₅·1.0 H₂O: | | | |
|---|---|---|---|
| Calculated: | C, 56.54; | H, 4.56; | N, 12.21 |
| Found: | C, 56.40; | H, 4.52; | N, 12.05 |

Example 27.a.: Chromatography solvent: dichloromethane:methanol (gradient, 99.5:0.5 to 97:3); TLC: R_{f}=0.65, dichloromethane:methanol; (95:5) MS: m/z=672(M+1).

4-Cyanopyrid-2-ylcarbinol was prepared by the method of Katz et al. and purified by chromatography, eluting with dichloromethane:methanol (95:5); TLC: R_{f}=0.20, dichloromethane:methanol (96:4); NMR: 4.62 (d,2), 5.65 (t,2), 7.73 (d,1), 7.80 (s,1), 8.75 (d,1); MS: m/z=135(M+1).

Example 27.b.: Chromatography solvent:

dichloromethane:methanol (gradient, 99.5:0.5 to 95:5); TLC: R_{f}=0.50, dichloromethane:methanol (95:5); 300 MHz NMR: 0.83 (d,3), 0.90 (d,3), 1.67-1.87 (m,1), 3.83 (t,1), 4:10 (q,1), 4.30-4.63 (m,2), 5.29 (s,2), 6.21 (d,1, J=7.7), 6.50 (d,1, J=6.9), 7.37-7.47 (m,5), 7.81-7.96 (m,3), 8.19 (s,1), 8.82 (d,1), 9.04 (s,1); MS: m/z=558(M+1).

Example 28: R=4-methylpyrid-2-ylmethoxycarbonyl: Chromatography solvent: dichloromethane:methanol (98:2), using three successive columns to afford a pale yellow solid; TLC: R_{f}=0.10; dichloromethane:methanol (96:4); 300 MHz NMR: 0.78-0.91 (m,6), 2.22 (broad s,1), 2.34 (s,3), 4.03-4.66 (m,3), 5.20 (s,2), 6.22 (d,1), 7.16 (d,1), 7.37-7.48 (m,5), 7.93 (d,1), 8.40 (d,1), 8.72 (s,1); MS: m/z=545(M+1).

| Analysis for C₂₇H₂₇F₃N₄O₅·1.5 H₂O: | | | |
|---|---|---|---|
| Calculated: | C, 56.74; | H, 5.29; | N, 9.80 |
| Found: | C, 56.60; | H, 5.10; | N, 9.59 |

Example 28.a.: Chromatography solvent: dichloromethane:methanol (gradient, 97.5:2.5 to 95:5); TLC: R_{f}=0.10, dichloromethane:methanol (99:1); MS: m/z=661(M+1).

4-Methylpyrid-2-ylcarbinol was prepared by the method of Katz, et al. and purified by chromatography, eluting with dichloromethane:methanol (95:5); TLC: R_{f}=0.15; dichloromethane:methanol (96:4); 300 MHz NMR: 2.31 (s,3), 4.51 (d,2), 5.35 (t,1), 7.07 (d,1), 7.29 (s,1), 8.31 (d,1); MS: m/z=124(M+1).

Example 28.b.: Isolated and used without further purification; TLC: R_{f}=0.25, dichloromethane:methanol (96.4); 300 MHz NMR: 0.86 (d,3), 0.93 (d,3), 1.73-1.88 (m,1), 2.38 (s,3), 3.86 (m,1), 4.14 (m,1), 4.33 (broad d,1), 4.60 (broad d,1), 5.24 (s,2), 6.25 (d,1), 6.56 (d,1), 7.20 (d,1), 7.35-7.57 (m,6), 7.94 (d,1), 7.97 (d,1), 8.75 (s,1); MS: m/z=547(M+1).

Example 29: R=2-(2-pyridyl)ethylaminocarbonyl: Chromatography solvent: dichloromethane:methanol (gradient, 99.5:0.5 to 97:3); TLC: R_{f}=0.35, dichloromethane:methanol (95:5); 300 MHz NMR: 0.78-0.90 (m,6), 2.11-2.30 (m,1), 2.88 (t,2), 3.47 (q, 1), 4.03-4.67 (m,3), 6.12-6.17 (m,1), 7.13-7.46 (m,8), 7.68-7.75 (m,1), 8.08 (dd,1), 8.35 (s,1), 8.50-8.52 (m,1), 8.73 (d,1, J=7.1); MS: m/z=544(M+1).

| Analysis for C₂₇H₂₈F₃N₅O₄·0.3 H₂O: | | | |
|---|---|---|---|
| Calculated: | C, 59.07; | H, 5.25; | N, 12.75 |
| Found: | C, 58.98; | H, 5.23; | N, 12.65 |

Example 29.a.: Chromatography solvent: dichloromethane:methanol (gradient, 99.5:0.5 to 97:3); TLC: R_{f}=0.30, dichloromethane:methanol (95:5); 300 MHz NMR: 0.78-0.90 (m,6), 2.11-2.30 (m,1), 2.88 (t,2), 3.47 (q,1), 4.03-4.67 (m,3), 6.12-6.17 (m,1), 7.13-7.46 (m,8), 7.68-7.75 (m,1), 8.08 (dd,1), 8.35 (s,1), 8.50-8.52 (m,1), 8.73 (d,1, J=7.1); MS: m/z=660(M+1).

Example 29.b.: Chromatography solvent: dichloromethane:methanol (gradient, 99:1 to 95:5); TLC: R_{f}=0.50, dichloromethane:methanol (9:1); 300 MHz NMR: 0.81 (d,3, J=6.7), 0.88 (d,3, J=6.7), 1.63-1.83 (m,1), 2.97 (t,2), 3.4-3.57 (d,1), 3.82 (t,1), 4.00-4.17 (m,1), 4.20-4.57 (m,2), 6.12 (d,1, J=7.7), 6.46 (d,1, J=6.8), 7.10-7.53 (m,5), 7.67-7.90 (m,2), 8.05 (d,1, J=7.7), 8.35 (s,1), 8.50 (s,1); MS: m/z=546(M+1).

Example 30: R=2-pyridylmethylaminocarbonyl: Chromatography solvent: dichloromethane:ethyl acetate:methanol (gradient 100:0:0 to 30:69:1); TLC: R_{f}=0.28, dichloromethane:methanol (95:5); MS: m/z=530(M+1).

| Analysis for C₂₆H₂₆F₃N₅O₄: | | | |
|---|---|---|---|
| Calculated: | C, 59.98; | H, 4.95; | N, 13.23 |
| Found: | C, 59.22; | H, 5.32; | N, 12.87 |

Example 30.a.: Except the reaction mixture was worked up after five hours; chromatography solvent: dichloromethane:ethyl acetate (gradient, 100:0 then 1:1); TLC: R_{f}=0.18, dichloromethane:methanol (95:5); MS: m/z=646(M+1).

Example 30.b.: Isolated and used without further purification; TLC: R_{f}=0.19, dichloromethane:methanol (95:5); MS: m/z=532(M+1).

Example 31: R=4-pyridylaminocarbonyl: Chromatography solvent: dichloromethane:methanol (95:5) after preadsorption onto silica gel and packing in dichloromethane; TLC: R_{f}=0.09, dichloromethane:methanol (95:5); HPLC: t_{R}=26.60, FR=1.0, column A, water:acetonitrile (1:1); MS: m/z=516(M+1).

| Analysis for C₂₅H₂₄F₃N₅O₄·1.7 H₂O: | | | |
|---|---|---|---|
| Calculated: | C, 54.98; | H, 5.06; | N, 12.82 |
| Found: | C, 55.27; | H, 4.88; | N, 12.39 |

Example 31.a.: Except the reaction mixture was stirred overnight, then heated under reflux for 3 hours, then diluted with ethyl acetate, resulting in the precipitation of a solid (triethylamine hydrochloride) which was filtered and washed with ethyl acetate. The ethyl acetate solution was washed (saturated sodium bicarbonate solution, then brine) and the aqueous phases back-washed with ethyl acetate. The combined ethyl acetate solution was dried and evaporated before chromatography. Chromatography solvent: dichloromethane:methanol (gradient, 100:0, then 97:3); TLC: R_{f}=0.17, dichloromethane:methanol (95:5); MS: m/z=632(M+1).

Example 31.b.: Except the reaction mixture was diluted with ethyl acetate and washed (saturated sodium bicarbonate solution), which resulted in crystallization. The solid product was filtered and dried under vacuum; TLC: R_{f}=0.27, dichloromethane:methanol (95:5); MS: m/z=518(M+1); 424 (base peak, loss of 4-pyridylamino).

Example 32: R=2-morpholinoethoxycarbonyl: Except that the reaction mixture was partitioned between ethyl acetate and saturated sodium bicarbonate solution before the organic phase was washed (brine), dried, evaporated and purified by chromatography; chromatography solvent: dichloromethane:methanol (gradient, 100:0, 98:2, 97:3, to 95:5), then the product was dried under vacuum; TLC: R_{f}=0.14, dichloromethane:methanol (95:5); HPLC: t_{R}=6.62, FR=0.5, column A, water:acetonitrile (1:1); MS: m/z=553(M+1).

| Analysis for C₂₆H₃₁F₃N₄O₆·0.4 H₂O: | | | |
|---|---|---|---|
| Calculated: | C, 55.79; | H, 5.73; | N, 10.01 |
| Found: | C, 55.75; | H, 5.66; | N, 9.88 |

Example 32.a.: Except using a 5 h reaction time and washing with saturated sodium bicarbonate solution and brine; chromatography solvent: dichloromethane:ethyl acetate:methanol (gradient, 100:0:0, 50:50:0, 45:55:0, 40:60:0, to 36:54:10); TLC: R_{f}=0.11, dichloromethane:ethyl acetate (60:40); MS: m/z=669(M+1).

Example 32.b.: Except using a 1.5 h reaction time, partitioning the reaction mixture between saturated sodium bicarbonate solution and ethyl acetate. The ethyl acetate solution was washed (brine), dried and evaporated to give a product which was used without further purification; TLC: R_{f}=0.22, dichloromethane:methanol (95:5); MS: m/z=555(M+1).

Example 33: R=bis(morpholinomethyl)methoxycarbonyl: Chromatography solvent: dichloromethane:methanol (gradient, 97:3 to 95:5), then trituration with hexane and ether; TLC: R_{f}=0.30, dichloromethane:methanol (95:5); MS: m/z=652(M+1).

| Analysis for C₃₁H₄₀F₃N₅O₇·0.7 H₂O: | | | |
|---|---|---|---|
| Calculated: | C, 56.05; | H, 6.28; | N, 10.54 |
| Found: | C, 55.98; | H, 6.06; | N, 10.25 |

Example 33.a.: Except the alcohol was added to the reaction mixture as a solution in methylene chloride, and the reaction was allowed to proceed overnight before it was diluted with methylene chloride, washed (saturated ammonium chloride solution, water, brine), dried, evaporated, and chromatographed twice; chromatography solvent: first column, dichloromethane:ethyl acetate:methanol (gradient, 60:40:0, 50:50:0, 50:50:1 to 90:0:10); second column, dichloromethane:ethyl acetate (gradient, 60:40, 30:70 to 0:100); TLC: R_{f}=0.25, dichloromethane:methanol (95:5); MS: m/z=768(M+1), 766(M-1) by FAB.

Example 33.b.: Except dried, evaporated and used without further purification; TLC: R_{f}=0.59, chloroform:methanol (9:1); MS: m/z=654(M+1).

Example 34: R=4-tetrahydropyranyloxycarbonyl: Chromatography solvent: first column, dichloromethane:methanol (gradient, 100:0, 98:2, 96:4), second column, dichloromethane:methanol (gradient, 100:0, 97.5:2.5), third column, dichloromethane:ethyl acetate (gradient, 100:0, 80:20); TLC: R_{f}=0.34, dichloromethane:methanol (95:5); HPLC: t_{R}=9.15, FR=1.0, column A, water:acetonitrile (1:1); MS: m/z=524(M+1).

| Analysis for C₂₅H₂₈F₃N₃O₆·0.6 H₂O: | | | |
|---|---|---|---|
| Calculated: | C, 56.20; | H, 5.51; | N, 7.86 |
| Found: | C, 56.26; | H, 5.56; | N, 7.86 |

Example 34.a.: Except the reaction mixture was diluted with methylene chloride, washed (10% aqueous hydrochloric acid, saturated sodium bicarbonate solution, brine), dried, evaporated and chromatographed twice; chromatography solvent: first column, dichloromethane:methanol (gradient, 100:0, 96:4, 95:5), second column, dichloromethane:ethyl acetate:methanol (gradient, 100:00:0, 95:5:0 [95:5]:5 [90:10]:5); TLC: R_{f}=0.40, dichloromethane:methanol (95:5); MS: m/z= 640(M+1).

Example 34.b.: Except the ethyl acetate solution was washed (10% aqueous hydrochloric acid, water, saturated sodium bicarbonate solution, brine), dried and evaporated to give a product which was used without further purification; TLC: R_{f}=0.32, dichloromethane:methanol (95:5); MS: m/z 526(M+1).

### EXAMPLES 35-40

Using similar procedures to that described in Example 1, the following compounds of formula I wherein R⁰ is isopropyl, R is the indicated acyl group, R⁵ is hydrogen and R⁶ is phenyl were prepared by oxidation of the corresponding alcohols of formula II.

Example 35: R=carbamoylmethylaminocarbonyl: After overnight stirring, dilution of the reaction mixture with ethyl acetate afforded a suspension. The solid was filtered, washed (ether), dried under vacuum and triturated with methanol/ether to afford a white solid; TLC: R_{f}=0.29, dichloromethane:methanol (9:1); HPLC: t_{R}=6.09, FR=1, column A, water:acetonitrile (3:2); MS: m/z=496(M+1).

| Analysis for C₂₂H₂₄F₃N₅O₅·1.25 H₂O: | | | |
|---|---|---|---|
| Calculated: | C, 51.01; | H, 5.16; | N, 13.52 |
| Found: | C, 50.73; | H, 5.05; | N, 13.84 |

Example 36: R=2-methoxyethoxycarbonyl: Except using a 2 h reaction time; chromatography solvent: dichloromethane:ethanol (99:1); TLC: R_{f}=0.25, dichloromethane:ethanol (98:2); HPLC: t_{R}=6.09, FR=1, column A, water:acetonitrile (1:1); MS: m/z=498(M+1).

| Analysis for C₂₃H₂₆F₃N₃O₆·0.25 H₂O: | | | |
|---|---|---|---|
| Calculated: | C, 55.03; | H, 5.32; | N, 8.37 |
| Found: | C, 55.03; | H, 5.70; | N, 8.29 |

Example 37: R=acetyl: Chromatography solvent: dichloromethane:ethyl acetate (gradient, 100:0, 60:40, 50:50) then dichloromethane:isopropranol (95:5); TLC: R_{f}=0.09, dichloromethane:methanol (95:5); HPLC: t_{R}=5.47, FR=1, column A, water:acetonitrile (1:1); MS: m/z=438(M+1).

| Analysis for C₂₁H₂₂F₃N₃O₄·0.3 H₂O: | | | |
|---|---|---|---|
| Calculated: | C, 55.01; | H, 5.46; | N, 9.04 |
| Found: | C, 55.02; | H, 5.31; | N, 8.32 |

Example 38: R=cyanoacetyl: Except 45 min reaction time; chromatography solvent: first column, dichloromethane:methanol (98:2), second column, dichloromethane:isopropanol (97:3) then dichloromethane:methanol (9:1), then recrystallized from ethyl acetate/hexane; TLC: R_{f}=0.43, dichloromethane:methanol (96:4); HPLC: t_{R}= 6.26, FR=1, column A, water:acetonitrile (1:1) MS: m/z=463(M+1).

| Analysis for C₂₂H₂₁F₃N₄O₄·0.25 H₂O: | | | |
|---|---|---|---|
| Calculated: | C, 56.59; | H, 4.64; | N, 12.00 |
| Found: | C, 56.64; | H, 4.61; | N, 11.85 |

Example 39: R=6-quinolylmethoxycarbonyl: Except omitting the hydrochloric acid wash; chromatography solvent: chloroform:methanol (first column, gradient 98:2, 97:3, second column, 98.5:1.5); TLC: R_{f}=0.27, chloroform:methanol (98:2); HPLC: t_{R}=6.77, FR=2, column A, water:acetonitrile (1:1); MS: m/z=581(M+1).

| Analysis for C₃₀H₂₇F₃N₄O₅·0.75 H₂O: | | | |
|---|---|---|---|
| Calculated: | C, 60.65; | H, 4.84; | N, 9.43 |
| Found: | C, 60.53; | H, 4.75; | N, 9.35 |

Example 40: R=3-methylisonicotinoyl: Chromatography solvent: chloroform:methanol (gradient, 60:1, 50:1, 40:1, 30:1); TLC: R_{f}=0.17, chloroform:methanol (20:1); HPLC: t_{R}=7.71, FR=1, column A, water:acetonitrile (1:1):

| Analysis for C₂₆H₂₅F₃N₄O₄·0.5 H₂O: | | | |
|---|---|---|---|
| Calculated: | C, 59.65; | H, 5.01; | N, 10.70 |
| Found: | C, 59.72; | H, 5.17; | N, 10.30 |

The corresponding alcohols of formula II for Examples 35-40 were prepared as follows:

### Examples 35.a.-40.a.

2-(3-Acylamino-2-oxo-6-phenyl-1,2-dihydro-1-pyridyl)-N-(2-tert-butyldimethysilyloxy-3,3,3-trifluoro-1-isopropylpropyl)acetamides having the indicated acyl group R were prepared by acylating 2-(3-amino-2-oxo-6-phenyl-1-1,2-dihydro-1-pyridyl)-N-(2-tert-butyldimethylsilyloxy-3,3,3-trifluoro-1-isopropylpropyl)acetamide using the acylation method indicated or described.

Example 35.a.: R=carbamoylmethylaminocarbonyl: Acylation Method D using 1.5 equivalents triphosgene, 10 equivalents triethylamine and 2 equivalents glycinamide hydrochloride. After 20 min, the reaction mixture was diluted with dichloromethane, washed (three times with saturated ammonium chloride solution, then with brine), dried, evaporated, and dried under vacuum to afford the product which was used without further purification; TLC: R_{f}=0.16, dichloromethane:methanol (96:4); MS: m/z=612(M+1).

Example 36.a.: R=2-methoxyethoxycarbonyl: Acylation Method D using 1.5 equivalents triphosgene, 7 equivalent triethylamine and 2.2 equivalents 2-methoxyethanol. After the cold reaction mixture was stirred 45 min, it was allowed to warm to ambient temperature over 3 h before it was diluted with dichloromethane, washed (three times with saturated ammonium chloride solution, then with brine), dried, evaporated and purified by chromatography: Chromatography solvent: dichloromethane:methanol (95:5); TLC: R_{f}=0.23, dichloromethane:ethyl acetate (9:1); MS: m/z=614(M+1).

Example 37.a.: R=acetyl: Acylation Method A using acetyl chloride and using the product isolated without chromatography; TLC R_{f}=0.42, dichloromethane:methanol (95:5); MS: m/z=554(M+1).

Example 38.a.: R=cyanoacetyl. Acylation Method B, as follows: To a solution of the amine (1.05 g) in dry dimethylformamide was added cyanoacetic acid (0.79 g), 1-(3-dimethylaminopropyl)-3-ethylcarbodimide hydrochloride (1.97 g), 1-hydroxybenzotriazole hydrate (1.39 g) and triethylamine (1.71 mL). After stirring overnight, the reaction mixture was diluted with ethyl acetate, washed (three times with 10% aqueous hydrochloric acid, twice with saturated sodium bicarbonate solution, water, and twice with brine), dried and evaporated before purification by chromatography. Chromatography solvent: dichloromethane:ethyl acetate (9:1); TLC: R_{f}=0.18, dichloromethane:ethyl acetate (9:1); MS: m/z=579(M+1).

Example 39.a.: R=6-quinolylmethoxycarbonyl: Acylation Method D using 2.2 equivalents of triethylamine and 2 equivalents of quinolin-6-ylcarbinol; chromatography solvent: chloroform:methanol (98:2); TLC: R_{f}=0.41, chloroform:methanol (97:3); MS: m/z=697(M+1).

Example 40.a.: R=3-methylisonicotinoyl: Acylation Method A as follows: The amine was added to a solution of 3-methylisonicotinoyl chloride hydrochloride (2.5 equivalents) in dry dimethylformamide; then triethylamine (2.2 equivalents) was added to the red solution, resulting in formation of a precipitate. After the reaction mixture was stirred overnight, it was worked up in a manner similar to that described for Acylation Method A. Chromatography solvent: first column, dichloromethane:methanol (gradient, 100:0, 98:2, 95:5), second column, dichloromethane:ethyl acetate (gradient, 100:0, 9:1, 8:2, 65:35) then dichloromethane:methanol (9:1); TLC: R_{f}=0.25, chloroform:methanol (20:1); MS: m/z=631(M+1).

The starting material 3-methylisonicotinoyl chloride hydrochloride was prepared as follows:

### i. 3-Methylisonicotinic acid.

3,4-Lutidine (9.0 g) was dissolved in diphenyl ether (84 mL), under nitrogen, and immersed in a preheated oil bath (155 °C). Selenium dioxide (15 g) was added in 5 portions of 3.0, 2.0, 3.0, 3.5 and 3.5 grams over a 1 hour period. The reaction mixture exothermed vigorously following each addition. The reaction mixture, which turned dark brown, was heated at 155 °C for 0.5 h, then the reflux condenser was replaced with a distillation head. The temperature was raised to 195 °C, but no water was distilled. After 35 min, the reaction mixture was cooled and filtered. The residue on the filter was washed with 200 mL hot water, in 5 portions. The combined aqueous washes were then washed (CHCl₃, 4 x 50 mL) and evaporated. The residue was dried under vacuum at 40 °C to afford 3-methylisonicotinic acid (61%); TLC: R_{f}=0.29, dichloromethane:methanol:acetic acid (9O:7:3); MS: m/z=139(M+1).

### ii. 3-Methylisonicotinoyl chloride hydrochloride.

To a suspension of 3-methylisonicotinic acid (0.676 g) in toluene was added thionyl chloride (0.5 mL), and the reaction mixture was heated under reflux 70 min. The resulting solution was decanted from a gummy residue on the bottom of the reaction flask, evaporated and further dried under high vacuum to afford 3-methylisonicotinoyl chloride hydrochloride which was used for the acylation without further purification.

### Examples 35.b.-40.b.

2-(3-Acylamino-2-oxo-6-phenyl-1,2-dihydro-1-pyridyl)-N-(3,3,3-trifluoro-2-hydroxyisopropylpropyl)acetamides of formula II having the corresponding acyl group R, R⁰ as isopropyl, R⁵ as hydrogen and R⁶ as phenyl were prepared by cleavage of the corresponding silyl ethers described above using a similar procedure to that described in Example 1.e., except as noted.

Example 35.b.: R=carbamoylmethylaminocarbonyl: Except using a 1 h reaction time and the diluted reaction mixture was washed with water and brine, resulting in the formation of a precipitate which was filtered, washed (ether) and dried under vacuum to afford the product as a white solid; TLC: R_{f}=0.28, dichloromethane:methanol (9:1); MS: m/z=498(M+1).

Example 36.b.: R=2-methoxyethoxycarbonyl: Except using a 2 h reaction time and the diluted reaction mixture was washed four times with brine:water (1:1) and once with brine. Chromatography solvent: dichloromethane:methanol (98.5:1.5); TLC: R_{f}=0.30, dichloromethane:methanol (97:3); MS: m/z=500(M+1).

Example 37.b.: R=acetyl: Except the diluted reaction mixture was washed only with water and brine, dried, evaporated and used without further purification; TLC: R_{f}=0.09, dichloromethane:methanol (95.5); MS: m/z=440(M+1).

Example 38.b.: R=cyanoacetyl: Except using a 2.5 h reaction time and the diluted reaction mixture was washed four times with brine:water (1:1) and once with brine. Chromatography solvent: dichloromethane:methanol (98:2); TLC: R_{f}=0.35, dichloromethane:methanol (96:4); MS: m/z=465(M+1).

Example 39.b.: R=6-quinolylmethoxycarbonyl: Except using a 1.5 h reaction time and washing the diluted reaction mixture twice with brine:water (1:1) and once with brine, and purifying the isolated solid by trituration with ethyl acetate/chloroform, rather than chromatography, to afford the product; TLC: R_{f}=0.31, chloroform:methanol (97:3); MS: m/z=583(M+1).

Example 40.b.: R=3-methylisonicotinoyl: Except the diluted reaction mixture was washed with water and brine and the isolated product was used without further purification; TLC: R_{f}=0.12, chloroform:methanol (20:1); MS: m/z=517(M+1).

### EXAMPLES 41-44

Using similar procedures to that described in Example 1, the following compounds of formula I wherein R⁰ is isopropyl, R is the indicated acyl group, R⁵ is hydrogen and R⁶ is phenyl were prepared by oxidation of the corresponding alcohols of formula II:

Example 41: R=4-methoxycarbonylbenzyloxycarbonyl: Except using a 2 h reaction time; chromatography solvent: dichloromethane:ethyl acetate (9:1); TLC: R_{f}=0.29, dichloromethane:ethyl acetate (9:1); NMR: 8.75 (d,1), 7.99 (d,2), 7.92 (d,1), 7.59 (d,2), 7.47-7.34 (m,6), 6.23 (d,1), 5.28 (s,2), 4.63 (t,1), 4.50 (AB q,2) 3.86 (s,3), 2.2-2.0 (m,1), 0.89 (d,3), 0.83 (d,3); MS: m/z=588(M+1).

Example 42: R=ethoxycarbonylmethoxycarbonyl: Except using a 4 h reaction time; chromatography solvent: dichloromethane:methanol (99:1); TLC: R_{f}=0.44, dichloromethane:methanol (98:2); NMR: 7.93 (d,1), 7.50-7.41 (m,5), 6.26 (d,1), 4.74 (s,2), 4.56 (AB q,2), 4.20 (q,2), 4.10 (d,1), 2.2-2.0 (m,1), 0.89 (d,3), 0.83 (d,3); MS: m/z=526(M+1).

Example 43: R=methoxycarbonylmethylaminocarbonyl: Not chromatographed, but isolated and recrystallized from dichloromethane/petroleum ether; TLC: R_{f}=0.39, dichloromethane:methanol (95:5); NMR: 8.03 (d,1), 7.48-7.37 (m,5), 6.18 (d,1), 4.50 (AB q,2), 4.08 (d,1), 3.90 (s,3), 3.81 (s,3), 2.2-2.1 (m,1), 0.87 (d,3), 0.81 (d,3); MS: m/z=511(M+1).

Example 44: R=3-methoxycarbonylbenzyloxycarbonyl: Chromatography solvent: dichloromethane:methanol (gradient, 100:0, 97:3, 95:5); TLC: R_{f}=0.30, dichloromethane:ethyl acetate (9:1); NMR: 8.03 (s,1), 7.93 (t,2), 7.73 (d,1), 7.57 (t,1), 7.49-7.37 (m,5), 6.24 (d,1), 5.26 (s,2), 4.49 (AB q,2), 4.05 (d,1), 3.87 (s,3), 2.2-2.1 (m,1), 0.87 (d,3), 0.79 (d,3); MS: m/z=588(M+1).

The corresponding alcohols of formula II for Examples 41-44 were prepared as follows:

### Examples 41.a.-44.a.

2-(3-Acylamino-2-oxo-6-phenyl-1,2-dihydro-1-pyridyl)-N-(2-tert-butyldimethylsilyloxy-3,3,3-trifluoro-1-isopropylpropyl)acetamides having the indicated acyl group R were prepared from 2-(3-amino-2-oxo-6-phenyl-1,2-dihydro-1-pyridyl)-N-(2-tert-butyldimethylsilyloxy-3,3,3-trifluoro-1-isopropylpropyl)acetamide using triphosgene, triethylamine and an alcohol of formula A.OH or an amine of formula A.NH₂ by Acylation Method D, as described in Example 22.f., except as otherwise noted or described. Certain of the requisite alcohols of formula A.OH which were not commercially available were prepared by the methods described.

Example 41.a.: R=4-methoxycarbonylbenzyloxycarbonyl: Except to a chilled solution of the amine (1.0 g) in dry dichloromethane (18 mL) was added triphosgene (0.29 g) and triethylamine (1.9 mL). After stirring the chilled mixture for 20 min, the alcohol (0.98 g) was added. The reaction mixture was stirred at 5 °C for 2 h before it was allowed to warm to ambient temperature over 4 h, diluted with dichloromethane (100 mL), washed (three times with saturated aqueous ammonium chloride solution and once with brine), dried and evaporated before purification by chromatography. Chromatography solvent: first column, dichloromethane:diethyl ether (97:3), second column, dichloromethane:ethyl acetate (9:1); TLC: R_{f}=0.48, dichloromethane:ethyl acetate (9:1); MS: m/z=704(M+1).

Example 42.a.: R=ethoxycarbonylmethoxycarbonyl: Except the reaction was carried out and worked up in a similar manner to that described in Example 41.a., but allowing the reaction mixture to stir 1.5 h before the cold bath was removed and it was allowed to warm to room temperature over 2 h before work up. Chromatography solvent: dichloromethane:ethyl acetate (9:1); TLC: R_{f}=0.48, dichloromethane:ethyl acetate (9:1); MS: m/z=642(M+1).

Example 43.a.: R=methoxycarbonylmethylaminocarbonyl: Except using a similar procedure to that described in Example 41.a., with a reaction time of 3.5 h; and the product was not chromatographed, but triturated successively with 10% aqueous hydrochloric acid, water and ether before drying under vacuum to afford the product; TLC: R_{f}=0.35, dichloromethane:methanol (95:5); MS: m/z=627(M+1).

Example 44.a.: R=3-methoxycarbonylbenzyloxycarbonyl: Except using a similar procedure to that described in Example 41.a., with a reaction time of 3.5 h. Chromatography solvent:
dichloromethane:ethyl acetate (gradient, 100:0, 95:5, 93:7, 90:10), then rechromatographing the mixed fractions (gradient, 100:0, 95:5, 90:10); TLC: R_{f}=0.11, dichloromethane:ethyl acetate (9:1); MS: m/z=704(M+1).

The methyl 3-hydroxymethylbenzoate for the above procedure was obtained as follows, using literature procedures. (See U.S. Patent 4,130,719; and Yoon et al., J. Org. Chem. (1973) 38(16), 2786-2792.)

To a suspension of 3-methoxycarbonylbenzoic acid (4.68 g, prepared by the method of Kasina and Nematollahi, Tetrahedron Lett. (1978) 1403) in tetrahydrofuran (12.5 mL) at 0 °C was added dropwise borane-tetrahydrofuran complex (1.0 M in tetrahydrofuran, 25 mL) over 40 min. The reaction mixture, which had become a solution, was allowed to warm slowly to room temperature as it was stirred overnight. When examination by TLC after 24 h showed little conversion to the alcohol, the reaction mixture was cooled with an ice bath and the reaction quenched with water (20 mL). The mixture was saturated with potassium carbonate and the phases were separated. The organic phase was washed (saturated potassium carbonate solution) and evaporated. The aqueous phase was extracted with ethyl acetate; and the ethyl acetate solution was used to redissolve the residue from the tetrahydrofuran solution. The resulting ethyl acetate solution was washed (water), dried and evaporated to afford a portion of crude alcohol.

The original aqueous phase was acidified with 10% aqueous hydrochloric acid to pH 2, resulting in precipitation of unreacted acid as a white solid, which was filtered, washed with water and ether, and dried under vacuum to provided recovered starting acid (2.7 g).

To a suspension of the recovered acid (2.7 g) in tetrahydrofuran was added sodium borohydride (0.49 g), resulting in evolution of gas. To the mixture was added dropwise boron trifluoride etherate (2.05 mL) over 5 min, causing further gas evolution. The reaction mixture, which was heated to a gentle reflux by the exothermic reaction, was stirred 4 h, quenched with water (2.5 mL) and evaporated. The residue was partitioned between dichloromethane and water. After the aqueous phase was further extracted with dichloromethane, the combined extracts were washed (brine), dried and evaporated. The residue was partitioned between ethyl acetate and saturated sodium bicarbonate solution; and the organic phase was washed (brine), dried and evaporated to afford crude alcohol which was combined with the material obtained from the borane reduction for purification by chromatography, eluting with dichloromethane:ethyl acetate (gradient, 100:0, 95:5, 91.5:9.5, 91:1, 85:15) to afford methyl 3-hydroxymethylbenzoate; TLC: 0.42, dichloromethane:ethyl acetate (9:1); MS: m/z=167(M+1).

### EXAMPLES 41.b.-44.b.

2-(3-acylamino-2-oxo-6-phenyl-1,2-dihydro-1-pyridyl)-N-(3,3,3-trifluoro-2-hydroxy-1-isopropylpropyl)acetamides of formula II having the indicated acyl group R, R⁰ as isopropyl, R⁵ as hydrogen and R⁶ as phenyl were prepared by cleavage of the corresponding silyl ethers described above using a similar procedure to that described in Example 1.e., except as noted or described.

Example 41.b.: R=4-methoxycarbonylbenzyloxycarbonyl: Except using a 2 h reaction time and the diluted reaction mixture was washed 3 times with brine:water (1:1) and once with brine. The dried solution was evaporated and dried under vacuum to provide a product which was used without further purification; TLC: R_{f}=0.12, dichloromethane:ethyl acetate (9:1); MS: m/z=590(M+1).

Example 42.b.: R=ethoxycarbonylmethoxycarbonyl: Except a buffered deprotection was used. To a solution of the silyl ether (0.452 g) in dry tetrahydrofuran (7 mL) was added acetic acid (0.05 mL) and tetrabutylammonium fluoride (0.70 mL). After the reaction mixture was stirred 45 min (reaction deemed complete), it was diluted with ethyl acetate, washed (water, three times, then brine), dried and evaporated to a residue which was dried under vacuum to give a product which was used without further purification; TLC: R_{f}=0.28, dichloromethane:methanol (97:3); MS: m/z=528 (M+1).

Example 43.b.: R=methoxycarbonylmethylaminocarbonyl: Not chromatographed, but crystallized from ethyl acetate (with cooling for one crop), washed (water, then ether) and dried under vacuum; TLC: R_{f}=0.06, dichloromethane:ethyl acetate (60:40); MS: m/z=513 (M+1).

Example 44.b.: R=3-methoxycarbonylbenzyloxycarbonyl: Isolated and triturated with ether; TLC: R_{f}=0.05, dichloromethane:ethyl acetate (95:5); MS: m/z=590(M+1).

### EXAMPLES 45-48

The following compounds of formula I wherein R⁰ is isopropyl, R is the indicated acyl group which contains a carboxy moiety, R⁵ is hydrogen and R⁶ is phenyl were prepared by hydrolysis of the ester groups of corresponding compounds of formula I in which the acyl group R contains an ester moiety, prepared as described in Examples 41-44, respectively. In each example, the hydrolysis was carried out using lithium hydroxide in aqueous tetrahydrofuran, followed by acidification, as described or indicated.

Example 45: R=4-carboxybenzyloxycarbonyl: To a solution of the starting ester (Example 41, 0.589 g) in dry tetrahydrofuran (8 mL) was added a solution of lithium hydroxide monohydrate (0.094 g) in water (2 mL). The biphasic mixture was rapidly stirred for 3 h. The mixture was diluted with water (5 mL) and the pH adjusted to about 3 with 10% aqueous hydrochloric acid to give a white precipitate. This mixture was extracted with ethyl acetate (3 times); and the combined extracts were washed (water, brine), dried, evaporated, and dried under vacuum. The product contained unhydrolyzed ester and was again subjected to the same reaction conditions for 5.5 h. The reaction mixture was worked-up as before to give a white solid which was crystallized from hot ethyl acetate/hexane to give the title acid as a white solid; TLC: R_{f}=0.41, dichloromethane:methanol:acetic acid (95.5:4:0.5); MS: m/z=574(M+1).

| Analysis for C₂₈H₂₆F₃N₃O₇: | | | |
|---|---|---|---|
| Calculated: | C, 58.65; | H, 4.57; | N, 7.33 |
| Found: | C, 58.87; | H, 4.65; | N, 7.13 |

Example 46: R=carboxymethoxycarbonyl: To a solution of the starting ester (Example 42, 0.586 g) in tetrahydrofuran (8 mL) and water (3 mL) was added lithium hydroxide monohydrate (0.10 g) to give a cloudy, biphasic mixture. After stirring for 45 min, the reaction was deemed complete. The reaction mixture was diluted with water (5 mL), acidified to about pH 3 with 10% aqueous hydrochloric acid, and extracted with ethyl acetate (three times). The combined extracts were washed (brine), dried, and evaporated. The residue was dried under vacuum to give a solid. The impure solid was crystallized from hot ethyl acetate/hexane to give the title product as an off-white powder; TLC: R_{f}=0.26, dichloromethane:methanol:acetic acid (89:10:1); MS: m/z=498(M+1).

| Analysis for C₂₂H₂₂F₃N₃O₇: | | | |
|---|---|---|---|
| Calculated: | C, 53.12; | H, 4.46; | N, 8.45 |
| Found: | C, 53.09; | H, 4.57; | N, 8.51 |

Example 47: R=carboxymethylaminocarbonyl: The ester (Example 43, 0.2 g) was dissolved in aqueous tetrahydrofuran (water:tetrahydrofuran, 20:80) such that the concentration was about 0.1 molar (4 mL); then lithium hydroxide monohydrate (2.2 equivalent) was added and the reaction mixture was stirred until the hydrolysis was deemed complete. The reaction mixture was diluted with water, washed with ether and acidified to about pH 2 with 10% aqueous hydrochloric acid. The resulting precipitate was filtered, washed (water) and dried under vacuum to afford the title product (77%); TLC: R_{f}=0.46, chloroform:methanol:acetic acid (85:10:5); MS: m/z=497(M+1).

| Analysis for C₂₂H₂₃F₃N₄O₆·1.2 H₂O·0.35 CH₃OH: | | | |
|---|---|---|---|
| Calculated: | C, 50.72; | H, 5.10; | N, 10.59 |
| Found: | C, 50.60; | H, 4.72; | N, 10.43 |

Example 48: R=3-carboxybenzyloxycarbonyl: The ester (Example 44) was hydrolyzed using a similar procedure to that of Example 47 except the crude product was purified by chromatography, eluting with dichloromethane:ethyl acetate:acetic acid (73:36:1), followed by redissolving the product in ethyl acetate, filtration, and evaporation to give the title acid as a solid; TLC: R_{f}=0.10, chloroform:methanol (9:1); MS: m/z=574(M+1).

| Analysis for C₂₈H₂₆F₃N₃O₇: | | | |
|---|---|---|---|
| Calculated: | C, 58.64; | H, 4.57; | N, 7.33 |
| Found: | C, 58.95; | H, 4.90; | N, 6.63 |

### EXAMPLE 49

### 2-(3-Amino-2-oxo-6-phenyl-1,2-dihydro-1-pyridyl)-N-(3,3,3-trifluoro1-isopropyl-2-oxopropyl)acetamide.

To a solution of 2-(3-benzyloxycarbonylamino-2-oxo-6-phenyl-1,2-dihydro-1-pyridyl)-N-(3,3,3-trifluoro-1-isopropyl-2-oxopropyl)acetamide (10 g) and anisole (6.6 g) in dichloromethane (100 mL) at 0 °C was added trifluoromethanesulphonic acid (9 mL, 15.3 g) while maintaining the temperature below 2 °C. The reaction mixture was allowed to warm to room temperature in 30 min and kept at room temperature for a further 45 min before saturated aqueous sodium bicarbonate was added slowly to pH 7. Ethyl acetate was added, the phases separated and the aqueous phase extracted further with ethyl acetate. The combined organic extract was washed (brine), dried (MgSO₄) and evaporated. The crude product was purified by trituration with hexane followed by trituration with diethyl ether to give the title product (which can be recrystallized from dichloromethane/hexane) as a white solid (4.85 g); TLC: R_{f}=0.4O, ethyl acetate; 300 MHz NMR: 0.78-0.91 (m,6), 2.07-2.30 (m,1), 4.39 (d,1), 4.49 (d,1), 4.61 (t,1), 5.17 (s,1), 5.98 (d,1), 6.51 (d,1), 7.28-7.41 (m,5), 8.68 (d,1); MS: m/z=396(M+1).

| Analysis for C₁₉H₂₀F₃N₃O₃·0.25 H₂O: | | | |
|---|---|---|---|
| Calculated: | C, 57.07; | H, 5.17; | N, 10.51 |
| Found: | C, 57.25; | H, 5.10; | N, 10.47 |

The benzyloxycarbonyl group can alternatively be removed using the following procedure:

To a solution of 2-(3-benzyloxycarbonylamino-2-oxo-6-phenyl-1,2-dihydro-1-pyridyl)-N-(3,3,3-trifluoro-1-isopropyl-2-oxopropyl)acetamide (22.2 g) in ethanol (500 mL) was added 10% (w/w) palladium on carbon (5.55 g). The mixture was shaken under a hydrogen atmosphere overnight. Catalyst was removed by filtration through diatomaceous earth. The filter pad was washed successively with ethanol and methanol. Concentration of the filtrate gave the crude product as an off-white solid (17.2 g). This material was combined with 0.61 g of product generated from a separate run, dissolved in a minimum volume of methanol, diethylether was added, and the mixture was allowed to stand overnight. The precipitate was collected and washed with ether to give the amine as an off-white solid (13.9 g).

The starting material ketone for the above preparation may be obtained as described in Example 1 or as follows:

### a. 2-(3-Benzyloxycarbonylamino-2-oxo-6-phenyl-1,2-dihydro-1-pyridyl)-N-(2-tert-butyldimethylsilyloxy-3,3,3-trifluoro-1-isopropylpropyl)acetamide.

To a slurry of 2-(3-amino-2-oxo-6-phenyl-1,2-dihydro-1-pyridyl)-N-(2-tert-butyldimethylsilyoxy-3,3,3-trifluoro-1-isopropylpropyl)acetamide (20.0 g) (Example 14.a. or 22.e.) and sodium carbonate (9.53 g) in tetrahydrofuran (200 mL) and dimethylformamide (4 mL) at 0 °C was added benzyl chloroformate (10.2 mL) dropwise in 5 min. After warming to room temperature, the reaction mixture was stirred overnight, then filtered and the filtrates evaporated. Ethyl acetate was added and the solution washed (water, brine). The combined aqueous was back extracted with ethyl acetate and then the combined organic washed (brine) and evaporated. Chromatography, eluting with dichloromethane, gave the title compound as a near colorless oil (20.9 g); TLC: R_{f}=0.60, dichloromethane:ethyl acetate (5:1); 300 MHz NMR: 0.08 (s,3), 0.10 (s,3), 0.82 (d,3), 0.85 (s,9), 0.92 (d,3), 1.70-1.76 (m,1), 3.82 (t,1), 3.70-4.00 (m,2), 4.25 (m,2), 4.60 (broad d,1), 5.19 (s,2), 6.22 (d,1), 7.25-7.51 (m,10), 7.64 (d,1), 7.92 (d,1), 8.54 (s, 1); MS: m/z=646(M+1).

### b. 2-(3-Benzyloxycarbonylamino-2-oxo-6-phenyl-1,2-dihydro-1-pyridyl)-N-(3,3,3-trifluoro-2-hydroxy-1-isopropylpropyl)acetamide.

To a solution of 2-(3-benzyloxycarbonylamino-2-oxo-6-phenyl-1,2-dihydro-1-pyridyl)-N-(2-tert-butyldimethylsilyoxy-3,3,3-trifluoro-1-isopropylpropyl)acetamide (20.5 g) in dry tetrahydrofuran (120 mL) was added acetic acid (2 mL) followed by tetrabutylammonium fluoride (1 M in tetrahydrofuran, 47.6 mL). After stirring for 5 min, the reaction mixture was diluted with ethyl acetate (1 L), washed (three times with water, brine), dried (MgSO₄) and evaporated to give the title product as a white solid (16.1 g) which was used without further purification; TLC: R_{f}=0.20, chloroform:ethyl acetate (20:1); 300 MHz NMR: 0.80 (d,3), 0.88 (d,3), 1.63-1.74 (m,1), 3.8 (t,1), 4.25-4.40 (m,1), 4.48-4.60 (broad d,1), 5.19 (s,2), 6.20 (d,1), 6.49 (d,1), 7.31-7.46 (m,10), 7.86 (d,1), 7.90 (d,1), 8.54 (s,1); MS: 532(M+1).

### c. 2-(3-Benzyloxycarbonylamino-2-oxo-6-phenyl-1,2-dihydro-1-pyridyl)-N-(3,3,3-trifluoro-1-isopropyl-2-oxopropyl)acetamide.

To a solution of 2-(3-benzyloxycarbonylamino-2-oxo-6-phenyl-1,2-dihyro-1-pyridyl)-N-(3,3,3-trifluoro-2-hydroxy-1-isopropylpropyl)acetamide (10.4 g) in dry dimethyl sulfoxide (50 mL) and toluene (50 mL) was added 1-(3-dimethylaminopropyl)-3-ethylcarbodiimide hydrochloride (39 g). To this cooled mixture (3 °C) was added dichloroacetic acid (6 mL, 9.2 g) while maintaining the temperature below 10 °C. The reaction mixture was warmed to room temperature in 30 min, then diluted with ethyl acetate (300 mL) and water (300 mL). The pH was adjusted to 6 with 1N HCl (10 mL), the phases separated and the organic phase washed (water and twice with brine), dried (MgSO₄) and evaporated to give the title product as a white solid (10.4 g) which was used without further purification; TLC: R_{f}=0.45, dichloromethane:ethyl acetate (5:1); 300 MHz NMR: 0.83 (d,3), 0.89 (d,3), 2.05-2.19 (m,1), 4.50 (q,1), 4.63 (t,3), 5.19 (s,2), 6.23 (d,1), 7.30-7.50 (m,10), 7.92 (d,1), 8.56 (s,1), 8.74 (d,1); MS: m/z=530(M+1).

2-(3-Benzyloxycarbonylamino-2-oxo-6-phenyl-1,2-dihydro-1-pyridyl)-N-(3,3,3-trifluoro-2-hydroxy-1-isopropylpropyl)acetamide can alternatively be prepared as follows:

### d. 3-Aza-4-phenylpent-3-enal dimethyl acetal.

Acetophenone (60.6 g) and aminoacetaldehyde dimethyl acetal (78.9 g) were dissolved in toluene (650 mL) in a 1 L round-bottomed flask. A Dean-Stark trap, fitted with a reflux condenser, was attached to the reaction vessel and the solution was brought to reflux. The trap was drained after 17 h, 41 h, and 48 h (30 mL each time). After 65 h, the reaction was cooled and volatiles were evaporated to leave a yellow liquid (103.3 g). Fractional distillation gave two major fractions: fraction 1, 10.5 g (60-126 °C, 20-24 Pa); fraction 2, 78.66 g (126-130 °C, 17-20 Pa). Fraction 1 contained a significant amount of acetophenone and amino acetaldyhyde dimethyl acetal. Fraction 2 contained less than 5% acetophenone and acetal, and was used directly in the next step. The NMR spectrum was obtained from a clean fraction of imine produced in a different run; 300 MHz NMR: 2.20 (s,6), 3.54 (d,2), 4.70 (t,1), 7.38-7.43 (m,3), 7.79-7.82 (m,2).

### e. Dimethyl 4-aza-6,6-dimethoxy-3-phenylhex-2-enylidinemalonate.

A dry, 2 L, 3-necked flask was equipped with a mechanical stirrer, an addition funnel and a Claisen adapter fitted with a thermometer and a nitrogen inlet. To the reaction vessel was added a solution of lithium diisopropylamide (230 mL, 2.0 M in hexane/tetrahydrofuran) and tetrahydrofuran (700 mL). To the cooled (5 °C) solution was added the crude material from Example 49.d. (78.5 g) in tetrahydrofuran (150 mL) over 30 min. The internal temperature was maintained at 5 °C during the addition and for 45 minutes thereafter. A solution of dimethyl methoxymethylenemalonate (70.5 g) in dry tetrahydrofuran (150 mL) was added dropwise over 30 min. The dark amber reaction mixture was allowed to warm to room temperature and was stirred overnight. The mixture was diluted with methylene chloride (2 L) and washed (saturated ammonium chloride). The aqueous washes were back extracted with methylene chloride. The combined methylene chloride layers were washed (brine) and dried (MgSO₄). Evaporation gave the crude diene ester as a red oil (147.6 g). This material was used without further purification. A separate iteration of this procedure provided a clean sample for characterization after chromatography; chromatography solvent: ethyl acetate:chloroform (5:95); TLC: R_{f}=0.32, ethyl acetate:chloroform:methanol (5:95:1); 300 MHz NMR: 3.33 (s,6), 3.48 (s,3), 3.68 (s,3), 4.63 (broad s,1), 6.17 (d,1), 7.33-7.35 (m,3), 7.52-7.54 (m,3), 7.90 (broad s,1); MS: m/z=350(M+1).

### f. 1-(2,2-Dimethoxyethyl)-6-phenylpyrid-2-one-3-carboxylic acid.

A 3 L round-bottomed flask was equipped with a stir bar and fitted with a Claisen adapter holding a thermometer and a nitrogen inlet. The flask was charged with a solution of the product from Example 49.e. in methanol (1.5 L). Sodium methoxide (32.4 g) was added in one portion and caused a mild warming. After 3 h, aqueous sodium hydroxide (750 mL, 10% w/v) was added to the mixture in one portion. The mixture was stirred at room temperature for 2 h, the methanol was evaporated, and the aqueous residue was acidified with hydrochloric acid and extracted with methylene chloride. The extracts were washed (brine), dried (MgSO₄), and evaporated to give a red-brown oil (99.6 g) which partially solidified. This material was used without further purification. A sample of the pyridone, after purification, was characterized; TLC: R_{f}=0.41, methanol:chloroform:acetic acid (1.5:98:0.5); 300 MHz NMR: 3.13 (s,6), 4.14 (d,2), 4.63 (t,1), 6.64 (d,1), 7.51-7.58 (m,5), 8.41 (d,1).

### g. 3-Benzyloxycarbonylamino-2-oxo-6-phenyl-1,2-dihydro-1-pyridyl-acetaldehyde dimethyl acetal.

An oven-dried, 3 L, three-necked flask was equipped with a mechanical stirrer, a thermometer and a reflux condenser capped with a nitrogen inlet. The reaction vessel was charged with a dioxane (1 L) solution of the product from Example 49.f (99.6 g). Diphenylphosphoryl azide (103.9 g) and triethylamine (39.8 g) were each added to the reaction vessel in one portion and washed down with dioxane (50 mL each). The resulting solution was heated at gentle reflux (100 °C) for 1 h. Gas evolution was vigorous at first but then subsided. The reaction mixture was cooled to 70 °C, and benzyl alcohol (38.9 g) was added in one portion along with a dioxane wash (100 mL). The reaction was heated at reflux for 18 h, cooled and evaporated. The residual oil was dissolved in ethyl acetate (1 L) and washed with 1 N hydrochloric acid:brine (1:1), followed by brine. The organic layer was dried (MgSO₄) and evaporated to give the crude mixture (249.5 g). This material was purified by chromatography, with ethyl acetate:dichloromethane as the eluent (gradient, 0:100, 5:95), to yield the amide (43.1 g); TLC: R_{f}=0.49, ethyl acetate:chloroform (5:95); 300 MHz NMR: 3.09 (s,6), 4.02 (d,2), 4.54 (t,1), 5.19 (s,2), 6.19 (d,1), 7.34-7.50 (m,5), 7.89 (d,1), 8.54 (s,1).

### h. 3-Benzyloxycarbonylamino-2-oxo-6-phenyl-1,2-dihydro-1-pyridyl-acetaldehyde.

The product from Example 49.g. (43.1 g) was dissolved in a mixture of tetrahydrofuran (700 mL) and aqueous hydrochloric acid (225 mL 3 N). The mixture was held at reflux under nitrogen for 3.5 h. The mixture was cooled and the tetrahydrofuran was evaporated. The aqueous residue was extracted with methylene chloride, washed (saturated aqueous sodium bicarbonate) and dried (MgSO₄). Evaporation gave the crude product as a tan solid. Trituration with ether (300 mL) gave the aldehyde as a white solid (27.3 g); TLC: R_{f}=0.32, ethyl acetate:dichloromethane (5:95); 300 MHz NMR: 4.66 (s,2), 5.19 (s,2), 6.28 (d,1), 7.32-7.49 (m,10), 7.94 (d,1), 8.61 (s,1), 9.50 (s,1); MS: m/z=363(M+1).

### i. 3-Benzyloxycarbonylamino-2-oxo-6-phenyl-1,2-dihydro-1-pyridyl-acetic acid.

A 2 L, three-necked flask was equipped with a mechanical stirrer, an addition funnel and a Claisen adapter holding a thermometer and a reflux condenser capped with a nitrogen inlet. The flask was charged with a tetrahydrofuran (275 mL) solution of the product from Example 49.h. (40.5 g). The addition of tert-butanol (275 mL) caused precipitation of the aldehyde starting material. The reaction mixture was cooled to 15 °C with an ice-water bath, and 2-methyl-2-butene (250 mL) was added in one portion. A solution of sodium chlorite (80%, 88.5 g) and sodium dihydrogen phosphate monohydrate (108.0 g) in water (400 mL) was added dropwise to the reaction mixture over 45 min. The internal temperature was maintained at 20 °C during the addition. Stirring at room temperature was continued for 2 h. The mixture was partially evaporated to leave an aqueous suspension of white solid. The mixture was diluted with brine and extracted with chloroform. The combined extracts were dried (MgSO₄) and evaporated. The residue was dissolved in diethyl ether and evaporated to give an off-white solid, which was triturated with hexane:diethyl ether (9:1) to give the acid as an off-white solid (43.1 g); TLC: R_{f}=0.20, methanol:dichloromethane (2:98); 300MHz NMR: 4.44 (s,2), 5.19 (s,2), 5.24 (d,1), 7.33-7.51 (m,10), 7.92 (d,1), 8.59 (s,1), 13.07 (broad s,1); MS: m/z=363(M+1). NMR showed that this material was pure but contained diethyl ether, which was not removed by prolonged drying in a vacuum oven.

### j. 2-(3-Benzyloxycarbonylamino-2-oxo-6-phenyl-1,2-dihydro-1-pyridyl)-N-(3,3,3-trifluoro-2-hydroxy-1-isopropylpropyl)acetamide.

To a solution of the acid of Example 49.i. (19.0 g), 1-hydroxybenzotriazole hydrate (13.6 g), 3-amino-1,1,1-trifluoro-4-methyl-2-pentanol hydrochloride (11.5 g) and triethylamine (14.0 mL) in dimethyl-formamide (100 mL) was added 1-(3-dimethylaminopropyl)3-ethyl-carbodiimide hydrochloride (14.5 g). The mixture was stirred overnight diluted with 1 N hydrochloric acid and extracted with ethyl acetate. The organics were washed (saturated sodium bicarbonate), dried, and evaporated to give a white solid. The solid was triturated with ether and dried in a vacuum oven overnight to yield the alcohol (20.85 g). The triturate was concentrated and the residue purified by flash chromatography (dichloromethane:methanol, 99:1) to yield additional alcohol (1.70 g).

The title compound of Example 49 can alternatively be prepared as follows:

To a solution of 2-(3-trifluoroacetylamino-2-oxo-6-phenyl-1,2-dihydro-1-pyridyl)-N-(3,3,3-trifluoro-1-isopropyl-2-oxopropyl)acetamide (9.5 g) in tetrahydrofuran (92 mL) was added water (184 mL) followed by potassium carbonate (13.4 g). This mixture was allowed to stir overnight. A first crop of the amine (4.2 g) was obtained after filtration and washing with diethyl ether. The organic filtrates were evaporated, and the residue recrystallized from dichloromethane:hexane to give a second crop of the amine (2.9 g).

### EXAMPLES 50-55

The following compounds of formula I wherein R⁰ is isopropyl, R is the indicated acyl group, R⁵ is hydrogen and R⁶ is phenyl were prepared by acylation of 2-(3-amino-2-oxo-6-phenyl-1,2-dihydro-1-pyridyl)-N-(3,3,3-trifluoro-1-isopropyl-2-oxopropyl)acetamide using the indicated acylation method, except as otherwise noted or described. Certain starting materials which were not commercially available were prepared by the methods described.

Example 50: R=3-methylpyrid-4-ylmethoxycarbonyl: Acylation Method D using 3-methylpyrid-4-ylcarbinol; chromatography solvent: first column, dichloromethane:methanol (gradient, 100:0, 97:3, 95:5), second column, dichloromethane:ethyl acetate:methanol (gradient, 100:0:0, 60:40:0, 60:39:1), third column, dichloromethane:ethyl acetate (gradient, 100:0, 60:40, 40:60, 0:100); TLC: R_{f}=0.31, dichloromethane:methanol (9:1); MS: m/z=545(M+1).

| Analysis for C₂₇H₂₇F₃N₄O₅·0.9 H₂O: | | | |
|---|---|---|---|
| Calculated: | C, 57.83; | H, 5.18; | N, 9.99 |
| Found: | C, 57.96; | H, 5.13; | N, 9.86 |

The 3-methylpyrid-4-ylcarbinol was prepared as follows:

### a. Ethyl 3-methylisonicotinate.

To a solution prepared by saturating absolute ethanol (25 mL) cooled in an ice bath (ca. 0 °C) with HCl gas was added 3-methylisonicotinic acid (Example 40.a.i.) (5.7 g). The yellow suspension was heated under reflux for 4.5 h, at which time esterification was complete. The reaction mixture was cooled and evaporated; and the residue was redissolved in water, affording a dark red solution. The aqueous phase was basified with sodium bicarbonate solution, resulting in formation of a precipitate. The mixture was then extracted with ether (solids still insoluble) and the ether phase was washed with brine, at which time the solids were filtered. The filtered ether solution was dried, evaporated and further dried under vacuum before purification by chromatography, eluting with dichloromethane:ethyl acetate (gradient, 100:0, 97:3) to afford the ester (42%); TLC: 0.41, eluted twice, first hexane:ethyl acetate (6:1), then dichloromethane:methanol (9:1); MS: m/z=166(M+1).

### b. 3-Methylpyrid-4-ylcarbinol.

A 500 mL, 3-necked round bottomed flask, equipped with a reflux condenser with a gas inlet tube, a dropping funnel, and a stopper was purged with nitrogen; and then lithium aluminum hydride (1.2 g) was weighed into it. The hydride was wet with distilled tetrahydrofuran (60 mL). The suspension was cooled in an ice bath (0 °C) before a solution of ethyl 3-methylisonicotinate (2.6 g) in tetrahydrofuran was added dropwise over 30 min. Following the addition, the reaction mixture was allowed to warm to room temperature. After one hour, the reduction was complete. The reaction mixture was again cooled in an ice bath before water (4 mL) was added very slowly to quench the reaction. 10% Aqueous sodium hydroxide solution (4 mL) and water (10 mL) were added, and the mixture was stirred for 0.5 h before diatomaceous earth was added. After further stirring, the mixture was filtered through more diatomaceous earth. The filtrate was evaporated, redissolved in dichloromethane and evaporated, and further dried under high vacuum. After storage overnight in the freezer, the milky residue was dissolved in ethyl acetate, dried, evaporated and further dried under high vacuum, before the oil was chromatographed, eluting with dichloromethane:methanol (gradient, 95:5, 92:8, 9:1), to afford the alcohol (6%); TLC: R_{f}=0.19, eluted twice, first hexane:ethyl acetate (6:1), then dichloromethane:methanol (9:1); MS: m/z=124(M+1).

Example 51: R=2-dimethylaminoethoxycarbonyl: Acylation Method D using 2-dimethylaminoethanol and diluting the reaction mixture with ethyl acetate before washing it with water, saturated sodium bicarbonate solution and brine. Chromatography solvent: dichloromethane:methanol (gradient, 100:0, 95:5, 9:1);TLC: R_{f}=0.21, dichloromethane:methanol (9:1); MS: m/z=311(M+1).

| Analysis for C₂₄H₂₉F₃N₄O₅: | | | |
|---|---|---|---|
| Calculated: | C, 56.47; | H, 5.73; | N, 10.97 |
| Found: | C, 56.44; | H, 5.74; | N, 10.89 |

Example 52: R=4-methoxyphenoxycarbonyl: Acylation Method A as follows: A solution of the amine in tetrahydrofuran was cooled in an ice bath and treated with 4-methoxyphenyl chloroformate, resulting in formation of a precipitate. The reaction mixture was allowed to warm to room temperature. After 1 h, the reaction mixture was diluted with ethyl acetate, washed (10% aqueous hydrochloric acid, water, saturated sodium bicarbonate solution and brine), dried and evaporated before purification by chromatography. Chromatography solvent: dichloromethane:ethyl acetate (gradient, 100:0, 9:1); TLC: R_{f}=0.67, dichloromethane:ethyl acetate (9:1); HPLC: t_{R}=17.14. FR=1, column A, water:acetonitrile (1:1); MS: m/z=415(M+1).

| Analysis for C₂₇H₂₆F₃N₃O₆: | | | |
|---|---|---|---|
| Calculated: | C, 59.45; | H, 4.80; | N, 7.70 |
| Found: | C, 59.63; | H, 5.03; | N, 7.28 |

Example 53: R=4-pyridylacetyl: Acylation Method B using 4-pyridylacetic acid hydrochloride. Upon quenching the reaction with water, a bit of the product precipitated and was filtered and redissolved in ethyl acetate. The filtrate was basified with sodium bicarbonate and extracted with ethyl acetate. The pH of the aqueous phase was further increased, and it was again extracted with ethyl acetate. The combined ethyl acetate solutions were washed (brine), dried and evaporated before purification by chromatography. Chromatography solvent: dichloromethane:methanol (9:1); TLC: R_{f}=0.38, dichloromethane:methanol (9:1); HPLC: t_{R}=7.41, FR=1, column A, water:acetonitrile (1:1); MS: m/z=515(M+1).

| Analysis for C₂₆H₂₅F₃N₄O₄·0.55 H₂O: | | | |
|---|---|---|---|
| Calculated: | C, 59.55; | H, 5.02; | N, 10.68 |
| Found: | C, 59.63; | H, 4.99; | N, 10.75 |

Example 54: R=1-methylimidazol-4-ylacetyl: Acylation Method B as follows: To a solution of the 3-aminopyridine (0.308 g) in dry dimethylformamide (3.5 mL) was added 1-hydroxybenzotriazole hydrate (0.263 g), triethylamine (0.32 mL), 1-(3-dimethylaminopropyl)-3-ethylcarbodiimide hydrochloride (0.373 g) and 1-methylimidazol-4-ylacetic acid (0.275 g); and the reaction mixture was stirred overnight, at which time the coupling was deemed complete. The reaction mixture was diluted with ethyl acetate, washed (three times with saturated sodium bicarbonate solution, then brine), dried and evaporated before purification by chromatography to afford the title compound as an off-white solid. Chromatography solvent: dichloromethane:methanol, (first column, 97:3), (second column, gradient, 98:2, 96:4); TLC: R_{f}=0.13, dichloromethane:methanol (96:4); HPLC: t_{R}=4.13, FR=3, column A, water:acetonitrile (2:3); MS: m/z=518(M+1).

| Analysis for C₂₅H₂₆F₃N₅O₄·1.2 H₂O: | | | |
|---|---|---|---|
| Calculated: | C, 55.70; | H, 5.31; | N, 12.99 |
| Found: | C, 55.68; | H, 5.16; | N, 12.90 |

Example 55: R=1-tert-butoxycarbonylimidazol-4-ylmethoxycarbonyl: Acylation Method D using 1-tert-butoxycarbonylimidazol-4-ylcarbinol, but using tetrahydrofuran instead of dichloromethane as the reaction solvent and diluting the reaction mixture with ethyl acetate instead of dichloromethane. Chromatography solvent: first column, chloroform:methanol (97:3), second column dichloromethane:ethyl acetate:methanol (70:29.5:0.5); TLC: R_{f}=0.47, dichloromethane:ethyl acetate:methanol (50:48:2); MS: m/z=620(M+1).

The starting material alcohol for the acylation was obtained as follows, using a procedure from European Patent Application, Publication Number 284 174.

To a solution of 4-imidazolylcarbinol hydrochloride (1.01 g) in dry dichloromethane (5 mL) was added pyridine (1.82 mL) and di-tert-butyl dicarbonate (2.46 g). The resulting solution was stirred overnight, evaporated and resuspended/dissolved in 100 mL of ethyl acetate/tetrahydrofuran (9:1). The organic phase was washed (three times with water, brine), dried, evaporated and dried under high vacuum to give 1-tert-butoxycarbonylimidazol-4-ylcarbinol as a colorless oil; TLC: R_{f}=0.30, dichloromethane:ethyl acetate:methanol (50:49:1); MS: m/z=199(M+1).

### EXAMPLE 56

### 2-[3-(4-Imidazolylmethoxycarbonylamino)-2-oxo-6-phenyl-1,2-dihydro-1-pyridyl]-N-(3,3,3-trifluoro-1-isopropyl-2-oxopropyl)acetamide trifluoroacetate.

To a solution of 2-[3-(1-tert-butoxycarbonylimidazol-4-ylmethoxycarbonylamino)-2-oxo-6-phenyl-1,2-dihydro-1-pyridyl]-N-(3,3,3-trifluoro-1-isopropyl-2-oxopropyl)acetamide (0.134 g) in dry dichloromethane was added trifluoroacetic acid (0.018 mL). After stirring for 1 h, the reaction was not complete; so additional trifluoracetic acid (0.018 mL) was added. Following 17 h more stirring, the reaction was still incomplete. Additional trifluoroacetic acid (0.036 mL) was added and the reaction mixture was stirred 6 h (complete deprotection), evaporated and dried under high vacuum to give the title compound as a trifluoroacetate salt; TLC: R_{f}=0.10, dichloromethane:methanol (94:6); MS: 520(M+1).

| Analysis for C₂₄H₂₄F₃N₅O₅·1.3 CF₃CO₂H: | | | |
|---|---|---|---|
| Calculated: | C, 47.62; | H, 3.79; | N, 10.40 |
| Found: | C, 47.62; | H, 3.92; | N, 10.10 |

### EXAMPLE 57

### 2-[3-[4-(2-Dimethylaminoethoxycarbonyl)benzyloxycarbonylamino]-2-oxo-6-phenyl-1,2-dihydro-1-pyridyl]-N-(3,3,3-trifluoro-1-isopropyl-2-oxopropyl)acetamide.

To a solution of 2-[3-(4-carboxybenzyloxycarbonylamino)-2-oxo-6-phenyl-1,2-dihydro-1-pyridyl]-N-(3,3,3-trifluoro-1-isopropyl-2-oxopropyl)acetamide (0.281 g) in dry tetrahydrofuran (5 ml) was added 1-(3-dimethylaminopropyl)-3-ethylcarbodiimide hydrochloride (0.141 g), 1-hydroxybenzotriazole hydrate (0.066 g) and 2-dimethylaminoethanol (0.06 mL). The reaction mixture was stirred overnight before it was diluted with ethyl acetate, washed (twice with saturated sodium bicarbonate solution, then brine), dried and evaporated before purification by chromatography, eluting with dichloromethane:methanol (97:3), to afford the title compound; TLC: R_{f}=0.18, dichlormethane:methanol (97:3); HPLC: t_{R}=6.51, FR=1, column A, water:acetonitrile (3:2); MS: m/z=645(M+1).

| Analysis for C₃₂H₃₅F₃N₄O₇: | | | |
|---|---|---|---|
| Calculated: | C, 59.62; | H, 5.47; | N, 8.69 |
| Found: | C, 59.44; | H, 5.69; | N, 8.36 |

### EXAMPLE 58

### 2-[3-[4-(N-methylsulfonylcarbamoyl)benzyloxycarbonylamino]-2-oxo-6-phenyl-1,2-dihydro-1-pyridyl]-N-(3,3,3-trifluoro-1-isopropyl-2-oxopropyl)acetamide.

To a solution of 2-[3-(4-carboxybenzyloxycarbonylamino)-2-oxo-6-phenyl-1,2-dihydro-1-pyridyl]-N-(3,3,3-trifluoro-1-isopropyl-2-oxopropyl)acetamide (0.387 g), 1-(3-dimethylaminopropyl)-3-ethylcarbodiimide hydrochloride (0.43 g) and 4-dimethylaminopyridine (0.27 g) in dry dichloromethane was added methanesulfonamide; and the reaction mixture was stirred for 5 days. The mixture was diluted with ethyl acetate, washed (three times with 10% aqueous hydrochloric acid, then brine), dried and evaporated before purification by chromatography, eluting with dichloromethane:ethyl acetate:methanol:acetic acid (50:48.9:1.0:0.1), to give the title compound; TLC: R_{f}=0.38, dichloromethane:ethyl acetate:methanol:acetic acid (50:48.9:1.0:0.1); HPLC: t_{R}=5.38, FR=2, column A, water:acetonitrile (7:3); MS: m/z=651(M+1).

| Analysis for C₂₉H₂₉F₃N₄O₈S: | | | |
|---|---|---|---|
| Calculated: | C, 53.53; | H, 4.49; | N, 8.61 |
| Found: | C, 54.08; | H, 4.81; | N, 8.26 |

### EXAMPLE 59

### 2-(3-Benzyloxycarbonylamino-6-methyl-2-oxo-1,2-dihydro-1-pyridyl)-N-(3,3,3-trifluoro-1-isopropyl-2-oxopropyl)acetamide.

Using a similar procedure to that described in Example 1, using chloroform:methanol (99:1) for elution in the chromatography, 2-(3-benzyloxycarbonylamino-6-methyl-2-oxo-1,2-dihydro-1-pyridyl)-N-(3,3,3-trifluoro-2-hydroxy-1-isopropylpropyl)acetamide was oxidized to afford the title compound; TLC: R_{f}=0.32, chloroform:methanol (97:3); HPLC: t_{R}=4.60, FR=2.0, column B, acetonitrile:water (1:1); MS: m/z=468(M+1).

| Anaylsis for C₂₂H₂₄F₃N₃O₅·0.4 H₂O: | | | |
|---|---|---|---|
| Calculated: | C, 55.67; | H, 5.27; | N, 8.85 |
| Found: | C, 55.59; | H, 5.31; | N, 8.68 |

The intermediate 2-(3-benzyloxycarbonylamino-6-methyl-2-oxo-1,2-dihydro-1-pyridyl)-N-(3,3,3-trifluoro-2-hydroxy-1-isopropylpropyl)acetamide may be prepared as follows:

### a. 6-Methylpyrid-2-one-3-carboxylic acid.

A suspension of 6-methylpyrid-2-one-3-carbonitrile (Example 3.a.) (16.9 g) in 20% NaOH (w/w; 63 mL) was heated at 140-145 °C overnight in a sealed bomb. The cooled reaction mixture was acidified to about pH 8 with concentrated hydrochloric acid and extracted with dichloromethane (three times). The aqueous phase was acidified, precipitating a yellow solid which was filtered, washed with water, and dried overnight in a vacuum oven at about 80 °C. The dried 6-methylpyrid-2-one-3-carboxylic acid (15.68 g) required no further purification; NMR: 2.38 (s,3), 6.54 (d,1, J=9), 8.27 (d,1, J=9), 13.27 (broad s, 1), 14.67 (broad s, 1); MS: m/z=154(M+1).

### b. 3-Benzyloxycarbonylamino-6-methylpyrid-2-one.

Using a similar procedure to that described in Example 1.c. and using chloroform:ethyl acetate (80:20, then 70:30) for the chromatography, 6-methylpyrid-2-one-3-carboxylic acid was converted into 3-benzyloxycarbonylamino-6-methylpyrid-2-one; TLC: R_{f}=0.47, chloroform:methanol (97:3); MS: m/z=259(M+1).

### c. Ethyl (3-Benzyloxycarbonylamino-6-methyl-2-oxo-1,2-dihydro-1-pyridyl)acetate.

Using a similar procedure to that of Example 1.d., above, 3-benzyloxycarbonylamino-6-methylpyrid-2-one (1.80 g) was added to a suspension of NaH (0.33 g) in dry dimethylformamide (50 mL). After the mixture had been stirred for 45 min, ethyl iodoacetate (1.48 g) was added; and the mixture was stirred overnight, diluted with 10% hydrochloric acid (300 mL) and extracted with ethyl acetate (3 x 150 mL). The organic phase was washed with brine (twice), dried and evaporated. The resulting yellow, waxy solid was chromatographed, eluting with ethyl acetate:dichloromethane (3:97), to give ethyl (3-benzyloxycarbonylamino-6-methyl-2-oxo-1,2-dihydro-1-pyridyl)acetate (1.28 g); TLC: R_{f}=0.52, dichloromethane:ethyl acetate (95:5); NMR: 1.21 (t,3, J=8.6), 2.26 (s,3), 4.16 (q,2, J=8.6), 4.84 (s,2), 5.15 (s,2), 6.20 (d,1, J=9), 7.32-7.43 (m,5), 7.76 (d,1, J=9), 8.38 (s,1); MS: m/z=345(M+1).

### d. (3-Benzyloxycarbonylamino-6-methyl-2-oxo-1,2-dihydro-1-pyridyl)acetic acid.

Ethyl (3-benzyloxycarbonylamino-6-methyl-2-oxo-1,2-dihydro-1-pyridyl)acetate (1.20 g) was dissolved in methanol (50 mL), and 20% NaOH (10 mL) was added. More methanol (25 mL) was added to facilitate stirring as white solid precipitated from the mixture. After stirring for 5 h, the mixture was evaporated, and the residue was partitioned between ethyl acetate and 10% hydrochloric acid. After the layers were separated, the aqueous phase was extracted further with ethyl acetate (twice). The combined organic extracts were washed with brine, dried and evaporated to give 1.43 g of crude product. Examination of this material showed a mixture of what was presumed to be the corresponding methyl carbamate along with the expected benzyl carbamate. The mixture proved to be inseparable by column chromatography and therefore was carried through to the next step without purification.

### e. 2-(3-Benzyloxycarbonylamino-6-methyl-2-oxo-1,2-dihydro-1-pyridyl)-N-(3,3,3-trifluoro-2-hydroxy-1-isopropylpropyl)acetamide.

A portion of the above mixture (1.10 g) was dissolved in dry dimethylformamide (25 mL) along with 3-amino-1,1,1-trifluro-4-methyl-2-pentanol hydrochloride (0.72 g), 1-(3-dimethylaminopropylpropyl)-3-ethylcarbodiimide hydrochloride (0.73 g) and 4-dimethylaminopyridine (0.93 g). The mixture was stirred overnight, diluted with 10% hydrochloric acid and extracted with ethyl acetate (three times). The extracts were washed with saturated sodium bicarbonate (twice) and brine, dried and evaporated to a white solid. Chromatography, eluting with acetone:dichloromethane (5:95), gave 2-(3-benzyloxycarbonylamino-6-methyl-2-oxo-1,2-dihydro-1-pyridyl)-N-(3,3,3-trifluoro-2-hydroxy-1-isopropylpropyl)acetamide (0.80 g); TLC: R_{f}=0.48, dichloromethane:acetone (85:15); MS: m/z=470(M+1).

### EXAMPLE 60

### 2-(3-Benzyloxycarbonylamino-6-methyl-2-oxo-5-phenyl-1,2-dihydro-1-pyridyl)-N-(3,3,3-trifluoro-1-isopropyl-2-oxopropyl)acetamide.

Using a similar procedure to that described in Example 1, using chloroform:ethyl acetate for elution in the chromatography, 2-(3-benzyloxycarbonylamino-6-methyl-2-oxo-5-phenyl-1,2-dihydro-1-pyridyl)-N-(3,3,3-trifluoro-2-hydroxy-1-isopropylpropyl)acetamide was oxidized to afford the title compound; TLC: R_{f}=0.23, chloroform:ethyl acetate (20:1); HPLC: t_{R}=11.26, FR=2.0, column A, water:acetonitrile:tetrahydrofuran:trifluoroacetic acid (55:35:15:0.1); MS: m/z=544(M+1).

| Analysis for C₂₈H₂₈F₃N₃O₅: | | | |
|---|---|---|---|
| Calculated: | C, 61.87; | H, 5.19; | N, 7.73 |
| Found: | C, 61.75; | H, 5.29; | N, 7.44 |

The intermediate 2-(3-benzyloxycarbonylamino-6-methyl-2-oxo-5-phenyl-1,2-dihydro-1-pyridyl)-N-(3,3,3-trifluoro-2-hydroxy-isopropylpropyl)acetamide may be prepared as follows:

### a. 6-Methyl-5-phenylpyrid-2-one-3-carbonitrile.

Using phenylacetone and Cyclization Method A, 6-methyl-5-phenylpyrid-2-one-3-carbonitrile was obtained; MS: m/z=211(M+1).

| Analysis for C₁₃H₁₀N₂O: | | | |
|---|---|---|---|
| Calculated: | C, 74.27; | H, 4.79; | N.13.32 |
| Found: | C, 74.10; | H, 4.88; | N, 13.15 |

### b. 6-Methyl-5-phenylpyrid-2-one-3-carboxylic acid.

Using Hydrolysis Method A, 6-methyl-5-phenylpyrid-2-one-3-carbonitrile was converted into 6-methyl-5-phenylpyrid-2-one-3-carboxylic acid; TLC: R_{f}=0.29, chloroform:methanol:acetic acid (50:1:trace); MS: m/z=230(M+1).

### c. 3-Benzyloxycarbonylamino-6-methyl-5-phenylpyrid-2-one.

Using a similar procedure to that described in Example 1.c., 6-methyl-5-phenylpyrid-2-one-3-carboxylic acid was converted into 3-benzyloxycarbonylamino-6-methyl-5-phenylpyrid-2-one; purified by chromatography, eluting with chloroform:methanol (20:1), or by recrystallization from methanol; TLC: R_{f}=0.46, chloroform:methanol (20:1); MS: m/z=335(M+1).

### d. 2-(3-Benzyloxycarbonylamino-6-methyl-2-oxo-5-phenyl-1,2-dihydro-1-pyridyl)-N-(2-tert-butyldimethylsilyloxy-3,3,3-trifluoro-1-isopropylpropyl) acetamide.

Using a similar procedure to that described in Example 1.d., but using dichloromethane:ethyl acetate (20:1) for chromatography, 3-benzyloxycarbonylamino-6-methyl-5-phenylpyrid-2-one was converted into 2-(3-benzyloxycarbonylamino-6-methyl-2-oxo-5-phenyl-1,2-dihydro-1-pyridyl)-N-(2-tert-butyldimethylsilyloxy-3,3,3-trifluoro-1-isopropylpropyl)acetamide; TLC: R_{f}=0.41, dichloromethane:ethyl acetate (20:1); MS: m/z=660(M+1).

### e. 2-(3-Benzyloxycarbonylamino-6-methyl-2-oxo-5-phenyl-1,2-dihydro-1-pyridyl)-N-(3,3,3-trifluoro-2-hydroxy-1-isopropylpropyl)acetamide.

Using a similar procedure to that described in Example 1.e., 2-(3-benzyloxycarbonylamino-6-methyl-2-oxo-5-phenyl-1,2-dihydro-1-pyridyl)-N-(2-tert-butyldimethylsilyloxy-3,3,3-trifluoro-1-isopropylpropyl)acetamide was converted into 2-(3-benzyloxycarbonylamino-6-methyl-2-oxo-5-phenyl-1,2-dihydro-1-pyridyl)-N-(3,3,3-trifluoro-2-hydroxy-1-isopropylpropyl)acetamide; chromatography solvent: chloroform:methanol (40:1); TLC: R_{f}=0.14, chloroform:ethyl acetate (20:1); MS: m/z=546(M+1).

### EXAMPLE 61

### 2-[3-Benzyloxycarbonylamino-5-(3-fluorobenzyl)-2-oxo-1,2-dihydro-1-pyridyl]-N-(3,3,3-trifluoro-1-isopropyl-2-oxopropyl)acetamide.

To a solution of 1-(3-dimethylaminopropyl)-3-ethylcarbodiimide hydrochloride (0.51 g) in dimethyl sulfoxide (2 mL), toluene (4 mL) and dichloroacetic acid (0.14 g) was added 2-[3-benzyloxycarbonylamino-5-(3-fluorobenzyl)-2-oxo-1,2-dihydro-1-pyridyl]-N-(3,3,3-trifluoro-2-hydroxy-1-isopropylpropyl)acetamide (0.15 g). The mixture was allowed to stir overnight and was partitioned between ethyl acetate and saturated aqueous sodium bicarbonate. The organics were washed (brine), dried, evaporated, and purified using chromatography, with dichloromethane as the eluent to yield the title compound (80%); NMR: 0.79-0.97 (m,6), 2.21 (m,1), 4.06 (m, NHCH hydrate), 4.49-4.81 (m,3, NHCH keto, CH₂CO), 5.13 (s,2, CH₂), 6.95 (m, OH hydrate), 7.01-7.07 (m,3, phenyl), 7.22-7.40 (m,7, phenyl), 7.72 (m,1, pyridone), 7.82 (d,J=10.5, NH hydrate), 8.36 (s, NH hydrate), 8.40 (s, NH keto), 8.92 (d,J=6.5, NH keto form).

| Analysis for C₂₈H₂₇F₄O₅N₃·0.25 H₂O: | | | |
|---|---|---|---|
| Calculated: | C, 59.41; | H, 4.90; | N, 7.42 |
| Found: | C, 59.34; | H, 4.84; | N, 7.15 |

The intermediate 2-[3-benzyloxycarbonylamino-5-(3-fluorobenzyl)-2-oxo-1,2-dihydro-1-pyridyl]-N-(3,3,3-trifluoro-2-hydroxy-1-isopropylpropyl)acetamide may be prepared as follows:

### a. 3-Aminopyrid-2-one.

Ethanol (300 mL) was added to a mixture of 10% (w/w) palladium on carbon catalyst (1 g) and 3-nitropyrid-2-one (10 g). The mixture was hydrogenated at atmospheric pressure and room temperature for 8 h. The catalyst was removed by filtration, washed with ethanol, and the ethanol evaporated to give the amine as a brown crystalline solid (98%).

### b. 3-Benzyloxycarbonylaminopyrid-2-one.

Benzyl chloroformate (13.085 g) was added dropwise to a stirred suspension of sodium carbonate (16.26 g) and 3-aminopyrid-2-one (7.67 g) in tetrahydrofuran. The mixture was stirred overnight, poured into ethyl acetate (400 mL), washed (saturated aqueous sodium bicarbonate, brine), dried and evaporated. The resulting residue was purified by crystallization from methanol to give the benzyl carbonate as a white crystalline solid (10.7 g).

The benzylcarbamate can alternatively be prepared as follows:
3-Carboxypyrid-2-one (5 g), diphenylphosphorylazide (9.9 g), benzyl alcohol (4.7 g), and triethylamine (3.6 g) were added to dioxane (50 mL). The mixture was allowed to stir at 90 °C for 20 h, was cooled, and the dioxane was evaporated. The residue was dissolved in ethyl acetate (400 mL), was washed (1 N hydrochloric acid, brine), dried, evaporated and the resulting oil was purified by chromatography using ethyl acetate:dichloromethane (gradient, 0:100, 10:90, 20:80) as eluent to give the benzylcarbamate (6.2 g).

### c. 3-Benzyloxycarbonylamino-5-iodopyrid-2-one.

To a stirred suspension of 3-benzyloxycarbonylaminopyrid-2-one (8.0 g) in dry dichloromethane (150 mL) was added N-iodosuccinimide (8.4 g). The mixture was allowed to stir overnight and the resulting precipitate was filtered to give the iodo compound (3.1 g). The filtrate was concentrated to 30 mL and purified by chromatography using ethyl acetate:dichloromethane as an eluent (gradient 0:100, 20:80, 25:75, 33:66, 50:50) to give additional iodo compound (5.7 g).

### d. Ethyl 3-benzyloxycarbonylamino-5-iodo-2-oxo-1,2-dihydro-1-pyridylacetate.

A suspension of 3-benzyloxycarbonylamino-5-iodopyrid-2-one (2.0 g) in dimethylformamide (10 mL) was added to a suspension of NaH (0.156 g) in dimethylformamide (10 mL) maintaining the temperature between 15 and 25 °C. After stirring for 20 min, ethyl iodoacetate (1.453 g) was added dropwise, maintaining the temperature below 20 °C. The mixture was stirred at room temperature for 3 h, poured into iced 1 N hydrochloric acid (100 mL) and extracted with ethyl acetate. The organic layer was washed (brine), dried, and evaporated to give a residue, which was purified by chromatography using ethyl acetate:dichloromethane as the eluent (gradient, 0:100, 3:97, 6:94) to give the ester (1.44 g).

### e. Ethyl 3-benzyloxycarbonylamino-5-(3-fluorobenzyl)-2-oxo-1,2-dihydro-1-pyridylacetate.

To a solution of freshly activated zinc dust (0.39 g) was added 3-fluorobenzyl bromide (0.76 g) in tetrahydrofuran (10 mL), maintaining the temperature at 20 °C. The solution was allowed to stir for 3 h and dichloro[1,1′-bis(diphenylphosphino)ferrocene]-palladium(II) (0.076 g) was added followed by a solution of ethyl 3-benzyloxycarbonylamino-5-iodo-2-oxo-1,2-dihydro-1-pyridylacetate (0.46 g) in tetrahydrofuran (10 mL), which was added dropwise. The mixture was stirred at room temperature for 5 h, at 50 °C for 4.5 h, and at room temperature overnight. It was poured into 1 N hydrochloric acid and partitioned into ethyl acetate. The organic extracts were dried, evaporated, and the resulting oil was purified using chromatography with ethyl acetate:dichloromethane (gradient, 0:100, 5:95, 10:90) to give ethyl 2-[3-benzyloxycarbonylamino-5-(3-fluorobenzyl)-2-oxo-1,2-dihydro-1-pyridyl]acetate (0.225 g).

### f. 2-[3-Benzyloxycarbonylamino-5-(3-fluorobenzyl)-2-oxo-1,2-dihydro-1-pyridyl]-N-(3,3,3-trifluoro-2-hydroxy-1-isopropylpropyl)acetamide.

Aqueous 2 N sodium hydroxide (1.25 mL) was added with stirring to a solution of ethyl 3-benzyloxycarbonylamino-5-(3-fluorophenyl)-2-oxo-1,2-dihydro-1-pyridylacetate (0.22 g) in methanol (8 mL). The mixture was allowed to stir for 4 h, was evaporated, and the resulting residue was titrated with 1 N hydrochloric acid and partitioned into ethyl acetate. The combined organic extracts were dried (MgSO₄) and evaporated to yield the crude acid (0.21 g).

To the crude acid (0.21 g) in dimethylformamide (35 mL) was added 3-amino-4-methyl-1,1,1-trifluoro-2-pentanol hydrochloride (0.117 g), dimethylaminopyridine (0.140 g) and 1-(3-dimethylaminopropyl)-3-ethylcarbodiimide hydrochloride (0.113 g). The mixture was allowed to stir overnight, was added to 1 N hydrochloric acid (100 mL), and was partitioned into ethyl acetate. The combined organic extracts were washed (saturated aqueous sodium bicarbonate, brine), dried, and evaporated to give a residue which was purified by chromatography, using ethyl acetate:dichloromethane (gradient, 0:100, 10:90, 20:80) as an eluent, to give 2-[3-benzyloxycarbonylamino-5-(3-fluorobenzyl)-2-oxo-1,2-dihydro-1-pyridyl]-N-(3,3,3-trifluoro-2-hydroxy-1-isopropyl-propyl)acetamide (0.155 g).

### EXAMPLE 62

### 2-[3-Benzyloxycarbonylamino-2-oxo-5-(4-pivaloyloxybenzyl)-1,2-dihydro-1-pyridyl]-N-(3,3,3-trifluoro-1-isopropyl-2-oxopropyl)acetamide.

2-[3-Benzyloxycarbonylamino-2-oxo-5-(4-pivaloyloxybenzyl)-1,2-dihydro-1-pyridyl]-N-(3,3,3-trifluoro-2-hydroxy-1-isopropylpropyl)acetamide was oxidized using a similar procedure to that described in Example 61 to give the title compound; NMR: 0.77-0.97 (m,6), 1.30 (s,9), 2.20 (m,1), 4.50-4.75 (m,3, NHCH keto, CH₂CO), 5.13 (s,2), 6.95 (m, OH hydrate), 7.02 (d,2, J=8.5), 7.73 (m,1, pyridone), 7.22-7.73 (m,8), 7.74 (d, J=10), 8.35 (s, NH hydrate), 8.37 (s, NH keto), 8.91 (d, J=6.5, NH keto).

| Analysis for C₃₃H₃₆N₃O₇F₃·0.5 H₂O: | | | |
|---|---|---|---|
| Calculated: | C, 60.72; | H, 5.71; | N, 6.44 |
| Found: | C, 60.81; | H, 5.63; | N, 6.14 |

The intermediate 2-[3-benzyloxy carbonylamino-2-oxo-5-(4-pivaloyloxybenzyl)-1,2-dihydro-1-pyridyl]-N-(3,3,3-trifluoro-2-hydroxy-1-isopropylpropyl)acetamide was prepared as follows:

### a. tert-Butyl 3-benzyloxycarbonylamino-5-iodo-2-oxo-1,2-dihydro-1-pyridylacetate.

A suspension of 3-benzyloxycarbonylamino-5-iodo-pyrid-2-one (5.0 g) in dimethylformamide (15 mL) was added to a stirred suspension of NaH (0.389 g), in dimethylformamide (10 mL), while maintaining the temperature between 15 and 25 °C. After stirring for 1 h, a solution of tert-butyl bromoacetate (3.294 g), in dimethylformamide (5 mL) was added dropwise, while maintaining the reaction temperature below 20 °C. The reaction mixture was stirred for 4 h at room temperature, poured into iced 1 N HCl (100 mL) and extracted with ethyl acetate (200 mL). The organic layer was washed (saturated aqueous sodium bicarbonate, brine), dried, and evaporated. The resulting residue was purified by chromatography, eluting with dichloromethane:hexane (gradient, 0:100, 50:50, 100:0). The product-containing fractions were rechromatographed to give the desired ester as a red solid (4.3 g).

### b. tert-Butyl 3-benzyloxycarbonylamino-2-oxo-5-(4-pivaloyloxybenzyl)-1,2-dihydro-1-pyridylacetate.

A solution of 4-pivaloyloxybenzyl bromide (1.6 g) in tetrahydrofuran (10 mL) was added dropwise to freshly activated zinc dust (0.576 g) with stirring, maintaining the temperature at about 20°C. After 1 h, dichloro[1,1′-bis(diphenylphosphino)ferrocene]-palladium(II) (0.112 g) was added followed by a solution of tert-butyl 3-benzyloxycarbonylamino-5-iodo-2-oxo-1,2-dihydro-1-pyridylacetate (0.714 g) in tetrahydrofuran (10 mL). The mixture was heated at 45-50 °C for 5 h, stirred at room temperature overnight, poured into cold 1 N hydrochloric acid (100 mL) and extracted with ethyl acetate. The combined extracts were dried and evaporated. The resulting residue was purified by chromatography, eluting with ethyl acetate:dichloromethane (0:100, 10:90), to give tert-butyl 3-benzyloxycarbonylamino-2-oxo-5-(4-pivaloyloxybenzyl)1,2-dihydro-1-pyridylacetate (0.35 g).

### c. 2-[3-Benzyloxycarbonylamino-2-oxo-5-(4-pivaloyloxybenzyl)-1,2-dihydro-1-pyridyl]-N-(3,3,3-trifluoro-2-hydroxy-1-isopropylpropyl]acetamide.

Trifluoroacetic acid (3 mL) was added dropwise to tert-butyl 3-benzyloxycarbonylamino-2-oxo-5-(4-pivaloyloxybenzyl)-1,2-dihydro-1-pyridylacetate (0.320 g) with stirring. After 30 min, the reaction mixture was diluted with dichloromethane (50 mL) and evaporated, diluted with chloroform (50 mL) and evaporated, diluted with diethyl ether (50 mL) and evaporated twice, and dried under high vacuum to give the crude acid. (2RS,3SR)-3-Amino-4-methyl-1,1,1-trifluoro-2-pentanol hydrochloride (0.149 g), 1-(3-dimethyl aminopropyl)-3-ethylcarbodiimide hydrochloride (0.143), and 4-dimethylaminopyridine (0.238 g) were added to a solution of the crude acid (0.32 g) in dimethylformamide and stirred overnight. The mixture was added to 1 N HCl (100 mL), extracted with ethyl acetate (100 mL), washed (saturated aqueous sodium bicarbonate, brine), and evaporated to give a residue which was purified by chromatography, eluting with ethyl acetate:dichloromethane (0:100, 10:90), to give the alcohol (0.220 g).

The intermediate 4-pivaloyloxybenzyl bromide may be prepared as follows:

### d. 4-Pivaloyloxybenzyl bromide.

N-Bromosuccinimide (3.43 g) and benzoyl peroxide (0.01 g) were added to a solution of 4-pivaloyloxytolene (3.70 g) in carbon tetrachloride (100 mL). The mixture was irradiated (sun lamp) with heating (60 °C) for 0.5 h. The succinimide was removed by filtration and the carbon tetrachloride evaporated. The residue was purified by chromatography, eluting with hexane:dichloromethane (100:0, 90:10, 88:12, 30:20), to give the benzyl bromide (4.1 g).

The title compound can alternatively be prepared using a procedure similar to that outlined in Example 64 by substituting 4-pivaloyloxybenzyl bromide for 4-methoxycarbonylbenzyl bromide at the step corresponding to Example 64.b.

### EXAMPLE 63

### 2-[3-Benzyloxycarbonylamino-2-oxo-5-(4-hydroxybenzyl)-1,2-dihydro-1-pyridyl]-N-(3,3,3-triluoro-1-isopropyl-2-oxopropyl]acetamide.

2-[3-Benzyloxycarbonylamino-2-oxo-5-(4-pivaloyloxybenzyl)-1,2-dihydro-1-pyridyl]-N-(3,3,3-trifluoro-1-isopropyl-2-oxopropyl]-acetamide was dissolved in methanol and excess 2 N sodium hydroxyde was added. The mixture was allowed to stir for 3 h, was evaporated, and partitioned between ethyl acetate and 1 N hydrochloric acid. The organic layer was washed (brine), dried, evaporated and purified by chromatography, using dichloromethane:methanol (gradient, 99:1, 95:5) as the eluent, to give the title compound; NMR: 0.78-0.91 (m,6), 2.20 (m,1), 4.09 (m, NHCH hydrate), 4.50-4.77 (m,3, CH₂CO, NHCH keto), 5.13 (s,2), 6.68 (d,2, J=8), 6.95 (m, OH hydrate), 6.96 (d,2, J=8), 7.20 (m,1) 7.29-7.42 (m,5), 7.70 (m,1), 7.73 (d, J=10), 8.33 (s, NH hydrate), 8.37 (S, NH keto), 8.93 (d, J=6.5, NH keto), 9.24 (s,1, OH).

| Analysis for C₂₈H₂₈F₃N₃O₆·0.75 H₂O: | | | |
|---|---|---|---|
| Calculated: | C, 58.69; | H, 5.19; | N, 7.33 |
| Found: | C, 58.77; | H, 5.28; | N, 7.00 |

### EXAMPLE 64

### 2-[3-Benzyloxycarbonylamino-5-(4-methoxycarbonylbenzyl)-2-oxo-1,2-dihydro-1-pyridyl]-N-(3,3,3-trifluoro-1-isopropyl-2-oxopropyl)acetamide.

2-[3-Benzyloxycarbonylamino-5-(4-methoxycarbonylbenzyl)-2-oxo-1,2-dihydro-1-pyridyl]-N-(3,3,3-trifluoro-2-hydroxy-1-isopropylpropyl)acetamide was oxidized using a similar procedure to that described in Example 61 to give the title compound; NMR: 0.78-0.97 (m,6), 2.20 (m,1), 3.84 (s,3, CH₃), 4.08 (m, NHCH hydrate), 4.62-4.78 (m, NHCH keto, CH₂CO), 5.11 (S, 2), 6.90 (m, OH hydrate), 7.27-7.40 (m, OH), 7.71 (m,1), 7.89 (d,2, J=8), 8.37 (s, NH hydrate), 8.40 (s, NH keto), 8.92 (d, J=6.5, NH keto).

| Analysis for C₃₀H₃₀F₃N₃O₇·0.75 H₂O: | | | |
|---|---|---|---|
| Calculated: | C, 58.58; | H, 5.16; | N, 6.83 |
| Found: | C, 58.62; | H, 5.04; | N, 6.90 |

The intermediate 2-[3-benzyloxycarbonylamino-5-(4-methoxycarbonylbenzyl)-2-oxo-1,2-dihydro-1-pyridyl]-N-(3,3,3-trifluoro-2-hydroxy-1-isopropylpropyl)acetamide was prepared as follows:

### a. 2-(3-Benzyloxycarbonylamino-5-iodo-2-oxo-1,2-dihydro-1-pyridyl)-N-(2-tert-butyldimethylsilyloxy-3,3,3-trifluoro-1-isopropylpropyl)acetamide.

A suspension of 3-benzyloxycarbonylamino-5-iodopyrid-2-one (1.0 g) in dimethylformamide (10 mL) was added to a suspension of sodium hydride (0.071 g), in dimethylformamide (5 mL) maintaining the temperature between 15 and 25 °C. After stirring for 1 h, a solution of N-(2-tert-butyldimethylsiloxy-3,3,3-trifluoro-1-isopropylpropyl)2-iodoacetamide (1.40 g) in dimethylformamide (5 mL) was added dropwise, maintaining the temperature below 20 °C. The mixture was stirred for 2 h, poured into iced 1 N HCl (100 mL) and extracted with ethyl acetate (200 mL). The organic layer was washed (saturated aqueous sodium bicarbonate, brine), dried, and evaporated. The resulting residue was purified by chromatography, eluting with dichloromethane, to give the iodoamide (1.74 g).

### b. 2-[3-Benzyloxycarbonylamino-5-(4-methoxycarbonylbenzyl)-2-oxo-1,2-dihydro-1-pyridyl]-N-(2-tert-butyldimethylsilyloxy-3,3,3-trifluoro-1-isopropylpropyl)acetamide.

2-(3-Benzyloxycarbonylamino-5-iodo-2-oxo-1,2-dihydro-1-pyridyl)-N-(2-tert-butyldimethylsilyloxy-3,3,3-trifluoro-1-isopropylpropyl)acetamide was coupled with 4-methoxycarbonylbenzyl bromide using a similar procedure to that described in Example 62.b. to give the 5-benzyl compound.

### c. 2-[3-Benzyloxycarbonylamino-5-(4-methoxycarbonylbenzyl)2-oxo-1,2-dihydro-1-pyridyl]-N-(3,3,3-trifluoro-2-hydroxyl-isopropylpropyl]acetamide.

A 1 N solution of tetrabutylammonium fluoride (1.20 mL) in tetrahydrofuran was added dropwise to a solution of 2-[3-benzyloxycarbonylamino-5-(4-methoxycarbonylbenzyl)-2-oxo-1,2-dihydro-1-pyridyl]-N-(2-tert-butyldimethylsilyloxy-3,3,3-trifluoro-1-isopropylpropyl)acetamide in tetrahydrofuran (5 mL) with stirring. After 15 min, the reaction mixture was diluted with ethyl acetate (100 mL), washed (1 N HCl, saturated aqueous sodium bicarbonate, brine), dried, and evaporated. The resulting solid was purified by chromatography, eluting with ethyl acetate:dichloromethane (0:100, gradient 5:90 to 50:50), to give the alcohol (0.590 g).

### EXAMPLE 65

### 2-[3-Benzyloxycarbonylamino-5-(4-fluorobenzyl)-2-oxo-1,2-dihydro-1-pyridyl]-N-(3,3,3-trifluoro-1-isopropyl-2-oxopropyl)acetamide.

Using a procedure similar to that outlined in Example 61, but substituting 4-fluorobenzyl bromide for 3-fluorobenzyl bromide in the step corresponding to Example 61.e., the title compound was obtained; mp 66-72 °C.

| Analysis for C₂₈H₂₇F₄N₃O₅·0.75 H₂O: | | | |
|---|---|---|---|
| Calculated: | C, 58.48; | H, 5.00; | N, 7.31 |
| Found: | C, 58.37; | H, 4.83; | N, 7.23 |

### EXAMPLES 66-67

Using a procedure similar to that described in Example 61, the following compounds of formula I wherein R⁰ is isopropyl, R is benzyloxycarbonylamino, R⁵ is the indicated aryl containing group, and R⁶ is hydrogen were prepared by oxidation of the corresponding alcohols of formula II:

Example 66: R⁵=3-methylbenzyl.

| Analysis for C₂₉H₃₀F₃N₃O₅·0.5 H₂O: | | | |
|---|---|---|---|
| Calculated: | C, 61.48; | H, 5.51; | N, 7.42 |
| Found: | C, 61.55; | H, 5.43; | N, 7.41 |

Example 67: R⁵=2-methylbenzyl: MS: m/z=557(M+1).

| Analysis for C₂₉H₃₀F₃N₃O₅·0.5 H₂O: | | | |
|---|---|---|---|
| Calculated: | C, 61.48; | H, 5.51; | N, 7.42 |
| Found: | C, 61.31; | H, 5.61; | N, 7.14 |

The corresponding alcohols of formula II for Examples 66-67 were prepared as follows:

### EXAMPLES 66.a.-67.a.

2-(3-Benzyloxycarbonylamino-5-iodo-2-oxo-1,2-dihydro-1-pyridyl)-N-(2-tert-butyldimethylsilyloxy-3,3,3-trifluoro-1-isopropylpropyl)-acetamide was coupled with a benzyl bromide possessing the requisite substitution using a procedure similar to that described in Example 64.b. to give the corresponding tert-butyldimethylsilyl ethers:
Example 66.a.: R⁵=3-methylbenzyl.
Example 67.a.: R⁵=2-methylbenzyl.

### EXAMPLES 66.b.-67.b.

The corresponding alcohols of formula II were prepared from their tert-butyldimethylsilyl ethers using a procedure similar to that described in Example 64.c.
Example 66.b.: R⁵=3-methylbenzyl.
Example 67.b.: R⁵=2-methylbenzyl.

### EXAMPLE 68

### 2-[3-Benzyloxycarbonylamino-2-oxo-5-(3-pivaloyloxybenzyl)-1,2-dihydro-1-pyridyl]-N-(3,3,3-trifluoro-1-isopropyl-2-oxopropyl)acetamide.

Using a procedure similar to that described in Example 62 and substituting 3-pivaloyloxytolene for 4-pivaloyloxytolene at Example 62.d., the title compound was prepared; NMR: 1.29 (d,9), 3.72 (s,2 CH₂); MS: m/z=645(M+1).

| Analysis for C₃₃H₃₆F₃N₃O₇·0.5 H₂O: | | | |
|---|---|---|---|
| Calculated: | C, 60.31; | H, 5.75; | N, 6.39 |
| Found: | C, 60.30; | H, 5.63; | N, 6.23 |

### EXAMPLE 69

### 2-[3-Benzyloxycarbonylamino-5-(3-hydroxybenzyl)-2-oxo-1,2-dihydro-1-pyridyl]-N-(3,3,3-trifluoro-1-iso-propyl-2-oxopropyl)acetamide.

2-[3-Benzyloxycarbonylamino-2-oxo-5-(3-pivaloyloxybenzyl)-1,2-dihydro-1-pyridyl]-N-(3,3,3-trifluoro-1-isopropyl-2-oxopropyl)acetamide was hydrolyzed using a procedure similar to that described in Example 63 to afford the title compound; NMR: 3.60 (s, 2H), 9.32 (s,OH); MS: m/z=561(M+1).

| Analysis for C₂₈H₂₈F₃N₃O₆·H₂O: | | | |
|---|---|---|---|
| Calculated: | C, 58.23; | H, 5.24; | N, 7.28 |
| Found: | C, 58.45; | H, 5.12; | N, 6.99 |

### EXAMPLE 70

### 2-[3-Benzyloxycarbonylamino-5-(3-acetoxybenzyl)-2-oxo-1,2-dihydro-1-pyridyl]-N-(3,3,3-trifluoro-1-isopropyl-2-oxopropyl)acetamide.

2-[3-Benzyloxycarbonylamino-5-(3-hydroxybenzyl)-2-oxo-1,2-dihydro-1-pyridyl]-N-(3,3,3-trifluoro-1-isopropyl-2-oxopropyl)acetamide was acylated using conditions similar to those described in Example 14.b., substituting acetic anhydride for phenylacetyl chloride, to give the title compound; NMR: 2.25 (s,3 and m,1), 6.95-7.41 (m,10); MS: m/z=602(M+1).

| Analysis for C₃₀H₃₀F₃N₃O₇·0.5 H₂O: | | | |
|---|---|---|---|
| Calculated: | C, 59.01; | H, 5.12; | N, 6.88 |
| Found: | C, 58.88; | H, 5.15; | N, 6.75 |

### EXAMPLE 71

### 2-(5-Benzyl-3-benzyloxycarbonylamino-2-oxo-1,2-dihydro-1-pyridyl)-N-(3,3,3-trifluoro-1-isopropyl-2-oxopropyl)acetamide.

2-(5-Benzyl-3-benzyloxycarbonylamino-2-oxo-1,2-dihydro-1-pyridyl)-N-(3,3,3-trifluoro-2-hydroxy-1-isopropylpropyl)acetamide was oxidized using a procedure similar to that described in Example 61 to give the title compound; mp 58-64 °C; NMR: 3.69 (s,2, CH₂), 5.12 (s,2, CH₂), 7.20-7.40 (m,11).

| Analysis for C₂₈H₂₈F₃N₃O₅·0.75 H₂O: | | | |
|---|---|---|---|
| Calculated: | C, 60.37; | H, 5.34; | N, 7.54 |
| Found: | C, 60.36; | H, 5.35; | N, 7.44 |

The intermediate 2-(5-benzyl-3-benzyloxycarbonylamino-2-oxo-1,2-dihydro-1-pyridyl)-N-(3,3,3-trifluoro-2-hydroxy-1-isopropyl)acetamide was prepared as follows:

### a. 2-(5-Benzyl-3-benzyloxycarbonylamino-2-oxo-1,2-dihydro-1-pyridyl)-N(2-tert-butyldimethylsiloxy-3,3,3-trifluoro-1-isopropylpropyl)acetamide.

2-(3-Benzyloxycarbonylamino-5-iodo-2-oxo-1,2-dihydro-1-pyridyl)-N-(2-tert-butyldimethylsiloxy-3,3,3-trifluoro-1-isopropylpropyl)acetamide was coupled with benzyl bromide using a procedure similar to that described in Example 62.b. to give the 5-benzyl compound.

### b. 2-(5-Benzyl-3-benzyloxycarbonylamino-2-oxo-1,2-dihydro-1-pyridyl)-N-(3,3,3-trifluoro-2-hydroxy-1-isopropyl)acetamide.

2-(5-Benzyl-3-benzyloxycarbonylamino-2-oxo-1,2-dihydro-1-pyridyl)N-(2-tert-butyl-dimethylsiloxy-3,3,3-trifluoro-1-isopropylpropyl)acetamide was deprotected using a procedure similar to that described in Example 64.c. to afford the corresponding alcohol.

The intermediate 2-(5-benzyl-3-benzyloxycarbonylamino-2-oxo-1,2-dihydro-1-pyridyl)-N-(3,3,3-trifluoro-2-hydroxy-1-isopropyl)acetamide was alternatively prepared as follows:

### c. 5-Benzyl-3-benzyloxycarbonylamino-pyrid-2-one.

3-Benzyloxycarbonylamino-5-iodopyrid-2-one was coupled with benzyl bromide using a procedure similar to that described in Example 62.b. to give the 5-benzyl compound.

### d. Ethyl 5-benzyl-3-benzyloxycarbonylamino-2-oxo-1,2-dihydro-1-pyridylacetate.

5-Benzyl-3-benzyloxycarbonylaminopyrid-2-one was alkylated using a procedure similar to that described in Example 61.d. to give the ester.

### e. 2-(5-Benzyl-3-benzyloxycarbonylamino-2-oxo-1,2-dihydro-1-pyridyl)-N-(3,3,3-trifluoro-2-hydroxy-1-isopropyl)acetamide.

Ethyl 2-(5-benzyl-3-benzyloxycarbonylamino-2-oxo-1,2-dihydro-1-pyridyl)acetate was subjected to a procedure similar to that described in Example 61.f. to yield the alcohol.

### EXAMPLE 72

### 2-[3-Benzyloxycarbonylamino-5-(4-trifluoromethylbenzyl)-2-oxo-1,2-dihydro-1-pyridyl]-N-(3,3,3-trifluoro-1-isopropyl-2-oxopropyl)acetamide.

Using a procedure similar to that outlined in Example 61, but substituting 4-trifluoromethylbenzyl bromide for 3-fluorobenzyl bromide in the step corresponding to Example 61.e., the title compound was obtained; mp 61-65 °C.

| Analysis for C₂₉H₂₇F₆N₃O₅·H₂O: | | | |
|---|---|---|---|
| Calculated: | C, 55.33; | H, 4.64; | N, 6.67 |
| Found: | C, 55.23; | H, 4.54; | N, 6.63 |

### EXAMPLE 73

### 2-(2-Oxo-6-phenyl-3-succinimidomethoxycarbonylamino-1,2-dihydro-1-pyridyl)-N-(3,3,3-trifluoro-1-isopropyl-2-oxopropyl)acetamide.

To a suspension of 2-(3-amino-2-oxo-6-phenyl-1,2-dihydro-1-pyridyl)-N-(3,3,3-trifluoro-1-isopropyl-2-oxopropyl)acetamide (0.50 g) in dichloromethane (15 mL) cooled in an ice bath, was added dropwise a solution of bis(trichloromethyl) carbonate (0.198 g) in dichloromethane (7 mL). The reaction mixture was stirred 15 min in the ice bath, 15 min with the ice bath removed, then the reaction was cooled to a temperature of less than 4 °C. A solution of triethylamine (0.95 mL) in dichloromethane was added dropwise and the mixture was stirred for 25 min. A solution of N-hydroxymethyl succinimide (0.338 g) in dichloromethane (2 mL) was added dropwise and the mixture was stirred 3 h. Bis(trichloromethyl) carbonate (0.032 g) was added and the mixture stirred 1 h. The reaction mixture was diluted with dichloromethane (15 mL) and washed with saturated aqueous ammonium chloride (4 times). The aqueous solutions were combined and extracted with dichloromethane. The combined dichloromethane extracts were washed with brine, dried and evaporated to yield a solid (0.6 g) which was purified by chromatography, eluting with acetonitrile:dichloromethane (2:8), followed by trituration with diethyl ether and drying overnight under high vacuum (75 °C at 13.3 Pa) to give the title compound as an off white powder (0.27 g); mp 216-218 °C (dec); TLC: R_{f}=0.53, dichloromethane:methanol (9:1); NMR: 0.82 (d,3, J=6.6), 0.88 (d,3, J=6.7), 2.12 (m,1), 2.70 (s,4), 4.5 (m,3), 5.45 (s,2), 6.23 (d,1, J=7.7), 7.42 (m,5), 7.85 (d,1, J=7.6), 9.40 (s,1), 9.72 (d,1, J=6.9); IR(KBr): 1725, 1645, 161O cm⁻¹; MS: m/z=551(M+1), 549(M-1) by FAB.

| Analysis for C₂₅H₂₅F₃N₄O₇: | | | |
|---|---|---|---|
| Calculated: | C, 54.55; | H, 4.58; | N, 10.18 |
| Found: | C, 54.30; | H, 4.59; | N, 10.14 |

### EXAMPLE 74

### 2-(3-Acetylamino-2-oxo-1,2-dihydro-1-pyridyl)-N-(3,3,3-trifluoro-1-isopropyl-2-oxopropyl)acetamide.

2-(3-Acetylamino-2-oxo-1,2-dihydro-1-pyridyl)-N-(3,3,3-trifluoro-2-hydroxy-1-isopropylpropyl)acetamide was oxidized using a procedure similar to that described in Example 61 to yield the title compound; mp 76-80 °C; NMR: 0.81-1.24 (m,6), 2.12 (s,3), 2.22 (m,1), 4.13 (m, NHCH hydrate), 4.53-4.89 (m, CH₂CO, NHCH keto), 6.22 (m,1), 6.91 (m, OH hydrate), 7.30 (m,1), 8.21 (d,1, J=7.5), 8.93 (d,CH₂CONH), 9.24 (s, NHCOCH₃), 9.26 (s, NHCOCH₃).

| Analysis for C₁₅H₁₈F₃N₃O₄·H₂O: | | | |
|---|---|---|---|
| Calculated: | C, 47.49; | H, 5.31; | N, 11.08 |
| Found: | C, 47.79; | H, 5.16; | N, 11.07 |

The intermediate 2-(3-acetylamino-2-oxo-1,2-dihydro-1-pyridyl)-N-(3,3,3-trifluoro-2-hydroxyl-1-isopropylpropyl)acetamide can be prepared as follows:

### a. Ethyl 3-amino-2-oxo-1,2-dihydro-1-pyridylacetate.

Ethyl 3-nitro-2-oxo-1,2-dihydro-1-pyridylacetate was reduced using a procedure similar to that outlined in Example 61.a. to give the amine.

### b. Ethyl 3-acetylamino-2-oxo-1,2-dihydro-1-pyridylacetate.

Ethyl 3-amino-2-oxo-1,2-dihydro-1-pyridylacetate was acylated using a procedure similar to Acylation Method A, substituting acetic anhydride for the acid chloride, to give the acetylamino compound.

### c. 2-(3-Acetylamino-2-oxo-1,2-dihydro-1-pyridyl)-N-(3,3,3-trifluoro-2-hydroxy-1-isopropylpropyl)acetamide.

Ethyl 3-acetylamino-2-oxo-1,2-dihydro-1-pyridylacetate was subjected to a procedure similar to that described in Example 61.f. to yield the amide.

### EXAMPLE 75

### 2-(3-Amino-2-oxo-1,2-dihydro-1-pyridyl)-N-(3,3,3-trifluoro-1-isopropyl-2-oxopropyl)acetamide.

2-(3-Nitro-2-oxo-1,2-dihydro-1-pyridyl)-N-(3,3,3-trifluoro-1-isopropyl-2-oxopropyl)acetamide was hydrogenated using a procedure similar to that shown in Example 61.a to yield the title compound; mp 135 °C (dec); NMR: 0.77-0.95 (m,6), 2.02-2.21 (m,1), 4.07 (m, NHCH hydrate), 4.40-4.74 (m, NHCH keto), 5.05 (s br, NH₂), 6.02 (m,1), 6.42 (m,1), 6.78 (m,1), 6.95 (m, OH hydrate), 8.63 (d, J=7).

| Analysis for C₁₃H₁₆F₃N₃O₃·0.5 H₂O: | | | |
|---|---|---|---|
| Calculated: | C, 47.6; | H, 5.2; | N, 12.8 |
| Found: | C, 47.6; | H, 5.5; | N, 11.8 |

The intermediate 2-(3-nitro-2-oxo-1,2-dihydro-1-pyridyl)-N-(3,3,3-trifluoro-1-isopropyl-2-oxopropyl)acetamide was prepared as follows:

### a. Ethyl 3-nitro-2-oxo-1,2-dihydro-1-pyridylacetate.

3-Nitropyrid-2-one was alkylated using a procedure similar to that described in Example 61.d. to yield the ester.

### b. 2-(3-Nitro-2-oxo-1,2-dihydro-1-pyridyl)-N-(3,3,3-trifluoro-2-hydroxy-1-isopropylpropyl)acetamide.

Ethyl 3-nitro-2-oxo-1,2-dihydro-1-pyridylacetate was subjected to a procedure similar to that described in Example 61.f. to yield the amide.

The nitro acid intermediate used in Example 75.b. alternatively can be prepared as follows:

### i. 3-Nitro-2-oxo-1,2-dihydro-1-pyridylacetic acid.

A solution of concentrated sulphuric acid (10 mL) in water (90 mL) was added dropwise to a solution of 1-allyl-3-nitropyrid-2-one (4.0 g) in acetone (40 mL) at about 0 °C. Potassium permanganate (17.42 g) was added in portions to the stirred reaction mixture at 0 °C. After 2 h, sodium bisulphate (9.25 g) was added portionwise at 0 °C. The solution was filtered and the salts washed with water. The acetone was evaporated from the combined filtrates and the resulting aqueous solution was extracted with ethyl acetate. The combined extracts were dried and evaporated to give the nitro acid (2.45 g).

The intermediate 1-allyl-3-nitropyrid-2-one may be prepared as follows:

### ii. 1-Allyl-3-nitropyrid-2-one.

3-Nitropyrid-2-one was alkylated with allyl bromide using a procedure similar to that described in Example 61.d.

### c. 2-(3-Nitro-2-oxo-1,2-dihydro-1-pyridyl)-N-(3,3,3-trifluoro-1-isopropyl-2-oxopropyl)acetamide.

2-(3-Nitro-2-oxo-1,2-dihydro-1-pyridyl)-N-(3,3,3-trifluoro-2-hydroxy-1-isopropylpropyl)acetamide was oxidized using a procedure similar to that described in Example 61 to yield the ketone; mp 60-65°C; NMR: 0.80-1.02 (m,6), 2.24 (m,1), 4.08 (m, NHCH hydrate), 4.66-4.99 (m, NHCH, CH₂CO), 6.48 (m,1), 6.95 (m, OH hydrate), 8.02 (d, J=10, NH hydrate), 8.15 (m,1), 8.45 (m,1), 9.03 (d, J=6.5, NH keto).

| Analysis for C₁₃H₁₄F₃N₃O₅·H₂O: | | | |
|---|---|---|---|
| Calculated: | C, 42.51; | H, 4.39; | N, 11.44 |
| Found: | C, 42.95; | H, 4.08; | N, 11.24 |

### EXAMPLE 76

### 2-[3-(3-phenylpropionylamino)-2-oxo-1,2-dihydro-1-pyridyl]-N-(3,3,3-trifluoro-1-isopropyl-2-oxopropyl)acetamide.

To a stirred suspension of 2-(3-amino-2-oxo-1,2-dihydro-1-pyridyl)-N-(3,3,3-trifluoro-1-isopropyl-2-oxopropyl)acetamide (0.399) in dichloromethane at 0 °C was added 3-phenylpropionyl chloride (0.247 g) and triethylamine (0.247 g). The mixture was allowed to stir for 2 h, was diluted with dichloromethane (100 mL), washed (1 N hydrochloric acid, saturated aqueous sodium bicarbonate, brine), dried, and evaporated to give a residue which was purified by chromatography, using methanol:dichloromethane as an eluent (gradient, 0.5:99.5, 2:98), to yield the title compound; mp 154-155 °C; NMR: 0.80-0.98 (m,6), 2.21 (m,1), 4.09(m, NHCH hydrate), 4.52-4.82 (m, NHCH keto, CH₂CO), 6.23 (m), 7.15-7.34 (m), 7.91 (d, J=10, NH hydrate), 8.24 (dd,1, J=7.5, 1.5), 8.93 (d, NH keto), 9.26 (s, NH hydrate), 9.29 (s, NH keto).

| Analysis for C₂₂H₂₄F₃N₃O₄·0.25 H₂O | | | |
|---|---|---|---|
| Calculated: | C, 57.95; | H, 5.42; | N, 9.22 |
| Found: | C, 57.98; | H, 5.55; | N, 8.90 |

### EXAMPLE 77

### 2-(3-Phenylacetylamino-2-oxo-1,2-dihydro-1-pyridyl)-N-(3,3,3-trifluoro-1-isopropyl-2-oxopropyl)acetamide.

2-(3-Amino-2-oxo-1,2-dihydro-1-pyridyl)-N-(3,3,3-trifluoro-1-isopropyl-2-oxopropyl)acetamide was acylated using a procedure similar to that described in Example 76, substituting phenylacetyl chloride for 3-phenylpropionyl chloride, to give the title compound; mp 70-72 °C.

| Analysis for C₂₁H₂₂F₃N₃O₄·0.50 H₂O: | | | |
|---|---|---|---|
| Calculated: | C, 56.50; | H, 5.19; | N, 9.41 |
| Found: | C, 56.61; | H, 5.28; | N, 9.34 |

### EXAMPLES 78-80

Using a procedure similar to that described in Example 76, the following compounds of Formula I wherein R⁰ is isopropyl, R is the indicated acyl group, R⁵ is hydrogen and R⁶ is hydrogen were prepared by acylation of 2-(3-amino-2-oxo-1,2-dihydro-1-pyridyl)-N-(3,3,3-trifluoro-1-isopropropyl-2-oxopropyl)acetamide with the requisite acid chloride:

Example 78: R=ethoxymalonyl: mp 126-128 °C; NMR: 1.19 (t,3, J=7.1, CH₂CH₃), 3.64 (s,2, COCH₂CO), 4.12 (q,2, J=7.1, CH₂CH₃); MS: m/z=434(M+1).

| Analysis for C₁₈H₂₂F₃N₃O₆·0.25 H₂O: | | | |
|---|---|---|---|
| Calculated: | C, 49.37; | H, 5.18; | N, 9.60 |
| Found: | C, 49.18; | H, 5.21; | N, 9.37 |

Example 79: R=methoxyoxalyl: mp 74-79 °C; NMR: 3.84 (s,3, CO₂CH₃); MS: m/z=406(M+1).

| Analysis for C₁₆H₁₈F₃N₃O₆·0.75 H₂O: | | | |
|---|---|---|---|
| Calculated: | C, 45.88; | H, 4.69; | N, 10.03 |
| Found: | C, 46.01; | H, 4.85; | N, 10.00 |

Example 80: R= methoxysuccinyl.

| Analysis for C₁₈H₂₂F₃N₃O₆·0.75 H₂O: | | | |
|---|---|---|---|
| Calculated: | C, 48.38; | H, 5.30; | N, 9.40 |
| Found: | C, 48.15; | H, 5.28; | N, 9.06 |

### EXAMPLES 81-83

Using a procedure similar to that described in Example 63, the following acids of Formula I wherein R⁰ is isopropyl, R is the indicated acyl group, and R⁵ and R⁶ are hydrogen were prepared by hydrolysis of the corresponding esters described in Examples 78-80:

Example 81: R=hydroxymalonyl: NMR: 3.48 (d,2); MS. m/z=406(M+1).

| Analysis for C₁₆H₁₈F₃N₃O₆·2 H₂O: | | | |
|---|---|---|---|
| Calculated: | C, 43.38; | H, 4.32; | N, 9.37 |
| Found: | C, 43.51; | H, 4.53; | N, 9.34 |

Example 82: R=hydroxyoxalyl; mp 168-170 °C; MS: m/z=392(M+1).

| Analysis for C₁₅H₁₆F₃N₃O₆·0.33 H₂O: | | | |
|---|---|---|---|
| Calculated: | C, 45.35; | H, 4.22; | N, 10.58 |
| Found: | C, 45.33; | H, 4.19; | N, 10.36 |

Example 83: R=hydroxysuccinyl: mp 98-100 °C; NMR: 2.25 (d,2, J=6.0), 2.67 (m,2); MS: m/z=420(M+1).

| Analysis for C₁₇H₂₀N₃O₆F₃·H₂O·HCl: | | | |
|---|---|---|---|
| Calculated: | C, 43.09; | H, 4.89; | N, 8.87 |
| Found: | C, 43.41; | H, 4.56; | N, 8.61 |

### EXAMPLE 84

### 2-(3-Benzyloxyoxalylamino-2-oxo-1,2,-dihydro-1-pyridyl)-N-(3,3,3-trifluoro-1-isopropyl-2-oxopropyl)acetamide.

2-(3-Hydroxyalylamino-2-oxo-1,2-dihydro-1-pyridyl)-N-(3,3,3-trifluoro-1-isopropyl-2-oxopropyl-2-oxopropyl)acetamide (0.15 g), benzyl alcohol (0.49 mL), 4-dimethylaminopyridine (0.14 g), and 1-(3-dimethylaminopropyl)-3-ethylcarbodiiide hydrochloride (0.98 g) were combined in dimethylformamide (4 mL) and allowed to stir for 72 h. The mixture was partitioned between ethyl acetate and 1 N hydrochloric acid, and the organic layer was subsequently washed (saturated aqueous sodium bicarbonate, water, brine), dried, evaporated, and purified using chromatography, with ethyl acetate:dichloromethane (gradient, 10:90, 15:85, 20:80, 30:70) as the eluent, to give the title compound as an off white solid (0.037 g); MS: m/z=482(M+1).

| Analysis for C₂₂H₂₂F₃N₃O₆·0.5 H₂O: | | | |
|---|---|---|---|
| Calculated: | C, 53.88; | H, 4.73; | N, 8.57 |
| Found: | C, 54.02; | H, 4.78; | N, 8.30 |

### EXAMPLE 85

### 2-(3-Aminooxalylamino-2-oxo-1,2-dihydro-1-pyridyl)-N-(3,3,3-trifluoro-1-isopropyl-2-oxopropyl)acetamide.

2-(3-Hydroxyoxalylamino-2-oxo-1,2-dihydro-1-pyridyl)-N-(3,3,3-trifluoro-1-isopropyl-2-oxopropyl)acetamide (0.26 g), the ammonium salt of 1-hydroxybenzotriazole (0.20 g, Bajusz, S. FEBS Lett. (1977), 76, 91), and 1-(3-dimethylaminopropyl)-3-ethylcarbodiimide hydrochloride (0.14 g) were combined in dimethylformamide (5 mL) and allowed to stir overnight. The mixture was partitioned between ethyl acetate and 1 N hydrochloric acid, and the organic layer was subsequently extracted (saturated aqueous sodium bicarbonate, water, brine), dried, evaporated and purified using chromatography, with ethyl acetate:dichloromethane (gradient, 1:1, 3:1, ethyl acetate) as the eluent, to give the title compound as a white solid; mp 258-260 °C; NMR: 8.15 (s,1, NH₂), 8.45 (s,1, NH₂), 9.92 (s,1, NH); MS: m/z=391(M+1).

| Analysis for C₁₅H₁₇F₃N₄O₅·0.50 H₂O: | | | |
|---|---|---|---|
| Calculated: | C, 45.12; | H, 4.54; | N, 14.03 |
| Found: | C, 45.42; | H, 4.63; | N, 13.78 |

### EXAMPLE 86

### 2-(3-Benzylaminooxalylamino-2-oxo-1,2-dihydro-1-pyridyl)-N-(3,3,3-trifluoro-1-isopropyl-2-oxopropyl)acetamide.

2-(3-Hydroxyoxalylamino-2-oxo-1,2-dihydro-1-pyridyl)-N-(3,3,3-trifluoro-1-isopropyl-2-oxopropyl)acetamide, 1-(3-dimethylaminopropyl)-3-ethylcarbodiimide hydrochloride, and benzylamine (1:1.2:1.2) were combined with 4-dimethylaminopyridine (catalyst) in dimethylformamide and the mixture was allowed to stir overnight. The mixture was poured into ethyl acetate, washed (1 N hydrochloric acid, water, brine), dried, evaporated and purified by chromatography to give the title compound; mp 160-164 °C; NMR: 4.39 (d,2, J=6.4, CH₂O), 9.69 (t,1, J=6.4, CH₂NHCO); MS: m/z=481(M+1).

| Analysis for C₂₂H₂₃F₃N₄O₅·0.25 H₂O: | | | |
|---|---|---|---|
| Calculated: | C, 54.49; | H, 4.88; | N, 11.55 |
| Found: | C, 54.66; | H, 4.97; | N, 11.34 |

### EXAMPLE 87

### 2-[3-(4-Chlorophenoxycarbonylamino)-2-oxo-1,2-dihydro-1-pyridyl]-N-(3,3,3-trifluoro-1-isopropyl-2-oxopropyl)acetamide.

2-[3-(4-Chlorophenoxycarbonylamino)-2-oxo-1,2-dihydro-1-pyridyl)-N-(3,3,3-trifluoro-2-hydroxy-1-isopropylpropyl)acetamide was oxidized using a procedure similar to that described in Example 61 to give the title compound; mp 137-139 °C; MS: m/z=474(M+1).

| Analysis for C₂₀H₁₉ClF₃N₃O₅: | | | |
|---|---|---|---|
| Calculated: | C, 50.70; | H, 4.04; | N, 8.87 |
| Found: | C, 50.60; | H, 4.11; | N, 8.81 |

The intermediate 2-[3-(4-chlorophenoxycarbonylamino)-2-oxo-1,2-dihydro-1-pyridyl]-N-(3,3,3-trifluoro-2-hydroxy-1-isopropylpropyl)acetamide was prepared as follows:

### a. 2-(3-Amino-2-oxo-1,2-dihydro-1-pyridyl)-N-(3,3,3-trifluoro-2-hydroxy-1-isopropylpropyl)acetamide.

2-(3-Nitro-2-oxo-1,2,-dihydro-1-pyridyl)-N-(3,3,3-trifluoro-2-hydroxy-1-isopropylpropyl)acetamide was hydrogenated using a procedure similar to that described in Example 61 to give the amine.

### b. 2-[3-(4-Chlorophenoxycarbonylamino)-2-oxo-1,2-dihydro-1-pyridyl]-N-(3,3,3-trifluoro-2-hydroxy-1-isopropylpropyl)acetamide.

2-(3-Amino-2-oxo-1,2-dihydro-1-pyridyl)-N-(3,3,3-trifluoro-2-hydroxy-1-isopropylpropyl)acetamide was acylated using a procedure similar to that described in Example 14.a., except sodium carbonate (2 equivalents) was used as a base instead of triethylamine and 4-chlorophenyl chloroformate was used in place of phenylacetyl chloride, to give the 4-chlorophenoxy compound.

### EXAMPLES 88-94

Using procedures similar to that described in Example 87, the following compounds of Formula I, wherein R⁰ is isopropyl, R is the indicated acyl group and R⁵ and R⁶ are hydrogen, were prepared by substituting the requisite acid chloride in the steps corresponding to Example 87.b.

Example 88: R=4-methoxyphenoxycarbonyl: mp 74-87 °C; NMR: 3.76 (s,3); MS: m/z=470(M+1).

| Analysis for C₂₁H₂₂F₃N₃O₆·0.5 H₂O: | | | |
|---|---|---|---|
| Calculated: | C, 52.72; | H, 4.85; | N, 8.78 |
| Found: | C, 52.61; | H, 4.82; | N, 8.61 |

Example 89: R=isobutoxycarbonyl: mp 68-84 °C; NMR: 0.93 (m, OCH₂CHCH₃), 1.90 (m,1, OCH₂CH), 3.87 (m,2, OCH₂CH); MS: m/z=420(M+1).

| Analysis for C₁₃H₂₄F₃N₃O₅·0.5 H₂O: | | | |
|---|---|---|---|
| Calculated: | C, 50.47; | H, 5.88; | N, 9.81 |
| Found: | C, 50.45; | H, 5.85; | N, 9.56 |

Example 90: R=4-methylphenoxycarbonyl: mp 72-82 °C; NMR: 2.31 (s, CH₃); MS: m/z=454(M+1).

| Analysis for C₂₁H₂₂F₃N₃O₅·0.5 H₂O: | | | |
|---|---|---|---|
| Calculated: | C, 54.55; | H, 5.01; | N, 9.09 |
| Found: | C, 54.39; | H, 5.07; | N, 8.95 |

Example 91: R=4-fluorophenoxycarbonyl: mp 147-150 °C; MS: m/z=458(M+1).

| Analysis for C₂₀H₁₉F₄N₃O₅: | | | |
|---|---|---|---|
| Calculated: | C, 52.52; | H, 4.19; | N, 9.19 |
| Found: | C, 52.30; | H, 4.26; | N, 9.13 |

Example 92: R=phenoxycarbonyl: mp 74-76 °C.

| Analysis for C₂₀H₂₀F₃N₃O₅·0.5 H₂O: | | | |
|---|---|---|---|
| Calculated: | C, 53.57; | H, 4.72; | N, 9.37 |
| Found: | C, 53.66; | H, 4.66; | N, 9.20 |

Example 93: R=cyclopentyloxycarbonyl: mp 71-76 °C; NMR: 1.55-1.84 (m,8, CH₂), 5.07 (m,1, OCH).

| Analysis for C₁₉H₂₄F₃N₃O₅·0.5 H₂O: | | | |
|---|---|---|---|
| Calculated: | C, 51.80; | H, 5.72; | N, 9.50 |
| Found: | C, 51.62; | H, 5.74; | N, 9.30 |

Example 94: R=benzyloxycarbonyl: mp 58-61 °C.

| Analysis for C₂₁H₂₂F₃N₃O₅·0.33 H₂O: | | | |
|---|---|---|---|
| Calculated: | C, 54.55; | H, 5.01; | N, 9.09 |
| Found: | C, 54.86; | H, 5.04; | N, 8.65 |

### EXAMPLE 95

### 2-[2-Oxo-3-(3-phenylureido)-1,2-dihydro-1-pyridyl]-N-(3,3,3-trifluoro-1-isopropyl-2-oxopropyl)acetamide.

2-[2-Oxo-3-(3-phenylureido)-1,2-dihydro-1-pyridyl]-N-(3,3,3-trifluoro-2-hydroxy-1-isopropylpropyl)acetamide was oxidized using a procedure similar to that described in Example 61 to give the title compound; mp 94-106 °C; NMR: 7.25 (m,3, phenyl), 7.45 (m,2, phenyl).

| Analysis for C₂₀H₂₁F₃N₄O₄·0.33 H₂O: | | | |
|---|---|---|---|
| Calculated: | C, 54.05; | H, 4.91; | N, 12.61 |
| Found: | C, 54.36; | H, 5.02; | N, 12.34 |

The intermediate 2-[2-oxo-3-(3-phenylureido)-1,2-dihydro-1-pyridyl]-N-(3,3,3-trifluoro-2-hydroxy-1-isopropylpropyl)acetamide was prepared as follows:

2-(3-Amino-2-oxo-1,2-dihydro-1-pyridyl)-N-(3,3,3-trifluoro-2-hydroxy-1-isopropylpropyl)acetamide (0.43 g) and phenylisocyanate (0.175 g) were combined in dichloromethane (5 mL) and allowed to stir overnight. The mixture was poured into ethyl acetate, washed (saturated aqueous sodium bicarbonate, brine), evaporated, and purified using chromatography, eluting with ethyl acetate:dichloromethane (gradient, 0:100, 50:50, 100:0), to give the urea (0.54 g).

### EXAMPLE 96

### 2-(2-Oxo-6-phenyl-3-ureido-1,2-dihydro-1-pyridyl)-N-(3,3,3-trifluoro-1-isopropyl-2-oxopropyl)acetamide.

To a solution of 2-(3-amino-2-oxo-6-phenyl-1,2-dihydro-1-pyridyl)-N-(3,3,3-trifluoro-1-isopropyl-2-oxopropyl)acetamide (0.30 g) in tetrahydrofuran (5 mL), cooled to 0 °C, was added, dropwise, chlorosulfonyl isocyanate (0.12 g). The reaction mixture was stirred for 10 min, neutralized with saturated aqueous sodium bicarbonate solution (1 mL), diluted with ethyl acetate (10 mL), and the organic phase washed (water, brine), dried (MgSO₄) and evaporated. Purification by chromatography, using an eluant of methylene chloride:methanol (30:1), followed by overnight vacuum-drying (50 °C at 27 Pa), yielded the title product as a white solid (0.23 g); mp 227-230 °C (dec); TLC: R_{f}=0.16, dichloromethane:methanol (20:1); 300 MHz NMR: 0.88 (2d,6), 2.15 (m,1), 4.50 (q,2), 4.65 (d,1), 6.18 (d,1), 6.40 (broad s,2), 7.40 (m,5), 8.08 (d,1), 8.35 (s,1), 8.75 (d,1); IR(KBr): 3470 (broad), 3360, 2980, 1760, 1700, 1535, 1490, 1210, 1160, 1020 cm⁻¹; MS: m/z=439(M+1).

| Analysis for C₂₀H₂₁F₃N₄O₄·0.25 H₂O: | | | |
|---|---|---|---|
| Calculated: | C, 54.24; | H, 4.89; | N, 12.65 |
| Found: | C, 54.12; | H, 4.76; | N, 12.66 |

### EXAMPLE 97

### 2-(5-Benzyl-3-methoxyoxalylamino-2-oxo-1,2-dihydro-1-pyridyl)-N-(3,3,3-trifluoromethyl-1-isopropyl-2-oxopropyl)acetamide.

2-(3-Amino-5-benzyl-2-oxo-1,2-dihydro-1-pyridyl)-N-(3,3,3-trifluoromethyl-1-isopropyl-2-oxopropyl)acetamide was acylated using a procedure similar to that outlined in Example 79 to give the title compound; mp 155-157 °C; NMR: 3.82 (s,3, CH₃OCO); MS: m/z=496(M+1).

| Analysis for C₂₃H₂₄F₃N₃O₆: | | | |
|---|---|---|---|
| Calculated: | C, 55.76; | H, 4.88; | N, 8.48 |
| Found: | C, 55.64; | H, 4.97; | N, 8.47 |

The intermediate amine (which is also an Example of the invention) was prepared as follows:

### a. 2-(3-Amino-5-benzyl-2-oxo-1,2-dihydro-1-pyridyl)-N-(3,3,3-trifluoromethyl-1-isopropyl-2-oxopropyl)acetamide

2-(5-Benzyl-3-benzyloxycarbonylamino-2-oxo-1,2-dihydro-1-pyridyl)-N-(3,3,3-trifluoromethyl-1-isopropyl-2-oxopropyl)acetamide was hydrogenolyzed using conditions similar to those outlined in Example 61.a., except the reaction was carried out at 3 bar in a shaking hydrogenator for 12 h, to give the amine.

### EXAMPLE 98

### 2-(5-Benzyl-3-hydroxyoxalylamino-2-oxo-1,2-dihydro-1-pyridyl)-N-(3,3,3-trifluoromethyl-1-isopropyl-2-oxopropyl)acetamide.

2-(5-Benzyl-3-methoxyoxalylamino-2-oxo-1,2-dihydro-1-pyridyl)N-(3,3,3-trifluoromethyl-1-isopropyl-2-oxopropyl)acetamide was hydrolysed using a procedure similar to that outlined in Example 63 to give the title compound; mp 82-93 °C; MS: m/z=482(M+1).

| Analysis for C₂₂H₂₂F₃N₃O₆·0.5 H₂O: | | | |
|---|---|---|---|
| Calculated: | C, 54.89; | H, 4.61; | N, 8.73 |
| Found: | C, 54.64; | H, 4.69; | N, 8.66 |

### EXAMPLE 99

### 2-(3-Aminooxalylamino-5-benzyl-2-oxo-1,2-dihydro-1-pyridyl)-N-(3,3,3-trifluoromethyl-1-isopropyl-2-oxopropyl)acetamide.

2-(5-Benzyl-3-methoxyoxalylamino-2-oxo-1,2-dihydro-1-pyridyl)-N-(3,3,3-trifluoromethyl-1-isopropyl-2-oxopropyl)acetamide (0.35 g) and concentrated ammonium hydroxide (1 mL) were combined in methanol (1 mL) and allowed to stir for 3 h. The mixture was poured into ethyl acetate, washed (1 N hydrochloric acid), dried, evaporated, and purified by chromatography, eluting with ethyl acetate:dichloromethane (gradient, 25:75, 50:50, 75:25), to give the title compound as a white solid (0.21 g); mp 147-156 °C; NMR: 8.13 (m,2, NH₂COCO); MS: m/z=481(M+1).

| Analysis for C₂₂H₂₃F₃N₄O₅·0.5 H₂O: | | | |
|---|---|---|---|
| Calculated: | C, 53.99; | H, 4.94; | N, 11.45 |
| Found: | C, 54.18; | H, 5.08; | N, 11.63 |

### EXAMPLE 100

### 2-[5-Benzyl-3-(methylaminooxalyl)amino-2-oxo-1,2-dihydro-1-pyridyl]-N-(3,3,3-trifluoromethyl-1-isopropyl-2-oxapropyl)acetamide.

2-(5-Benzyl-3-methoxyoxalylamino-2-oxo-1,2-dihydro-1-pyridyl)-N-(3,3,3-trifluoromethyl-1-isopropyl-2-oxopropyl)acetamide (0.15 g) and 40% aqueous methylamine (2 mL) were combined in methanol (2 mL) and allowed to stir for 2 h. The mixture was poured into ethyl acetate, washed (1 N hydrochloric acid, saturated aqueous sodium bicarbonate, brine), dried, evaporated, and purified by chromatography, eluting with ethyl acetate:dichloromethane (gradient, 50:50, 75:25), to give the title compound as a white solid (0.11 g); mp 177-179 °C; NMR: 2.71 (d,3, J=4.8, CH₃NH); MS: m/z=495(M+1).

| Analysis for C₂₂H₂₅F₃N₄O₅: | | | |
|---|---|---|---|
| Calculated: | C, 55.87; | H, 5.10; | N, 11.33 |
| Found: | C, 55.67; | H, 5.20; | N, 11.02 |

### EXAMPLE 101

### 2-[3-Benzyloxycarbonylamino-5-(3-trifluoroacetylaminobenzyl)-2-oxo-1,2-dihydro-1-pyridyl]-N-(3,3,3-trifluoro-1-isopropyl-2-oxopropyl)acetamide.

2-[3-Benzyloxycarbonylamino-5-(3-trifluoroacetylaminobenzyl)-2-oxo-1,2-dihydro-1-pyridyl]-N-(3,3,3-trifluoro-2-hydroxy-1-isopropylpropyl)acetamide was oxidized using a procedure similar to that outlined in Example 61 to give the title compound; MS: m/z=655(M+1).

| Analysis for C₃₀H₂₈F₆N₄O₆·0.5 H₂O: | | | |
|---|---|---|---|
| Calculated: | C, 54.30; | H, 4.40; | N, 8.44 |
| Found: | C, 54.36; | H, 4.41; | N, 8.40 |

The intermediate 2-[3-benzyloxycarbonylamino-5-(3-trifluoroacetylaminobenzyl)-2-oxo-1,2-dihydro-1-pyridyl]-N-(3,3,3-trifluoro-2-hydroxy-1-isopropylpropyl)acetamide was prepared as follows:

### a. N-Trifluoroacetyl-m-toluidine.

m-Toluidine was acylated using a procedure similar to Acylation Method A, but substituting trifluoroacetic anhydride for the acid chloride, to give the amide.

### b. 3-(Trifluoroacetylamino)benzyl bromide.

N-Trifluoroacetyl-m-toluidine was brominated using a procedure similar to that outlined in Example 62.d. to give the benzyl bromide.

### c. tert-Butyl 3-benzyloxycarbonylamino-5-(3-trifluoroacetylaminobenzyl)-2-oxo-1,2-dihydro-1-pyridylacetate.

3-(Trifluoroacetylamino)benzyl bromide was coupled with tert-butyl 3-benzyloxycarbonylamino-5-iodo-2-oxo-1,2-dihydro-1-pyridylacetate using a procedure similar to that outlined in Example 62.b. to give the benzyl substituted tert-butyl ester.

### d. 2-[3-Benzyloxycarbonylamino-5-(3-trifluoroacetylaminobenzyl)-2-oxo-1,2-dihydro-1-pyridyl]-N-(3,3,3-trifluoro-2-hydroxy-1-isopropylpropyl)acetamide.

tert-Butyl 3-benzyloxycarbonylamino-5-(3-trifluoroacetylaminobenzyl)-2-oxo-1,2-dihydro-1-pyridylacetate was subjected to a procedure similar to that outlined in Example 61.f. to give the alcohol.

### EXAMPLE 102

### 2-[5-(3-Aminobenzyl)-3(methoxycarbonylamino-2-oxo-1,2-dihydro-1-pyridyl]-N-(3,3,3-trifluoro-1-isopropyl-2-oxopropyl)acetamide.

2-[3-Benzyloxycarbonylamino-5-(3-trifluoroacetylaminobenzyl)-2-oxo-1,2-dihydro-1-pyridyl]-N-(3,3,3-trifluoro-1-isopropyl-2-oxopropyl)acetamide (0.19 g), and 1 N sodium hydroxide (3 mL) were combined in methanol and allowed to stir for 6 h. The methanol was evaporated, the residue was dissolved in ethyl acetate, dried, evaporated, and purified by chromatography, eluting with ethyl acetate:dichloromethane (gradient, 0:100, 5:95), to give the title compound (0.070 g).

| Analysis for C₂₂H₂₅F₃N₄O₆·0.66 H₂O: | | | |
|---|---|---|---|
| Calculated: | C, 53.44; | H, 5.37; | N, 11.33 |
| Found: | C, 53.57; | H, 5.33; | N, 11.18 |

### EXAMPLES 103-121

Using a similar procedure to that described in Example 1, the following compounds of formula I wherein R⁰ is isopropyl, R is the indicated acyl group, R⁵ is hydrogen and R⁶ is phenyl were prepared by oxidation of the corresponding alcohols of formula II.

Example 103: R=2-benzyloxycarbonylphenylacetyl: Chromatography solvent: ethyl acetate:dichloromethane (30:70); TLC: R_{f}=0.6, ethyl acetate:dichloromethane (30:70).

Example 104: R=2-methoxycarbonylphenylacetyl: Chromatography solvent: ethyl acetate:hexane (gradient, 30:70 to 100:0); TLC: R_{f}=0.5, methanol:dichloromethane (5:95); MS: m/z=572(M+1).

| Analysis for C₂₉H₂₈F₃N₃O₆·H₂O: | | | |
|---|---|---|---|
| Calculated: | C, 59.08; | H, 5.12; | N, 7.12 |
| Found: | C, 59.06; | H, 5.09; | N, 7.08 |

Example 105: R=(diethoxyphosphoryl)methylaminocarbonyl: Chromatography solvent: methanol:dichloromethane (0:100, 2.5:97.5, 5:95, 7:93); TLC: R_{f}=O.52, methanol:dichloromethane (10:90); MS: m/z=589(M+1).

| Analysis for C₂₅H₃₂F₃N₄O₇·0.75 H₂O: | | | |
|---|---|---|---|
| Calculated: | C, 49.88; | H, 5.61; | N, 9.31 |
| Found: | C, 49.75; | H, 5.38; | N, 8.95 |

Example 106: R=4-methoxycarbonylphenylacetyl: Chromatography solvent: ethyl acetate:dichloromethane (gradient, 0:100, 10:90); TLC: R_{f}=0.46, ethyl acetate:dichloromethane (20:80); MS: m/z=572(M+1).

Example 107: R=3-methoxycarbonylphenylacetyl: Chromatography solvent: ethyl acetate:dichloromethane (gradient, 0:100, 20:80, 40:60); TLC: R_{f}=0.28, ethyl acetate:dichloromethane (20:80); MS: m/z=572(M+1).

Example 108: R=4-pyridylmethylaminocarbonyl: Chromatography solvent: methanol:dichloromethane (10:90); TLC: R_{f}=0.25, methanol:dichloromethane (10:90); MS: m/z=530(M+1).

| Analysis for C₂₆H₂₆F₃N₅O₄: | | | |
|---|---|---|---|
| Calculated: | C, 58.98; | H, 4.95; | N, 13.23 |
| Found: | C, 58.93; | H, 4.93; | N, 13.46 |

Example 109: R=3-pyridylmethylaminocarbonyl: Chromatography solvent: methanol:dichloromethane (gradient, 0:100 to 20:80); MS: m/z=530(M+1).

| Analysis for C₂₆H₂₆F₃N₅O₅·0.75 H₂O: | | | |
|---|---|---|---|
| Calculated: | C, 57.51; | H, 5.10; | N, 12.90 |
| Found: | C, 57.52; | H, 5.03; | N, 12.21 |

Example 110: R=2-(4-pyridyl)ethoxycarbonyl: Chromatography solvent: methanol:dichloromethane (gradient, 3:97 to 5:95); MS: m/z=55(M+1).

| Analysis for C₂₇H₂₇F₃N₄O₅·0.3 H₂O: | | | |
|---|---|---|---|
| Calculated: | C, 58.97; | H, 5.06; | N, 10.19 |
| Found: | C, 58.95; | H, 4.98; | N, 10.13 |

Example 111: R=2-morpholinoethylaminocarbonyl: Chromatography solvent: methanol:dichloromethane (10:90); MS: m/z=552(M+1).

| Analysis for C₂₆H₃₂F₃N₅O₅: | | | |
|---|---|---|---|
| Calculated: | C, 56.62; | H, 5.85; | N, 12.70 |
| Found: | C, 56.35; | H, 6.06; | N, 12.98 |

Example 112: R=ethoxycarbonylaminocarbonyl: Chromatography solvent: methanol:dichloromethane (5:95); MS: m/z=511(M+1).

| Analysis for C₂₃H₂₅F₃N₄O₆·H₂O: | | | |
|---|---|---|---|
| Calculated: | C, 52.27; | H, 5.15; | N, 10.60 |
| Found: | C, 52.08; | H, 4.93; | N, 10.74 |

Example 113: R=3-methoxycarbonylanilinocarbonyl: Chromatography solvent: ethyl acetate:dichloromethane (10:90); MS m/z=587(M+1).

Example 114: R=2-benzyloxycarbonylanilinocarbonyl: Chromatography solvent: ethyl acetate:dichloromethane (gradient, 10:90, 20:80); TLC: R_{f}=0.66, ethyl acetate:dichloromethane (25:75); MS: m/z=649(M+1).

| Analysis for C₂₉H₃₁F₃N₃PO₈·0.25 H₂O: | | | |
|---|---|---|---|
| Calculated: | C, 54.25; | H, 4.94; | N, 6.54 |
| Found: | C, 54.14; | H, 5.00; | N, 6.43 |

Example 115: R=4-(dimethoxyphosphoryl)benzyloxycarbonyl: Chromatography solvent: methanol:dichloromethane (5:95); TLC: R_{f}=0.4, methanol:dichloromethane (10:90); MS: m/z=638(M+1).

Example 116: R=2-pyridylaminocarbonyl: Chromatography solvent: methanol:dichloromethane (gradient, 0:100 to 10:90); TLC: R_{f}=0.65, methanol:dichloromethane (10:90); MS: m/z=516(M+1).

| Analysis for C₂₅H₂₄F₃N₅O₄·0.75 H₂O | | | |
|---|---|---|---|
| Calculated: | C, 56.76; | H, 4.86; | N, 13.24 |
| Found: | C, 56.71; | H, 4.80; | N, 13.16 |

Example 117: R=2-(2-pyridyl)ethoxycarbonyl: MS m/z=545(M+1).

| Analysis for C₂₇H₂₇F₃N₄O₅·0.5 H₂O: | | | |
|---|---|---|---|
| Calculated: | C, 59.59; | H, 5.10; | N, 10.12 |
| Found: | C, 59.38; | H, 4.94; | N, 10.06 |

Example 118: R=2-(2-tert-butoxycarbonylaminothiazol-4-yl)-ethoxycarbonyl: Chromatography solvent: ethyl acetate; MS: m/z=666(M+1).

Example 119: R=1-methylpiperid-4-yloxycarbonyl: Chromatography solvent: methanol:dichloromethane (gradient, 5:95 to 15:85); MS: m/z=537(M+1).

| Analysis for C₂₆H₃₁F₃N₄O₅·H₂O: | | | |
|---|---|---|---|
| Calculated: | C, 56.31; | H, 6.00; | N, 10.10 |
| Found: | C, 56.31; | H, 5.70; | N, 9.97 |

Example 120: R=2-piperidinoethylaminocarbonyl: Chromatography solvent: methanol:dichloromethane:ammonium hydroxide (10:89:1); MS: m/z=550(M+1).

| Analysis for C₂₇H₃₄F₃N₅O₄·0.75 H₂O: | | | |
|---|---|---|---|
| Calculated: | C, 56.68; | H, 6.43; | N, 12.24 |
| Found: | C, 56.67; | H, 6.21; | N, 12.11 |

Example 121: R=5-methylpyrid-2-ylmethoxycarbonyl: Chromatography solvent: methanol:dichloromethane (5:95); MS: m/z=545(M+1).

| Analysis for C₂₇H₂₇F₃N₄O₅·0.5 H₂O: | | | |
|---|---|---|---|
| Calculated: | C, 58.59; | H, 5.10; | N, 10.12 |
| Found | C, 58.54; | H, 5.00; | N, 10.06 |

The corresponding alcohols of Formula II for Examples 103-121 were prepared as follows:

### EXAMPLES 103.a.-121.a.

2-(3-Acylamino-2-oxo-6-phenyl-1,2-dihydro-1-pyridyl)-N-(2-tert-butyldimethylsilyloxy-3,3,3-trifluoro-1-isopropylpropyl)acetamides having the indicated acyl group R were prepared by acylation of 2-(3-amino-2-oxo-6-phenyl-1,2-dihydro-1-pyridyl)-N-(2-tert-butyldimethylsilyloxy-3,3,3-trifluoro-1-isopropylpropyl)acetamide using the Acylation Method noted:

Example 103.a.: R=2-benzyloxycarbonylphenylacetyl: Acylation Method B; Chromatography solvent: diethyl ether:hexane (gradient 30:70, 40:60); TLC: R_{f}=0.7, diethyl ether (40:60);
MS: m/z=764(M+1).

The 2-benzyloxycarbonylphenylacetic acid for the acylation described in 103.a. was prepared as follows:

To a solution of homophthalic acid (15 g) in dimethylformamide (40 mL) was added potassium carbonate (34 g) and then benzyl bromide (21 mL). The resulting solution was allowed to stir for 12 h. The mixture was diluted with ether, washed (saturated aqueous sodium bicarbonate, water, 1 N hydrochloric acid), dried, and evaporated to give an oil which was dissolved in tetrahydrofuran (240 mL) and H₂O (120 mL). To this was added lithium hydroxide and the solution was allowed to stir for 2 h and was acidified to pH 2. The product was extracted into ether and dried. The solvent was evaporated and the resulting oil was crystallized from ether/hexane to yield the mono-benzyl acid (3.7 g); TLC: R_{f}=0.5, ether; MS: m/z=271(M+1).

Example 104.a.: R=2-methoxycarbonylphenylacetyl: Acylation Method B; chromatography solvent: diethyl ether:hexane; (gradient, 60:40 to 100:0), TLC: R_{f}=0.8, methanol:dichloromethane (5:95); MS: m/z=688(M+1).

The 2-methoxycarbonylphenylacetic acid for the acylation described in Example 104.a. was prepared as follows:

Using a procedure similar to that described in Example 103.a.i., for preparing the acid, but substituting methyl iodide for benzyl bromide, the mono-methyl acid was prepared; chromatography solvent: methanol:dichloromethane (10:90), TLC: R_{f}=0.5, methanol:dichloromethane (15:85); MS: m/z=195(M+10).

Example 105.a.: R=(diethoxyphosphoryl)methylaminocarbonyl: Acylation Method D; TLC: R_{f}=0.19, methanol:dichloromethane (4:96); MS: m/z=705(M+1).

The amine for the acylation was prepared as follows.

### i. Benzyloxycarbonylaminomethylphosphonic acid.

To a solution of aminomethylphosphonic acid (2.5 g) in 2 N aqueous sodium hydroxide (23 mL) containing sodium carbonate (4.77 g) and sodium bicarbonate (3.78 g) at 50 °C was added benzyl chloroformate (9.64 mL), portionwise over 1 h. The resulting solution was allowed to stir overnight. The solution was diluted with water (100 mL) and the pH lowered to 1. The product was extracted into ethyl acetate and the organic solvent dried (MgSO₄). The solvent was evaporated and the product crystallized from ethyl acetate to provide the carbamate (4.64 g); MS: m/z=246(M+1).

### ii. Diethyl benzyloxycarbonylaminomethylphosphonate.

A solution of benzyloxycarbonylaminomethylphosphonic acid (3.0 g) and triethyl orthoformate (40 mL) was heated to 90 °C for 48 h. The reaction was diluted with ethyl acetate and washed with 1 N hydrochloric acid. The mixture was dried (MgSO₄) and evaporated to give an oil which was chromatographed, eluting with ethanol:ethyl acetate (gradient, 0.25:99.75 to 2:98), to yield the diethyl phosphonate (1.3 g); TLC: R_{f}=0.36, ethanol:ethyl acetate (0.5:99.5); MS: m/z=302(M+1).

### iii. Diethyl aminomethylphosphonate.

To a solution of diethyl benzyloxycarbonylaminomethyl phosphonate (3.56 g) in tetrahydrofuran (125 mL) and ethanol (100 mL) was added 10% (w/w) palladium on carbon (15% by weight) and the mixture was shaken under a hydrogen atmosphere (2.8 bar) overnight. The catalyst was removed by filtration and the solvent evaporated. The resulting oil was chromatographed, eluting with methanol:dichloromethane (gradient, 1:99 to 10:90), to yield the amine (0.45 g); TLC: R_{f}=0.38, methane:dichloromethane (10:90); MS: m/z=168(M+1).

Example 106.a.: R=4-methoxycarbonylphenylacetyl: Acylation Method B; chromatography solvent: ethyl acetate:dichloromethane (gradient, 0:100, 10:90); TLC: R_{f}=0.59, methanol:dichloromethane (5:95); MS: m/z=688(M+1).

The 4-methoxycarbonylphenylacetic acid for the acylation was prepared as follows:

### i. Methyl 4-hydroxyphenylacetate.

To a solution of 4-hydroxyphenylacetic acid (10 g) in methylene chloride (66 mL) and methanol (26 mL) was added p-toluenesulfonic acid (1.25 g), and the resulting solution was heated at reflux overnight. The solvent was evaporated, the residue dissolved in ethyl acetate, and the the solution washed (H₂O), dried (MgSO₄), and evaporated to give crude methyl 4-hydroxyphenylacetate (10.4 g); TLC: R_{f}=0.72, ethyl acetate:dichloromethane (10:90); MS: m/z=167(M+1).

### ii. Methyl 4-trifluoromethylsulfonyloxyphenylacetate.

To a solution of methyl 4-hydroxyphenyl acetate (5 g) and triethylamine (3.97 g) in methylene chloride (60.2 mL) was added a solution of N-phenyltrifluoromethanesulfonimide (13.97 g) in methylene chloride (20 mL), and the resulting mixture was allowed to stir overnight. The reaction was diluted with ethyl acetate, washed (1 N hydrochloric acid), dried (MgSO₄), evaporated, and the resulting oil was purified by chromatography, eluting with ethyl acetate:dichloromethane:hexane (gradient, 30:15:55, 15:15:70), to afford the-trifluoromethylsulfonyloxy compound (8.56 g); TLC: R_{f}=0.68; MS: m/z=299(M+1).

### iii. Methyl 4-methoxycarbonylphenylacetate.

To a solution of methyl 4-trifluoromethylsulfonyloxyphenylacetate (6 g) dissolved in dimethyl sulfoxide (30 mL) and methanol (5 mL) was added 1,3-bis(diphenylphosphino)propane (0.25 g) and bis(triphenylphosphine)palladium(II) chloride (0.43 g). The reaction was placed under a carbon monoxide atmosphere and the solution heated at 60 °C for 24 h. The reaction was diluted with ethyl acetate, washed (1 N hydrochloric acid, water, brine), dried (MgSO₄), and evaporated to give an oil which was purified by chromatography, eluting with diethyl ether:hexane (7:3), to afford the di-ester (3.15 g); TLC: R_{f}=0.32, diethyl ether:hexane (30:70).

### iv. 4-Methoxycarbonylphenylacetic acid.

To a solution of methyl 4-methoxycarbonylphenylacetate (3.15 g) dissolved in tetrahydrofuran (100 mL), methanol (25 mL) and water (25 mL) at 0 °C was added lithium hydroxide (0.7 g) and the solution was allowed to stir for 2 h. The solution was acidified to pH 2.5 and the product extracted into ethyl acetate. The organic layer was dried (MgSO₄) and evaporated to give an oil which was crystallized from hexane to give the mono-acid; TLC: R_{f}=0.65, methanol:dichloromethane (20:80); MS: m/z=195(M+1).

Example 107.a.: R=3-methoxycarbonylphenylacetyl: Acylation Method B; TLC: R_{f}=0.73, methanol:dichloromethane (5:45); MS: m/z=688(M+1).

The 3-methoxycarbonylphenylacetic acid for the acylation was prepared using procedures similar to those described in Example 106.a.i.-.iv., except 3-hydroxyphenylacetic acid was substituted for 4-hydroxyphenylacetic acid in the step corresponding to 106.a.i:
i. Methyl 3-hydroxyphenylacetate: TLC: R_{f}=0.87, ethyl acetate:dichloromethane (10:90); MS: m/z=167(M+1).
ii. Methyl 3-trifluoromethylsulfonyloxyphenylacetate: Chromatography solvent: diethyl ether:hexane (gradient 10:90, 40:60); TLC: R_{f}=0.5, diethyl ether:hexane (25:75); MS: m/z=299(M+1).
iii. Methyl 3-methoxycarbonylphenylacetate: Chromatography solvent: diethyl ether:hexane (30:70); TLC: R_{f}=0.2, diethyl ether:hexane (30:70).
iv. 3-Methoxycarbonylphenylacetic acid: TLC: R_{f}=0.54, methanol:dichloromethane (20:80); MS: m/z=195(M+1).

Example 108.a.: R=4-pyridylmethylaminocarbonyl: Acylation Method D; chromatography solvent: methanol:dichloromethane (10:90); TLC: R_{f}=0.15, methanol:dichloromethane (5:95); MS: miz=646(M+1).

Example 109.a.: R=3-pyridylmethylaminocarbonyl: Acylation Method D; chromatography solvent: methanol:dichloromethane (5:95); TLC: R_{f}=0.25, methanol:dichloromethane (5:95); MS: m/z=646(M+1).

Example 110.a.: R=2-(4-pyridyl)ethoxycarbonyl: Acylation Method D; chromatography solvent: ethyl acetate; MS: m/z=661(M+1).

Example 111.a.: R=2-morpholinoethylaminocarbonyl: Acylation Method D; chromatography solvent: methanol:dichloromethane (10:90); TLC: R_{f}=0.3, methanol:dichloromethane (10:90); MS: m/z=668(M+1).

Example 112.a.: R=ethoxycarbonylaminocarbonyl: Acylation Method C; chromatography solvent: methane:dichloromethane (10:90); TLC: R_{f}=0.4, ethyl acetate:dichloromethane (10:90); MS: m/z=627(M+1).

Example 113.a.: R=3-methoxycarbonylanilinocarbonyl: Acylation Method C; chromatography solvent: methanol:dichloromethane (5:95); TLC: R_{f}=0.5, methanol:dichloromethane (5:95); MS: m/z=703(M+1).

Example 114.a.: R=2-benzyloxycarbonylanilinocarbonyl: Acylation Method D; chromatography solvent: diethyl ether; MS: m/z=765(M+1).

Example 115.a.: R=4-(dimethoxyphosphoryl)benzyloxycarbonyl: Acylation Method D; chromatography solvent: ethyl acetate; TLC: R_{f}=0.25, ethyl acetate; MS: m/z=754(M+1).

The alcohol for the acylation was prepared as follows:

### i. 4-Iodobenzyl alcohol.

To a solution of 4-iodobenzoic acid (22 g) in tetrahydrofuran (450 mL) was added borane dimethylsulfide complex (35.5 mL, 10 M in tetrahydrofuran), and the solution was allowed to stir overnight. The reaction was quenched by addition of methanol, and the solvent was evaporated. The residue was dissolved in ethyl acetate and filtered. The solvent was evaporated to give a solid, which was recrystallized from ether to yield a white solid, which was chromatographed, eluting with ether, to give 4-iodobenzyl alcohol (15.1 g).

### ii. 4-(tert-Butyldimethylsilyloxymethyl)iodobenzene.

To a solution of 4-iodobenzyl alochol (7 g) in dimethylformamide (30 mL) was added imidazole (4.08 g) and tert-butyldimethylsilyl chloride (5.28 g) and the solution allowed to stir for 0.25 h. The reaction mixture was diluted with ethyl acetate, washed (saturated aqueous ammonium chloride, water, brine), dried (MgSO₄), and evaporated; and the resulting material was chromatographed, eluting with ether:hexane (20:80), to give the tert-butyldimethylsilyl ether as a white solid (8.76 g).

### iii. Dimethyl 4-(tert-butyldimethylsiloxymethyl)phenylphosphonate.

To a solution of 4-(tert-butyldimethylsiloxymethyl)iodobenzene (6.96 g) in dimethylformamide (90 mL) was added dimethyl phosphite (2.64 g) and diisopropylethylamine (4.53 mL). The mixture was placed under an argon atmosphere and tetrakis(triphenylphosphine)-palladium(0) (0.9 g) was added. The solution was heated for 2 h at 80 °C, and the solvent was evaporated. The residue was dissolved in ethyl acetate, washed (water, brine), dried (MgSO₄), and evaporated. The product was purified by chromatography, eluting with ethyl acetate, to give the phosphonate (3.45 g) as an oil; TLC: R_{f}=0.35, ethyl acetate; MS: m/z=331(M+1).

### iv. Dimethyl 4-(hydroxymethyl)phenylphosphonate.

To a solution of dimethyl 4-(tert-butyldimethylsiloxymethyl)-phenylphosphonate (3.4 g) in tetrahydrofuran (20 mL) was added acetic acid (0.6 mL) and tetrabutylammonium floride (15 mL, 1 M in THF), and the resulting solution was allowed to stir for 1 h. The solution was evaporated and the residue dissolved in ethyl acetate, washed (saturated aqueous ammonium chloride, brine), dried (MgSO₄), and evaporated. The product was purified by chromatography (ethyl acetate) to provide the alcohol (1.5 g); TLC: R_{f}=0.15, ethyl acetate; MS: m/z=217(M+1).

Example 116.a.: R=2-pyridylaminocarbonyl: Acylation Method D; TLC: R_{f}=0.65, methanol:dichloromethane (10:90); MS: m/z=632(M+1).

Example 117.a.: R=2-(2-pyridyl)ethoxycarbonyl: Acylation Method D; chromatography solvent: ethyl acetate:dichloromethane (gradient, 20:80, 40:60); TLC: R_{f}=0.6, ethyl acetate; MS: m/z=661(M+1).

Example 118.a.: R=2-[2-(tert-butoxycarbonylamino)thiazol-4-yl]ethoxycarbonyl: Acylation Method D; chromatography solvent: ethyl acetate:hexane (50:50); MS: m/z=782(M+1).

The alcohol for the acylation was prepared as follows:

### i. Ethyl 2-tert-butoxycarbonylamino-4-thiazoleacetate.

To a solution of ethyl 2-amino-4-thiazoleacetate (4.66 g) in tetrahydrofuran (100 mL) was added di-tert-butyl dicarbonate (5.9 g), and the solution was allowed to reflux for 4 h. The solvent was evaporated, and the residue was redissolved in ethyl acetate, washed (saturated aqueous ammonium chloride, brine), dried (MgSO₄), and evaporated. The product was purified by chromatography, eluting with ether, to provide a mixture of monoprotected and diprotected compounds (9.65 g); MS: m/z=287(M+1), monoprotected, m/z=387(M+1), diprotected.

### ii. 2-(2-tert-Butoxycarbonylaminothiazol-4-yl)ethanol.

To the crude product from step i. (9.65 g) in tetrahydrofuran (200 mL) at -78 °C was added dropwise a solution of diisobutylaluminum hydride (75 mL, 1 M in toluene). The mixture was allowed to warm to room temperature and then recooled to -78 °C. The reaction was quenched by addition of ethyl acetate and then brought to room temperature. The mixture was diluted with ethyl acetate, washed (saturated aqueous sodium potassium tartrate), dried (MgSO₄), and evaporated to give an oil which was purified by chromatography, eluting with diethyl ether, to give the alcohol (0.97 g); MS: m/z=245(M+1).

Example 119.a.: R=1-methylpiperid-4-yloxycarbonyl: Acylation Method D; chromatography solvent: methanol:dichloromethane (10:90); MS: m/z=653(M+1).

Example 120.a.: R=2-piperidinoethylaminocarbonyl: Acylation Method D; chromatography solvent: ethyl acetate; TLC: R_{f}=0.15, ethyl acetate.

Example 121.a.: R=5-methylpyrid-2-ylmethoxycarbonyl: Acylation Method D; chromatography solvent: methanol:dichloromethane (5:95); MS: m/z=661(M+1).

### EXAMPLES 103.b.-121.b.

The following alcohols of formula II wherein R⁰ is isopropyl, R is the indicated acyl group, R⁵ is hydrogen and R⁶ is phenyl were prepared by deprotection of the corresponding tert-butyldimethylsilyl ethers using a procedure similar to either that outlined in Example 1.e. or that outlined in Example 19.b. as noted.

Example 103.b.: R=2-benzyloxycarbonylphenylacetyl: Deprotection as in Example 19.b.; chromatography solvent: ethyl acetate:dichloromethane (20:80); TLC: R_{f}=0.35, methanol:dichloromethane (5:95);

Example 104.b.: R=2-methoxycarbonylphenylacetyl: Deprotection as in Example 19.b.; TLC: R_{f}=0.5, diethyl ether.

Example 105.b.: R=(diethoxyphosphoryl)methylaminocarbonyl: Deprotection as in Example 1.e.; chromatography solvent: methanol:dichloromethane (gradient, 3:97, 7:93); TLC: R_{f}=0.38, methanol:dichloromethane (10:90); MS: m/z=591(M+1).

Example 106.b.: R=4-methoxycarbonylphenylacetyl: Deprotection as in Example 19.b.; TLC: R_{f}=0.33, ethyl acetate:dichloromethane (20:80); MS: m/z=574(M+1).

Example 107.b.: R=3-methoxycarbonylphenylacetyl: Deprotection as in Example 19.b.; TLC: R_{f}=0.19, ethyl acetate:dichloromethane (20:80); MS: m/z=574(M+1).

Example 108.b.: R=4-pyridylmethylaminocarbonyl: Deprotection as in Example 1.e.; chromatography solvent: methanol:dichloromethane (10:90); TLC: R_{f}=0.15, methanol:dichloromethane (10:90); MS: m/z=532(M+1).

Example 109.b.: R=3-pyridylmethylaminocarbonyl: Deprotection as in Example 1.e.; chromatography solvent: methanol:dichloromethane (10:90); TLC: R_{f}=0.1, methanol:dichloromethane (5:95); MS: m/z=532(M+1).

Example 110.b.: R=2-(4-pyridyl)ethoxycarbonyl: Deprotection as in Example 1.e.; chromatography solvent: ethyl acetate; MS: m/z=547(M+1).

Example 111.b.: R=2-morpholinoethylaminocarbonyl: Deprotection as in Example 1.e.; chromatography solvent: methanol:dichloromethane (5:95); TLC: R_{f}=0.5, methanol:dichloromethane (5:95).

Example 112.b.: R=ethoxycarbonylaminocarbonyl: Deprotection as in Example 1.e.; chromatography solvent: ethyl acetate:dichloromethane (10:90); MS: m/z=513(M+1).

Example 113.b.: R=3-methoxycarbonylanilinocarbonyl: Deprotection as in Example 1.e.; chromatography solvent: methanol:dichloromethane (5:95); TLC: R_{f}=0.18, methanol:dichloromethane (5:95); MS: m/z=589(M+1).

Example 114.b.: R=2-benxyloxycarbonylanilinocarbonyl: Deprotection as in Example 19.b.; chromatography solvent: ethyl acetate:dichloromethane (25:75); TLC: R_{f}=0.5, ethyl acetate:dichloromethane (25:75); MS: m/z=651(M+1).

Example 115.b.: R=4-(dimethoxyphosphoryl)benzyloxycarbonyl: Deprotection as in Example 1.e.; chromatography solvent: methanol:dichloromethane (10:90); TLC: R_{f}=0.45, methanol:dichloromethane (10:90); MS: m/z=640(M+1).

Example 116.b.: R=2-pyridylaminocarbonyl: Deprotection as in Example 1.e.; chromatography solvent: methanol:dichloromethane (5:95); TLC: R_{f}=0.55, methanol:dichloromethane (10:90); MS: m/z=518(M+1).

Example 117.b.: R=2-(2-pyridyl)ethoxycarbonyl: Deprotection as in Example 19.b.; chromatography solvent: ethyl acetate; TLC: R_{f}=0.4, ethyl acetate; MS: m/z=547(M+1).

Example 118.b.: R=2-(2-tert-butoxycarbonylaminothiazol-4-yl)ethoxycarbonyl: Deprotection as in Example 1.e.; chromatography solvent: ethyl acetate; MS: m/z=668(M+1).

Example 119.b.: R=1-methylpiperid-4-yloxycarbonyl: Deprotection as in Example 1.e.; chromatography solvent: methanol:dichloromethane (gradient, 10:90, 20:80); MS: m/z=539(M+1).

Example 120.b.: R=2-piperidinoethylaminocarbonyl: Deprotection as in Example 1.e.; chromatography solvent: methanol:dichloromethane (gradient, 10:90, 15:85); MS: m/z=552(M+1).

Example 121.b.: R=6-methylpyrid-2-ylmethoxycarbonyl: Deprotection as in Example 1.e.; chromatography solvent: methanol:dichloromethane (5:95); MS: m/z=547(M+1).

### EXAMPLE 122

### 2-[3-(2-Carboxyphenylacetylamino)-2-oxo-6-phenyl-1,2-dihydro-1-pyridyl]-N-(3,3,3-trifluoro-1-isopropyl-2-oxopropyl)acetamide.

To 2-[3-(2-Benzyloxycarbonylphenylacetylamino)-2-oxo-6-phenyl-1,2-dihydro-1-pyridyl]-N-(3,3,3-trifluoro-1-isopropyl-2-oxopropyl)-acetamide (0.6 g) in methanol (50 mL) was added 10% (w/w) palladium on carbon (0.2 g), and the mixture was shaken under a hydrogen atmosphere (3.4 bar) for 3 h. The catalyst was filtered and the solvent evaporated to give a solid which was chromatographed, eluting with methanol:dichloromethane (gradient, 2:98 to 10:90), to give the title compound (0.5 g); TLC: R_{f}=0.25, methanol:dichloromethane (10:90); MS: m/z=588(M+1).

| Analysis for: C₂₈H₂₆F₃O₆N₃·0.5 H₂O: | | | |
|---|---|---|---|
| Calculated: | C, 59.36; | H, 4.80; | N, 7.41 |
| Found: | C, 59.40; | H, 4.91; | N, 7.28 |

### EXAMPLE 123

### 2-[3-[3-(2-Carboxyphenyl)ureido]-2-oxo-6-phenyl-1,2-dihydro-1-pyridyl]-N-(3,3,3-trifluoro-1-isopropyl-2-oxopropyl)acetamide.

2-[3-[3-(2-Benzyloxycarbonylphenyl)ureido]-2-oxo-6-phenyl-1,2-dihydro-1-pyridyl]-N-(3,3,3-trifluoro-1-isopropyl-2-oxopropyl)acetamide was hydrogenated using a procedure similar to that outlined in Example 122 to give the title compound; chromatography solvent: methanol:dichloromethane (gradient, 5:95, 10:90); MS: m/z=559(M+1).

| Analysis for: C₂₇H₂₅F₃N₄O₆·0.75 H₂O: | | | |
|---|---|---|---|
| Calculated: | C, 56.69; | H, 4.67; | N, 9.79 |
| Found: | C, 56.62; | H, 4.61; | N, 9.63 |

### EXAMPLES 124-126

The following compounds of formula I wherein R⁰ is isopropyl, R is the indicated acyl group which contains a carboxy moiety, R⁵ is hydrogen and R⁶ is phenyl were prepared by hydrolysis of the ester groups of corresponding compounds of formula I in which the acyl group R contains an ester moiety, prepared as described in Examples 106, 107, and 113, respectively. In each example, the hydrolysis was carried out using a procedure similar to that described in Example 106.a.iv. to give the acid:

Example 124: R=4-carboxyphenylacetyl: Chromatography solvent: methanol:dichloromethane (gradient, 0:0:100, 35:5:60); TLC: R_{f}=0.45, methanol:dichloromethane (20:80); MS: m/z=558(M+1).

| Analysis for: C₂₇H₂₆F₃N₃O₆: | | | |
|---|---|---|---|
| Calculated: | C, 60.32; | H, 4.70; | N, 7.54 |
| Found: | C, 60.52; | H, 4.87; | N, 7.22 |

Example 125: R=3-carboxyphenylacetyl: Chromatography solvent: methanol:ethyl acetate:dichloromethane (gradient, 0:0:100, 5:35:10, 10:35:55); TLC: R_{f}=0.43, methanol:dichloromethane (20:80), MS: m/z=558(M+1).

| Analysis for: C₂₇H₂₆F₃N₃O₆: | | | |
|---|---|---|---|
| Calculated: | C, 60.32; | H, 4.70; | N, 7.54 |
| Found: | C, 60.04; | H, 4.81; | N, 7.32 |

Example 126: R=3-carboxyanilinocarbonyl: MS: m/z=559(M+1).

| Analysis for: C₂₇H₂₅F₃N₄O₆·0.5 H₂O: | | | |
|---|---|---|---|
| Calculated: | C, 57.14; | H, 4.62; | N, 9.87 |
| Found: | C, 57.24; | H, 4.65; | N, 9.70 |

### EXAMPLE 127

### 2-[2-Oxo-[3-(1-oxopyrid-4-ylmethyl)ureido]-6-phenyl-1,2-dihydro-1-pyridyl]-N-(3,3,3-trifluoro-1-isopropyl-2-oxopropyl)acetamide.

2-[2-Oxo-6-phenyl-3-[3-(4-pyridylmethyl)ureido]-1,2-dihydro-1-pyridyl]-N-(3,3,3-trifluoro-1-isopropyl-2-oxopropyl)acetamide (0.83 g) was combined with dioxirane (40 mL, 0.035 M, Murry J. Org. Chem. (1985), 2847) in acetone (10 mL), and the mixture was allowed to stir for 0.5 h. The solvent was evaporated and the residue purified by chromatography, eluting with methanol:dichloromethane (gradient, 10:90, 20:80), to provide the title compound (0.42 g); TLC: R_{f}=0.5, methanol:dichloromethane (20:80); MS: m/z=546(M+1).

| Analysis for C₂₆H₂₆F₃N₅O₅·0.5 H₂O: | | | |
|---|---|---|---|
| Calculated: | C, 56.32; | H, 4.91; | N, 12.63 |
| Found: | C, 56.13; | H, 4.92; | N, 11.69 |

### EXAMPLE 128

### 2-[2-Oxo-3-[3-(1-oxopyrid-3-ylmethyl)ureido]-6-phenyl-1,2-dihydro-1-pyridyl]-N-(3,3,3-trifluoro-1-isopropyl-2-oxopropyl)-acetamide.

2-[2-Oxo-6-phenyl-3-[3-(3-pyridylmethyl)uriedo]-1,2-dihydro-1-pyridyl]-N-(3,3,3-trifluoro-1-isopropyl-2-oxopropyl)-acetamide was oxidized using a procedure similar to that described in Example 127 to give the title compound; chromatography solvent: methanol:dichloromethane (gradient, 10:90, 20:80); TLC: R_{f}=0.35, methanol:dichloromethane (20:80); MS: m/z=546(M+1), 544(M-1) by FAB.

| Analysis for C₂₆H₂₆F₃N₅O₅·H₂O: | | | |
|---|---|---|---|
| Calculated: | C, 55.53; | H, 5.23; | N, 12.10 |
| Found: | C, 55.70; | H, 5.03; | N, 11.72 |

### EXAMPLE 129

### 2-[2-Oxo-3-[3-(1-oxopyrid-2-yl)ureido]-6-phenyl-1,2-dihydrol-1-pyridyl]-N-(3,3,3-trifluoro-1-isopropyl-2-oxopropyl)-acetamide.

2-[2-Oxo-6-phenyl-3-[3-(2-pyridyl)ureido]-1,2-dihydro-1-pyridyl]-N-(3,3,3-trifluoro-1-isopropyl-2-oxopropyl)acetamide was oxidized using a procedure similar to that described in Example 127 to give the title compound; chromatography solvent: methanol:dichloromethane (10:90); MS: m/z=532(M+1), 530(M-1) by FAB.

| Analysis for C₂₅H₂₄F₃N₅O₅: | | | |
|---|---|---|---|
| Calculated: | C, 56.50; | H, 4.55; | N, 13.18 |
| Found: | C, 54.66; | H, 4.64; | N, 12.72 |

### EXAMPLE 130

### 2-[2-Oxo-6-phenyl-3-[4-(N-phenylsulfonylcarbamoyl)phenylacetylamino]-1,2-dihydro-1-pyridyl]-N-(3,3,3-trifluoro-1-isopropyl-2-oxopropyl)acetamide.

2-[2-Oxo-6-phenyl-3-[4-(N-phenylsulfonylcarbamoyl)phenylacetylamino]-1,2-dihydro-1-pyridyl]-N-(3,3,3-trifluoro-2-hydroxy-1-isopropylpropyl)acetamide was oxidized using a procedure similar to that described in Example 61 to give the title compound; chromatography solvent: methanol:dichloromethane (gradient, 1:99, 3:97); TLC: R_{f}=0.3, methanol:dichloromethane (5:95); MS: m/z=695 (M-1) by FAB.

The intermediate alcohol was prepared as follows:

### a. 2-[3-(4-Carboxyphenylacetylamino)-2-oxo-6-phenyl-1,2-dihydro-1-pyridyl]-N-(3,3,3-trifluoro-2-hydroxy-1-isopropylpropyl)acetamide.

2-[3-(4-Methoxycarbonylphenylacetylamino)-2-oxo-6-phenyl-1,2-dihydro-1-pyridyl]-N-(2-tert-butyldimethylsilyloxy-3,3,3-trifluoro-1-isopropylpropyl)acetamide (1.0 g) was dissolved in tetrahydrofuran:methanol:water (4:1:1) and cooled to 0 °C. Lithium hydroxide (0.312 g) was added and the mixture was allowed to stir for 6 h. The mixture was cooled to -78 °C for 12 h and then allowed to warm to room temperature for 4 h. The mixture was diluted with water and 1N hydrochloric acid to pH 2, extracted with ethyl acetate, dried, evaporated and crystallized from ethyl acetate/hexane to give the hydroxy acid (0.75 g).

### b. 2-[2-Oxo-6-phenyl-3-[4-(N-phenylsulfonylcarbamoyl)phenylacetylamino]-1,2-dihydro-1-pyridyl]-N-(3,3,3-trifluoro-2-hydroxy-1-isopropylpropyl)acetamide.

To a solution of 2-[3-(4-carboxyphenylacetylamino]-2-oxo-6-phenyl-1,2-dihydro-1-pyridyl]-N-(3,3,3-trifluoro-2-hydroxy-1-isopropylpropyl)acetamide (0.52 g), benzenesulfonamide (0.16 g) and 4-dimethylaminopyridine (0.13 g) dissolved in dichloromethane (43 mL) and dimethylformamide (3 mL) was added 1-(3-dimethylaminopropyl)-3-ethylcarbodiimide (0.2 g), and the mixture was allowed to stir for 5 days. The mixture was diluted with ethyl acetate, washed (1N hydrochloric acid), dried (MgSO₄), evaporated, and the resulting material chromatographed, eluting with dichloromethane:methanol (gradient, 0:100, 2.5:97.5, 5:95), to provide material which was rechromatographed, eluting with ethyl acetate:dichloromethane (gradient, 25:75.0, 30:70:0; 0:99:1, 0:97.5:2.5, 0:95:5), to provide the alcohol (0.32 g); TLC: R_{f}=0.74, methanol:dichloromethane (15:85); MS: m/z=699(M+1), 697(M-1) by FAB.

### EXAMPLE 131

### 2-[2-Oxo-6-phenyl-3-[3-(N-phenylsulfonylcarbamoyl)phenylacetylamino]-1,2-dihydro-1-pyridyl]-N-(3,3,3-trifluoro-1-isopropyl-2-oxopropyl)acetamide.

Using a procedure similar to that described in Example 130, substituting 2-[3-(3-methoxycarbonylphenylacetylamino)-2-oxo-6-phenyl-1,2-dihydro-1-pyridyl]-N-(2-tert-butyldimethylsilyloxy-3;3,3-trifluoro-1-isopropylpropyl)acetamide for 2-[3-(4-methoxycarbonylphenylacetylamino)-2-oxo-6-phenyl-1,2,-dihydro-1-pyridyl]-N-(2-tert-butyldimethylsilyloxy-3,3,3-trifluoro-1-isopropylpropyl)acetamide at the step corresponding to Example 130.a., the title compound was prepared; TLC: R_{f}=0.5, methanol:dichloromethane (20:80); MS: m/z=697(M+1), 695(M-1) by FAB.

| Analysis for C₂₂H₂₂F₃N₇O₄·H₂O: | | | |
|---|---|---|---|
| Calculated: | C, 50.47; | H, 4.62; | N, 18.73 |
| Found: | C, 50.71; | H, 4.37; | N, 18.70 |

### EXAMPLE 132

### 2-[2-Oxo-6-phenyl-3-(tetrazol-5-ylacetylamino)-1,2-dihydro-1-pyridyl]-N-(3,3,3-trifluoro-1-isopropyl-2-oxopropyl)acetamide.

2-[2-Oxo-6-phenyl-3-(1-triphenylmethyltetrazol-5-ylacetylamino)-1,2-dihydro-1-pyridyl)-N-(3,3,3-trifluoro-2-hydroxy-1-isopropylpropyl)acetamide was oxidized using a procedure similar to that outlined in Example 61. The material obtained from extraction and solvent removal was redissolved in tetrahydrofuran and treated with 1 N hydrochloric acid to remove the triphenylmethyl protecting group. The solution was allowed to stir for 0.5 hr. The product was extracted into ethyl acetate, dried, evaporated and chromatographed, eluting with methanol:dichloromethane (gradient, 5:95 to 30:70) to give the title compound; TLC: R_{f}=0.5, methanol:dichloromethane (20:80); MS: m/z=506(M+1).

| Analysis for C₂₂H₂₂F₃N₇O₄·H₂O: | | | |
|---|---|---|---|
| Calculated: | C, 50.47; | H, 4.62; | N, 18.73 |
| Found: | C, 50.71; | H, 4.37; | N, 18.70 |

The intermediate alcohol was prepared as follows:

### a. Ethyl 1-triphenylmethyltetrazol-5-ylacetate.

To a solution of ethyl 5-tetrazolylacetate (1 g) (Lofquist et al. J. Amer. Chem. Soc. (1958), 80, 3908) in pyridine (6 mL) was added triphenylmethyl chloride (2.2 g) and the resulting solution allowed to stir for 3 h. The solvent was evaporated and the product crystallized from ether/hexane to give ethyl 3-triphenylmethyltetrazol-5-ylacetate as a white solid (2 g); TLC: R_{f}=0.5, ether:hexane (50:50).

### b. 1-Triphenylmethyltetrazol-5-ylacetic acid.

To a solution of ethyl 1-triphenylmethyltetrazol-5-ylacetate (1.95 g) in tetrahydrofuran (12 mL), methanol (4 mL), and H₂O (4 mL) was added lithium hydroxide (0.61 g) and the resulting solution was allowed to stir for 2 h. The solution was acidified to pH 2, the product extracted into dichloromethane, dried, and evaporated to give the acid as a white solid (1.61 g); TLC: R_{f}=0.2, ether.

### c. 2-[2-Oxo-6-phenyl-3-(1-triphenylmethyltetrazol-5-ylacetylamino)1,2-dihydro-1-pyridyl]-N-(2-tert-butyldimethylsilyloxy-3,3,3-trifluoro-1-isopropylpropyl)acetamide.

2-(3-Amino-2-oxo-6-phenyl-1,2-dihydro-1-pyridyl)-N-(2-tert-butyldimethylsilyloxy-3,3,3-trifluoro-1-isopropylpropyl)acetamide was acylated using a procedure similar to Acylation Method B, with 2-(3-triphenylmethyltetrazol-5-yl)acetic acid as an acylating agent, to give the amide; chromatography solvent: diethyl ether:hexane (70:30); TLC: R_{f}=0.5, diethyl ether.

### d. 2-[2-Oxo-6-phenyl-3-(1-triphenylmethyltetrazol-5-ylacetylamino)-1,2-dihydro-1-pyridyl]-N-(3,3,3-trifluoro-2-hydroxy-1-isopropylpropyl)acetamide.

The tert-butyldimethylsilyl group was removed from 2-[2-oxo-6-phenyl-3-(1-triphenylmethyltetrazol-5-ylacetylamino)-1,2,-dihydro-1-pyridyl]-N-(2-tert-butyldimethylsilyloxy-3,3,3-trifluoro-1-isopropylpropyl)acetamide using a procedure similar to that outlined in Example 1.e. to give the alcohol; chromatography solvent: ethyl acetate:dichloromethane (30:70); TLC: R_{f}=0.5, ethyl acetate:dichloromethane (30:70).

### EXAMPLE 133

### 2-[2-Oxo-6-phenyl-3-[3-(5-tetrazolyl)ureido]-1,2-dihydro-1-pyridyl]-N-(3,3,3-trifluoro-1-isopropyl-2-oxopropyl)acetamide.

2-[2-Oxo-6-phenyl-3-[3-(1-triphenylmethyltetrazol-5-yl)ureido]-1,2-dihydro-1-pyridyl)-N-(3,3,3-trifluoro-2-hydroxy-1-isopropylpropyl)acetamide was subjected to a procedure similar to that outlined in Example 132 to give the title compound; chromatography solvent: methanol:dichloromethane (10:90); MS: m/z=507(M+1), 505(M-1) by FAB.

| Analysis for C₂₁H₂₁F₃N₈O₄·0.75 H₂O: | | | |
|---|---|---|---|
| Calculated: | C, 48.51; | H, 4.36; | N, 21.55 |
| Found: | C, 48.35; | H, 4.12; | N, 21.17 |

The intermediate 2-[2-oxo-6-phenyl-3-[3-(1-triphenylmethyltetrazol-5-yl)ureido]-1,2-dihydro-1-pyridyl)-N-(3,3,3-trifluoro-2-hydroxy-1-isopropylpropyl)acetamide was prepared as follows:

### a. 2-[2-Oxo-6-phenyl-3-[3-(5-tetrazolyl)ureido]-1,2-dihydro-1-pyridyl]-N-(2-tert-butyldimethylsilyloxy-3,3,3-trifluoro-1-isopropylpropyl)acetamide.

2-(3-Amino-2-oxo-6-phenyl-1,2-dihydro-1-pyridyl)-N-(2-tert-butyldimethylsilyloxy-3,3,3-trifluoro-1-isopropylpropyl)acetamide was combined with 5-aminotetrazole using a procedure similar to Acylation Method D to give the urea; chromatography solvent: methanol:dichloromethane (15:85); TLC: R_{f}=0.2, ethyl acetate; MS: m/z=623(M+1), 621(M-1) by FAB.

### b. 2-[2-Oxo-6-phenyl-3-[3-(1-triphenylmethyltetrazol-5-yl)ureido]-1,2-dihydro-1-pyridyl]-N-(2-tert-butyldimethylsilyloxy-3,3,3-trifluoro-1-isopropylpropyl)acetamide.

To a solution of 2-[2-oxo-6-phenyl-3-[3-(5-tetrazolyl)ureido]-1,2-dihydro-1-pyridyl]-N-(2-tert-butyldimethylsilyoxy-3,3,3-trifluoro-1-isopropylpropyl)acetamide (0.15 g) in pyridine (2 mL) was added triphenylmethyl chloride (0.13 g) and the resulting solution was allowed to stir for 3 h. The pyridine was evaporated and the residue was dissolved in ethyl acetate, washed (H₂O, brine), dried (MgSO₄), and evaporated. The product was purified by chromatography, with diethyl ether as the eluent, to give the triphenylmethyl compound; TLC: R_{f}=0.7, diethyl ether; MS: m/z=864(M-1) by FAB.

### c. 2-[2-Oxo-6-phenyl-3-[3-(1-triphenylmethyltetrazol-5-yl)ureido]-1,2-dihydro-1-pyridyl]-N-(3,3,3-trifluoro-2-hydroxy-1-isopropylpropyl)acetamide.

2-[2-Oxo-6-phenyl-3-[3-(1-triphenylmethyltetrazol-5-yl)ureido]-1,2-dihydro-1-pyridyl]-N-(2-tert-butyldimethylsilyloxy-3,3,3-trifluoro-1-isopropylpropyl)acetamide was subjected to a procedure similar to that described in Example 1.e. to give the alcohol; chromatography solvent: diethyl ether; TLC: R_{f}=0.2, diethyl ether.

### EXAMPLE 134

### 2-[3-[2-(2-Aminothiazol-5-yl)ethoxycarbonylamino]-2-oxo-6-phenyl-1,2-dihydro-1-pyridyl]-N-(3,3,3-trifluoro-1-isopropyl-2-oxopropyl)acetamide.

To 2-[3-[2-(2-tert-butoxycarbonylaminothiazol-5-yl)ethoxycarbonylamino]-2-oxo-6-phenyl-1,2-dihydro-1-pyridyl]-N-(3,3,3-trifluoro-1-isopropyl-2-oxopropyl)acetamide (0.9 g) in methylene chloride (5 mL) was added trifluoroacetic acid (1 mL), and the resulting solution was allowed to stir for 3 h. The solvents were evaporated and the residue chromatographed, eluting with methanol:methylene chloride (5:95), to give the title compound (0.46 g); MS: m/z=566(M+1).

### EXAMPLE 135

### 2-[2-Oxo-6-phenyl-3-(3-phenylsulfonylureido)-1,2-dihydro-1-pyridyl]-N-(3,3,3-trifluoro-1-isopropyl-2-oxopropyl)acetamide.

To 2-(3-amino-2-oxo-6-phenyl-1,2-dihydro-1-pyridyl)-N-(3,3,3-trifluoro-1-isopropyl-2-oxopropyl)acetamide (0.5 g) in dichloromethane (40 mL) was added benzenesulfonyl isocyanate (0.23 g), and the resulting solution was allowed to stir for 1 h. The solvent was evaporated and the product was purified by chromatography to provide the title compound (0.7 g); chromatography solvent: methanol:dichloromethane (5:95); TLC: R_{f}=0.6, methanol:dichloromethane (10:90); MS: m/z=579(M+1), 577(M-1) by FAB.

| Analysis for C₂₆H₂₅F₃N₄O₆S·0.5 H₂O: | | | |
|---|---|---|---|
| Calculated: | C, 53.15; | H, 4.46; | N, 9.54 |
| Found: | C, 53.07; | H, 4.48; | N, 9.40 |

### EXAMPLES 136-145

Using a similar procedure to that described in Example 1, the following compounds of Formula I wherein R⁰ is isopropyl, R⁵ is hydrogen, R⁶ is phenyl and R has the indicated value were prepared by oxidation of the corresponding alcohols of formula II.

Example 136: R=methoxymalonyl: Chromatography solvent: dichloromethane:methanol (98:2), then recrystallized from ethyl acetate/hexane; mp 164.5-167 °C; TLC: R_{f}=0.63, chloroform:methanol (9:1); NMR: 0.84 (d,3, J=6.7), 0.89 (d,3, J=6.7), 2.16 (m,1), 3.65 (s,3), 3.70 (s,2), 4.23-4.86 (m,2), 6.22 (d,1, J=7.6), 7.34-7.50 (m,5), 8.30 (d,1, J=7.7), 8.76 (d,1), 9.81 (s,1); IR(KBr): 1645, 1605, 1600, 1530 cm⁻¹; MS: m/z=496(M+1).

| Analysis for C₂₃H₂₄F₃N₃O₆·0.25 H₂O: | | | |
|---|---|---|---|
| Calculated: | C, 55.26; | H, 4.94; | N, 8.40 |
| Found: | C, 55.21; | H, 4.88; | N, 8.33 |

Example 137: R=methoxysuccinyl: Purified by recrystallization from ethyl acetate:2-butanone (10:1); mp 173-175 °C; TLC: R_{f}=0.35, dichloromethane:methanol (20:1); 300 MHz NMR: 0.89 (2d,6), 2.20 (m,1), 2.58 (t,2), 2.75 (t,2), 3.60 (s,3), 4.50 (q,2), 4.63 (t,1), 6.20 (d,1), 7.43 (m,5), 8.27 (d,1), 8.76 (d,1), 9.45 (s,1); IR(KBr): 3320 (broad), 1745, 1650, 1530, 1375, 1220, 1155, 700 cm⁻¹; MS: m/z=510(M+1).

| Analysis for C₂₄H₂₆F₃N₃O₆·0.50 H₂O: | | | |
|---|---|---|---|
| Calculated: | C, 55.60; | H, 5.25; | N, 8.10 |
| Found: | C, 55.71; | H, 5.06; | N, 7.99 |

Example 138: R=oxazolidin-2-on-3-ylacetyl: Chromatography solvent: dichloromethane:methanol (20:1), then recrystallization from ethyl acetate; mp 172-182 °C; TLC: R_{f}=0.4, dichloromethane:methanol (95:5); NMR: 0.83 (d,3), 0.89 (d,3), 2.15 (m,1), 3.61 (t,2), 4.14 (s,2), 4.37 (t,2), 4.46 (d,1), 4.56 (d,1), 4.63 (dd,2), 6.21 (d,1), 7.3-7.5 (m,6), 8.26 (d,1), 8.75 (d,1), 9.67 (s,1); IR(KBr): 1740, 1530 cm⁻¹; MS: m/z=523(M+1).

| Analysis for C₂₄H₂₅F₃N₄O₆: | | | |
|---|---|---|---|
| Calculated: | C, 55.17; | H, 4.82; | N, 10.72 |
| Found: | C, 54.84; | H, 4.82; | N, 10.56 |

Example 139: R=dimethylaminosuccinyl: Chromatography solvent: dichloromethane:methanol (98:2); mp 167-170 °C (dec); TLC: R_{f}=0.35, dichloromethane:methanol (20:1); NMR: 0.88 (2d,6), 2.18 (m,6), 2.65 (2d,4), 2.85 (s,3), 3.00 (s,3), 4.52 (q,2), 4.65 (t,1), 6.22 (d,1), 7.45 (m,5), 8.30 (d,1), 8.77 (d,1), 9.35 (s,1); IR(KBr): 3300, 1760, 1650, 1530 cm⁻¹; MS: m/z=523(M+1).

| Analysis for C₂₄H₂₉F₃N₄O₅·0.75 H₂O: | | | |
|---|---|---|---|
| Calculated: | C, 56.02; | H, 5.73; | N, 10.45 |
| Found: | C, 56.07; | H, 5.46; | N, 10.31 |

Example 140: R=2-benzoxazolinon-3-ylacetyl: Purified by trituration with dietyl ether:hexane (5:95), then with ethyl acetate; mp 252-254 °C (dec); TLC: R_{f}=0.63, chloroform:methanol (9:1); NMR: 0.86 (d,3, J=6.7), 0.92 (d,3, J=6.8), 2.16 (m,1), 4.5-4.75 (m,3), 6.20 (d,1, J=7.7), 7.1-7.34 (m,4), 7.34-7.56 (m,5), 8.22 (d,1, J=7.7), 8.77 (d,1, J=6.5), 10.05 (s,1); IR(KBr): 1775, 1700, 1650, 1610 cm⁻¹; MS: m/z=571(M+1).

| Analysis for C₂₅H₂₅F₃N₄O₆: | | | |
|---|---|---|---|
| Calculated: | C, 58.97; | H, 4.42; | N, 9.82 |
| Found: | C, 58.76; | H, 4.55; | N, 9.81 |

Example 141: R=succinimidoacetyl: Chromatography solvent: dichloromethane:ethyl acetate (1:1); mp 217-222 °C (dec); TLC: R_{f}=0.42, ethyl acetate; NMR: 0.84 (d,3), 0.90 (d,3), 2.14 (m,1), 2.73 (s,4), 4.35 (s,1), 6.19 (d,1, J=7.6), 7.49 (m,5), 8.20 (d,1, J=7.6), 8.76 (d,1, J-7.0), 9.91 (s,1); IR(KBr): 1710, 1645, 1600 cm⁻¹; MS: m/z=535(M+1).

| Analysis for C₂₅H₂₅F₃N₄O₆: | | | |
|---|---|---|---|
| Calculated: | C, 56.18; | H, 4.71; | N, 10.66 |
| Found: | C, 55.98; | H, 4.85; | N, 10.31 |

Example 142: R=2-pyrrolidinon-1-ylacetyl: Chromatography solvent: dichloromethane:methanol (96:4), then preparative (thick layer) TLC, dichloromethane:methanol (9:1); mp 198.5-200.5 °C; TLC: R_{f}=0.65, chloroform:methanol (9:1); NMR: 0.82 (d,3), 0.88 (d,3), 1.95 (broad t,2), 2.15 (m,1), 2.22 (broad t,2, J=7.5), 3.39 (broad t,2, J=6.7), 6.19 (d,1, J=7.4), 7.41 (m,5), 8.23 (d,1, J=7.6), 8.73 (d,1, J=6.7), 9.53 (s,1); MS: m/z=521(M+1).

| Analysis for C₂₅H₂₇F₃N₄O₅: | | | |
|---|---|---|---|
| Calculated: | C, 57.69; | H, 5.23; | N, 10.76 |
| Found: | C, 57.42; | H, 5.27; | N, 10.71 |

Example 143: R=phthalimidoacetyl: Chromatography solvent: dichloromethane:methanol (98:2), then trituration with diethyl ether; mp 229-230 °C; TLC: R_{f}=0.68, dichloromethane:methanol (9:1); 300 MHz NMR: 0.85 (d,3, J=6.7), 0.91 (d,3, J=6.7), 2.33 (m,1), 6.20 (d,1, J=7.7), 7.40 (m,5), 7.92 (m,4), 8.19 (d,1, J=7.6), 8.78 (d,1, J=7.0), 10.05 (s,1); IR(KBr): 1720, 1650 cm⁻¹; MS: m/z=583(M+1).

| Analysis for C₂₉H₂₅F₃N₄O₆: | | | |
|---|---|---|---|
| Calculated: | C, 59.79; | H, 4.32; | N, 9.62 |
| Found: | C, 59.41; | H, 4.33; | N, 9.59 |

Example 144: R=cis-hexahydrophthalimidoacetyl: Chromatography solvent: dichloromethane:tetrahydrofuran (9:1); mp 126-131 °C; TLC: R_{f}=0.61, dichloromethane:tetrahydrofuran (8:2); NMR: 0.82 (d,3, J=6.8), 0.88 (d,3, J=6.7), 1.36 (broad d,4), 1.72 (broad d,4), 2.14 (m,1), 2.98 (broad t,2), 4.32 (s,2), 6.16 (d,1 J=7.7), 7.38 (m,5), 8.18 (d,1 J=7.7), 8.72 (d,1 J=7.1), 9.88 (s,1); IR(KBr): 1710, 1645, 1520 cm⁻¹; EI MS: m/z=588(M).

| Analysis for C₂₉H₃₁F₃N₄O₆·0.5 H₂O: | | | |
|---|---|---|---|
| Calculated: | C, 58.29; | H, 5.40; | N, 9.38 |
| Found: | C, 58.46; | H, 5.36; | N, 9.32 |

Example 145: R=methoxyoxalyl: Chromatography solvent: dichloromethane:methanol (98:2); mp 227-228 °C; TLC: R_{f}=0.30, dichloromethane:methanol (95:5); NMR: 0.82 (d,3 J=6.8), 0.87 (d,3 J=6.7), 2.12 (m,1), 3.94 (s,3), 6.29 (d,1, J=8.0), 7.41 (m,5), 8.26 (d,1, J=7.6), 8.75 (d,1, J=6.9), 9.72 (s,1); IR(KBr): 1755, 1710, 1642 cm⁻¹; MS: m/z=482(M+1).

| Analysis for C₂₂H₂₂F₃N₃O₆·0.25 H₂O: | | | |
|---|---|---|---|
| Calculated: | C, 54.38; | H, 4.67; | N, 8.65 |
| Found: | C, 54.28; | H, 4.64; | N, 8.55 |

The corresponding alcohols of formula II for Examples 136-145 were prepared as follows:

### EXAMPLES 136.a.-145.a.

2-(3-Acylamino-2-oxo-6-phenyl-1,2-dihydro-1-pyridyl-N-(2-tert-butyldimethylsilyloxy-3,3,3-trifluoro-1-isopropylpropyl)acetamides having the indicated acyl group R were prepared from 2-(3-amino-2-oxo-6-phenyl-1,2-dihydro-1-pyridyl)-N-(2-tert-butyl-dimethylsilyloxy-3,3,3-trifluoro-1-isopropylpropyl)acetamide using the indicated acylation method, except as otherwise noted or described.

Example 136.a.: R=methoxymalonyl: Acylation Method A using methyl malonyl chloride and using sodium carbonate instead of triethylamine; not purified, but used directly; TLC: R_{f}=0.69, dichloromethane:methanol (9:1); MS: m/z=612(M+1).

Example 137.a.: R=methoxysuccinyl: Acylation Method A using methyl succinoyl chloride and using sodium carbonate instead of triethylamine; not purified, but used directly; TLC: R_{f}=0.46, dichloromethane:methanol (95:5); MS: m/z=626(M+1).

Example 138.a.: R=oxazolidin-2-on-3-ylacetyl: Acylation Method B using oxazolindin-2-on-3-ylacetic acid (K. Potts J. Org. Chem. (1980), 45, 4985); used directly without further purification; TLC: R_{f}=0.6, dichloromethane:ethyl acetate (1:1); MS: m/z=639(M+1).

Example 139.a.: R=dimethlyaminosuccinyl: Acylation Method B using N,N-dimethylsuccinamic acid; used directly without purification; TLC: R_{f}=0.32, dichloromethane:ethyl acetate (3:1); MS: m/z=639(M+1).

Example 140.a.: R=2-benzoxazolinon-3-ylacetyl: Acylation Method B using 2-benzoxazolinon-3-ylacetic acid (K. Potts J. Org. Chem. (1980), 45, 4985); used directly without further purification; TLC: R_{f}=0.84, dichloromethane:methanol (9:1); MS: m/z=687(M+1).

Example 141.a.: R=succinimidoacetyl: Acylation Method B using succinimidoacetic acid (Sheehan and Loubach J. Amer. Chem. Soc. (1975), 173, 4376); used directly without purification; TLC: R_{f}=0.40, toluene:ethyl acetate (1:1); MS: m/z=651(M+1).

Example 142.a.: R=2-pyrrolidinon-1-ylacetyl: Acylation Method B using 2-pyrrolidinon-1-ylacetyl acid (prepared from the methyl ester); obtained as a 48:52 mixture with methyl 2-pyrrolidinon-1-ylacetate and not purified, but used directly; TLC R_{f}=0.42, ethyl acetate; MS: m/z=637(M+1).

Example 143.a.: R=phthalimidoacetyl: Acylation Method B using phthalimidoacetic acid (Nefkins et al. Recueil (1960), 79, 688); used directly without purification; TLC: R_{f}=0.70, toluene:ethyl acetate (1:1); MS: m/z=699(M+1).

Example 144.a.: R=cis-hexahydrophthalimidoacetyl: Acylation Method B using cis-hexahydrophthalimidoacetic acid (T. Nagase Chem. Pharm. Bull. (1964), 37, 1175); used directly without purification; TLC: R_{f}=0.56, toluene:ethyl acetate (1:1); MS: m/z=705(M+1).

Example 145.a.: R=methoxyoxalyl: Acylation Method A using methyl oxalyl chloride and using sodium carbonate instead of triethylamine; used directly without purification; TLC: R_{f}=0.85, dichloromethane:methanol (95:5); MS: m/z=598(M+1).

### EXAMPLES 136.b.-145.b.

The following alcohols of formula II having the indicated acyl group R, in which R⁰ is isopropyl, R⁵ is hydrogen and R⁶ is phenyl were prepared by cleavage of the corresponding silyl ethers described above using a similar procedure to that described in Example 19.b. (fluoride buffered with acetic acid).

Example 136.b.: R=methoxymalonyl: Used directly without further purification; TLC: R_{f}=0.53, dichloromethane:methanol (9:1); MS: m/z=498(M+1).

Example 137.b.: R=methoxysuccinyl: Chromatography solvent: dichloromethane:methanol (99:1); TLC: R_{f}=0.31, dichloromethane:methanol (95:5); MS: m/z=512(M+1).

Example 138.b.: R=oxazolidin-2-on-3-ylacetyl: Chromatography solvent: dichloromethane:ethyl acetate (gradient, 1:1 to 1:3); TLC: R_{f}=0.25, dichloromethane:methanol (20:1); MS: m/z=525(M+1).

Example 139.b.: R=dimethylaminosuccinyl: Chromatography solvent: dichloromethane:methanol (99:1); TLC: R_{f}=0.32, dichloromethane:methanol (20:1); MS: m/z=525(M+1).

Example 140.b.: R=2-benzoxazolinon-3-ylacetyl: Chromatography solvent: dichloromethane:ethyl acetate (gradient, 3:1, 2:1); TLC: R_{f}=0.67, dichloromethane:ethyl acetate (1:1); MS: m/z=573(M+1).

Example 141.b.: R=succinimidoacetyl: Chromatography solvent: dichloromethane:ethyl acetate (1:2); TLC: R_{f}=0.54, dichloromethane:methanol (9:1); MS: m/z=537(M+1).

Example 142.b.: R=2-pyrrolidinon-1-ylacetyl: Chromatography solvent: dichloromethane:methanol (96:4); TLC: R_{f}=0.54, dichloromethane:methanol (9:1); MS: m/z=523(M+1).

Example 143.b.: R=phthalimidoacetyl: Chromatography solvent: dichloromethane:methanol (98:2); TLC: R_{f}=0.58, dichloromethane:methanol (9:1); MS: m/z=585(M+1).

Example 144.b.: R=cis-hexahydrophthalimidoacetyl: Chromatography solvent: dichloromethane:ethyl acetate (3:2); TLC: R_{f}=0.51, dichloromethane:methanol (9:1); MS: m/z=590(M+1).

Example 145.b.: R=methoxyoxalyl: Chromatography solvent: dichloromethane:methanol (95:5); TLC: R_{f}=0.23, dichloromethane:methanol (95:5); MS: m/z=484(M+1).

### EXAMPLE 146

### 2-(3-Methanesulfonylacetylamino-2-oxo-6-phenyl-1,2-dihydro-1-pyridyl)-N-(3,3,3-trifluoro-1-isopropyl-2-oxopropyl)acetamide.

A flask was charged with 2-(3-amino-2-oxo-6-phenyl-1,2-dihydro-1-pyridyl)-N-(3,3,3-trifluoro-1-isopropyl-2-oxopropyl)acetamide (0.30 g), methanesulfonylacetic acid (0.14 g), 1-(3-dimethylaminopropyl)-3-ethylcarbodiimide hydrochloride (0.19 g), 4-dimethylaminopyridine(0.12 g), and methylene chloride (8 mL); and the mixture was stirred. Over the next 15 min the initial suspension became completely soluble, and the reaction mixture was stirred overnight. The reaction mixture was diluted with ethyl acetate (25 mL), acidified with 1 N aqueous hydrochloric acid, and the organic phase washed (water, brine), dried (MgSO₄) and evaporated to yield a yellow/green solid (0.35 g), which was purified by chromatography, eluting with dichloromethane:tetrahydrofuran (9:1), followed by drying overnight under high vacuum at 50 °C to yield an off-white solid (0.16 g); TLC: R_{f}=0.29, dichloromethane:methanol (20:1); NMR: 0.82 (2d,6), 2.17 (m,1), 3.15 (s,3), 4.55 (m,3), 4.62 (s,2), 6.25 (d,1), 7.42 (m,5), 8.32 (d,1), 8.76 (d,1), 10.0 (S,1); IR(KBr): 3280, 2940, 1770, 1690, 1640, 1530, 1310, 1215, 1150 cm⁻¹; MS: m/z=516(M+1).

| Analysis for C₂₂H₂₄F₃N₃O₆: | | | |
|---|---|---|---|
| Calculated: | C, 51.26; | H, 4.69; | N, 8.15 |
| Found: | C, 51.54; | H, 4.80; | N, 8.29 |

### EXAMPLE 147

### 2-(3-Methoxycarbonylamino-2-oxo-6-phenyl-1,2-dihydro-1-pyridyl)-N-(3,3,3-trifluoro-1-isopropyl-2-oxopropyl)acetamide.

To a suspension of 2-(3-amino-2-oxo-6-phenyl-1,2-dihydro-1-pyridyl)-N-(3,3,3-trifluoro-1-isopropyl-2-oxopropyl)acetamide (0.10 g) and anhydrous sodium carbonate (0.60 g) in tetrahydrofuran (1.5 mL) was added a 1.5 mL tetrahydrofuran solution of methyl chloroformate (0.30 g), and the mixture was stirred overnight. The reaction mixture was diluted with ethyl acetate (5 mL), acidified with 1 N aqueous hydrochloric acid, and the organic phase washed (water, brine), dried (MgSO₄) and evaporated to yield a light-yellow foam (0.08 g). Silica (preparative-plate) chromatography, eluting with methylene chloride:ethyl acetate (6:1), followed by drying overnight under high vacuum at 40 °C, yielded an off-white solid (0.04 g); mp 204-206 °C (dec); TLC: R_{f}=0.24, dichloromethane:ethyl acetate (4:1); NMR: 0.90 (2d,6), 2.20 (m,1), 3.70 (s,3), 4.50 (q,2), 4.65 (t,1), 6.25 (d,1), 7.40 (m,5), 7.90 (d,1), 8.40 (s,1), 8.75 (d,1); IR(KBr): 3380, 3280, 1730, 1680, 1645, 1520, 1370, 1200, 1160 cm⁻¹; MS: m/z=454(M+1).

| Analysis for C₂₁H₂₂F₃N₃O₅: | | | |
|---|---|---|---|
| Calculated: | C, 55.63; | H, 4.89; | N, 9.27 |
| Found: | C, 55.41; | H, 4.91; | N, 8.93 |

### EXAMPLE 148

### 2-(3-Hydroxymalonylamino-2-oxo-6-phenyl-1,2-dihydro-1-pyridyl)-N-(3,3,3-trifluoro-1-isopropyl-2-oxopropyl)acetamide.

A solution of 2-(3-methoxymalonylamino-2-oxo-6-phenyl-1,2-dihydro-1-pyridyl)-N-(3,3,3-trifluoro-1-isopropyl-2-oxopropyl)acetamide (0.87 g) in methanol (10.7 mL) was treated with 1 N sodium hydroxide (3.52 mL). After 1.5 h of stirring the reaction was diluted with water (40 mL), made acidic (pH 1) by addition of 1 N hydrochloric acid, and was extracted with ethyl acetate (40 mL). The extract was washed with brine, dried and evaporated to give a solid. Purification was by reverse phase flash chromatography of the sodium salt over octadecylsilane coated support (from REGIS), using methanol:water (1:1) as the eluent. The appropriate fractions were combined, the methanol evaporated and the product precipitated by addition of 1 N hydrochloric acid. Filtration and drying overnight (75 °C at 13.3 Pa) gave the title compound as a white solid (0.846 g); mp 203-205 °C (dec); TLC: R_{f}=0.5, on ODS reversed phase plates, methanol:water (65:35) adjusted to pH 6.7 with 0.1% aqueous ammonium acetate; NMR: 0.84 (d,3, J=6.7), 0.90 (d,3, J=6.8), 2.14 (m,1), 3.59 (s,2), 4.51 (dd,2), 4.65 (d,1, J=6.5), 6.21 (d,1, J=7.6), 7.42 (m,5), 8.30 (d,1, J=7.2), 8.76 (d,1, J=7.0), 9.82 (s,1); IR(KBr): 1640, 1535, 1500 cm⁻¹; MS: m/z=482(M+1).

| Analysis for C₂₂H₂₂F₃N₃O₆·0.1 H₂O: | | | |
|---|---|---|---|
| Calculated: | C, 54.68; | H, 4.63; | N, 8.70 |
| Found: | C, 54.73; | H, 4.62; | N, 8.70 |

### EXAMPLES 149-150

Using similar procedures to that described in Example 148, the following acids of formula I wherein R⁰ is isopropyl, R⁵ is hydrogen, R₆ is phenyl and R has the indicated values were prepared by hydrolysis of the corresponding esters of formula I described in Examples 137 and 145, respectively.

Example 149: R=hydroxysuccinyl: Purified by trituration with hexane; mp 213 °C (dec); TLC: R_{f}=0.54, on ODS reversed phase plates, methanol:water (60:40); 300 MHz NMR: 0.88 (2d,6), 2.15 (m,1), 3.00 (t,2), 3.20 (t,2), 4.50 (m,2), 4.65 (t,1), 6.20 (t?,1), 7.43 (m,5), 8.25 (d,1), 8.75 (d,1), 9.40 (s,1), 12.15 (broad s,1), IR(KBr): 3310 (broad), 2990, 1765, 1680, 1645, 1530, 1400, 1150 cm⁻¹; MS: m/z=496(M+1).

| Analysis for C₂₃H₂₄F₃N₃O₆·0.5 H₂O: | | | |
|---|---|---|---|
| Calculated: | C, 54.70; | H, 4.99; | N, 8.32 |
| Found: | C, 54.98; | H, 4.84; | N, 8.33 |

Example 150: R=hydroxyoxalyl: Purified by trituration with ethyl acetate; mp 216-218 °C (dec); TLC: R_{f}=0.64, on ODS reversed phase plates, methanol:water (65:35) adjusted to pH 5.7 with 0.1% aqueous ammonium acetate; NMR: 0.84 (d.3, J=6.8), 0.90 (d,3, J=6.7), 2.15 (m,1), 4.53 (q,2), 4.64 (t,1), 6.32 (t,1, J=7.6), 7.43 (m,5), 8.30 (d,1, J=7.6), 8.78 (d,1, J=7.0), 9.75 (s,1); IR(KBr): 1760, 1690, 1680, 1640 cm⁻¹; MS: m/z=468(M+1).

| Analysis for C₂₁H₂₀F₃N₃O₆·0.5 H₂O: | | | |
|---|---|---|---|
| Calculated: | C, 52.94; | H, 4.44; | N, 8.82 |
| Found: | C, 53.19; | H, 4.38; | N, 8.73 |

### EXAMPLES 151-152

Using a similar procedure to that described in Example 1, the following compounds of formula I wherein R⁰ is isopropyl, R⁵ is hydrogen, R⁶ is phenyl and R has the indicated value were prepared by oxidation of the corresponding alcohols of formula II.

Example 151: R=oxazolidin-2-on-3-ylmethoxycarbonyl: Chromatography solvent: dichloromethane:ethyl acetate (3:1); mp 159-161 °C (with gas evolution); TLC: R_{f}=0.66, ethyl acetate; NMR: 0.82 (d,3, J=6.8), 0.88 (d,3, J=6.7), 2.13 (m,1), 3.72 (t,2, J=7.9), 4.32 (t,2, J=7.9), 4.49 (d,1, J=9.5), 4.82 (t,2, J=6.9), 5.32 (s,2), 8.22 (d,1, J=7.6), 7.4-7.5 (m,5), 7.87 (d,1, J=7.6), 8.82 (s,1), 8.74 (d,1, J=6.5); IR(KBr): 1775, 1650, 1610 cm⁻¹; MS: m/z=539(M+1).

| Analysis for C₂₄H₂₅F₃N₄O₇: | | | |
|---|---|---|---|
| Calculated: | C, 53.53; | H, 4.68; | N, 10.40 |
| Found: | C, 53.51; | H, 4.68; | N, 10.29 |

Example 152: R=5-methyl-1,3-dioxacyclohex-5-ylmethoxycarbonyl: Chromatography solvent: dichloromethane:methanol (99:1); mp 82-84 °C; TLC: R_{f}=0.28, dichloromethane:methanol (20:1); NMR: 0.88 (2d,6), 2.18 (m,1), 3.45 (d,2), 3.85 (d,2), 4.15 (s,2), 4.50 (q,2), 4.65 (2d,2), 4.90 (d,1), 6.25 (d,1), 7.42 (m,5), 7.95 (d,1), 8.0 (d,1); IR(KBr): 332O (broad), 2980, 1740, 1650, 1610, 1530, 1500, 1210, 1165 cm⁻¹; MS: m/z=554(M+1).

| Analysis for C₂₆H₃₀F₃N₃O₇·0.33 H₂O: | | | |
|---|---|---|---|
| Calculated: | C, 55.82; | H, 5.52; | N, 7.51 |
| Found: | C, 55.83; | H, 5.55; | N, 7.16 |

The corresponding alcohols of formula II for Examples 151-152 were prepared as follows.

### Examples 151.a.-152.a.

2-(3-Acylamino-2-oxo-6-phenyl-1,2-dihydro-1-pyridyl)-N-(2-tert-butyldimethylsilyloxy-3,3,3-trifluoro-1-isopropylpropyl)acetamides having the indicated acyl group R were prepared from 2-(3-amino-2-oxo-6-phenyl-1,2-dihydro-1-pyridyl)-N-(2-tert-butyldimethylsilyloxy-3,3,3-trifluoro-1-isopropylpropyl)acetamide using triphosgene, triethylamine and an alcohol of formula A.OH by Acylation Method D, as described in Example 22.f.

Example 151.a.: R=oxazolidin-2-on-3-ylmethoxycarbonyl: Using oxazolidin-2-on-3-ylcarbinol (Endo et al. Macromol. Chem. (1968), 112, 49-57; Chem. Abstr. (1968), 69, 3198, Abstract 3190x); used directly without purification; TLC: R_{f}=0.79, dichloromethane:methanol (9:1); MS: m/z=655(M+1).

Example 152.a.: R=5-methyl-1,3-dioxacyclohex-5-ylmethoxycarbonyl: From 5-methyl-1,3-dioxacyclohex-5-ylcarbinol; used directly without purification; TLC: R_{f}=0.61, dichloromethane:ethyl acetate (4:1); MS: m/z=670(M+1).

### Examples 151.b.-152.b.

The following alcohols of formula II having the indicated acyl group R, in which R⁰ is isopropyl, R⁵ is hydrogen and R⁶ is phenyl, were prepared by cleavage of the corresponding silyl ethers described above. The cleavage was carried out using a similar procedure to that described in Example 19.b. (fluoride buffered with acetic acid).

Example 151.b.: R=oxazolidin-2-on-3-ylmethoxycarbonyl: Used directly without purification; TLC: R_{f}=0.46, dichloromethane:methanol (9:1); MS: m/z=424(M+1) for isocyanate resulting from elimination of oxazolidin-2-on-3-ylcarbinol.

Example 152.b.: R=5-methyl-1,3-dioxacyclohex-5-ylmethoxycarbonyl: Chromatography solvent: dichloromethane:ethyl acetate (4:1); TLC: R_{f}=0.28, dichloromethane:methanol (20:1); MS: m/z=556(M+1).

### EXAMPLE 153

### 2-[3-[2,2-bis(hydroxymethyl)propoxycarbonylamino]-2-oxo-6-phenyl-1,2-dihydro-1-pyridyl]-N-(3,3,3-trifluoro-1-isopropyl-2-oxopropyl)acetamide.

To a solution of 2-[3-(5-methyl-1,3-dioxacyclohex-5-ylmethoxycarbonylamino)-2-oxo-6-phenyl-1,2-dihydro-1-pyridyl]-N-(3,3,3-trifluoro-1-isopropyl-2-oxopropyl)acetamide (0.35 g) in methylene chloride (6 mL), cooled to 0 °C, was added dropwise 1.27 mL of a 1.0 M methylene chloride solution of boron trichloride, and the reaction mixture allowed to warm to room temperature over 30 min. The reaction mixture was quenched by pouring into 25 mL of a 15% aqueous sodium chloride solution and stirring 15 min. Ethyl acetate (10 mL) was added, and the organic phase washed with brine, dried (MgSO₄) and evaporated. Chromatography, using as eluant methylene chloride:methanol (20:1), followed by overnight vacuum-drying (50 °C at 27 Pa) yielded the title compound as a white solid (0.25 g); mp 94-97 °C (dec); TLC: R_{f}=0.11, dichloromethane:methanol (95:5); 300 MHz NMR: 0.80 (s,3), 0.85 (s,3), 2.15 (m,1), 3.32 (2d,4), 3.98 (s,2), 4.4-4.6 (m,4), 4.65 (t,1), 6.25 (dd,1), 7.43 (m,5), 7.90 (d,1), 8.40 (d,1), 8.75 (d,1); IR(KBr): 3400 (broad), 2980, 1700, 1650, 1600, 1525, 1215 cm⁻¹; MS: m/z=542(M+1).

| Analysis for C₂₅H₃₀F₃N₃O₇·0.5 H₂O: | | | |
|---|---|---|---|
| Calculated: | C, 54.54; | H, 5.68; | N, 7.63 |
| Found: | C, 54.76; | H, 5.68; | N, 7.50 |

### EXAMPLES 154-158

The following compounds of formula I wherein R⁰ is isopropyl, R is the indicated acyl group, R⁵ is hydrogen and R⁶ is phenyl were prepared by acylation of 2-(3-amino-2-oxo-6-phenyl-1,2-dihydro-1-pyridyl)-N-(3,3,3-trifluoro-1-isopropyl-2-oxopropyl)acetamide using the acylating agent and the Acylation Method noted.

Example 154: R=2-methylphenylcarbonyl: Acylation Method A using 2-methylbenzoyl chloride. Chromatography solvent: hexane:ethyl acetate (2:1); TLC: R_{f}=0.60, dichloromethane:methanol (96:4); MS: m/z=514(M+1).

| Analysis for C₂₇H₂₆F₃N₃O₄·0.3 H₂O: | | | |
|---|---|---|---|
| Calculated: | C, 62.49; | H, 5.17; | N, 8.10 |
| Found: | C, 62.41; | H, 5.31; | N, 7.91 |

Example 155: R=pyrid-4-ylcarbonyl: Acylation Method A using isonicotinoyl chloride hydrochloride and eliminating the work-up wash with 10% hydrochloric acid; purified by precipitation from acetone:hexane. TLC: R_{f}=0.31, dichloromethane:methanol (96:4); MS: m/z=501(M+1).

| Analysis for C₂₅H₂₃F₃N₄O₄·0.65 H₂O: | | | |
|---|---|---|---|
| Calculated: | C, 58.63; | H, 4.78; | N, 10.94 |
| Found: | C, 58.66; | H, 4.81; | N, 10.98 |

Example 156: R=4-fluorophenoxycarbonyl: Acylation Method A using 4-fluorophenyl chloroformate; purified by chromatographing twice, eluting the first column with dichloromethane:methanol (96:4) and eluting the second with dichloromethane:ethyl acetate (97:3); TLC: R_{f}=0.13, dichloromethane:ethyl acetate (97:3); MS: m/z=534(M+1).

| Analysis for C₂₆H₂₃F₄N₃O₅·0.35 H₂O: | | | |
|---|---|---|---|
| Calculated: | C, 57.85; | H, 4.43; | N, 7.78 |
| Found: | C, 57.89; | H, 4.47; | N, 7.83 |

Example 157: R=4-bromophenoxycarbonyl: Acylation Method A using 4-bromophenyl chloroformate. Chromatography solvent: Dichloromethane:ethyl acetate (97:3); TLC: R_{f}=0.17, dichloromethane:ethyl acetate (97:3); MS: m/z=594 (M+1) for ⁷⁹Br.

| Analysis for C₂₆H₂₃BrF₃N₃O₅·0.3 H₂O: | | | |
|---|---|---|---|
| Calculated: | C, 52.07; | H, 3.97; | N, 7.01 |
| Found: | C, 52.07; | H, 4.05; | N, 6.84 |

Example 158: R=4-(dimethylamino)phenoxycarbonyl: Acylation Method D using 4-dimethylaminophenol purified by chromatographing twice, eluting the first column with hexane:ethyl acetate (2:1), then dichloromethane:ethyl acetate (2:1), and eluting the second column with dichloromethane:methanol (99:1). TLC: R_{f}=0.30, dichloromethane:methanol (99:1); MS: m/z=559(M+1).

| Analysis for C₂₈H₂₉F₃N₄O₅·0.2 H₂O: | | | |
|---|---|---|---|
| Calculated: | C, 59.82; | H, 5.27; | N, 9.97 |
| Found: | C, 59.79; | H, 5.36; | N, 9.80 |

The 4-dimethylaminophenol was prepared as follows:

A mixture of 4-methylaminophenol sulfate (1.00 g) and potassium carbonate (0.88 g) in dry tetrahydrofuran (29 mL) was stirred for 45 minutes at room temperature before iodomethane (0.36 mL) was added. After the reaction was stirred for 18 h, TLC showed incomplete reaction. Dimethylformamide (5 mL) was added to make the reaction mixture homogeneous and stirring was continued. After a total of 42 h, the reaction was evaporated and the residue was suspended in ethyl acetate (75 mL). The suspension was washed (saturated sodium bicarbonate, brine), dried, evaporated and dried under vacuum to give the crude product as an oil. The oil was preadsorbed onto silica gel; and chromatography, eluting with hexane:ethyl acetate (2:1), gave 4-dimethylaminophenol as a white solid (0.26 g); TLC: R_{f}=0.35, hexane:ethyl acetate (2:1); MS: m/z=138(M+1).

### EXAMPLE 159

### 2-[3-(4-Aminophenylacetylamino)-2-oxo-6-phenyl-1,2-dihydro-1-pyridyl]-N-(3,3,3-trifluoro-1-isopropyl-2-oxopropyl)acetamide hydrochloride.

2-[3-(4-Nitrophenylacetylamino)-2-oxo-6-phenyl-1,2-dihydro-1-pyridyl]-N-(3,3,3-trifluoro-1-isopropyl-2-oxopropyl)acetamide (0.318 g) was dissolved in absolute ethanol (5 mL). To the solution was added 10% (w/w) palladium on carbon (0.051 g) and the mixture was stirred under a hydrogen atmosphere overnight. The mixture was filtered through diatomaceous earth and evaporated to give a yellow-orange oil. The crude oil was purified by chromatography, eluting with dichloromethane:methanol (gradient, 98.5:1.5, 92:8). The amine was dissolved in dichloromethane and hydrogen chloride gas was bubbled through the solution. Evaporation gave the salt, which was purified by trituration with ether:dichloromethane, followed by crystallization from ethyl acetate:hexane:ether to give the title compound as a tan powder; free base TLC: R_{f}=0.45, dichloromethane: methanol (96:4); MS: m/z=529(M+1).

| Analysis for C₂₇H₂₇F₃N₄O₄·1.0 HCl·0.5 H₂O: | | | |
|---|---|---|---|
| Calculated: | C, 56.50; | H, 5.09; | N, 9.76 |
| Found: | C, 56.36; | H, 5.25; | N, 9.71 |

The starting nitro compound was prepared by using Acylation Method A (but excluding triethyl amine), using N-succinimidyl 4-nitrophenylacetate and 2-(3-amino-2-oxo-6-phenyl-1,2-dihydro-1-pyridyl)-N-(3,3,3-trifluoro-1-isopropyl-2-oxopropyl)-acetamide and used without purification; TLC: R_{f}=0.41, dichloromethane:methanol (97.3); MS: m/z=559(M+1).

### EXAMPLE 160

### 2-(3-Aminoacetylamino-2-oxo-6-phenyl-1,2-dihydro-1-pyridyl)-N-(3,3,3-trifluoro-1-isopropyl-2-oxopropyl)acetamide.

Using a procedure similar to that described in Example 49 for removal of the benzyloxycarbonyl group with trifluoromethane sulfonic acid, and purifying by trituration with ether:dichloromethane followed by partitioning between water and ethyl acetate, drying, evaporating, and drying under vacuum, 2-(3-benzyloxycarbonylaminoacetylamino-2-oxo-6-phenyl-1,2-dihydro-1-pyridyl)-N-(3,3,3-trifluoro-1-isopropyl-2-oxopropyl)acetamide was converted into the title compound; TLC: R_{f}=0.47, dichloromethane:methanol (9:1); MS: m/z=453(M+1).

| Analysis for C₂₁H₂₃F₃N₄O₄·0.5 H₂O: | | | |
|---|---|---|---|
| Calculated: | C, 54.66; | H, 5.24; | N, 12.14 |
| Found: | C, 54.70; | H, 5.19; | N, 11.82 |

The starting material ketone was prepared as follows:

### a. 2-(3-Benzyloxycarbonylaminoacetylamino-2-oxo-6-phenyl-1,2-dihydro-1-pyridyl)-N-(2-tert-butyldimethylsilyloxy-3,3,3-trifluoro-1-isopropylpropyl)acetamide.

Using N-benzyloxycarbonylglycine and a procedure similar to that described in Acylation Method B, the amide was prepared. Chromatography solvent: Dichloromethane:methanol (99:1, 90:10); TLC: R_{f}=0.38, dichloromethane:methanol (98:2); MS: m/z=703(M+1).

### b. 2-(3-Benzyloxycarbonylaminoacetylamino-2-oxo-6-phenyl-1,2-dihydro-1-pyridyl)-N-(3,3,3-trifluoro-2-hydroxy-1-isopropylpropyl)acetamide.

Cleavage of the silyl ether of 2-(3-benzyloxycarbonylaminoacetylamino-2-oxo-6-phenyl-1,2-dihydro-1-pyridyl)-N-(2-tert-butyldimethylsilyloxy-3,3,3-trifluoro-1-isopropylpropyl)acetamide was carried out using a similar procedure to that described in Example 1.e. The alcohol was purified by trituration with dichloromethane; TLC: R_{f}=0.32, dichloromethane:methanol (96:4); MS: m/z=589(M+1).

### c. 2-(3-Benzyloxycarbonylaminoacetylamino-2-oxo-6-phenyl-1,2-dihydro-1-pyridyl)-N-(3,3,3-trifluoro-1-isopropyl-2-oxopropyl)acetamide.

Using a similar procedure to that described in Example 1, 2-(3-benzyloxycarbonylaminoacetylamino-2-oxo-6-phenyl-1,2-dihydro-1-pyridyl)-N-(3,3,3-trifluoro-2-hydroxy-1-isopropylpropyl)acetamide was oxidized to give the ketone which was purified by trituration with dichloromethane followed by chromatography, eluting with dichloromethane:methanol (98:2, 90:10); TLC: R_{f}=0.37, dichloromethane:methanol (96:4); MS: m/z=587(M+1).

### EXAMPLE 161

### 2-(3-Amino-5-methyl-2-oxo-6-phenyl-1,2-dihydro-1-pyridyl)-N-(3,3,3-trifluoro-1-isopropyl-2-oxopropyl)acetamide.

2-(3-benzyloxycarbonylamino-5-methyl-2-oxo-6-phenyl-1,2-dihydro-1-pyridyl)-N-(3,3,3-trifluoro-1-isopropyl-2-oxopropyl)acetamide was subject to conditions similar to those described in Example 49. The resulting mixture was purifying by trituration with ether to give the title compound; TLC: R_{f}=0.08, chloroform:methanol (98:2); MS: m/z=410(M+1).

| Analysis for C₂₀H₂₂F₃N₃O₃·0.9 H₂O: | | | |
|---|---|---|---|
| Calculated: | C, 56.44; | H, 5.64; | N, 9.87 |
| Found: | C, 56.66; | H, 5.33; | N, 9.86 |

### EXAMPLE 162

### 2-[3-Amino-6-(3,5-dimethoxyphenyl)-2-oxo-1,2-dihydro-1-pyridyl]-N-(3,3,3-trifluoro-1-isopropyl-2-oxopropyl)acetamide.

2-[3-Benzyloxycarbonylamino-6-(3,5-dimethoxyphenyl)-2-oxo-1,2-dihydro-1-pyridyl]-N-(3,3,3-trifluoro-1-isopropyl-2-oxopropyl)acetamide was subjected to the conditions described in Example 159, except with shaking under a 2.7 bar hydrogen atmosphere, to give the title compound. Chromatography solvent: Dichloromethane:methanol (98:2, 95:5); TLC: R_{f}=0.35, dichloromethane:methanol (95:5); MS: m/z=456(M+1).

| Analysis for C₂₁H₂₄F₃N₃O₅·0.65 H₂O: | | | |
|---|---|---|---|
| Calculated: | C, 53.99; | H, 5.46; | N, 9.00 |
| Found: | C, 53.94; | H, 5.41; | N, 8.75 |

### Example 163

### 2-(3-Dimethylaminooxycarbonylamino-2-oxo-6-phenyl-1,2-dihydro-1-pyridyl)-N-(3,3,3-trifluoro-1-isopropyl-2-oxopropyl)acetamide.

Using a procedure similar to that described in Example 73 and substituting N,N-dimethylhydroxylamine for N-hydroxymethyl succinimide, the title compound was prepared: Chromatography solvent: dichloromethane:acetonitrile; TLC: R_{f}=0.55, dichloromethane:methanol (90:10); MS: m/z=483(M+1).

| Analysis for C₂₂H₂₅F₃N₄O₅: | | | |
|---|---|---|---|
| Calculated: | C, 54.76; | H, 5.22; | N, 11.61 |
| Found: | C, 54.54; | H, 5.22; | N, 11.56 |

### EXAMPLES 164-167

Using a procedure similar to that described in Example 1, the following compounds of formula I wherein R⁰ is isopropyl, R is the indicated acyl group, R⁵ is hydrogen and R⁶ is phenyl were prepared by oxidation of the corresponding alcohols of formula II.

Example 164: R=2,6-dichloropyrid-4-ylmethoxycarbonyl: Chromatography solvent: dichloromethane:methanol (99.5:0.5, 99:1); TLC: R_{f}=0.39, dichloromethane:methanol (98:2); MS: m/z=600(M+1).

| Analysis for C₂₆H₂₃Cl₂F₃N₄O₅·0.5 H₂O: | | | |
|---|---|---|---|
| Calculated: | C, 51.32; | H, 3.97; | N, 9.20 |
| Found: | C, 51.31; | H, 3.84; | N, 8.99 |

Example 165: R=2-thenyloxycarbonyl: Chromatography solvent: dichloromethane:methanol (99.5:0.5, 99:1, 98.5:1.5); TLC: R_{f}=0.51, dichloromethane:methanol (98:2); MS: m/z=536(M+1).

| Analysis for C₂₅H₂₄F₃N₃O₅S·0.7 H₂O: | | | |
|---|---|---|---|
| Calculated: | C, 54.77; | H, 4.67; | N, 7.66 |
| Found: | C, 54.72; | H, 4.85; | N, 7.40 |

Example 166: R=3-thenyloxycarbonyl: Chromatography solvent: dichloromethane:methanol (99.5:0.5, 99:1, 98.5:0.5); TLC: R_{f}=0.52, dichloromethane:methanol (98:2); MS: m/z=536(M+1).

| Analysis for C₂₅H₂₄F₃N₃O₅S·0.7 H₂O: | | | |
|---|---|---|---|
| Calculated: | C, 54.77; | H, 4.67; | N, 7.66 |
| Found: | C, 54.69; | H, 4.70; | N, 7.41 |

Example 167: R=trifluoroacetyl: Not chromatographed but purified by trituration with diethyl ether followed by recrystallization with ethyl acetate:hexane; TLC: R_{f}=0.30, dichloromethane:methanol (96:4); MS: m/z=492(M+1).

| Analysis for C₂₁H₁₉F₆N₃O₄·0.25 H₂O: | | | |
|---|---|---|---|
| Calculated: | C, 50.80; | H, 3.96; | N, 8.47 |
| Found: | C, 50.74; | H, 3.97; | N, 8.44 |

### EXAMPLES 164.a.-167.a.

2-(3-Acylamino-2-oxo-6-phenyl-1,2-dihydro-1-pyridyl)-N-(2-tert-butyldimethylsilyloxy-3,3,3-trifluoro-1-isopropyl)acetamides having the indicated acyl group R were prepared by acylation of 2-(3-amino-2-oxo-6-phenyl-1,2-dihydro-1-pyridyl)-N-(2-tert-butyldimethylsilyloxy-3,3,3-trifluoro-1-isopropyl)acetamide using the Acylation Method and the acylating agent noted.

Example 164.a.: R=2,6-dichloropyrid-4-ylmethoxycarbonyl: Acylation Method D with 2,6-dichloropyrid-4-ylcarbinol; chromatography solvent: dichloromethane:methanol (99:1); TLC: R_{f}=0.95, dichloromethane:methanol (9:1); MS: m/z=716(M+1).

Example 165.a.: R=2-thenyloxycarbonyl: Acylation Method D with 5 equivalents of 2-thienylcarbinol; chromatography solvent: dichloromethane:methanol (99.5:0.5) (first column) and dichloromethane:acetone (99.5:0.5) (second column); TLC: R_{f}=0.80, dichloromethane:methanol (98:2); MS: m/z=652(M+1).

Example 166.a.: R=3-thenyloxycarbonyl: Acylation Method D with 5 equivalents of 3-thienylcarbinol; chromatography solvent: dichloromethane:methanol (99.5:0.5) (two columns); TLC: R_{f}=0.76, dichloromethane:methanol (98:2); MS: m/z=652(M+1).

Example 167.a.: R=trifluoroacetyl: Acylation method A with trifluoroacetic anhydride, and dichloromethane in place of tetrahydrofuran; used without further purification; TLC: R_{f}=0.55, dichloromethane:methanol (99:1); MS: m/z=608(M+1).

### EXAMPLES 164.b.-167.b.

The following alcohols of formula II wherein R⁰ is isopropyl, R is the indicated acyl group, R⁵ is hydrogen and R⁶ is phenyl were prepared by deprotection of the corresponding tert-butyldimethylsilyl ethers using a procedure similar to either that outline in Example 1.e. or that outlined in Example 19.b. as noted.

Example 164.b.: R=2,6-dichloropyrid-4-ylmethoxycarbonyl: Deprotection as in Example 1.e.; chromatography solvent: dichloromethane:methanol (99:1, 98:2, 95:5); TLC: R_{f}=0.47, dichloromethane:methanol (95:5); MS: m/z=602(M+1).

Example 165.b.: R=2-thenyloxycarbonyl: Deprotection as in Example 1.e.; chromatography solvent: dichloromethane:methanol (gradient, 99.5:0.5, 99:1, 98.5:1.5); TLC: R_{f}=0.37, dichloromethane:methanol (98:2); MS: m/z=538(M+1).

Example 166.b.: R=3-thenyloxycarbonyl: Deprotection as in Example 1.e.; chromatography solvent: dichloromethane:methanol (gradient, 99.5:0.5, 99:1, 98.5:1.5); TLC: R_{f}=0.37, dichloromethane:methanol (98:2); MS: m/z=538(M+1).

Example 167.b.: R=trifluoroacetyl: Deprotection as in Example 19.b. and used without purification; TLC: R_{f}=0.25, dichloromethane:methanol (96:4); MS: m/z=494(M+1).

### EXAMPLE 168

### 2-(2-Oxo-6-phenyl-3-pyruvoylamino-1,2-dihydro-1-pyridyl)-N-(3,3,3-trifluoro-1-isopropyl-2-oxopropyl)acetamide.

2-(3-Amino-2-oxo-6-phenyl-1,2-dihydro-1-pyridyl)-N-(3,3,3-trifluoro-1-isopropyl-2-oxopropyl)acetamide was subjected to procedure similar to Acylation Method B, but substituting: dichloromethane for tetrahydrofuran, 4-dimethylaminopyridine for 1-hydroxybenzotriazole, and pyruvic acid for 4-methoxyphenyl acetic acid. After 72 h, dichloromethane was added and the mixture was washed (water, brine), dried (magnesium sulfate) and evaporated. Chromatography, eluting with dichloromethane:methanol (gradient, 99.5:0.5, 99:1), gave the title compound as a pale yellow solid; TLC: R_{f}=0.44, dichloromethane:methanol (98:2); MS: m/z=466(M+1).

| Analysis for C₂₂H₂₂F₃N₃O₅·0.6 H₂O: | | | |
|---|---|---|---|
| Calculated: | C, 55.48; | H, 4.91; | N, 8.82 |
| Found: | C, 55.43; | H, 4.83; | N, 8.77 |

### EXAMPLE 169

### 2-[3-(4-Aminobenzoylamino)-2-oxo-6-phenyl-1,2-dihydro-1-pyridyl]-N-(3,3,3-trifluoro-1-isopropyl-2-oxopropyl)acetamide.

To a suspension of 2-[3-(4-nitrobenzoylamino)-2-oxo-6-phenyl-1,2-dihydro-1-pyridyl]-N-(3,3,3-trifluoro-1-isopropyl-2-oxopropyl)acetamide (0.38 g) in ethanol (20 mL), was added tin(II) chloride dihydrate (0.79 g). This mixture was heated to reflux for 1 h then cooled to ambient temperature. The reaction mixture was poured into water and the pH adjusted to 7-8 with saturated sodium bicarbonate. The aqueous layer was extracted with ethyl acetate, and the organic extract washed (water, brine), dried (magnesium sulfate), and evaporated. The crude solid was triturated with diethyl ether:hexane (1:1), then further purified by chromatography, eluting with dichloromethane:methanol (gradient, 99.5:0.5, 99:1, 98.5:1.5), to give the title compound as a pale yellow solid; TLC: R_{f}=0.32, dichloromethane:methanol (98:2); MS: m/z=515(M+1).

| Analysis for C₂₆H₂₅F₃N₄O₄·0.40 H₂O | | | |
|---|---|---|---|
| Calculated: | C, 59.85; | H, 4.98; | N, 10.73 |
| Found: | C, 59.74; | H, 5.08; | N, 10.57 |

The intermediate nitro compound was prepared as follows:

2-(3-Amino-2-oxo-6-phenyl-1,2-dihydro-1-pyridyl)-N-(3,3,3-trifluoro-1-isopropyl-2-oxopropyl)acetamide and 4-nitrobenzoic acid were subjected to a procedure simlar to Acylation Method B, omitting 1-hydroxybenzotriazole hydrate and adding 4-dimethylaminopyridine. After stirring 24 h, dichloromethane was added and the organic layer was washed (1 N hydrochloric acid, saturated sodium chloride), dried (magnesium sulfate), and evaporated. Chromatography, eluting with dichloromethane:methanol (gradient, 99.5:0.5, 99:1), gave the nitro compound as a yellow solid; TLC: R_{f}=0.48, dichloromethane:methanol (98:2); MS: m/z=545(M+1).

### EXAMPLES 170-172

The following compounds of formula I wherein R⁰ is isopropyl, R is benzyloxycarbonyl, R⁵ is hydrogen and R⁶ is the indicated heteroaryl group were prepared using a procedure similar to that outline in Example 49 steps d.-j., which correspond to steps a.-g. below.

Example 170: R⁶=3-pyridyl: Using a procedure similar to that described in Example 1, 2-[3-benzyloxycarbonylamino-2-oxo-6-(3-pyridyl)-1,2-dihydro-1-pyridyl]-N-(3,3,3-trifluoro-2-hydroxy-1-isopropylpropyl)acetamide was oxidized to give the title compound; purified by crystallization from ethyl acetate; TLC: R_{f}=0.80, dichloromethane:methanol (90:10); MS: m/z=531(M+1).

| Analysis for C₂₆H₂₅F₃N₄O₅·1.5 H₂O: | | | |
|---|---|---|---|
| Calculated: | C, 56.01; | H, 5.06; | N, 10.05 |
| Found: | C, 55.98; | H, 5.13; | N, 10.24 |

The intermedicate alcohol was prepared as follows:

### a. 3-Aza-4-(3-pyridyl)pent-3-enal dimethyl acetal.

Using a procedure similar to that described in Example 49.d., 3-acetylpyridine and aminoacetaldehyde dimethyl acetal were converted to the imine, a yellow oil; bp 150-157 °C (120 Pa).

### b. 3-Ethoxycarbonyl-2-oxo-6-(3-pyridyl)-1,2-dihydro-1-pyridylacetaldehyde dimethyl acetal.

Using a procedure similar to that described in Example 49.e., but employing diethyl ethoxymethylenemalonate (in place of dimethyl methoxymethylenemalonate), including a methanol addition prior to the extractive work-up to complete the cyclization, and purifying the product by dry-column chromatography, eluting with ethyl acetate:hexane (gradient, 40:60, 50:50, 60:40, 70:30, 80:20, 100:0) then ethyl acetate:ethanol (90:10), the pyridone was obtained (as a mixture of methyl and ethyl esters); TLC: R_{f}=0.15, ethyl acetate; MS: m/z=333(M+1) (ethyl ester), 319(M+1) (methyl ester).

### c. 1-(2,2-Dimethoxyethyl)-6-(3-pyridyl)pyrid-2-one-3-carboxylic acid.

Using a procedure similar to that described in Example 49.f., but omitting the sodium methoxide addition and using the following modified work-up procedure, the acid was obtained. On completion of hydrolysis, water was added followed by 6 N hydrochloric acid to pH 6. On standing for 10 min, the acid crystallized and was collected by filtration, washed with water and dried overnight under vacuum. Additional acid was obtained by extraction of the aqueous phase with dichloromethane, which was dried (magnesium sulfate), evaporated, and further dried under vacuum overnight. The two crops were combined and used without further purification; TLC: R_{f}=0.10, ethyl acetate; MS: m/z=305(M+1).

### d. 3-Benzyloxycarbonylamino-2-oxo-6-(3-pyridyl)-1,2-dihydro-1-pyridylacetaldelyde dimethyl acetal.

Using a procedure similar to that described in Example 49.g., but omitting the acid wash, and purifying by dry-column chromatography, eluting with ethyl acetate:hexane (gradient, 50:50, 60:40, 80:20, 100:0), the benzyloxycarbonylamino compound was obtained; TLC: R_{f}=0.40, ethyl acetate; MS: m/z=410(M+1).

### e. 3-Benzyloxycarbonylamino-2-oxo-6-(3-pyridyl)-1,2-dihydro-1-pyridylacetaldehyde.

Using a procedure similar to that described in Example 49.h., but purifying by chromatography, eluting with ethyl acetate, the aldehyde was obtained; TLC: R_{f}=0.20, dichloromethane:methanol (96:4); MS: m/z=364(M+1).

### f. 3-Benzyloxycarbonylamino-2-oxo-6-(3-pyridyl)-1,2-dihydro-1-pyridylacetic acid.

Using a procedure similar to that described for Example 49.i., but with the following modified work-up, the acid was prepared. Upon completion of the oxidation, dichloromethane, followed by 6 N hydrochloric acid to pH 3-4, were added. The aqueous phase was separated and further extracted with dichloromethane. The combined organic extracts were washed (water), dried (magnesium sulfate), evaporated, and the resultant oil was triturated with diethyl ether to produce a pale-yellow solid, which was collected by filtration and dried under vacuum overnight; TLC: R_{f}=0.10, dichloromethane:methanol (90:10); MS: m/z=380(M+1).

### g. 2-[3-Benzyloxycarbonylamino-2-oxo-6-(3-pyridyl)-1,2-dihydro-1-pyridyl]-N-(3,3,3-trifluoro-2-hydroxy-1-isopropylpropyl)acetamide.

3-Benzyloxycarbonylamino-2-oxo-6-(3-pyridyl)-1,2-dihydro-1-pyridylaceticacid was subject to a procedure similar to that described for Example 171.g., but employing the following modified work-up, to give the alcohol. On addition of ethyl acetate and 1 N aqueous sodium hydroxide to the reaction mixture, the product crystallized in the organic phase. The aqueous layer phase was rapidly separated and the organic phase allowed to stand. The aqueous layer was further extracted with ethyl acetate, again separating the organic phase rapidly as product crystallized. The organic phases were combined and allowed to stand 2-3 h. A first crop of the crystals was collected by filtration and washed with ethyl acetate. The combined ethyl acetate layers were evaporated to give a dark-red oil, which on trituration with diethyl ether yielded a second crop of solid. The second crop was collected by filtration, washed with ethyl acetate, then combined with the first crop and dried under vacuum overnight; TLC: R_{f}=0.50, dichloromethane:methanol (90:10); MS: m/z=533(M+1).

Example 171: R⁶=2-thienyl: 2-[3-Benzyloxycarbonylamino-2-oxo-6-(2-thienyl)-1,2-dihydro-1-pyridyl]-N-(3,3,3-trifluoro-2-hydroxy-1-isopropylpropyl)acetamide was subjected to a procedure similar to that described in Example 1, but purifying by chromatography, with dichloromethane:methanol (98:2) as the eluent, to give the title compound as a white solid; TLC: R_{f}=0.55, dichloromethane:methanol (96:4); MS: m/z=536(M+1).

| Analysis for C₂₅H₂₄F₃N₃O₅S·0.75 H₂O: | | | |
|---|---|---|---|
| Calculated: | C, 54.69; | H, 4.68; | N, 7.65 |
| Found: | C, 54.69; | H, 4.52; | N, 7.55 |

The intermediate alcohol was prepared as follows:

### a. 3-Aza-4-(2-thienyl)pent-3-enal dimethyl acetal.

Using a procedure similar to that described in Example 49.d., 2-acetylthiophene and aminoacetaldehyde dimethyl acetate were converted to the imine, a pale yellow oil; bp 133-42 °C (47-67 Pa).

### b. 3-Methoxycarbonyl-2-oxo-6-(2-thienyl)-1,2-dihydro-1-pyridylacetaldehyde dimethyl acetal.

The imine from Example 171.a. was subjected to a procedure similar to that described in Example 49.e., but employing a methanol addition prior to the extractive work-up to complete cyclization, and purifying the product by dry-column chromatography, with ethyl acetate:hexane (gradient, 40:60, 50:50, 60:40) as the eluent, to give the pyridone as a dark-orange oil; TLC: R_{f}=0.20, ethyl acetate:hexane (50:50).

### c. 1-(2,2-Dimethoxyethyl)-6-(2-thienyl)pyrid-2-one-3-carboxylic acid.

Using a procedure similar to that described in Example 49.f., but omitting the sodium methoxide addition, the acid was obtained as a dark-orange oil and used without purification; TLC: R_{f}=0.10, ethyl acetate:hexane (50:50); MS: m/z=310(M+1).

### d. 3-Benzyloxycarbonylamino-2-oxo-6-(2-thienyl)-1,2-dihydro-1-pyridylacetaldehyde dimethyl acetal.

Using a procedure similar to that described in Example 49.g., but purifying by dry-column chromatography, with ethyl acetate:hexane (gradient, 20:80, 30:70, 40:60) as the eluent, the benzyloxycarbonyl compound was obtained; TLC: R_{f}=0.30, ethyl acetate:hexane; MS: m/z=415(M+1).

### e. 3-Benzyloxycarbonylamino-2-oxo-6-(2-thienyl)-1,2-dihydro-1-pyridylacetaldehyde.

The following procedure, based on that described by Huet F. et al., Synthesis (1978) 63, was used to prepare the aldehyde.

To a stirred solution of the product from Example 171.d. (9.3 g) in chloroform (200 mL) was added silica gel (36.4 g), followed by 3 N hydrochloric acid (18.6 mL). The mixture was stirred for 3 days, filtered through magnesium sulfate, washed with chloroform and evaporated. A second iteration of this procedure was required to complete the hydrolysis. Dry-column chromatography, with ethyl acetate:hexane (gradient, 20:80, 30:70, 100:0) as the eluent, followed by a second dry column, with ethyl acetate:hexane (gradient, 30:70, 40:60, 50:50) as the eluent, gave the aldehyde as a yellow solid (2.8 g); TLC: R_{f}=0.30, ethyl acetate:hexane; MS: m/z=369(M+1).

### f. 3-Benzyloxycarbonylamino-2-oxo-6-(2-thienyl)-1,2-dihydro-1-pyridylacetic acid.

The product from Example 171.e. was subjected to a procedure similar to that described in Example 49.i., with the following modifications to the work-up. Upon completion of the reaction, dichloromethane, followed by 1 N hydrochloric acid, was added. The organic phase was separated, washed (water), dried (magnesium sulfate), and evaporated. Chromatography, with dichloromethane:methanol (gradient, 100:0, 95:5) as the eluent, gave the acid as a beige solid; TLC: R_{f}=0.15, dichloromethane:methanol (90:10); MS: m/z=385(M+1).

### g. 2-[3-Benzyloxycarbonylamino-2-oxo-6-(2-thienyl)-1,2-dihydro-1-pyridyl]-N-(3,3,3-trifluoro-2-hydroxy-1-isopropylpropyl)acetamide.

To a solution of the product from Example 171.f., 3-amino-1,1,1-trifluoro-4-methyl-2-pentanol hydrochloride, 1-hydroxybenzotriazole hydrate and triethylamine in dimethylformamide was added 1-(3-dimethylaminopropyl)-3-ethylcarbodiimide hydrochloride. This mixture was stirred overnight. Ethyl acetate and 1.0 N sodium hydroxide were added, and the aqueous phase was separated and further extracted with ethyl acetate. The combined organic layers were washed (1:1 brine:water), dried (magnesium sulfate), and evaporated. Chromatography, with dichloromethane:methanol (98:2) as the eluent, gave alcohol as a white solid; TLC: R_{f}=0.25, dichloromethane:methanol (98:2); MS: m/z=538(M+1).

Example 172: R⁶=2-furyl: 2-[3-Benzyloxycarbonylamino-6-(2-furyl)-2-oxo-1,2-dihydro-1-pyridyl]-N-(3,3,3-trifluoro-2-hydroxy-1-isopropyl-propyl)acetamide was subjected to a procedure similar to that described in Example 1, but purifying by chromatography, with dichloromethane:methanol (98:2) as the eluent, to give the title compound; TLC: R_{f}=0.55, dichloromethane:methanol (96:4); MS: m/z=520(M+1).

| Analysis for C₂₅H₂₄F₃N₃O₆·0.1 H₂O: | | | |
|---|---|---|---|
| Calculated: | C, 57.60; | H, 4.68; | N, 8.06 |
| Found: | C, 57.39; | H, 4.78; | N, 7.98 |

The intermediate alcohol was prepared as follows:

### a. 3-Aza-4-(2-furyl)pent-3-enal dimethyl acetal.

Using a procedure similar to that described in Example 49.d., 2-acetylfuran and aminoacetaldehyde dimethyl acetal were converted to the imine, a pale yellow oil; bp 92-102 °C (93-106 Pa).

### b. 6-(2-Furyl)-3-methoxycarbonyl-2-oxo-1,2-dihydro-1-pyridylacetaldehyde dimethyl acetal.

The imine from 172.a. was subjected to a procedure similar to that described in Example 49.e., but employing a methanol addition to complete cyclization prior to extractive work-up, and purifying the product by dry-column chromatography, with ethyl acetate:hexane (gradient, 40:60, 50:50, 60:40) as the eluent, to give the pyridone as a dark-orange oil; TLC: R_{f}=0.20, ethyl acetate:hexane (50:50); MS: m/z=308(M+1).

### c. 1-(2,2-Dimethoxyethyl)-6-(2-furyl)pyrid-2-one-3-carboxylic acid.

Using a procedure similar to that described in Example 49.f., but omitting the sodium methoxide addition, the acid was obtained as a dark-orange oil and used without purification; TLC: R_{f}=0.10, ethyl acetate:hexane (50:50); MS: m/z=294(M+1).

### d. 3-Benzyloxycarbonylamino-6-(2-furyl)-2-oxo-1,2-dihydro-1-pyridylacetaldehyde dimethyl acetal.

Using a procedure similar to that described in Example 49.g., but purifying by dry-column chromatography, with ethyl acetate:hexane (gradient, 20:80, 30:70, 40:60) as the eluent, the benzyloxycarbonyl compound was obtained; TLC: R_{f}=0.60, ethyl acetate:hexane (50:50); MS: m/z=399(M+1).

### e. 3-Benzyloxycarbonylamino-6-(2-furyl)-2-oxo-1,2-dihydro-1-pyridylacetaldehyde.

3-Benzyloxycarbonylamino-6-(2-furyl)-2-oxo-1,2-dihydro-1-pyridylacetaldehyde dimethyl acetal was subjected to a procedure similar to that described in Example 171.e. Dry-column chromatography, with ethyl acetate:hexane (gradient, 10:90, 20:80, 30:70) as the eluent, gave the aldehyde as a white solid; TLC: R_{f}=0.20, ethyl acetate:hexane (30:70); MS: m/z=353(M+1).

### f. 3-Benzyloxycarbonylamino-6-(2-furyl)-2-oxo-1,2-dihydro-1-pyridylacetic acid.

The product from Example 172.e. was subjected to a procedure similar to that described in Example 49.i., with the following modifications to the work-up. After stirring the reaction mixture for 1 h, the solid was collected by filtration, washed (water, diethyl ether), and dried overnight under vacuum to give the acid, which was used without purification; TLC: R_{f}=0.15, dichloromethane:methanol (90:10); MS: m/z=369(M+1).

### g. 2-[3-Benzyloxycarbonylamino-6-(2-furyl)-2-oxo-1,2-dihydro-1-pyridyl]-N-(3,3,3-trifluoro-2-hydroxy-1-isopropylpropyl)acetamide.

3-Benzyloxycarbonylamino-6-(2-furyl)-2-oxo-1,2-dihydro-1-pyridylacetic acid was subjected to a procedure similar to that described in Example 171.g. to give the alcohol as a white solid; chromatography solvent: dichloromethane:methanol (98:2); TLC: R_{f}=0.65, dichloromethane:methanol (90:10); MS: m/z=522(M+1).

### EXAMPLES 173-175

The following compounds of formula I wherein R⁰ is isopropyl, R is hydrogen, R⁵ is hydrogen and R⁶ is the indicated heteroaryl group were prepared by removal of the benzyloxycarbonyl group from the corresponding compounds of formula I described in Examples 170-172, using a procedure similar to the one described in Example 49, with exceptions as noted:

Example 173: R⁶=3-pyridyl: Addition of trifluoromethanesulfonic acid to a stirred suspension of the urethane in dichloromethane caused deposition of title product as a sticky mass. Sodium bicarbonate solution was added to pH 8, and the reaction mixture was extracted with ethyl acetate. The combined organic extracts were washed (1:1 brine:water), dried (magnesium sulphate), and evaporated. The resulting solid was triturated with hexane, diethyl ether, purified by chromatography, with dichloromethane:methanol (gradient, 95:5 90:10 80:20) as the eluent, and trituration with refluxing ethyl acetate. Cooling overnight, gave the title compound, which was collected by filtration and dried under vacuum; TLC: R_{f}=0.40, dichloromethane:methanol (90:10); MS: m/z=397(M+1).

| Analysis for C₁₈H₁₉F₃N₄O₃·1.2 H₂O: | | | |
|---|---|---|---|
| Calculated: | C, 51.72; | H, 5.16; | N, 13.40 |
| Found: | C, 51.64; | H, 5.25; | N, 13.11 |

Example 174: R⁶=2-thienyl: Purified by trituration with hexane, then diethyl ether; TLC: R_{f}=0.30, dichloromethane:methanol (9:1); MS: m/z=402(M+1).

| Analysis for C₁₇H₁₈F₃N₃O₃S·0.75 H₂O: | | | |
|---|---|---|---|
| Calculated: | C, 49.21; | H, 4.74; | N, 10.13 |
| Found: | C, 48.99; | H, 4.48; | N, 9.79 |

Example 175: R⁶=2-furyl: Purified by trituration with hexane, then diethyl ether; TLC: R_{f}=0.50, dichloromethane:methanol (90:10); MS: m/z=386(M+1).

| Analysis for C₁₇H₁₈F₃N₃O₄·0.55 H₂O: | | | |
|---|---|---|---|
| Calculated: | C, 51.66; | H, 4.87; | N, 10.63 |
| Found: | C, 52.07; | H, 5.04; | N, 10.23 |

### EXAMPLES 176-178

The following compounds of formula I wherein R⁰ is isopropyl, R is trifluoroacetyl, R⁵ is hydrogen and R⁶ is the indicated heteroaryl group were prepared from the corresponding amines of formula I described in Examples 173-175 using Acylation Method A, with the exceptions noted:

Example 176: R=2-furyl: Omitting addition of triethylamine, employing dichloromethane in place of tetrahydrofuran, and purifying by chromatography, with dichloromethane:methanol (96:4) as the eluent, the title compound was prepared; TLC: R_{f}=0.55, dichloromethane:methanol (90:10); MS: m/z=493(M+1).

| Analysis for C₂₀H₁₈F₆N₄O₄·CF₃CO₂H·H₂O: | | | |
|---|---|---|---|
| Calculated: | C, 42.32; | H, 3.39; | N, 8.97 |
| Found: | C, 42.01; | H, 3.13; | N, 8.74 |

Example 177: R=3-pyridyl: Omitting addition of triethylamine, employing dichloromethane in place of tetrahydrofuran, and purifying by chromatography, with dichloromethane:methanol (98:2) as the eluent, the title compound was prepared; TLC: R_{f}=0.40, dichloromethane:methanol (96:4); MS: m/z=498(M+1).

| Analysis for C₁₉H₁₇F₆N₃O₄S: | | | |
|---|---|---|---|
| Calculated: | C, 45.88; | H, 3.44; | N, 8.45 |
| Found: | C, 45.51; | H, 3.66; | N, 8.25 |

Example 178: R=2-thienyl: Omitting addition of triethylamine, employing dichloromethane in place of tetrahydrofuran, and purifying by chromatography, with dichloromethane:methanol (98:2) as the eluent, the title compound was prepared; TLC: R_{f}=0.45, dichloromethane:methanol (96:4); MS: m/z=482(M+1).

| Analysis for C₁₉H₁₇F₆N₃O₅: | | | |
|---|---|---|---|
| Calculated: | C, 47.41; | H, 3.56; | N, 8.73 |
| Found: | C, 47.07; | H, 3.73; | N, 8.49 |

### EXAMPLE 179

### 2-[2-Oxo-6-phenyl-3-[3-(3-pyridylmethyl)thioureido]-1,2-dihydro-1-pyridyl]-N-(3,3,3-trifluoro-1-isopropropyl-2-oxopropyl)acetamide.

A slurry of 2-(3-amino-2-oxo-6-phenyl-1,2-dihydro-1-pyridyl)-N-(3,3,3-trifluoro-1-isopropropyl-2-oxopropyl)acetamide (0.30 g), 3-picolyl isothiocyanate hydrobromide (0.178 g) and triethylamine (0.21 mL) in dry tetrahydrofuran (3 mL) was heated at 60 °C for 4 h. Additional 3-picolyl isothiocyanate (0.36 g) and triethylamine (0.04 mL) were added, and the reaction was allowed to stir for 64 h. Additional isothiocyanate (0.16 g) was added and the reaction was heated at 60 °C for 3 hours and isothiocyanate (0.17 g) was again added. Three hours later, isothiocyanate (0.17 g) and triethylamine (0.02 mL) were added and the reaction was heated overnight. Ethyl acetate was added and the mixture was washed (water:sodium phosphate monobasic monohydrate:sodium phosphate dibasic (95 mL:5 g:5 g), water, brine), dried and evaporated. The residue was purified by chromatography, with dichloromethane:methanol (98:2) as the eluent, followed by trituration with methyl tert-butyl ether to yield the title compound (0.148 g); mp 124.5-127 °C; TLC: R_{f}=0.61, dichloromethane:methanol (90:10); MS: m/z=546(M+1).

| Analysis for C₂₆H₂₆F₃N₅O₃S·0.5 H₂O: | | | |
|---|---|---|---|
| Calculated: | C, 56.31; | H, 4.91; | N, 12.63 |
| Found: | C, 56.00; | H, 4.72; | N, 12.60 |

### EXAMPLES 180-183

Using a procedure similar to that described in Example I, the following compounds of formula I wherein R⁰ is isopropyl, R is the indicated acyl group, R⁵ is hydrogen and R⁶ is phenyl were prepared by oxidation of the corresponding alcohols of formula II.

Example 180: R=methoxyacetyl: Product was isolated directly from workup with no chromatographic purification; TLC: R_{f}=0.25, methanol:dichloromethane (5:95); MS: m/z=468(M+1).

| Analysis for C₂₂H₂₄F₃N₃O₅·1.0 H₂O: | | | |
|---|---|---|---|
| Calculated: | C, 54.43; | H, 5.40; | N, 8.66 |
| Found: | C, 54.46; | H, 5.42; | N, 8.69 |

Example 181: R=piperazin-2-ylaminocarbonyl: Chromatography solvent: methanol:dichloromethane (gradient, 1:99, 7:93); TLC: R_{f}=0.41, methanol:dichloromethane (5:95); MS: m/z=517(M+1).

| Analysis for C₂₄H₂₃F₃N₆O₄·0.8 H₂O: | | | |
|---|---|---|---|
| Calculated: | C, 54.29; | H, 4.67; | N, 15.83 |
| Found: | C, 54.37; | H, 4.83; | N, 15.49 |

Example 182: R=pyrid-4-yldimethylmethoxycarbonyl: Chromatography solvent: methanol:dichloromethane (1:99); TLC: R_{f}=0.30, methanol:dichloromethane (3:97); MS: m/z=559(M+1).

| Analysis for C₂₈H₂₉F₃N₄O₅: | | | |
|---|---|---|---|
| Calculated: | C, 60.21; | H, 5.23; | N, 10.03 |
| Found: | C, 59.95; | H, 5.48; | N, 9.60 |

Example 183: R=morpholinoacetyl: Chromatography solvent: methanol:dichloromethane (4:96), followed by trituration with diethylether; TLC: R_{f}=0.29, methanol:dichloromethane (4:96); MS: m/z=523(M+1).

| Analysis for C₂₅H₂₉F₃N₄O₅·0.1 H₂O: | | | |
|---|---|---|---|
| Calculated: | C, 57.27; | H, 5.61; | N, 10.69 |
| Found: | C, 56.97; | H, 5.59; | N, 10.60 |

The corresponding alcohols of Formula II for examples 180-183 were prepared as follows:

### EXAMPLES 180.a.-183.a

2-(3-Acylamino-2-oxo-6-phenyl-1,2-dihydro-1-pyridyl)-N-(2-tert-butyldimethylsilyloxy-3,3,3-trifluoro-1-isopropylpropyl)acetamides having the indicated acyl group R were prepared by acylation of 2-(3-amino-2-oxo-6-phenyl-1,2-dihydro-1-pyridyl)-N(2-tert-butyldimethylsilyloxy-3,3,3-trifluoro-1-isopropylpropyl)acetamide using the Acylation Method noted:

Example 180.a.: R=methoxyacetyl: Acylation Method A, with sodium carbonate in place of triethylamine; used without further purification; TLC: R_{f}=0.64, methanol:dichloromethane (5:95); MS: m/z=584(M+1).

Example 181.a.: R=piperazin-2-ylaminocarbonyl: Acylation Method D; chromatography solvent: methanol:dichloromethane (gradient, 0.5:99.5, 2:98); TLC: R_{f}=0.52, methanol:dichloromethane (5:95); MS: m/z=633(M+1).

Example 182.a.: R=pyrid-4-yldimethylmethoxycarbonyl: Acylation Method D. The required alcohol was prepared using a literature procedure; J. Chem. Soc. Perkin Trans. I (1985), 213. Chromatography: First column, methanol:dichloromethane (2:98); second column, methanol:dichloromethane (99:1); third column, methanol:diethyl ether:dichloromethane (0.5:25:74.5); fourth column, methanol:diethyl ether:dichloromethane (0.5:25:74.5); TLC: R_{f}=O.43, methanol:diethylether:dichloromethane (0.5:25:74.5); MS: m/z=675(M+1).

Example 183.a.: R=morpholinoacetyl: Acylation Method B; the reaction mixture was diluted with water and the resulting solid was washed with saturated aqueous sodium bicarbonate and water and dried under vacuum at 40 °C; TLC: R_{f}=0.43, methanol:dichloromethane (4:96); MS: m/z=639(M+1).

The morpholinoacetic acid used in Example 183.a. was prepared as follows:

Ethyl morpholinoacetate (5.0 g) in ethanol (115 mL) was added to a solution of sodium hydroxide (1.27 g) in water (12 mL) and the mixture was allowed to stir for 1 h. The mixture was evaporated, dissolved in water (125 mL), and extracted with ethyl acetate. The aqueous phase was acidified with 10% hydrochloric acid (pH 2) and lyophilized to give a brown oil. The oil was dried under vacuum to yield morpholinoacetic acid hydrochloride, which was used directly for the acylation above.

### EXAMPLES 180.b.-183.b.

The following alcohols of Formula II wherein R⁰ is isopropyl, R is the indicated acyl group, R⁵ is hydrogen and R⁶ is phenyl were prepared by deprotection of the corresponding tert-butyldimethylsilylethers using a procedure similar to that described in Example 1.e., unless otherwise noted:

Example 180.b.: R=methoxyacetyl: Deprotection as in Example 19.b.; used without further purification; TLC: R_{f}=0.26, methanol:dichloromethane (5:95); MS: m/z=470(M+1).

Example 181.b.: R=piperazin-2-ylaminocarbonyl: Chromatography solvent: methanol:dichloromethane (gradient, 0.5:99.5, 5:95); TLC: R_{f}=0.35, methanol:dichloromethane (5:95); MS: m/z=519(M+1).

Example 182.b.: R=pyrid-4-yldimethylmethoxycarbonyl: TLC: R_{f}=0.12, methanol:diethylether:dichloromethane (1:25:75); MS: m/z=561(M+1).

Example 183.b.: R=morpholinoacetyl: Chromatography solvent: methanol:dichloromethane (gradient, 0:100, 2:98); TLC: R_{f}=0.30, methanol:dichloromethane; MS: m/z=525(M+1).

### EXAMPLE 184

### 2-(3-Benzyloxycarbonylamino-2-oxo-6-phenyl-1,2-dihydro-1-pyridyl)-N-(1-ethyl-3,3,3-trifluoro-2-oxopropyl)acetamide.

2-(3-Benzyloxycarbonylamino-2-oxo-6-phenyl-1,2-dihydro-1-pyridyl)-N-(1-ethyl-3,3,3-trifluoro-2-hydroxypropyl)acetamide was oxidized using a procedure similar to that outlined in Example 1 to give the title compound, which was purified by chromatography, with methanol:dichloromethane (5:95) as the eluent; TLC: R_{f}=0.55, methanol:dichloromethane (5:95); MS: m/z=516(M+1).

| Analysis C₂₆H₂₄F₃O₅N₃: | | | |
|---|---|---|---|
| Calculated: | C, 60.58; | H, 4.69; | N, 8.15 |
| Found: | C, 60.05; | H, 4.76; | N, 7.97 |

The intermediate alcohol was prepared as follows:

### a. 1,1,1-Trifluoro-3-nitro-2-pentanol.

A mixture of 1-nitropropane (10 mL), trifluoroacetaldehyde ethyl hemiacetal (20.2 mL), and potassium carbonate (15.4 g) was stirred at 25 °C for 48 h. The solution was made acidic by addition of 1 N hydrochloric acid and the product extracted into dichloromethane. The solution was dried (MgSO₄) and the solvent evaporated to give an oil, which was distilled (60 °C, 133 Pa) to provide 1,1,1-trifluoro-3-nitro-2-pentanol (15 g) as a mixture of diastereoisomers; MS: m/z=188(M+1).

### b. 3-Amino-1,1,1-trifluoro-2-pentanol hydrochloride.

To a solution of 1,1,1-trifluoro-3-nitro-2-pentanol (3 g) in ethanol (100 mL) was added 10% (w/w) palladium on carbon (1 g) and the resulting mixture shaken under a hydrogen atmosphere (4 bar) for 3 days. The catalyst was removed by filtration and the solvent evaporated. To the resulting oil was added a saturated solution of ethanolic hydrochloric acid (5 mL) and the solvent was evaporated. Addition of ether gave a white solid which was collected and washed with ether to provide 3-amino-1,1,1-trifluoro-2-pentanol hydrochloride (1.3 g) as a white solid; MS: m/z=158(M+1-Cl); TLC: Rf=0.1, methanol:dichloromethane (10:90).

### c. 2-(3-Benzyloxycarbonylamino-2-oxo-6-phenyl-1,2-dihydro-1-pyridyl)-N-(1-ethyl-3,3,3-trifluoro-2-hydroxypropyl)acetamide.

Using a procedure similar to that described in Example 49.j., but substituting 3-amino-1,1,1-trifluoro-2-pentanol hydrochloride for 3-amino-1,1,1-trifluoro-4-methyl-2-pentanol hydrochloride, the hydroxy amide was prepared. The crude material was crystallized from ether to yield a white solid; TLC: R_{f}=0.60, methanol:dichloromethane, (10:90); MS: m/z=518(M+1).

### EXAMPLE 185

### 2-[3-Benzyloxycarbonylamino-6-(3-carboxyphenyl)-2-oxo-1,2-dihydro-1-pyridyl]-N-(3,3,3-trifluoro-1-isopropyl-2-oxopropyl)acetamide.

2-[3-Benzyloxycarbonylamino-6-(3-methoxycarbonylphenyl)-2-oxo-1,2-dihydro-1-pyridyl]-N-(3,3,3-trifluoro-1-isopropyl-2-oxopropyl)acetamide was hydrolyzed using a procedure similar to that described in Example 45 to give the title compound: Chromatography solvent: ethanol:dichloromethane:acetic acid (3:96.5:0.5); TLC: R_{f}=0.45, ethanol:dichloromethane:acetic acid, (3:96.5:0.5): MS: m/z=574(M+1).

| Analysis for C₂₈H₂₆F₃N₃O₇·0.5 H₂O: | | | |
|---|---|---|---|
| Calculated: | C, 57.73; | H, 4.67; | N, 7.21 |
| Found: | C, 57.78; | H, 4.70; | N, 7.19 |

The intermediate 2-[3-benzyloxycarbonylamino-6-(3-methoxycarbonylphenyl)-2-oxo-1,2-dihydro-1-pyridyl]-N-(3,3,3-trifluoro-1-isopropyl-2-oxopropyl)acetamide was prepared as follows:

### a. Methyl 3-acetylbenzoate.

To a solution of 3-acetylbenzoic acid (4.10 g) in dimethylformamide (50 mL) was added potassium carbonate (3.63 g) and methyl iodide (5.2 mL) and the mixture was allowed to stir for 18 h. Water was added and the mixture was extracted with ethyl acetate. The combined organic extracts were washed with brine, dried, evaporated, and dried under vacuum, to give the ester as a brown oil (4.10 g); TLC: R_{f}=0.36, hexane:ethyl acetate (6:1); MS: m/z=179(M+1).

### b. 6-(3-Methoxycarbonylphenyl)-3-nitropyrid-2-one.

A solution of methyl 3-acetylbenzoate (4.01 g) and dimethylformamide dimethylacetal (8.97 mL) in acetonitrile (100 mL) was allowed to reflux for 18 h. The mixture was evaporated and dried under vacuum to give a brown solid. This solid (4.16 g) was dissolved in dimethylformamide (50 mL), and the ammonium salt of nitro acetamide (2.90 g, prepared as described in J. Org. Chem. (1958), 113) was added. The mixture was heated to 100 °C for 24 h., cooled and diluted with water. The resulting precipitate was collected, washed with water, and dried under vacuum to give the pyridone (1.75 g); TLC: R_{f}=0.20, methanol:ethyl acetate:dichloromethane (2:3:95); MS: m/z=275(M+1).

### c. 3-Amino-6-(3-methoxycarbonylphenyl)pyrid-2-one.

A solution of 6-(3-methoxycarbonylphenyl)-3-nitropyrid-2-one (0.12 M, in dimethylformamide) was added to 10% (w/w) palladium on carbon (10% by weight) and the mixture was shaken under hydrogen (3.5 bar) for 18 h. The catalyst was removed by filtration and the resulting solution was evaporated and dried under vacuum to give the amine; TLC: R_{f}=0.21, methanol:ethyl acetate:dichloromethane (3:3:94); MS: m/z=245(M+1).

### d. 3-Benzyloxycarbonylamino-6-(3-methoxycarbonylphenyl)pyrid-2-one.

3-Amino-6-(3-methoxycarbonylphenyl)pyrid-2-one was acylated with benzyl chloroformate using conditions similar to those described in Acylation Method A. The crude material was triturated with methanol and crystallized from dimethylformamide and water to give the benzyloxycarbonylamino-compound; TLC: R_{f}=0.62, methanol:ethyl acetate:dichloromethane (3:3:94); MS: m/z=379(M+1).

### e. 2-[3-Benzyloxycarbonylamino-6-(3-methoxycarbonylphenyl)-2-oxo-1,2-dihydro-1-pyridyl]-N-(2-tert-butyldimethylsilyloxy-3,3,3-trifluoro-1-isopropylpropyl)acetamide.

3-Benzyloxycarbonylamino-6-(3-methoxycarbonylphenyl)pyrid-2-one was subjected to a procedure similar to that described in Example 1.d The crude material was purified by chromatography, with ethyl acetate:dichloromethane (1.5:98.5) as the eluent, to yield the N-alkylated pyridone; TLC: R_{f}=0.19, ethyl acetate:dichloromethane (1.5:98.5); MS: m/z=704(M+1).

### f. 2-[3-Benzyloxycarbonylamino-6-(3-methoxycarbonylphenyl)-2-oxo-1,2-dihydro-1-pyridyl]-N-(3,3,3-trifluoro-2-hydroxy-1-isopropylpropyl)acetamide.

2-[3-Benzyloxycarbonylamino-6-(3-methoxycarbonylphenyl)-2-oxo-1,2-dihydro-1-pyridyl]-N-(2-tert-butyldimethylsilyloxy-3,3,3-trifluoro-1-isopropylpropyl)acetamide was subjected to a procedure similar to that described in Example 1.e. The crude material was purified by chromatography, with ethanol:diethylether:dichloromethane (1:10:89) as the eluent, to give the alcohol; TLC: R_{f}=0.15, ethanol:diethylether:dichloromethane (1:10:89); MS: m/z=590(M+1).

### g. 2-[3-Benzyloxycarbonylamino-6-(3-methoxycarbonylphenyl)-2-oxo-1,2-dihydro-1-pyridyl]-N-(3,3,3-trifluoro-1-isopropyl-2-oxopropyl)acetamide.

2-[3-Benzyloxycarbonylamino-6-(3-methoxycarbonylphenyl)-2-oxo-1,2-dihydro-1-pyridyl]-N-(3,3,3-trifluoro-2-hydroxy-1-isopropylpropyl)acetamide was subjected to an oxidation procedure similar to that described in Example 1. The crude material was purified by chromatography, with ethanol:diethylether:dichloromethane (1:10:89) as the eluent, to give the ketone (which is also an example of the invention); TLC: R_{f}=0.30, ethanol:diethylether:dichloromethane (1:10:89); MS: m/z=588(M+1).

### EXAMPLE 186

### 2-[3-Benzyloxycarbonylamino-6-(4-carboxyphenyl)-2-oxo-1,2-dihydro-1-pyridyl]-N-(3,3,3-trifluoro-1-isopropyl-2-oxopropyl)acetamide.

2-[3-Benzyloxycarbonylamino-6-(4-methoxycarbonylphenyl)-2-oxo-1,2-dihydro-1-pyridyl]-N-(3,3,3-trifluoro-1-isopropyl-2-oxopropyl)acetamide was hydrolyzed using a procedure similar to that described in Example 45 to give the title compound, which crystallized from hot ethyl acetate:hexane; TLC: R_{f}=0.43,methanol:dichloromethane:acetic acid, (5:94.5:0.5): MS: m/z=574(M+1).

| Analysis for C₂₈H₂₆F₃N₃O₇: | | | |
|---|---|---|---|
| Calculated: | C, 58.64; | H, 4.57; | N, 7.33 |
| Found: | C, 58.37; | H, 4.57; | N, 7.30 |

The intermediate 2-[3-benzyloxycarbonylamino-6-(4-methoxycarbonylphenyl)-2-oxo-1,2-dihydro-1-pyridyl]-N-(3,3,3-trifluoro-1-isopropyl-2-oxopropyl)acetamide was prepared as follows:

### a. Methyl 4-acetylbenzoate.

To a solution of 4-acetylbenzoic acid (4.10 g) in dimethylformamide (50 mL) was added potassium carbonate (3.63 g) and methyl iodide (5.2 mL) and the mixture was allowed to stir for 18 h. Water was added and the mixture was extracted with ethyl acetate. The combined organic extracts were washed with brine, dried, evaporated, and dried under vacuum, to give the ester as a brown oil; TLC: R_{f}=0.33, hexane:ethyl acetate (6:1); MS: m/z=179(M+1).

### b. 6-(4-Methoxycarbonylphenyl)-3-nitropyrid-2-one.

Using a procedure similar to that described in Example 185.b., but substituting methyl 4-acetylbenzoate for methyl 3-acetylbenzoate, the nitro pyridone was prepared; TLC: R_{f}=0.14, methanol:ethyl acetate:dichloromethane (2:3:95); MS: m/z=275(M+1).

### c. 3-Amino-6-(4-methoxycarbonylphenyl)pyrid-2-one.

6-(4-Methoxycarbonylphenyl)-3-nitropyrid-2-one was subjected to a procedure similar to that outlined in Example 185.c. to give the amine; TLC: R_{f}=0.23, methanol:dichloromethane (3:97); MS: m/z=245(M+1).

### d. 3-Benzyloxycarbonylamino-6-(4-methoxycarbonylphenyl)pyrid-2-one.

3-Amino-6-(4-methoxycarbonylphenyl)pyrid-2-one was acylated with benzyl chloroformate using conditions similar to those described in Acylation Method A. After stirring overnight, the mixture was evaporated, suspended in ethyl acetate, and washed with saturated sodium bicarbonate solution. Solids suspended in the aqueous layers were removed by filtration, and dried to give a mixture of the starting amine and the benzyloxycarbonylamino pyridone. This material was subjected to a second iteration of the acylation procedure. The ethyl acetate from the above extraction was washed (1 N hydrochloric acid, brine), dried and evaporated to give crude material. This residue was combined with the material isolated from the second iteration and purified by chromatography, with methanol:ethyl acetate:dichloromethane (gradient, 0:5:95, 1:0:99, 2:0:98) as the eluent. The recovered solid was crystallized from dimethylformamide and water. The resulting material was washed (water, diethyl ether) and dried under vacuum to give the benzyloxycarbonylamino compound; TLC: R_{f}=0.52, methanol:dichloromethane (3:97); MS: m/z=379(M+1).

### e. 2-[3-Benzyloxycarbonylamino-6-(4-methoxycarbonylphenyl)-2-oxo-1,2-dihydro-1-pyridyl]-N-(2-tert-butyldimethylsilyloxy-3,3,3-trifluoro-1-isopropylpropyl)acetamide.

3-Benzyloxycarbonylamino-6-(4-methoxycarbonylphenyl)pyrid-2-one was subjected to a procedure similar to that described in Example 1.d. The crude material was purified by chromatography: First column, ethyl acetate:dichloromethane (3:97); secound column, ethyl acetate:dichloromethane (2:98); third column, ethyl acetate:dichloromethane (3:97); to yield the N-alkylated pyridone; TLC: R_{f}=0.20, ethyl acetate:dichloromethane (3:97); MS: m/z=704(M+1).

### f. 2-[3-Benzyloxycarbonylamino-6-(4-methoxycarbonylphenyl)-2-oxo-1,2-dihydro-1-pyridyl]-N-(3,3,3-trifluoro-2-hydroxy-1-isopropylpropyl)acetamide.

2-[3-Benzyloxycarbonylamino-6-(4-methoxycarbonylphenyl)-2-oxo-1,2-dihydro-1-pyridyl]-N-(2-tert-butyldimethylsilyloxy-3,3,3-trifluoro-1-isopropylpropyl)acetamide was subjected to a procedure similar to that described in Example 1.e. The crude material was crystallized from ethyl acetate and hexane to give the alcohol; TLC: R_{f}=0.35, methanol:dichloromethane (3:97); MS: m/z=590(M+1).

### g. 2-[3-Benzyloxycarbonylamino-6-(4-methoxycarbonylphenyl)-2-oxo-1,2-dihydro-1-pyridyl]-N-(3,3,3-trifluoro-1-isopropyl-2-oxopropyl)acetamide.

2-[3-Benzyloxycarbonylamino-6-(4-methoxycarbonylphenyl)-2-oxo-1,2-dihydro-1-pyridyl]-N-(3,3,3-trifluoro-2-hydroxy-1-isopropylpropyl)acetamide was subjected to an oxidation procedure similar to that described in Example 1. The crude material was crystallized from ethyl acetate and hexane to give the ketone (which is also an example of the invention); TLC: R_{f}=0.46, methanol:dichloromethane (2:98); MS: m/z=588(M+1).

### EXAMPLE 187

### 2-[3-[3-Tris(acetoxymethyl)methylureido]-2-oxo-6-phenyl-1,2-dihydro-1-pyridyl]-N-(3,3,3-trifluoro-1-isopropyl-2-oxopropyl)acetamide.

2-[3-[3-Tris(acetoxymethyl)methylureido]-2-oxo-6-phenyl-1,2-dihydro-1-pyridyl]-N-(3,3,3-trifluoro-2-hydroxy-1-isopropylpropyl)acetamide was oxidized using a procedure similar to that outlined in Example 1 to give crude material, which was purified by chromatography, with acetonitrile:dichloromethane (20:80) as the eluent, to give the title compound; TLC: R_{f}=0.62, methanol:chloroform (10:90); MS: m/z=669(M+1).

| Analysis for C₃₀H₃₅F₃N₄O₁₀: | | | |
|---|---|---|---|
| Calculated: | C, 53.89; | H, 5.28; | N, 8.38 |
| Found: | C, 53.57; | H, 5.31; | N, 8.32 |

The intermediate 2-[3-[3-tris(acetoxymethyl)methylureido]-2-oxo-6-phenyl-1,2-dihydro-1-pyridyl]-N-(3,3,3-trifluoro-2-hydroxy-isopropylpropyl)acetamide was prepared as follows:

### a. 2-[3-[3-Tris(hydroxymethyl)methylureido]-2-oxo-6-phenyl-1,2-dihydro-1-pyridyl]-N-(2-tert-butyldimethylsilyloxy-3,3,3-trifluoro-1-isopropylpropyl)acetamide.

A solution of triphosgene (0.450 g) in dichloromethane (6 mL) was added dropwise to a 3 °C solution of 2-(3-amino-2-oxo-6-phenyl-1,2-dihydro-1-pyridyl)-N-(2-tert-butyl-dimethylsilyloxy-3,3,3-trifluoro-1-isopropylpropyl)acetamide (1.5 g) in dichloromethane (6 mL). The mixture was allowed to stir for 10 min at 3 °C, 10 min at room temperature and was then cooled to 3 °C. Triethylamine (2.2 mL) as a solution in dichloromethane (2 mL) was added dropwise maintaining the internal temperature below 5 °C. The mixture was stirred for 15 min and was added dropwise to a room temperature suspension of tris(hydroxymethyl)methylamine (0.728 g) in tert-butanol (50 mL). The mixture was allowed to stir at room temperature for 75 min, was diluted with ethyl acetate and washed with saturated brine. The saturated brine was extracted with ethyl acetate and the combined organic layers were washed with saturated brine, dried and evaporated to yield the urea (2.49 g). The crude material was used without further purification; TLC: Rf=0.40, chloroform:methanol (90:10); MS: m/z=659 (M+1).

### b. 2-[3-[3-Tris(acetoxymethyl)methylureido]-2-oxo-6-phenyl-1,2-dihydro-1-pyridyl]-N-(2-tert-butyldimethylsilyloxy-3,3,3-trifluoro-1-isopropylpropyl)acetamide.

A solution of 2-[3-[3-tris-(hydroxymethyl)methylureido]-2-oxo-6-phenyl-1,2-dihydro-1-pyridyl]-N-(2-tert-butyldimethylsilyloxy-3,3,3-trifluoro-1-isopropylpropyl)acetamide (1.5 g), acetic anhydride (1.1 mL), triethylamine (1.6 mL) and 4-dimethylaminopyridine (0.045 g) in dichloromethane (7 mL) was stirred for 1 h. The mixture was diluted with ethyl acetate (75 mL), washed (10% acetic acid, half saturated sodium hydrogen carbonate, water, saturated brine), dried and evaporated to yield the triacetate (2.39 g). The material was used without further purification; TLC: Rf=0.75 chloroform:methanol (90:10); MS: m/z=785(M+1).

### c. 2-[3-[3-Tris(acetoxymethyl)methylureido]-2-oxo-6-phenyl-1,2-dihydro-1-pyridyl]-N-(3,3,3-trifluoro-2-hydroxy-1-isopropylpropyl) acetamide.

2-[3-[3-Tris(acetoxymethyl)methylureido]-2-oxo-6-phenyl-1,2-dihydro-1-pyridyl]-N-(2-tert-butyldimethylsilyloxy-3,3,3-trifluoro-1-isopropylpropyl)acetamide was subjected to a procedure similar to that described in Example 19.b. to yield the alcohol, which was purified by chromatography, with dichloromethane:acetonitrile (80:20) as the eluent; TLC: Rf=0.42, chloroform:methanol (90:10); MS: m/z=671 (M+1).

### EXAMPLE 188

### 2-[3-[3-Tris(hydroxymethyl)methylureido]-2-oxo-6-phenyl-1,2-dihydro-1-pyridyl]-N-(3,3,3-trifluoro-1-isopropyl-2-oxopropyl)acetamide.

A solution of lithium hydroxide monohydrate (0.419 g) in water (5 mL) was added to a solution of 2-[3-[3-tris(acetoxymethyl)methylureido]-2-oxo-6-phenyl-1,2-dihydro-1-pyridyl]-N-(3,3,3-trifluoro-1-isopropyl-2-oxopropyl)acetamide (1.11 g) in tetrahydrofuran (6 mL) and the cloudy mixture was allowed to stir for 1 h. The mixture was diluted with half saturated potassium phosphate monobasic and extracted with ethyl acetate. The ethyl acetate was washed with saturated brine, dried and evaporated. The residue was purified by chromatography, with dichloromethane:methanol (95:5) as the eluent, to yield the title compound (0.592 g); mp 148-151 °C; TLC: Rf=0.23, chloroform:methanol (90:10); MS: m/z=543 (M+1).

| Analysis for C₂₄H₂₉F₃N₄O₇·0.5 H₂O: | | | |
|---|---|---|---|
| Calculated: | C, 52.33; | H, 5.48; | N, 10.16 |
| Found: | C, 52.20; | H, 5.50; | N, 9.98 |

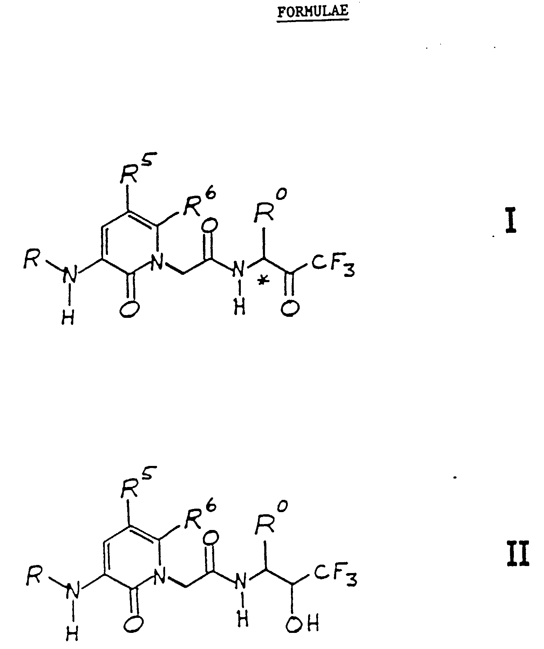

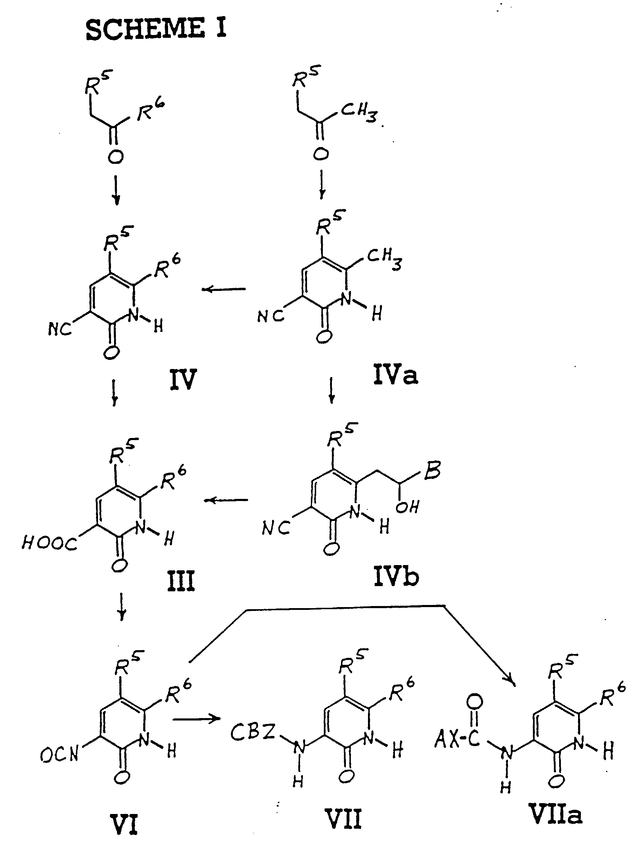

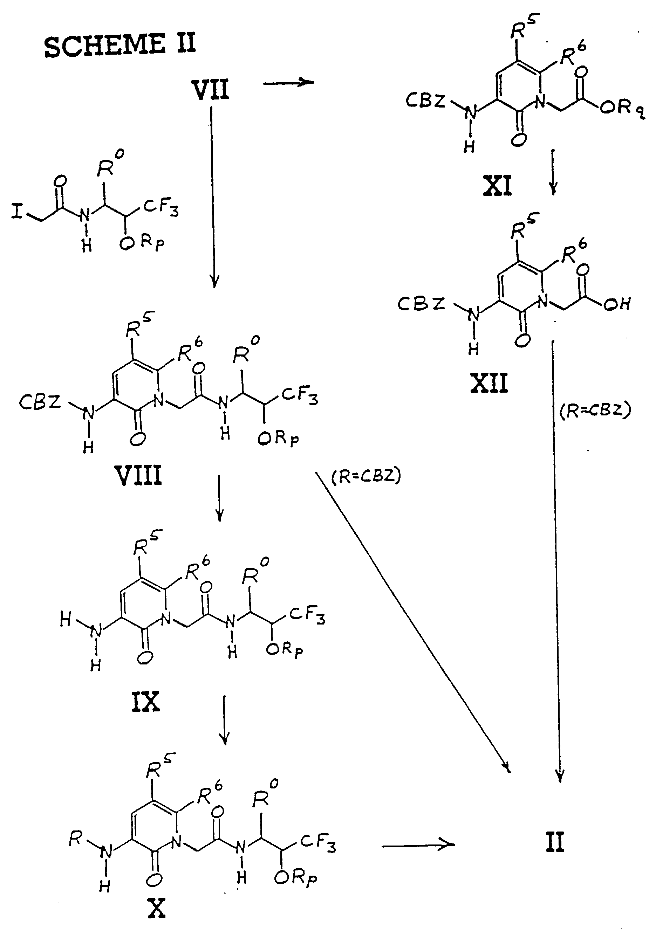

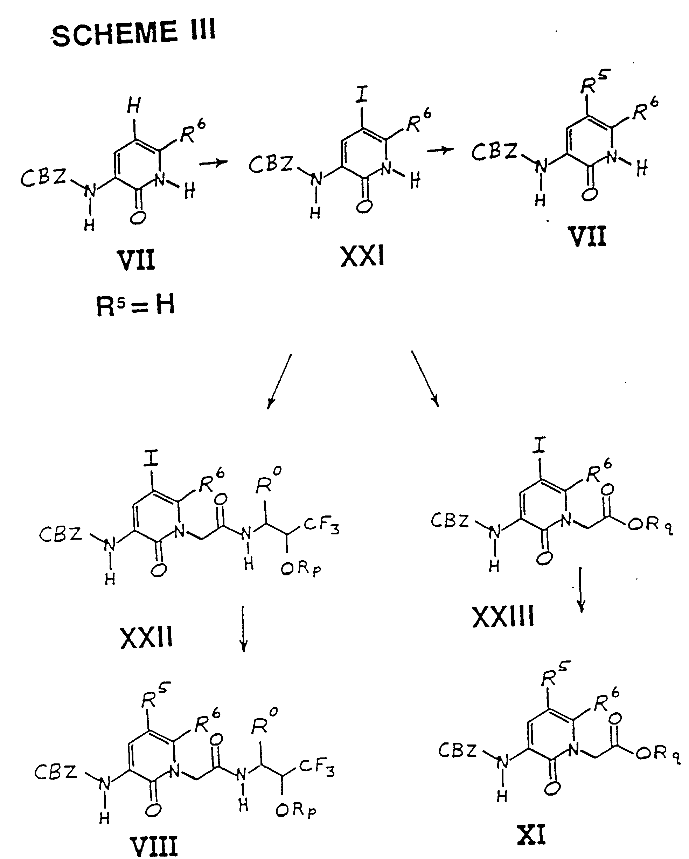

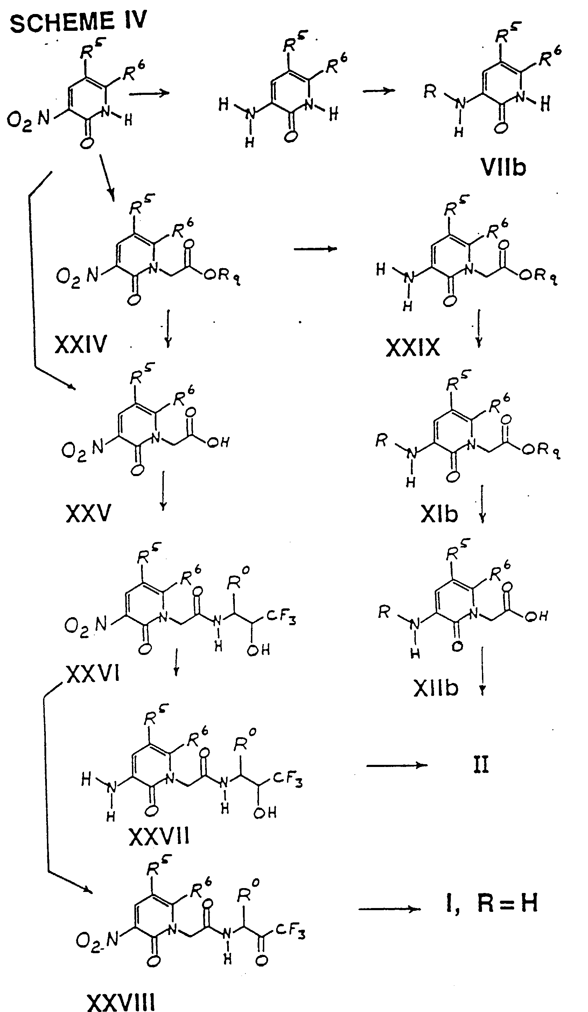

## Claims

1. A compound of formula I, 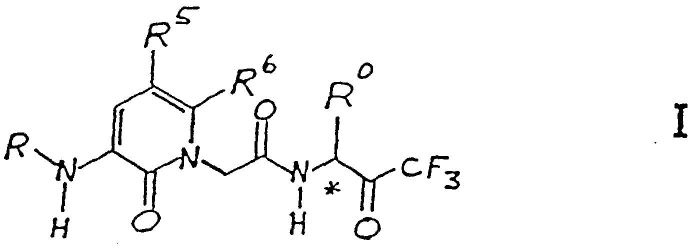
R⁰ is (1-5C)alkyl;
R is hydrogen, formyl or trifluoroacetyl; or
R is an acyl group of formula A.X.CO- in which A.X-, taken together, is amino, RbRcN.O-, RaOCONH-, R¹SO₂NH-, RaOCO-, RbRcNCO- or RaCO-; or
R is an acyl group of formula A.X.CJ- in which
J is oxygen or sulfur;
X is a direct bond, imino, oxy or thio; and
A is (1-6C)alkyl, (3-6C)cycloalkyl, (3-6C)cycloalkyl(1-3C)alkyl, aryl, aryl(1-3C)alkyl, heteroaryl or heteroaryl(1-3C)alkyl wherein an aryl or heteroaryl moiety may bear one to three halogeno, methyl or trifluoromethyl groups and further wherein the group A may bear one or more substituents selected from a group consisting of hydroxy, lower alkoxy, lower acyloxy, COORa, CONRbRc, COO(CH₂)₂NReRf, cyano, SO₂R¹, CONRdSO₂R¹, NReRf, NRgCOR², NRgCOOR², NRhCQNRiRj, NRkSO₂R³, SO₂NRlRm, SO₂NRnCOR⁴ and P(O)(ORa)₂ in which
Q is oxygen or sulfur;
Ra-Rn are independently hydrogen, benzyl or lower alkyl;
or, independently, a group NRbRc, NReRf, NRiRj or NRlRm is a cyclic radical selected from a group consisting of 1-pyrrolidinyl, piperidino, morpholino and 1-piperazinyl which may bear a lower alkyl substituent at the 4-position; or, independently, a group NReRf is a cyclic radical selected from a group consisting of 2-pyrrolidinon-1-yl, succinimido, oxazolidin-2-on-3-yl, 2-benzoxazolinon-3-yl, phthalimido and cis-hexahydrophthalimido; and
R¹-R⁴ are independently trifluoromethyl, (1-6C)alkyl, (3-6C)cycloalkyl, aryl or heteroaryl in which the aryl or heteroaryl may bear one or more substituents selected from a group consisting of lower alkyl, hydroxy, lower alkoxy, halogeno and trifluoromethyl; or
A is tetrahydropyran-4-yl, 1-methylpiperid-4-yl, or 5-methyl-1,3-dioxacyclohex-5-ylmethyl;
Each of R⁵ and R⁶ is, independently, hydrogen or lower alkyl; or
One of R⁵ and R⁶ is hydrogen or methyl and the other of R⁵ and R⁶ is a radical of formula B.Y- in which
B is aryl or heteroaryl, which aryl or heteroaryl independently may bear one or more of the substituents defined for A or an aryl or heteroaryl moiety thereof;
Y is a direct bond, methylene, ethylene or trans-vinylene; and
provided that no aliphatic carbon is bonded to more than one nitrogen or oxygen, except as part of a cyclic ketal or where the nitrogen bears a carbonyl group; or,
for a compound of formula I which is acidic or basic, a pharmaceutically acceptable salt thereof.

2. A compound as claimed in Claim 1 wherein
R⁰ is ethyl or isopropyl;
(1-6C)alkyl is methyl, ethyl, propyl, isopropyl, butyl, isobutyl, t-butyl, pentyl, 3-methylbutyl, 1-ethylpropyl, hexyl or 4-methylpentyl; (3-6C)cycloalkyl is cyclopropyl, cyclopentyl or cyclohexyl; the (1-3C)alkyl portion of (3-6C)cycloalkyl-(1-3C)alkyl, aryl(1-3C)alkyl or heteroaryl(1-3C)alkyl is methylene, ethylene or trimethylene; aryl is phenyl, indenyl or naphthyl; heteroaryl is furyl, imidazolyl, tetrazolyl, pyridyl (or its N-oxide), thienyl, pyrimidinyl (or its N-oxide), indolyl or quinolinyl (or its N-oxide); lower alkyl is methyl, ethyl, propyl, isopropyl, butyl, isobutyl or t-butyl; lower acyloxy is acetoxy; lower alkoxy is methoxy, ethoxy, propoxy, isoproxy or t-butoxy; halogeno is bromo, chloro or fluoro; COORa is carboxy or methoxycarbonyl; CONRbRc is carbamoyl or N,N-dimethylcarbamoyl; CONRdSO₂R¹ is N-phenylsulfonylcarbamoyl; and A.X-, when taken together, is tris(hydroxymethyl)methylamino, tris(acetoxymethyl)methylamino or 2,2-bis(hydroxymethyl)propoxy.

3. A compound as claimed in Claim 1 or 2 wherein
R⁰ is isopropyl; J is oxygen; X is a direct bond, imino or oxy; A is methyl, ethyl, phenyl, benzyl, phenethyl, pyridyl, thienyl, 5-tetrazolyl, thiazolyl, pyridylmethyl, thenyl, 5-tetrazolylmethyl, 2-(pyridyl)ethyl, 2-(thienyl)ethyl or 2-(thiazolyl)ethyl wherein the phenyl or heteroaryl group may bear one or two halogeno or methyl groups and further wherein the group A may bear a substituent selected from hydroxy, methoxy, t-butoxy, acetoxy, pivaloyloxy, carboxy, methoxycarbonyl, ethoxycarbonyl, carbamoyl, dimethylcarbamoyl, 2-(dimethylamino)ethoxycarbonyl, cyano, methylsulfonyl, phenylsulfonyl, N-methylsulfonylcarbamoyl, N-phenylsulfonylcarbamoyl, amino, dimethylamino, oxazolidin-2-on-3-yl, acetylamino, trifluoroacetylamino, ureido, sulfamoyl, dimethylphosphoryl or diethylphosphoryl.

4. A compound as claimed in any one of Claims 1-3 wherein
R is hydrogen, trifluoroacetyl, hydroxyoxalyl, methoxycarbonyl, ethoxycarbonyl, isopropoxycarbonyl, 4-fluorophenoxycarbonyl, 4-bromophenoxycarbonyl, 4-methoxyphenoxycarbonyl, benzyloxycarbonyl, 4-fluorobenzyloxycarbonyl, 4-pyridylmethoxycarbonyl, 3-methylpyrid-4-ylmethoxycarbonyl, 2,6-dimethylpyrid-4-ylmethoxycarbonyl, 2-pyridylmethoxycarbonyl, 6-methylpyrid-2-ylmethoxycarbonyl, 2-dimethylaminoethoxycarbonyl, acetyl, carbamoylmethylaminocarbonyl or 4-(N-phenylsulfonylcarbamoyl)phenylacetyl.

5. A compound as claimed in Claim 1 in which R⁰ and R, independently, have any of the values claimed in any one of Claims 1-4 and R⁵ and R⁶ are selected from
(a) a group in which R⁵ is benzyl, the phenyl ring of which may bear a 3-fluoro, 4-fluoro, 4-trifluoromethyl, 4-methoxycarbonyl, 3-acetoxy, 3-hydroxy, 3-pivaloyloxy, 4-hydroxy, 4-pivaloyloxy, 3-trifluoroacetylamino or 3-amino substituent, and R⁶ is hydrogen; and
(b) a group in which R⁵ is hydrogen, and R⁶ is 2-furyl, 2-thienyl, 3-pyridyl or phenyl in which the phenyl may bear one or two halogeno, trifluoromethyl, methyl, hydroxy, methoxy, tert-butoxy, methoxycarbonyl or carboxy substituents.

6. A compound as claimed in Claim 1 selected from
(a) 2-[3-[3-(carbamoylmethyl)ureido]-2-oxo-6-phenyl-1,2-dihydro-1-pyridyl]-N-(3,3,3-trifluoro-1-isopropyl-2-oxopropyl)-acetamide;
(b) 2-(3-amino-2-oxo-6-phenyl-1,2-dihydro-1-pyridyl)-N-(3,3,3-trifluoro-1-isopropyl-2-oxopropyl)acetamide;
(c) 2-[3-(4-bromophenoxycarbonylamino)-2-oxo-6-phenyl-1,2-dihydro-1-pyridyl]-N-(3,3,3-trifluoro-1-isopropyl-2-oxopropyl)-acetamide;
(d) 2-[3-(4-aminophenylacetyl)-2-oxo-6-phenyl-1,2-dihydro-1-pyridyl]-N-(3,3,3-trifluoro-1-isopropyl-2-oxopropyl)acetamide; and
(e) 2-[2-oxo-6-(2-thienyl)-3-trifluoroacetylamino-1,2-dihydro-1-pyridyl]-N-(3,3,3-trifluoro-1-isopropyl-2-oxopropyl)acetamide;
or a pharmaceutically acceptable salt thereof.

7. A salt as claimed in Claim 1 selected from
(a) for an acidic compound of formula I, alkali metal salts, alkaline earth metal salts, aluminum salts, ammonium salts, and salts made from organic bases triethylamine, morpholine, piperidine and triethanol amine; and
(b) for a basic compound of formula I, acid-addition salts made with a strong acid which provides a pharmaceutically acceptable anion.

8. A method of making a compound of formula I, or a pharmaceutically acceptable salt thereof, as claimed in any one of Claims 1-7 which is characterized by:
(A) oxidizing a corresponding alcohol of formula II: 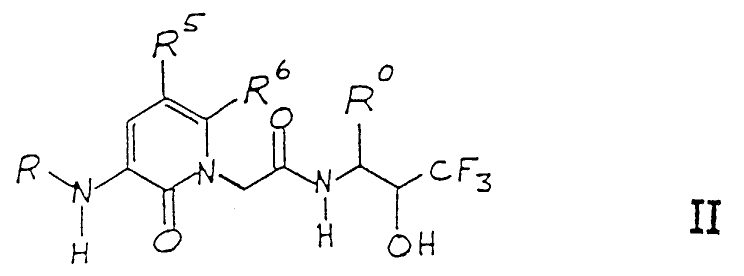
(B) for a compound of formula I which bears a hydroxy substituent on an aryl or heteroaryl group, cleaving the alkyl ether or acyloxy ester of a corresponding compound of formula I which bears a lower alkoxy or lower acyloxy substituent on an aryl or heteroaryl group;
(C) for a compound of formula I wherein R is not hydrogen, acylation of a corresponding amine of formula I wherein R is hydrogen;
(D) for a compound of formula I which bears a group of formula COORa in which Ra is hydrogen, decomposing the ester group of a corresponding ester made with a conveniently removed acid protecting group;
(E) for a compound of formula I which contains an amino N-H residue, removal of the nitrogen protecting group of a corresponding compound bearing a conventional nitrogen protecting group by using a conventional method;
(F) for a compound of formula I bearing a moiety of formula COORa, CONRbRc, COO(CH₂)₂NReRf or CONRdSO₂R¹, acylation of a corresponding compound of formula HORa, HNRbRc, HO(CH2)₂NReRf or HNRdSO₂R¹ with a corresponding acid of formula I bearing a moiety of formula COORa in which Ra is hydrogen, or an activated derivative thereof;
(G) for a compound of formula I bearing a lower acyloxy group or a group of formula NRgCOR², NRgCOOR², NRhCQNRiRj or NRkSO₂R³, acylation or sulfonation of a corresponding compound of formula I bearing a hydroxy group or an amino group of formula NHRg, NHRh or NHRk with an activated derivative of a corresponding acid of formula HOCOR², HOCOOR², HOCQNRiRj (including an isocyanate or isothiocyanate) or HOSO₂R³, respectively, using a conventional method;
(H) for a compound of formula I which bears a heteroaryl N-oxide group, oxidation of a corresponding compound of formula I which bears a heteroaryl group using a conventional oxidant;
(I) For a compound of formula I which bears a primary amino group, reduction of a corresponding compound bearing a nitro group using a conventional reducing method; and
whereafter, for any of the above procedures, when a pharmaceutically acceptable salt of an acidic or basic compound of formula I is required, reacting the acidic or basic form of such a compound of formula I with a base or acid affording a physiologically acceptable counterion or by any other conventional procedure;
and wherein the chemical formulae I and II are set out hereinbelow; and wherein A, B, J, Q, R⁰, R, R¹-R⁶, Ra-Rn, X and Y, except where more particularly described, have the meanings defined in any one of Claims 1-7.

9. A compound of formula II, set out hereinbelow, wherein R, R⁰, R⁵ and R⁶ are defined as defined in Claim 1, or a salt thereof.

10. A pharmaceutical composition comprising a compound as defined in Claim 1, or a pharmaceutically acceptable salt thereof, and a pharmaceutically acceptable diluent or carrier.

## Patentansprüche

1. Verbindung mit der folgenden Formel I: 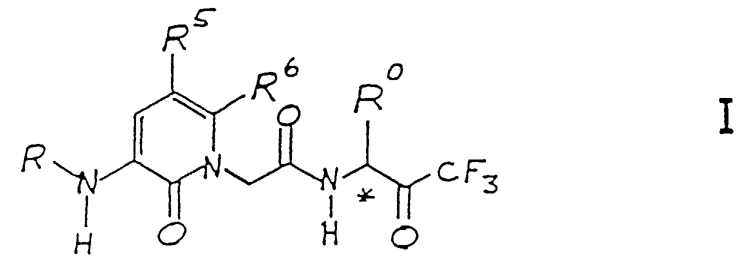 für die folgendes gilt:
R⁰ ist (1-5C)Alkyl,
R ist Wasserstoff, Formyl oder Trifluoracetyl, oder R ist eine Acyl-Gruppe mit der Formel A.X.CO-, in der A.X- zusammengenommen Amino, RbRcN.O-, RaOCONH-,
R¹SO₂NH-, RaOCO-, RbRcNCO- oder RaCO- ist, oder R ist eine Acyl-Gruppe mit der Formel A.X.CJ-, in der J Sauerstoff oder Schwefel ist,
X eine Direktbindung, Imino, Oxy oder Thio ist, und A (1-6C)Alkyl, (3-6C)Cycloalkyl, (3-6C)Cycloalkyl-(1-3C)alkyl, Aryl, Aryl(1-3C)alkyl, Heteroaryl oder Heteroaryl(1-3C)-alkyl ist, wobei ein Aryl- oder Heteroaryl-Rest eine bis drei Halogen-, Methyl- oder Trifluormethyl-Gruppen tragen kann, und wobei ferner die Gruppe A einen oder mehrere Substituenten tragen kann, die unter Hydroxy, Niederalkoxy, Niederacyloxy, COORa, CONRbRc, COO(CH₂)₂NReRf, Cyano, SO₂R¹, CONRdSO₂R¹, NReRf, NRgCOR², NRgCOOR², NRhCQNRiRj, NRkSO₂R³, SO₂NRlRm, SO₂NRnCOR⁴ und P(O)(ORa)₂ ausgewählt sind, wobei Q Sauerstoff oder Schwefel ist, Ra-Rn unabhängig voneinander Wasserstoff, Benzyl oder Niederalkyl sind, oder unabhängig davon ist eine Gruppe NRbRc, NReRf, NRiRj oder NRlRm ein cyclischer Rest, der unter 1-Pyrrolidinyl, Piperidino, Morpholino und 1-Piperazinyl ausgewählt ist, das einen Niederalkyl-Substituenten in 4-Stellung tragen kann, oder unabhängig davon ist eine Gruppe NReRf ein cyclischer Rest, der unter 2-Pyrrolidinon-1-yl, Succinimido, Oxazolidin-2-on-3-yl, 2-Benzoxazolinon-3-yl, Phthalimido und cis-Hexahydrophthalimido ausgewählt ist, und
R¹-R⁴ sind unabhängig voneinander Trifluormethyl, (1-6C)Alkyl, (3-6C)Cycloalkyl, Aryl oder Heteroaryl, wobei das Aryl oder Heteroaryl einen oder mehrere Substituenten tragen kann, die unter Niederalkyl, Hydroxy, Niederalkoxy, Halogen und Trifluormethyl ausgewählt sind, oder
A ist Tetrahydropyran-4-yl, 1-Methylpiperid-1-yl oder 5-Methyl-1,3-dioxacyclohex-5-ylmethyl;
R⁵ und R⁶ sind jeweils unabhängig voneinander Wasserstoff oder Niederalkyl, oder einer der Substituenten R⁵ und R⁶ ist Wasserstoff oder Methyl und der andere der Substituenten R⁵ und R⁶ ist ein Rest mit der Formel B.Y-, in der
B Aryl oder Heteroaryl ist, wobei das Aryl oder Heteroaryl unabhängig einen oder mehrere der für A oder einen Aryl- oder Heteroaryl-Rest davon definierten Substituenten tragen kann,
Y eine Direktbindung, Methylen, Ethylen oder trans-Vinylen ist,
mit der Maßgabe, daß kein aliphatischer Kohlenstoff an mehr als ein Stickstoff- oder Sauerstoffatom gebunden ist, ausgenommen als Teil eines cyclischen Ketals, oder wenn das Stickstoffatom eine Carbonyl-Gruppe trägt, oder ein pharmazeutisch geeignetes Salz davon, wenn die Verbindung mit der Formel I sauer oder basisch ist.

2. Verbindung nach Anspruch 1, wobei
R⁰ für Ethyl oder Isopropyl steht,
(1-6C)Alkyl für Methyl, Ethyl, Propyl, Isopropyl, Butyl, Isobutyl, t-Butyl, Pentyl, 3-Methylbutyl, 1-Ethylpropyl, Hexyl oder 4-Methylpentyl steht; (3-6C)Cycloalkyl für Cyclopropyl, Cyclopentyl oder Cyclohexyl steht,
der (1-3C)-Alkyl-Teil von (3-6C)Cycloalkyl-(1-3C)alkyl, Aryl(1-3C)alkyl oder Heteroaryl(1-3C)-alkyl für Methylen, Ethylen oder Trimethylen steht, Aryl für Phenyl, Indenyl oder Naphthyl steht;
Heteroaryl für Furyl, Imidazolyl, Tetrazolyl, Pyridyl (oder sein N-Oxid), Thienyl, Pyrimidinyl (oder sein N-Oxod), Indolyl oder Chinolinyl (oder sein N-Oxid) steht,
Niederalkyl für Methyl, Ethyl, Propyl, Isopropyl, Butyl, Isobutyl oder t-Butyl steht,
Niederacyloxy für Acetoxy steht,
Niederalkoxy für Methoxy, Ethoxy, Propoxy, Isopropoxy oder t-Butoxy steht,
Halogen für Brom, Chlor oder Fluor steht,
COORa für Carboxy oder Methoxycarbonyl steht,
CONRbRa für Carbamoyl oder N,N-Dimethylcarbamoyl steht,
CONRdSO₂R¹ für N-Phenylsulfonylcarbamoyl steht; und A.X- zusammengenommen für Tris(hydroxymethyl)-methylamino, Tris(acetoxymethyl)methylamino oder 2,2-Bis(Hydroxymethyl)propoxy steht.

3. Verbindung nach Anspruch 1 oder 2, wobei
R⁰ für Isopropyl steht,
J für Sauerstoff steht,
X für eine Direktbindung, Imino oder Oxy steht,
A für Methyl, Ethyl, Phenyl, Benzyl, Phenethyl, Pyridyl, Thienyl, 5-Tetrazolyl, Thiazolyl, Pyridylmethyl, Thenyl, 5-Tetrazolylmethyl, 2-(Pyridyl)ethyl, 2-(Thienyl)ethyl oder 2-(Thiazolyl)ethyl steht, wobei die Phenyl- oder Heteroaryl-Gruppe eine oder zwei Halogen- oder Methyl-Gruppen tragen kann, und wobei ferner die Gruppe A einen Substituenten tragen kann, der unter Hydroxy, Methoxy, t-Butoxy, Acetoxy, Pivaloyloxy, Carboxy, Methoxycarbonyl, Ethoxycarbonyl, Carbamoyl, Dimethylcarbamoyl, 2-(Dimethylamino)-ethoxycarbonyl, Cyano, Methylsulfonyl, Phenylsulfonyl, N-Methylsulfonylcarbamoyl, N-Phenylsulfonylcarbamoyl, Amino, Dimethylamino, Oxazolidin-2-on-3-yl, Acetylamino, Trifluoracetylamino, Ureido, Sulfamoyl, Dimethylphosphoryl oder Diethylphosphoryl ausgewählt ist.

4. Verbindung nach einem der Ansprüche 1 bis 3, wobei R für Wasserstoff, Trifluoracetyl, Hydroxyoxalyl, Methoxycarbonyl, Ethoxycarbonyl, Isopropoxycarbonyl, 4-Fluorphenoxycarbonyl, 4-Bromphenoxycarbonyl, 4-Methoxyphenoxycarbonyl, Benzyloxycarbonyl, 4-Fluorbenzyloxycarbonyl, 4-Pyridylmethoxycarbonyl, 3-Methylpyrid-4-ylmethoxycarbonyl, 2,6-Dimethylpyrid-4-yl-methoxycarbonyl, 2-Pyridylmethoxycarbonyl, 6-Methylpyrid-2-ylmethoxycarbonyl, 2-Dimethylaminoethoxycarbonyl, Acetyl, Carbamoylmethylaminocarbonyl oder 4-(N-Phenylsulfonylcarbamoyl)phenylacetyl steht.

5. Verbindung nach Anspruch 1, wobei R⁰ und R unabhängig voneinander einen beliebigen der in einem der Ansprüche 1 bis 4 beanspruchten Werte haben und R⁵ und R⁶ ausgewählt sind unter:
(a) einer Gruppe, in der R⁵ für Benzyl steht, dessen Phenyl-Ring einen 3-Fluor-, 4-Fluor-, 4-Trifluormethyl-, 4-Methoxycarbonyl-, 3-Acetoxy-, 3-Hydroxy-, 3-Picaloyoxy-, 4-Hydroxy-, 4-Pivaloyloxy-, 3-Trifluoracetylamino- oder 3-Amino-Substituenten tragen kann, und R⁶ für Wasserstoff steht, und
(b) einer Gruppe, in der R⁵ für Wasserstoff und R⁶ für 2-Furyl, 2-Thienyl, 3-Pyridyl oder Phenyl steht, wobei das Phenyl einen oder zwei Halogen-, Trifluormethyl-, Methyl-, Hydroxy-, Methoxy-, tert.-Butoxy-, Methoxycarbonyl- oder Carboxy-Substituenten tragen kann.

6. Verbindung nach Anspruch 1, ausgewählt unter:
(a) 2-[3-[3-(Carbamoylmethyl)ureido]-2-oxo-6-phenyl-1,2-dihydro-1-pyridyl]-N-(3,3,3-trifluor-1-isopropyl-2-oxopropyl)acetamid,
(b) 2-(3-Amino-2-oxo-6-phenyl-1,2-dihydro-1-pyridyl)-N-(3,3,3-trifluor-l-isopropyl-2-oxopropyl)acetamid,
(c) 2-[3-(4-Bromphenoxycarbonylamino)-2-oxo-6-phenyl-1,2-dihydro-1-pyridyl]-N-(3,3,3-trifluor-1-isopropyl-2-oxopropyl)acetamid,
(d) 2-[3-(4-Aminophenylacetyl)-2-oxo-6-phenyl-1,2-dihydro-l-pyridyl]-N-(3,3,3-trifluor-1-isopropyl-2-oxopropyl)acetamid, und
(e) 2-[2-Oxo-6-(2-thienyl)-3-trifluoracetylamino-1,2-dihydro-l-pyridyl]-N-(3,3,3-trifluor-1-isopropyl-2-oxopropyl)acetamid,
oder ein pharmazeutisch geeignet Salz davon.

7. Salz nach Anspruch 1, ausgewählt unter:
(a) für eine saure Verbindung mit der Formel I: Alkalimetallsalzen, Erdalkalimetallsalzen, Aluminiumsalzen, Ammoniumsalzen und Salzen der organischen Basen Triethylamin, Morpholin, Piperidin und Triethanolamin, und
(b) für eine basische Verbindung mit der Formel I: Säureadditionssalzen mit einer starken Säure, die ein pharmazeutisch geeignetes Anion liefert.

8. Verfahren zur Herstellung einer Verbindung mit der Formel I, oder eines pharmazeutisch geeigneten Salzes davon, nach einem der Ansprüche 1 bis 7, das dadurch **gekennzeichnet** ist, daß:
(A) ein entsprechender Alkohol mit der Formel II: 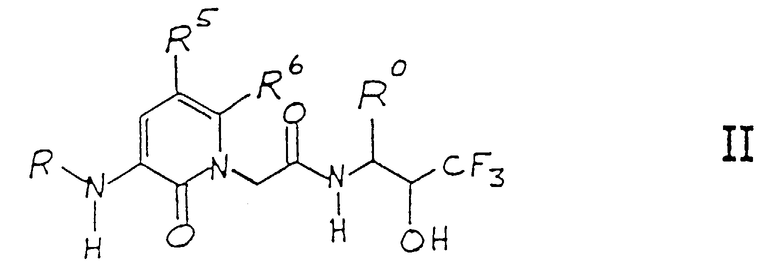 oxidiert wird,
(B) für eine Verbindung mit der Formel I, die einen Hydroxy-Substituenten an einer Aryl- oder Heteroaryl-Gruppe trägt, der Alkylether oder Acyloxyester einer entsprechenden Verbindung mit der Formel I, die einen Niederalkoxy- oder Niederacyloxy-Substituenten an einer Aryl- oder Heteroaryl-Gruppe trägt, gespalten wird,
(C) für eine Verbindung mit der Formel I, in der R nicht für Wasserstoff steht, ein entsprechendes Amin mit der Formel I, in der R für Wasserstoff steht, acyliert wird,
(D) für eine Verbindung mit der Formel I, die eine Gruppe mit der Formel COORa, in der Ra für Wasserstoff steht, trägt, die Ester-Gruppe eines entsprechenden Esters, der mit einer leicht zu entfernenden Säure-Schutzgruppe hergestellt wurde, zersetzt wird,
(E) für eine Verbindung mit der Formel I, die einen Amino-N-H-Rest enthält, die Stickstoff-Schutzgruppe aus einer entsprechenden Verbindung, die eine übliche Stickstoff-Schutzgruppe trägt, unter Anwendung eines üblichen Verfahrens entfernt wird,
(F) für eine Verbindung mit der Formel I, die einen Rest mit der Formel COORa, CONRbRc, COO(CH₂)₂NReRf oder CONRdSR¹ trägt, eine entsprechende Verbindung mit der Formel HORa, HNRbRc, HO(CH₂)₂NReRf oder HNRdSO₂R¹ mit einer entsprechenden Säure mit der Formel I, die einen Rest mit der Formel COORa, in der Ra für Wasserstoff steht, oder mit einem aktivierten Derivat davon acyliert wird,
(G) für eine Verbindung mit der Formel I, die eine Niederacyloxy-Gruppe oder eine Gruppe mit der Formel NRgCOR², NRgCOOR², NRhCQNRiRj oder NRkSO₂R³ trägt, eine entsprechende Verbindung mit der Formel I, die eine Hydroxy-Gruppe oder eine Amino-Gruppe mit der Formel NHRg oder NHRh oder NHRk trägt mit einem aktivierten Derivat einer entsprechenden Säure mit der Formel HOCOR², HOCOOR², HOCQNRiRj (ein Isocyanat oder Isothiocyanat eingeschlossen) bzw. HOSO₂R³ unter Anwendung eines üblichen Verfahrens acyliert oder sulfoniert wird,
(H) für eine Verbindung mit der Formel I, die eine Heteroaryl-n-oxid-Gruppe trägt, eine entsprechende Verbindung mit der Formel I, die eine Heteroaryl-Gruppe trägt, unter Verwendung eines üblichen Oxidationsmittels oxidiert wird,
(I) für eine Verbindung mit der Formel I, die eine primäre Amino-Gruppe trägt, eine entsprechende Verbindung, die eine Nitro-Gruppe trägt, unter Anwendung eines üblichen Reduktionsverfahrens reduziert wird,
woraufhin, und zwar für jedes der obigen Verfahren, wenn ein pharmazeutisch geeignetes Salz einer sauren oder basischen Verbindung mit der Formel I benötigt wird, die saure oder basische Form einer derartigen Verbindung mit der Formel I mit einer Base oder einer Säure, die ein physiologisch geeignetes Gegenion liefert, oder nach jedem anderen üblichen Verfahren, umgesetzt wird,
wobei A, B, J, Q, R⁰, R¹ - R⁶, Ra - Rn, X und Y die in einem der Ansprüche 1 bis 7 definierten Bedeutungen haben, sofern sie nicht konkreter beschrieben sind.

9. Verbindung mit der Formel II, wobei R, R⁰, R⁵ und R⁶ wie in Anspruch 1 definiert sind, oder ein Salz davon.

10. Pharmazeutische Zusammensetzung, die eine Verbindung nach Anspruch 1, oder ein pharmazeutisch geeignetes Salz davon, und ein pharmazeutisch geeignetes Streckmittel oder Trägermittel enthält.

## Revendications

1. Composé de formule I 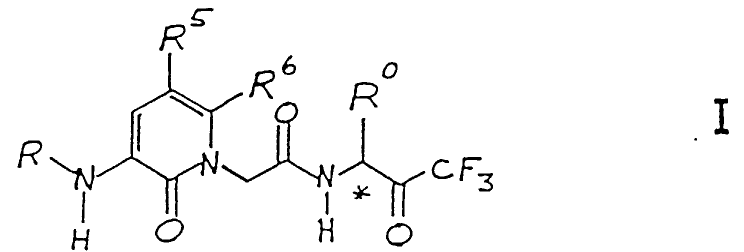 dans laquelle
R⁰ représente un groupe alkyle en C₁ à C₅ ;
R représente l'hydrogène, un groupe formyle ou trifluoracétyle ; ou
R représente un groupe acyle de formule A.X.CO- dans laquelle A.X-, pris conjointement, représentent un groupe amino, RbRcN.O-, RaOCONH-, R¹SO₂NH-, RaOCO-, RbRcNCO- ou RaCO- ; ou bien
R représente un groupe acyle de formule A.X.CJ- dans laquelle
J représente l'oxygène ou le soufre ;
X représente une liaison directe, un groupe imino, oxy ou thio ; et
A représente un groupe alkyle en C₁ à C₆, cycloalkyle en C₃ à C₆, (cycloalkyle en C₃ à C₆)-(alkyle en C₁ à C₃), aryle, aryl-(alkyle en C₁ à C₃), hétéroaryle ou hétéroaryl-(alkyle en C₁ à C₃), dans lequel un groupement aryle ou hétéroaryle peut porter un à trois groupes halogéno, méthyle ou trifluorométhyle, le groupe A pouvant porter en outre un ou plusieurs substituants choisis dans le groupe consistant en substituants hydroxy, alkoxy inférieur, acyloxy inférieur, COORa, CONRbRc, COO(CH₂)₂NReRf, cyano, SO₂R¹, CONRdSO₂R¹, NReRf, NRgCOR², NRgCOOR², NRhCQNRiRj, NRkSO₂R³, SO₂NRlRm, SO₂NRnCOR⁴ et P(O) (ORa)₂ dans lesquels
Q représente l'oxygène ou le soufre ;
Ra-Rn représentent, indépendamment, l'hydrogène, un groupe benzyle ou alkyle inférieur ; ou bien, indépendamment, un groupe NRbRc, NReRf, NRiRj ou NRlRm qui est un radical cyclique choisi dans le groupe consistant en les radicaux 1-pyrrolidinyle, pipéridino, morpholino et 1-pipérazinyle pouvant porter un substituant alkyle inférieur en position 4 ; ou bien, indépendamment, un groupe NReRf qui est un radical cyclique choisi dans le groupe consistant en radicaux 2-pyrrolidinone-l-yle, succinimido, oxazolidine-2-one-3-yle, 2-benzoxazolinone-3-yle, phtalimido et cis-hexahydrophtalimido ; et
R¹-R⁴ représentent, indépendamment, un groupe trifluorométhyle, alkyle en C₁ à C₆, cycloalkyle en C₃ à C₆, aryle ou hétéroaryle, le groupe aryle ou hétéroaryle pouvant porter un ou plusieurs substituants choisis dans le groupe consistant en substituants alkyle inférieur, hydroxy, alkoxy inférieur, halogéno et trifluorométhyle ; ou bien
A représente un groupe tétrahydropyranne-4-yle, 1-méthylpipérid-4-yle ou 5-méthyl-1,3-dioxacyclohex-5-ylméthyle ;
chacun des groupes R⁵ et R⁶ représente, indépendamment, l'hydrogène ou un groupe alkyle inférieur ; ou
un des groupes R⁵ et R⁶ représente l'hydrogène ou un groupe méthyle et l'autre des groupes R⁵ et R⁶ représente un radical de formule B.Y- dans laquelle
B représente un groupe aryle ou hétéroaryle, groupe aryle ou hétéroaryle pouvant porter, indépendamment, un ou plusieurs des substituants définis pour A ou un groupement aryle ou hétéroaryle de A ;
Y représente une liaison directe, un groupe méthylène, éthylène ou trans-vinylène ; et
sous réserve qu'aucun atome de carbone aliphatique ne soit lié à plus d'un atome d'azote ou d'oxygène, sauf en tant qu'une partie d'un cétal cyclique ou lorsque l'atome d'azote porte un groupe carbonyle ; ou
pour un composé de formule I qui est acide ou basique, un de ses sels pharmaceutiquement acceptables.

2. Composé suivant la revendication 1, dans lequel
R⁰ représente un groupe éthyle ou isopropyle ;
le groupe alkyle en C₁ à C₆ est un groupe méthyle, éthyle, propyle, isopropyle, butyle, isobutyle, tertio-butyle, pentyle, 3-méthylbutyle, l-éthylpropyle, hexyle ou 4-méthylpentyle ; le groupe cycloalkyle en C₃ à C₆ est un groupe cyclopropyle, cyclopentyle ou cyclohexyle ; la portion alkyle en C₁ à C₃ du groupe (cycloalkyle en C₃ à C₆)-(alkyle en C₁ à C₃), aryl-(alkyle en C₁ à C₃) ou hétéroaryl-(alkyle en C₁ à C₃) est un groupe méthylène, éthylène ou triméthylène ; le groupe aryle est un groupe phényle, indényle ou naphtyle ; le groupe hétéroaryle est un groupe furyle, imidazolyle, tétrazolyle, pyridyle (ou son N-oxyde), thiényle, pyrimidinyle (ou son N-oxyde), indolyle ou quinolinyle (ou son N-oxyde) ; le groupe alkyle inférieur est un groupe méthyle, éthyle, propyle, isopropyle, butyle, isobutyle ou tertio-butyle ; le groupe acyloxy inférieur est un groupe acétoxy ; le groupe alkoxy inférieur est un groupe méthoxy, éthoxy, propoxy, isopropoxy ou tertio-butoxy ; le groupe halogéno est un groupe bromo, chloro ou fluoro ; le groupe COORa est un groupe carboxy ou méthoxycarbonyle ; le groupe CONRbRc est un groupe carbamoyle ou N,N-diméthylcarbamoyle ; le groupe CONRdSO₂R¹ est un groupe N-phénylsulfonylcarbamoyle ; et A.X-, lorsqu'ils sont pris conjointement, représentent un groupe tris(hydroxyméthyl)méthylamino, tris(acétoxyméthyl)méthylamino ou 2,2-bis(hydroxyméthyl)propoxy.

3. Composé suivant la revendication 1 ou 2, dans lequel
R⁰ représente un groupe isopropyle ; J représente l'oxygène, X représente une liaison directe, un groupe imino ou oxy ; A représente un groupe méthyle, éthyle, phényle, benzyle, phénéthyle, pyridyle, thiényle, 5-tétrazolyle, thiazolyle, pyridylméthyle, thényle, 5-tétrazolylméthyle, 2-(pyridyl)éthyle, 2-(thiényl)éthyle ou 2-(thiazolyl)éthyle dans lequel le groupe phényle ou hétéroaryle peut porter un ou deux groupes halogéno ou méthyle et, en outre, le groupe A peut porter un substituant choisi entre les substituants hydroxy, méthoxy, tertio-butoxy, acétoxy, pivaloyloxy, carboxy, méthoxycarbonyle, éthoxycarbonyle, carbamoyle, diméthylcarbamoyle, 2-(diméthylamino)éthoxycarbonyle, cyano, méthylsulfonyle, phénylsulfonyle, N-méthylsulfonylcarbamoyle, N-phénylsulfonylcarbamoyle, amino, diméthylamino, oxazolidine-2-one-3-yle, acétylamino, trifluoracétylamino, uréido, sulfamoyle, diméthylphosphoryle ou diéthylphosphoryle.

4. Composé suivant l'une quelconque des revendications 1 à 3, dans lequel
R représente l'hydrogène, un groupe trifluoracétyle, hydroxyoxalyle, méthoxycarbonyle, éthoxycarbonyle, isopropoxycarbonyle, 4-fluorophénoxycarbonyle, 4-bromophénoxycarbonyle, 4-méthoxyphénoxycarbonyle, benzyloxycarbonyle, 4-fluorobenzyloxycarbonyle, 4-pyridylméthoxycarbonyle, 3-méthylpyrid-4-ylméthoxycarbonyle, 2,6-diméthylpyrid-4-ylméthoxycarbonyle, 2-pyridylméthoxycarbonyle, 6-méthylpyrid-2-ylméthoxycarbonyle, 2-diméthylaminoéthoxycarbonyle, acétyle, carbamoylméthylaminocarbonyle ou 4-(N-phénylsulfonylcarbamoyl)phénylacétyle.

5. Composé suivant la revendication 1, dans lequel R⁰ et R, indépendamment, ont n'importe laquelle des valeurs revendiquées dans l'une quelconque des revendications 1 à 4 et R⁵ et R⁶ sont choisis entre
(a) un groupe dans lequel R⁵ représente un groupe benzyle, dont le noyau phényle peut porter un substituant 3-fluoro, 4-fluoro, 4-trifluorométhyle, 4-méthoxycarbonyle, 3-acétoxy, 3-hydroxy, 3-pivaloyloxy, 4-hydroxy, 4-pivaloyloxy, 3-trifluoracétylamino ou 3-amino, et R⁶ représente l'hydrogène ; et
(b) un groupe dans lequel R⁵ représente l'hydrogène et R⁶ représente un groupe 2-furyle, 2-thiényle, 3-pyridyle ou phényle, le groupe phényle pouvant porter un ou deux substituants halogéno, trifluorométhyle, méthyle, hydroxy, méthoxy, tertio-butoxy, méthoxycarbonyle ou carboxy.

6. Composé suivant la revendication 1, choisi entre
(a) le 2-[3-[3-(carbamoylméthyl)uréido]-2-oxo-6-phényl-1,2-dihydro-1-pyridyl]-N-(3,3,3-trifluoro-1-isopropyl-2-oxopropyl)acétamide ;
(b) le 2-(3-amino-2-oxo-6-phényl-1,2-dihydro-1-pyridyl)-N-(3,3,3-trifluoro-1-isopropyl-2-oxopropyl)-acétamide ;
(c) le 2- [3- (4-bromophénoxycarbonylamino) -2-oxo-6-phényl-1,2-dihydro-1-pyridyl]-N-(3,3,3-trifluoro-1-isopropyl-2-oxopropyl)acétamide ;
(d) le 2-[3-(4-aminophénylacétyl)-2-oxo-6-phényl-1,2-dihydro-1-pyridyl]-N-(3,3,3-trifluoro-1-isopropyl-2-oxopropyl)acétamide ; et
(e) le 2-[2-oxo-6-(2-thiényl)-3-trifluoracétylamino-1,2-dihydro-1-pyridyl]-N-(3,3,3-trifluoro-1-isopropyl-2-oxopropyl)acétamide ;
ou un de ses sels pharmaceutiquement acceptables.

7. Sel suivant la revendication 1, choisi entre
(a) pour un composé acide de formule I, des sels de métaux alcalins, des sels de métaux alcalino-terreux, des sels d'aluminium, des sels d'ammonium et des sels préparés à partir des bases organiques consistant en triéthylamine, morpholine, pipéridine et triéthanolamine ; et
(b) pour un composé basique de formule I, des sels d'addition d'acides préparés avec un acide fort qui donne un anion pharmaceutiquement acceptable.

8. Procédé de préparation d'un composé de formule I, ou d'un de ses sels pharmaceutiquement acceptables, suivant l'une quelconque des revendications 1 à 7, qui est caractérisé par :
(A) l'oxydation d'un alcool correspondant de formule II : 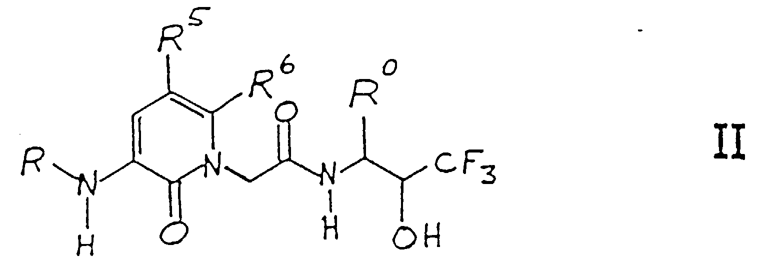
(B) pour un composé de formule I qui porte un substituant hydroxy sur un groupe aryle ou hétéroaryle, le clivage de l'éther alkylique ou de l'acyloxyester d'un composé correspondant de formule I qui porte un substituant alkoxy inférieur ou acyloxy inférieur sur un groupe aryle ou hétéroaryle ;
(C) pour un composé de formule I dans laquelle R ne représente pas l'hydrogène, l'acylation d'une amine correspondante de formule I dans laquelle R représente l'hydrogène ;
(D) pour un composé de formule I qui porte un groupe de formule COORa dans laquelle Ra représente l'hydrogène, la décomposition du groupe ester d'un ester correspondant préparé avec un groupe protecteur d'acide d'élimination commode ;
(E) pour un composé de formule I qui contient un résidu N-H de groupe amino, l'élimination du groupe protecteur de l'atome d'azote d'un composé correspondant portant un groupe protecteur classique d'atome d'azote au moyen d'un procédé classique ;
(F) pour un composé de formule I portant un groupement de formule COORa, CONRbRc, COO(CH₂)₂NReRf ou CONRdSO₂R¹, l'acylation d'un composé correspondant de formule HORa, HNRbRc, HO(CH₂)₂NeRf ou HNRdSO₂R¹ avec un acide correspondant de formule I portant un groupement de formule COORa dans laquelle Ra représente l'hydrogène, ou un de ses dérivés activés ;
(G) pour un composé de formule I portant un groupe acyloxy inférieur ou un groupe de formule NRgCOR², NRgCOOR², NRhCQNRiRj ou NRkSO₂R³, l'acylation ou la sulfonation d'un composé correspondant de formule I portant un groupe hydroxy ou un groupe amino de formule NHRg, NHRh ou NHRk avec un dérivé activé d'un acide correspondant de formule HOCOR², HOCOOR², HOCQNRiRj (y compris un isocyanate ou isothiocyanate) ou HOSO₂R³, respectivement, au moyen d'un procédé classique ;
(H) pour un composé de formule I qui porte un groupe hétéroaryl-N-oxyde, l'oxydation d'un composé correspondant de formule I qui porte un groupe hétéroaryle au moyen d'un oxydant classique ;
(I) pour un composé de formule I qui porte un groupe amino primaire, la réduction d'un composé correspondant portant un groupe nitro au moyen d'un procédé classique de réduction ; et
puis, pour n'importe lequel des modes opératoires précités, lorsqu'un sel pharmaceutiquement acceptable d'un composé acide ou basique de formule I est requis, la réaction de la forme acide ou basique d'un tel composé de formule I avec une base ou un acide donnant un ion complémentaire physiologiquement acceptable, ou n'importe quel autre mode opératoire classique ;
et dans lequel les formules chimiques I et II sont indiquées ci-dessus ; et A, B, J, Q, R⁰, R, R¹-R⁶, Ra-Rn, X et Y, sauf spécification contraire, répondent aux définitions figurant dans l'une quelconque des revendications 1 à 7.

9. Composé répondant à la formule II, indiquée ci-dessus, dans laquelle R, R⁰, R⁵ et R⁶ répondent aux définitions figurant dans la revendication 1, ou un de ses sels.

10. Composition pharmaceutique comprenant un composé répondant à la définition figurant dans la revendication 1, ou un de ses sels pharmaceutiquement acceptables, et un diluant ou support pharmaceutiquement acceptable.
